(19) Europäisches Patentamt
European Patent Office
Office européen des brevets

(11) EP 1 592 426 B1

(12) **EUROPEAN PATENT SPECIFICATION**

(45) Date of publication and mention of the grant of the patent:
**19.12.2007 Bulletin 2007/51**

(21) Application number: **04708332.4**

(22) Date of filing: **05.02.2004**

(51) Int Cl.:
***A61K 31/519*** *(2006.01)* ***A61P 29/00*** *(2006.01)*
***C07D 487/04*** *(2006.01)*

(86) International application number:
**PCT/EP2004/001081**

(87) International publication number:
**WO 2004/069256 (19.08.2004 Gazette 2004/34)**

(54) **2-CYANOPYRROLOPYRIMIDINES AND PHARMACEUTICAL USES THEREOF**

2-CYANOPYRROLOPYRIMIDINE UND DEREN PHARMAZEUTISCHE VERWENDUNG

2-CYANOPYRROLOPYRIMIDINES ET UTILISATIONS PHARMACEUTIQUES DE CELLES-CI

(84) Designated Contracting States:
**AT BE BG CH CY CZ DE DK EE ES FI FR GB GR HU IE IT LI LU MC NL PT RO SE SI SK TR**

(30) Priority: **06.02.2003 GB 0302748**
**28.02.2003 GB 0304642**
**28.02.2003 GB 0304641**

(43) Date of publication of application:
**09.11.2005 Bulletin 2005/45**

(73) Proprietors:
- **Novartis AG**
**4056 Basel (CH)**
Designated Contracting States:
**BE BG CH CY CZ DE DK EE ES FI FR GB GR HU IE IT LI LU MC NL PT RO SE SI SK TR**
- **Novartis Pharma GmbH**
**1230 Vienna (AT)**
Designated Contracting States:
**AT**

(72) Inventors:
- **BUXTON, Francis, Paul**
**Winchester, MA 01890 (US)**
- **EHARA, Takeru**
**Tsukuba-shi,**
**Ibaraki Pref. 305-0051 (JP)**
- **GANJU, Pamposh,**
**Novartis Inst. Medical Sciences**
**London WC1E 6BS (GB)**
- **HALLETT, Allan,**
**Novartis Inst. Medical Sciences**
**London WC1E 6BS (GB)**
- **IRIE, Ozamu**
**Tsukuba-shi,**
**Ibaraki Pref. 300-3253 (JP)**
- **IWASAKI, Atsuko**
**Tsukuba-shi,**
**Ibaraki Pref. 305-0005 (JP)**
- **KANAZAWA, Takanori**
**Tsukuba-shi,**
**Ibaraki Pref. 305-0033 (JP)**
- **MASUYA, Keiichi**
**Tsukuba-shi,**
**Ibarak i Pref. 300-3257 (JP)**
- **NONOMURA, Kazuhiko**
**Tsukuba-shi,**
**Ibaraki-Pref. 305-0831 (JP)**
- **SAKAKI, Junichi**
**Tsukuba-shi,**
**Ibaraki Pref. 305-0051 (JP)**
- **SNELL, Christopher, Robert**
**Runcton Holme,**
**Norfolk, PE33 0AF (GB)**
- **SONG, Chuanheng**
**Southborough, MA 01772 (US)**
- **TANABE, Keiko**
**Kashiwa-shi,**
**Chiba Pref. 277-08356 (JP)**
- **TENO, Naoki**
**Ushiku-shi,**
**Ibaraki Pref. 300-1232 (JP)**
- **UMEMURA, Ichiro**
**Tsukuba-shi,**
**Ibaraki Pref. 305-0051 (JP)**
- **YOKOKAWA, Fumiaki**
**Ibaraki Pref. 305-0047 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

(74) Representative: **Dressel, Jürgen**
**Novartis AG**
**4002 Basel (CH)**

(56) References cited:
**WO-A-00/00201**
**WO-A-02/055084**
**WO-A-03/020287**
**WO-A-03/020721**

## Description

This invention relates to novel pyrrolopyrimidine-2-carbonitrile derivatives, their preparation, their use as pharmaceuticals, pharmaceutical compositions containing them, and to the use of such a compound for the manufacture of a pharmaceutical preparation for the treatment of neuropathic pain.

Cathepsin S is a member of the family of lysosomal cysteine cathepsin enzymes, e.g. cathepsins B, K, L and S, which are implicated in various disorders including inflammation, rheumatoid arthritis, osteoarthritis, osteoporosis, tumors (especially tumor invasion and tumor metastasis), coronary disease, atherosclerosis (including atherosclerotic plaque rupture and destabilization), autoimmune diseases, respiratory diseases, infectious diseases and immunologically mediated diseases (including transplant rejection).

WO-A-03020721, published on 13.03.2003, discloses pyrrolo pyrimidines as agents for the inhibition of cystein proteases.

WO-A-03020287, published on 13.03.2003, discloses a class of pyrrolo pyrimidines as cathepsin inhibitors.

Surprisingly, it has now been found that the pyrrolopyrimidine-2-carbonitrile derivatives described herein have advantageous pharmacological properties and inhibit, for example, the activity of cathepsin S enzymes. The pyrrolopyrimidine-2-carbonitrile derivatives of formula I are hence suitable to be used in the treatment of diseases wherein the inhibition of cathepsin S activity causes a beneficial effect.

The pyrrolopyrimidine-2-carbonitrile derivatives of formula I are suitable, in particular, to be used in the treatment and also in the prevention of neuropathic pain.

Hence, the present invention provides a pyrrolo pyrimidine of formula I

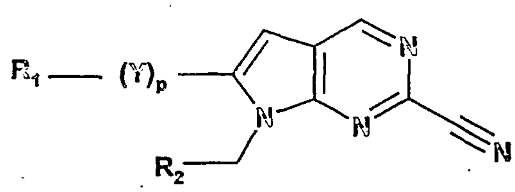

(I)

wherein

Y represents $-(CH_2)_t-O-$ or $-(CH_2)_r-S-$,

p is 1 or 2,

r is 1, 2 or 3,

t is 1, 2 or 3,

$R_1$ represents

(a) phenyl which is unsubstituted or mono-, di- or trisubstituted by

(α) halogen, carboxy, alkoxy, nitro, alkyl-C(O)-NH-, cycloalkyl-C(O)-NH-, alkyl-C(O)-N(alkyl)-, formyl, alkyl-C(O)-, alkyl-$S(O)_2$-NH-, $CF_3$-alkyl-$S(O)_2$-NH-, pyrrolidinyl carbonyl, piperidinyl carbonyl, morpholinyl carbonyl, N-alkyl piperazinyl carbonyl, piperidinyl, 1-(alkyl carbonyl) piperidinyl, 1,2,3,6-tetrahydropyridyl, alkyl carbonyl 1,2,3,6-tetrahydropyridyl, piperazinyl, alkyl piperazinyl, alkyl carbonyl piperazinyl, cycloalkyl carbonyl piperazinyl, alkoxy carbonyl piperazinyl, alkyl-$SO_2$-piperazinyl, diazacycloheptyl, alkyl carbonyl diazacycloheptyl, 2-oxo-1-pyrrolidinyl, 3,3-di-alkyl-2-oxo-1-pyrrolidinyl;

(β) $R_3$-alkyl, wherein $R_3$ represents hydrogen, hydroxy, carboxy, alkyl-N(alkyl)-, alkyl-NH-, 1-pyrrolidinyl, 1-piperidyl, 4-alkyl-1-piperazinyl carbonyl, 2,4-dioxa-5,5-(di-alkyl)-oxazolidin-3-yl, $R_4R_5N$-C(O)-, wherein $R_4$ and $R_5$ independently of each other represent hydrogen or alkyl; or

(γ) $R_6R_7N$-C(O)-, wherein $R_6$ and $R_7$ independently of each other represent hydrogen, alkyl, cycloalkyl alkyl, $CF_3$-alkyl or pyridyl alkyl;

(b) pyridyl, which is unsubstituted or mono-, di- or trisubstituted by halogen or alkyl which is mono-, di- or trisubstituted by halogen;

(c) pyrimidyl;

(d) indolyl, which is mono- or disubstituted by alkyl-C(O)-NH-alkyl;

(e) 2-(alkyl)-benzothiazolyl;

(f) a radical of subformula Ia

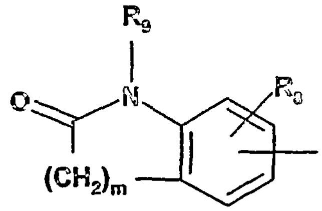

(Ia)

wherein $R_8$ is hydrogen, halogen or alkyl, $R_9$ is hydrogen or alkyl, and m is 1, 2, 3 or 4; or
(g) a radical of subformula Ib

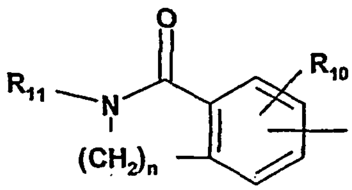

(Ib)

wherein $R_{10}$ is hydrogen, halogen or alkyl, $R_{11}$ is hydrogen or alkyl, and n is 1, 2, 3 or 4;

$R_2$ represents alkyl, which is unsubstituted or substituted by cycloalkyl, which is unsubstituted or mono- or disubstituted by halogen, or phenyl, which is mono- or disubstituted by halogen;
under the proviso that $R_2$ does not represent 1,1-dimethylethyl if Y is O and $R_1$ is selected from 3-pyridyl, 4-pyridyl, 5-chloro-3-pyridyl, 6-chloro-3-pyridyl, 2-chloro-4-pyridyl, 2-trifluoromethyl-4-pyridyl, 2-difluoromethyl-4-pyridyl, 4-acetyl-1-piperazinyl-phenyl, 4-methyl-1-piperazinyl-methyl-phenyl, and
under the proviso that $R_2$ does not represent 1,1-dimethylethyl, if Y is S and $R_1$ is 4-pyridyl; or
Y is $-(CH_2)_j$-or -CH=CH-,
j is 1 or 2;
p is 1 or 2,
$R_1$ represents

(a) thienyl, thiazolyl, 1-piperidinyl-carbonyl, or
(b) phenyl which is unsubstituted or mono-, di- or trisubstituted by

(i) alkoxy, $H_2N$-C(O)-, 4-(alkyl carbonyl) 1-piperazinyl, 2-oxo-1-pyrrolidinyl, or halogen;
(ii) $R_{12}$-O-C(O)-, wherein $R_{12}$ is hydrogen or alkyl, or
(iii) $R_{13}$NH-, wherein $R_{13}$ represents hydrogen or a radical $R_{14}$-alkyl-Z-, wherein Z is CO, SO or $SO_2$ and $R_{14}$ denotes hydrogen, trifluoromethyl or alkoxy,
(iv) $R_{15}$-alkyl, wherein $R_{15}$ denotes hydrogen, hydroxy, alkoxy, 1-pyrrolidinyl, 2-oxo-1-pyrrolidinyl, imidazolidin-2,5-dion-1-yl, 5,5-di-alkyl-oxazolidin-2,4-dion-3-yl or alkyl-N($R_{16}$)-, wherein $R_{16}$ represents hydrogen or alkyl; and

$R_2$ represents

(a) alkyl, which is unsubstituted or substituted by alkenyl, indanyl, cycloalkyl which is unsubstituted or mono- or disubstituted by halogen or alkyl, cycloalkenyl, phenyl, which is unsubstituted or mono- or disubstituted by halogen or by alkyl;
(b) cycloalkyl; or
(c) alkylcarbonyl;

under the proviso that, if Y is $CH_2$, $R_1$ represents 4-chlorophenyl and p is 1, $R_2$ does not denote 1,1-dimethylethyl, 1-methylethyl, cyclopropyl, cyclohexyl, 2-methyl-propyl or 2-ethyl-propyl;

under the proviso that $R_2$ does not represent 1,1-dimethylethyl, if p is 1, Y is $CH_2$ and $R_1$ represents thienyl, phenyl, methoxyphenyl, propoxyphenyl, 4-fluorophenyl, 4-methylphenyl, 4-ethylphenyl, 4-butylphenyl, hydroxymethylphenyl, 4-(5,5-dimethyl-oxazolipin-2,4-dion-3-yl-methyl)-phenyl, 4-(methylsulfonylamino)-phenyl, 4-(n-butylsulfonylamino)-phenyl, 4-(ethylsulfonylamino)-phenyl, 4-(n-propylsulfonylamino)-phenyl, 4-(iso-propylsulfonylamino)-phenyl, 4-aminophenyl, 4-(acetylamino)-phenyl, 4-(butanoylamino)-phenyl or 4-(diethylaminomethyl)-phenyl;
and under the proviso that that $R_2$ does not represent 1-methylethyl, if p is 1, Y is $CH_2$ and $R_1$ represents phenyl which is unsubstituted or substituted by 4-acetyl-1-piperazinyl; or
Y is $-(CH_2)_f-$,
f is 1 or 2;
pis1,
$R_1$ represents

(a) 1,2,3,6-tetrahydropyrid-1-yl, alkyl-1,2,3,6-tetrahydropyrid-1-yl, di-alkyl-1,2,3,6-tetrahydropyrid-1-yl, halo-1,2,3,6-tetrahydropyrid-1-yl, phenyl-1,2,3,6-tetrahydropyrid-1-yl, imidazolyl, alkyl imidazolyl, di-halo imidazolyl, imidazolidin-2,5-dion-1-yl, 5,5-dialkyl-oxazolidin-2,4-dion-3-yl, alkyl imidazolidin-2,5,-dion-1-yl, trifluoromethyl-3,4-pyrrolin-1-yl, pyrrolidinyl, alkyl 1-pyrrolidinyl, di-alkyl pyrrolidinyl, alkoxy pyrrolidinyl, alkyl 2-oxo-1-pyrrolidinyl, di-alkyl 2-oxo-1-pyrrolidinyl, halo 1-pyrrolidinyl, di-halo 1-pyrrolidinyl, di-halo 1-piperidinyl, triazolyl, nitro triazolyl, phenyl imidazolyl, tetrazolyl, benzo[b]imidazolyl, (1-(alkyl-$SO_2$)-4-piperidinyl)-2,3-dihydro-2-oxo-benzo[b]imidazolyl, 3-(alkyl carbonyl-4-piperidinyl)-2,3-dihydro-2-oxo-benzo[b]imidazolyl, indolyl, halo 1-indolyl, 1,3-dihydro-2-isoindolyl, 2,3-dihydro-1-indolyl, 2,3-dihydro-2-oxo-benzo[b]thiazolyl, di-alkoxy 1,2,3,4-tetrahydroquinnolin, alkoxy-1,2,3,4-tetrahydroisoquinnolin;
(b) a radical of substructure Ic

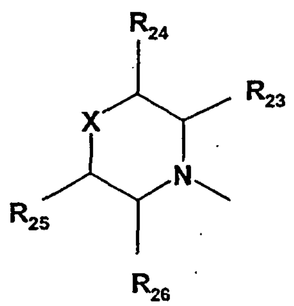
(Ic)

which is bound to the molecule via the nitrogen atom, wherein
X is -O-, $-(CH_2)_s-CR_{17}R_{18}-$ or $-NR_{18}$, wherein
s is 0, 1 or 2, $R_{17}$ and $R_{18}$ are independently selected from hydrogen, halogen, hydroxy, alkyl, phenyl alkyl carbonyl, carbamoyl, N-phenyl carbamoyl, cyano, pyridyl, piperidinyl and phenyl which is unsubstituted or mono- or disubstituted by halogen or alkoxy, or, if X is $CR_{17}R_{18}$, $R_{17}$ and $R_{18}$ and together form an oxo group or a group HO-C(O)-CH=, and $R_{23}$, $R_{24}$, $R_{25}$ and $R_{26}$ are independently selected from hydrogen and alkyl;
(c) a radical of substructure Id

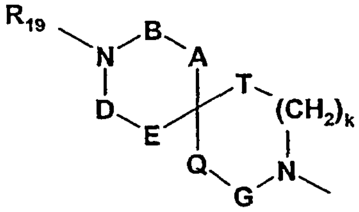
(Id)

which is bound to the molecule via the nitrogen atom, wherein
k is 0, 1 or 2, A is $CH_2$ or a bond, B is $CH_2$ or carbonyl, D is $CH_2$ or carbonyl, E is $CH_2$ or $NR_{22}$, G is $CH_2$ or a bond, Q is $CH_2$ or carbonyl, T is $CH_2$ or $NR_{29}$, $R_{19}$ represents hydrogen, alkyl, phenyl alkyl, alkyl carbonyl or alkyl-$SO_2$-, $R_{22}$ is hydrogen or alkyl and $R_{29}$ is phenyl;

(d) a radical of substructure Ie

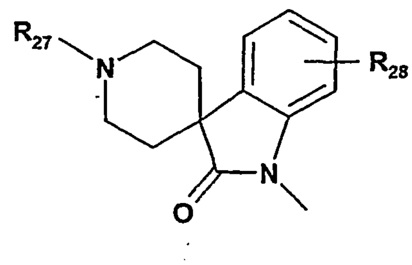

(Ie)

which is bound to the molecule via the nitrogen atom, wherein $R_{27}$ is alkyl or alkyl carbonyl and $R_{28}$ is hydrogen, alkoxy or halogen; or

(e) $NR_{20}R_{21}$, wherein $R_{20}$ and $R_{21}$ are independently selected from hydrogen, alkyl, cycloalkyl which is unsubstituted or mono- or disubstituted by hydroxy; and phenyl which is unsubstituted or mono- or disubstituted by 1,2,3-thiadiazolyl, under the proviso that not both $R_{20}$ and $R_{21}$ can represent hydrogen at the same time; and

$R_2$ denotes alkyl, which is unsubstituted or substituted by cycloalkyl which is unsubstituted or mono- or disubstituted by halogen; or phenyl, which is mono- or disubstituted by halogen; under the proviso that $R_2$ does not represent 1,1-dimethylethyl, if

(a) $R_1$ is benzo[b]imidazol-1-yl, 1-imidazolyl, 4,5-dichloro-1-imidazolyl, 2-($C_1$-$C_4$alkyl)-1-imidazolyl, imidazolidin-2,5-dion-1-yl, 5,5-dimethyl-oxazolidin-2,4-dion-3-yl, 1H-1,2,3-triazol-1-yl, 2H-1,2,3-triazol-2-yl, 3-nitro-1H-1,2,4-triazol-1-yl, 2H-tetrazol-2-yl or 1H-tetrazol-1-yl, or if $R_1$ is a radical of substructure Ic, $R_{23}$ to $R_{26}$ are hydrogen, X is $NR_{18}$ and $R_{18}$ is hydrogen, methyl, ethyl, acetyl, 4-pyridyl, 1-piperidinyl, phenyl, methoxyphenyl, ethoxyphenyl, fluorophenyl or chlorophenyl;

(b) $R_1$ is a radical of substructure Ic, $R_{23}$ to $R_{26}$ are hydrogen, X is $-(CH_2)_s-CR_{17}R_{18}-$, s is 0, and $R_{17}$ and $R_{18}$ are selected from hydroxyl and phenyl which is monosubstituted by chloro or $R_{17}$ and $R_{18}$ are selected from hydrogen, methoxyphenyl and N-phenylcarbamoyl; or

(c) $R_1$ is a radical of substructure Id, k is 1, A is a bond, E is $NR_{22}$, $R_{22}$ is hydrogen, G, Q and T are $CH_2$, B and D are carbonyl and $R_{19}$ is methyl, n-propyl or iso-butyl;

under the proviso that $R_2$ does not represent 2-methylpropyl, if $R_1$ is a radical of substructure Id, k is 1, A is a bond, E is $NR_{22}$, $R_{22}$ is hydrogen, G, Q and T are $CH_2$, B and D are carbonyl and $R_{19}$ is methyl, or if $R_1$ is a radical of substructure Ic, $R_{23}$ to $R_{26}$ are hydrogen, X is $-(CH_2)_s-CR_{17}R_{18}-$, s is 0, and $R_{17}$ and $R_{18}$ are selected from hydrogen and phenyl which is monosubstituted by methoxy;

and under the proviso that $R_2$ does not represent 1-methylethyl, if $R_1$ is a radical of substructure Ic, $R_{23}$ to $R_{26}$ are hydrogen, X is $NR_{18}$ and $R_{18}$ is methoxyphenyl or ethoxyphenyl, or X is $CR_{17}R_{18}$ and $R_{17}$ and $R_{18}$ are selected from hydrogen and methoxyphenyl;

or an N-oxide or a tautomer thereof,

or a salt of such pyrrolo pyrimidine, its N-oxide or its tautomer.

The general terms used hereinbefore and hereinafter preferably have within the context of this disclosure the following meanings, unless otherwise indicated:

Where the plural form is used for compounds, salts, and the like, this is taken to mean also a single compound, salt, or the like.

Halogen or halo is especially fluorine, chlorine, bromine, or iodine, especially fluorine, chlorine, or bromine.

Alkoxy is especially methoxy, ethoxy, propoxy or n-pentyloxy, but also benzyloxy or halogen-lower alkoxy, such as trifluoromethyloxy or 1,1,2,2-tetrafluoroethoxy. Preferably, alkoxy is methox, ethoxy or propoxy.

Alkyl is especially alkyl with from and including 1 up to and including 7, preferably from and including 1 to and including 4, C atoms and is linear or branched; preferably, alkyl is methyl, ethyl, propyl, such as n-propyl or isopropyl, butyl, such as n-butyl, sec-butyl, isobutyl or tert-butyl, 3-metyl-butyl or 2,2-dimethyl-butyl.

Alkenyl is preferably alkenyl with from and including 2 up to and including 7, preferably from and including 2 to and including 4, C atoms and is linear or branched. Alkenyl is preferably allyl, butenyl, e.g. 2-butenyl, methyl-butenyl, e.g. 3-methyl-2-butenyl, or dimethyl-butenyl, e.g. 2,2-dimethyl-4-butenyl.

Cycloalkyl is especially $C_3$-$C_8$cycloalkyl, e.g. cyclopropyl, cyclobutyl, cyclopentyl, cyclohexyl. Cycloheptyl or cyclooctyl.

Cycloalkenyl is especially $C_5$-$C_8$cycloalkyl, e.g cyclopentenyl, cyclohexenyl. Cycloheptenyl or cyclooctenyl.

In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient.

Salts are formed, for example, as acid addition salts, preferably with organic or inorganic acids, from compounds of formula I with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

The compounds of the invention exhibit valuable pharmacological properties in mammals and are particularly useful as inhibitors of cathepsin S. The cathepsin S inhibitory effects of the compound of the invention can be demonstrated in vitro by measuring the inhibition of e.g. recombinant human cathepsin S (In vitro cathepsin S assay).

The in vitro assay is carried out in clear, flat-bottomed, 96-well microtiter plates (Greiner GmbH, Germany) at ambient temperature using recombinant human cathepsin S. Inhibition of human cathepsin S is assayed at a constant enzyme and various substrate concentrations (substrate is Z-Leu-Leu-4-methylcoumaryl-7-amide (Bachem (Switzerland)) in 100 parts 0.2M sodium phosphate, pH 7:0, containing 2 mM EDTA, 2 parts 1% Triton X-100, 10 parts 20 mM dithiothreitol (DTT) and 58 parts distilled water. The assay is started by adding the enzyme solution (13 times higher concentration of final concentration of recombinant human Cathepsin S) to the reaction mixture containing various concentrations of the corresponding substrate and the compound. Substrate concentrations between 3.4 and 17 $\mu$M are used. The recombinant human Cathepsin S is used at a final concentration of 0.04 nM. Test compounds are used at concentrations between 0.4 and 2 times the determined IC50 of the compound at the enzyme. The relative fluorescence is continuously measured for 30 minutes and the initial velocity is obtained from each progress curve. The inhibition patterns and the $K_i$ values are determined by Dixon plot analysis.

Compounds of the Invention typically have $IC_{50}$s for inhibition of human cathepsin S of less than about 100 nM down to about 1 nM or less, preferably of about 5 nM or less, e.g. about 0.5 nM.

In view of their activity as inhibitors of cathepsin S, compounds of formula I are particularly useful in mammals as agents for the treatment and prophylaxis of diseases and medical conditions involving elevated levels of cathepsin S activity. Such diseases include chronic neuropathic pain, exemplified by conditions such as diabetic neuropathy, postherpetic neuralgia, trigeminal neuralgia, painful diabetic polyneuropathy, post-stroke pain (central pain), postamputation pain, myolopathic or radiculopathic pain (e.g. spinal stenosis, arachnoiditis, root sleeve fibrosis), atypical facial pain and causalgia-like syndromes (complex regional pain syndromes), autoimmune disorders, including, but not limited to juvenile onset diabetes and multiple sclerosis, allergic disorders, including, but not limited to, asthma, and allogeneic immune responses, including, but not limited to, organ transplant rejection.

Beneficial effects are evaluated in in vitro and in vivo pharmacological tests generally known in the art, and as illustrated herein. The above cited properties are demonstrable in in vitro and in vivo tests, using advantageously mammals, e.g. rats, mice, dogs, rabbits, monkeys or isolated organs and tissues, as well as mammalian enzyme preparations, either natural or prepared by e.g. recombinant technology. Compounds of the Invention can be applied in vitro in the form of solutions, e.g. preferably aqueous solutions or suspensions, and in vivo either enterally or parenterally, advantageously orally, e.g. as a suspension or in aqueous solution, or as a solid capsule or tablet formulation. The dosage in vitro may range between about $10^{-5}$ molar and $10^{-9}$ molar concentrations. The dosage in vivo may range, depending on the route of administration, between about 0.1 and 100 mg/kg.

The efficacy of the Compounds of the Invention for the treatment of chronic inflammatory or neuropathic pain can be determined using the following In vivo animal models:

Chronic inflammatory pain model:

The Complete Freund's Adjuvant -induced mechanical hyperalgesia may be used as a model of chronic inflammatory pain (Stein, C. et al. Pharmacol. Biochem. Behav. (1988) 31 :445-451). In this model, typically a male Sprague-Dawley or Wistar rat (200-250 g) receives an intraplantar injection of 25 μl complete Freund's adjuvant into one hind paw. A marked inflammation occurs in this hind paw. Drugs are generally administered for evaluation of efficacy, 24 hours after the inflammatory insult, when mechanical hyperalgesia is considered fully established.

Chronic neuropathic pain models:

Two animal models of chronic neuropathic pain may be used that involve some form of peripheral nerve damage. In the Seltzer model (Seltzer et al. (1990) Pain 43: 205-218) rats are anaesthetised and a small incision made mid-way up one thigh (usually the left) to expose the sciatic nerve. The nerve is carefully cleared of surrounding connective tissues at a site near the trochanter just distal to the point at which the posterior biceps semitendinosus nerve branches off the common sciatic nerve. A 7-0 silk suture is inserted into the nerve with a 3/8 curved, reversed-cutting mini-needle, and tightly ligated so that the dorsal 1/3 to 1/2 of the nerve thickness is held within the ligature. The muscle and skin are closed with sutures and clips and the wound dusted with antibiotic powder. In sham animals the sciatic nerve is exposed but not ligated and the wound closed as in nonsham animals.

In the Chronic Constriction Injury (CCI) model (Bennett, G.J. and Xie, Y.K. Pain (1988) 33: 87-107) rats are anaesthetised and a small incision is made mid-way up one thigh (usually the left) to expose the sciatic nerve. The nerve is cleared of surrounding connective tissue and four ligatures of 4/0 chromic gut are tied loosely around the nerve with approximately 1mm between each, so that the ligatures just barely constrict the surface of the nerve. The wound is closed with sutures and clips as described above. In sham animals the sciatic nerve is exposed but not ligated and the wound closed as in nonsham animals.

In contrast to the Seltzer and CCI models, the Chung model involves ligation of the spinal nerve. (Kim, S.O. and Chung, J.M. Pain (1992): 50:355-363). In this model, rats are anesthetized and placed into a prone position and an incision is made to the left of the spine at the L4-S2 level. A deep dissection through the paraspinal muscles and separation of the muscles from the spinal processes at the L4-S2 level will reveal part of the sciatic nerve as it branches to form the L4, L5 and L6 spinal nerves. The L6 transverse process is carefully removed with a small rongeur enabling visualisation of these spinal nerves. The L5 spinal nerve is isolated and tightly ligated with 7-0 silk suture. The wound is closed with a single muscle suture (6-0 silk) and one or two skin closure clips and dusted with antibiotic powder. In sham animals the L5 nerve is exposed as before but not ligated and the wound closed as before.

Behavioral index

In all chronic pain models (inflammatory and neuropathic) mechanical hyperalgesia is assessed by measuring paw withdrawal thresholds of both hindpaws to an increasing pressure stimulus using an Analgesymeter (Ugo-Basile, Milan). Mechanical allodynia is assessed by measuring withdrawal thresholds to non-noxious mechanical stimuli applied with von Frey hairs to the plantar surface of both hindpaws. Thermal hyperalgesia is assessed by measuring withdrawal latencies to a noxious thermal stimulus applied to the underside of each hindpaw. With all models, mechanical hyperalgesia and allodynia and thermal hyperalgesia develop within 1 - 3 days following surgery and persist for at least 50 days. For the assays described herein, drugs may be applied before and after surgery to assess their effect on the development of hyperalgesia, particularly approximately 14 days following surgery, to determine their ability to reverse established hyperalgesia.

The percentage reversal of hyperalgesia is calculated as follows:

$$\%\ reversal = \frac{postdose\ threshold - predose\ threshold}{naive\ threshold - predose\ threshold}\ X\ 100$$

In the experiments disclosed herein, Wistar rats (male) are employed in the pain models described above. Rats weigh approximately 120-140 grams at the time of surgery. All surgery is performed under enflurane/$O_2$ inhalation anaesthesia. In all cases the wound is closed after the procedure and the animal allowed to recover. In all pain models employed, after a few days in all but the sham operated animals, a marked mechanical and thermal hyperalgesia and allodynia develops in which there is a lowering of pain threshold and an enhanced reflex withdrawal response of the hind-paw to touch, pressure or thermal stimuli. After surgery the animals also exhibit characteristic changes to the affected paw. In the majority of animals the toes of the affected hind paw are held together and the foot turned slightly to one side; in some rats the toes are also curled under. The gait of the ligated rats varies, but limping is uncommon. Some rats are seen to raise the affected hind paw from the cage floor and to demonstrate an unusual rigid extension

of the hind limb when held. The rats tend to be very sensitive to touch and may vocalise. Otherwise the general health an condition of the rats is good.

The efficacy of the compounds of the invention for the treatment of osteoarthritis can be determined using models such as or similar to the rabbit partial lateral meniscectomy model, as described previously (Colombo et al. Arth. Rheum. 1993 26, 875-886). The efficacy of the compounds in the model can be quantified using histological scoring methods, as described previously (O'Byrne et al. Inflamm Res 1995, 44, S117-S118).

A compound of formula I can be administered alone or in combination with one or more other therapeutic agents, possible combination therapy taking the form of fixed combinations or the administration of a compound of the invention and one or more other therapeutic agents being staggered or given independently of one another, or the combined administration of fixed combinations and one or more other therapeutic agents.

The invention relates in particular to a pyrrolo pyrimidine of formula I, wherein
Y represents $-CH_2-O-$ or $-CH_2-S-$, p is 1,
$R_1$ represents

(a) phenyl which is unsubstituted or mono- or disubstituted by

(α) halogen, carboxy, $C_1$-$C_4$alkoxy, nitro, $C_1$-$C_4$alkyl-C(O)-NH-, $C_3$-$C_4$cycloalkyl-C(O)-NH-, $C_1$-$C_4$alkyl-C(O)-N($C_1$-$C_4$alkyl)-, formyl, $C_1$-$C_4$alkyl-C(O)-, $C_1$-$C_4$alkyl-$S(O)_2$-NH-, $CF_3$-$C_1$-$C_3$alkyl-$S(O)_2$-NH-, 1-pyrrolidinyl-carbonyl, 1-piperidinyl-carbonyl, 4-morpholinyl-carbonyl, 4-($C_1$-$C_4$alkyl)-1-piperazinyl carbonyl, 4-piperidinyl, 1-piperidinyl, 1-($C_1$-$C_4$alkyl-carbonyl)-4-piperidinyl, 1,2,3,6-tetrahydro-4-pyridyl, 1-($C_1$-$C_4$alkyl-carbonyl)-1,2,3,6-tetrahydro-4-pyridyl, 1-piperazinyl, 4-($C_1$-$C_4$alkyl)-1-piperazinyl, 4-($C_1$-$C_4$alkyl-carbonyl)-1-piperazinyl, 4-($C_3$-$C_5$cycloalkyl-carbonyl)-1-piperazinyl, 4-($C_1$-$C_4$alkoxy-carbonyl)-1-piperazinyl, 4-($C_1$-$C_4$alkyl-$SO_2$)-1-piperazinyl, 1,4-diazacyclohept-1-yl, 4-($C_1$-$C_4$alkyl-carbonyl)-1,4-diazacyclohept-1-yl, 2-oxo-1-pyrrolidinyl, 3,3-di-($C_1$-$C_4$alkyl)-2-oxo-1-pyrrolidinyl;
(β) $R_3$-$C_1$-$C_4$alkyl, wherein $R_3$ represents hydrogen, hydroxyl, carboxy, $C_1$-$C_4$alkyl-N($C_1$-$C_4$alkyl)-, $C_1$-$C_4$alkyl-NH-, 1-pyrrolidinyl, 1-piperidyl, 4-($C_1$-$C_4$alkyl)-1-piperazinyl carbonyl, 2,4-dioxa-5,5-(di-$C_1$-$C_4$alkyl)-oxazolidin-3-yl, $R_4R_5$N-C(O)-, wherein $R_4$ and $R_5$ independently of each other represent hydrogen or $C_1$-$C_4$alkyl; or
(γ) $R_6R_7$N-C(O)- wherein $R_6$ and $R_7$ independently of each other represent hydrogen, $C_1$-$C_4$alkyl, $C_5$-$C_7$cycloalkyl-$C_1$-$C_4$alkyl, $CF_3$-$C_1$-$C_3$alkyl or pyridyl-$C_1$-$C_4$alkyl;

(b) pyridyl, which is unsubstituted or mono- or disubstituted by halogen or $C_1$-$C_4$alkyl which is di- or trisubstituted by halogen;
(c) pyrimidyl;
(d) indolyl, which is monosubstituted by $C_1$-$C_4$alkyl-C(O)-NH-$C_1$-$C_4$alkyl;
(e) 2-($C_1$-$C_4$alkyl)-benzothiazolyl;
(f) a radical of subformula Ia
wherein $R_8$ is hydrogen, $R_9$ is hydrogen, and m is 2 or 3; or
(g) a radical of subformula Ib
wherein $R_{10}$ is hydrogen, $R_{11}$ is hydrogen, and n is 2 or 3;

$R_2$ represents $C_1$-$C_5$alkyl, which is unsubstituted or substituted by $C_5$-$C_7$cycloalkyl, which is unsubstituted or disubstituted by halogen, or phenyl; which is mono- or disubstituted by halogen;
under the proviso that $R_2$ does not represent 1,1-dimethylethyl if Y is O and $R_1$ is selected from 3-pyridyl, 4-pyridyl, 5-chloro-3-pyridyl, 6-chloro-3-pyridyl, 2-chloro-4-pyridyl, 2-trifluoromethyl-4-pyridyl, 2-difluoromethyl-4-pyridyl, 4-acetyl-1-piperazinyl-phenyl, 4-methyl-1-piperazinyl-methyl-phenyl, and
under the proviso that $R_2$ does not represent 1,1-dimethylethyl, if Y is S and $R_1$ is 4-pyridyl; and to a tautomer thereof, and to the salts of such a pyrrolo pyrimidine or its tautomer.

Furthermore, the invention relates in particular to a pyrrolo pyrimidine of formula I, wherein Y is $CH_2$ or -CH=CH-, p is 1 or 2,
$R_1$ represents

(a) thienyl, thiazolyl, 1-piperidinyl-carbonyl, or
(b) phenyl which is unsubstituted or mono- or disubstituted by

(i) $C_1$-$C_4$alkoxy, $H_2N$-C(O)-, 4-($C_1$-$C_4$alkyl-carbonyl)-1-piperazinyl, 2-oxo-1-pyrrolidinyl, or halogen;
(ii) $R_{12}$-O-C(O)-, wherein $R_{12}$ is hydrogen or $C_1$-$C_4$alkyl, or
(iii) $R_{13}$NH-, wherein $R_{13}$ represents hydrogen or a radical $R_{14}$-$C_1$-$C_4$alkyl-Z-, wherein Z is CO or $SO_2$ and $R_{14}$ denotes hydrogen, trifluoromethyl or $C_1$-$C_4$alkoxy,

(iv) $R_{15}$-$C_1$-$C_4$alkyl, wherein $R_{15}$ denotes hydrogen, hydroxy, lower alkoxy, 1-pyrrolidinyl, 2-oxo-1-pyrrolidinyl, imidazolidin-2,5-dion-1-yl, 5,5-dimethyl-oxazolidin-2,4-dion-3-yl or $C_1$-$C_4$alkyl-N($R_{16}$)-, wherein $R_{16}$ represents hydrogen or $C_1$-$C_4$alkyl; and

$R_2$ represents

(a) $C_1$-$C_7$alkyl, which is unsubstituted or substituted by $C_2$-$C_3$alkenyl, indanyl, $C_3$-$C_7$cycloalkyl which is unsubstituted or disubstituted by halogen or $C_1$-$C_4$alkyl, $C_3$-$C_7$cycloalkenyl, phenyl, which is unsubstituted or mono- or disubstituted by halogen or by $C_1$-$C_4$alkyl;
(b) $C_3$-$C_7$cycloalkyl; or
(c) $C_1$-$C_4$alkylcarbonyl;

under the proviso that, if Y is $CH_2$, $R_1$ represents 4-chlorophenyl and p is 1, $R_2$ does not denote 1,1-dimethylethyl, 1-methylethyl, cyclopropyl, cyclohexyl, 2-methyl-propyl or 2-ethylpropyl;
under the proviso that $R_2$ does not represent 1,1-dimethylethyl, if p is 1, Y is $CH_2$ and $R_1$ represents thienyl, phenyl, methoxyphenyl, propoxyphenyl, 4-fluorophenyl, 4-methylphenyl, 4-ethylphenyl, 4-butylphenyl, hydroxymethylphenyl, 4-(5,5-dimethyl-oxazolidin-2,4-dion-3-yl-methyl)-phenyl, 4-(methylsulfonylamino)-phenyl, 4-(n-butylsulfonylamino)-phenyl, 4-(ethylsulfonylamino)-phenyl, 4-(n-propylsulfonylamino)-phenyl, 4-(iso-propylsulfonylamino)-phenyl, 4-aminophenyl, 4-(acetylamino)-phenyl, 4-(butanoylamino)-phenyl or 4-(diethylaminomethyl)-phenyl;
and under the proviso that that $R_2$ does not represent 1-methylethyl, if p is 1, Y is $CH_2$ and $R_1$ represents phenyl which is unsubstituted or substituted by 4-acetyl-1-piperazinyl; or

Additionally, the invention relates in particular to a pyrrolo pyrimidine of formula I, wherein Y is $CH_2$, p is 1, $R_1$ represents

(a) 1,2,3,6-tetrahydropyrid-1-yl, 4-($C_1$-$C_4$alkyl)-1,2,3,6-tetrahydropyrid-1-yl, 4,5-di($C_1$-$C_4$alkyl)-1,2,3,6-tetrahydropyrid-1-yl, 5-chloro-1,2,3,6-tetrahydropyrid-1-yl; 4-phenyl-1,2,3,6-tetrahydropyrid-1-yl, 1-imidazolyl, 2-($C_1$-$C_4$alkyl)-1-imidazolyl, 4,5-dihalo-1-imidazolyl, imidazolidin-2,5-dion-1-yl, 5,5-dimethyl-oxazolidin-2,4-dion-3-yl, 3-($C_1$-$C_4$alkyl)-imidazolidin-2,5-dion-1-yl, 3-trifluoromethyl-3,4-pyrrolin-1-yl, 1-pyrrolidinyl, 3-$C_1$-$C_4$alkyl-1-pyrrolidinyl, 3,3-di-($C_1$-$C_4$alkyl)-1-pyrrolidinyl, 3-$C_1$-$C_4$alkoxy-1-pyrrolidinyl, 3-$C_1$-$C_4$alkyl-2-oxo-1-pyrrolidinyl, 3,3-di-($C_1$-$C_4$alkyl)-2-oxo-1-pyrrolidinyl, 3-halo-1-pyrrolidinyl, 3,3-di-halo-1-pyrrolidinyl, 3,3-di-halo-1-piperidinyl, 1H-1,2,3-triazol-1-yl, 2H-1,2,3-triazol-2-yl, 1H-1,2,4-triazol-1-yl, 3-nitro-1H-1,2,4-triazol-1-yl, 2-phenyl-1-imidazolyl, 2H-tetrazol-2-yl, 1 H-tetrazol-1-yl, benzo[b]imidazol-1-yl, 3-(1-($C_1$-$C_4$alkyl-$SO_2$)-4-piperidinyl)-2,3-dihydro-2-oxo-benzo[b]imidazol-1-yl, 3-(1-$C_1$-$C_4$alkylcarbonyl-4-piperidinyl)-2,3-dihydro-2-oxo-benzo[b]imidazol-1-yl, 1-indolyl, 6-halo-1-indolyl, 1,3-dihydro-2-isoindolyl, 2,3-dihydro-1-indolyl, 2]3-dihydro-2-oxo-benzo[b]thiazol-3yl, 6,7-di-($C_1$-$C_4$alkoxy)-1,2,3,4-tetrahydroquinnolin, 6-$C_1$-$C_4$alkoxy-1,2,3,4-tetrahydroisoquinnolin, 7-$C_1$-$C_4$alkoxy-1,2,3,4-tetrahydroisoquinnolin;
(b) a radical of substructure Ic
which is bound to the molecule via the nitrogen atom, wherein
X is -O-, -$(CH_2)_s$-$CR_{17}R_{18}$- or -$NR_{18}$, wherein
s is 0 or 1, $R_{17}$ and $R_{18}$ are independently selected from hydrogen, halogen, hydroxy, $C_1$-$C_4$alkyl, phenyl-$C_1$-$C_4$alkylcarbonyl, carbamoyl, N-phenyl-carbamoyl, cyano, 4-pyridyl, 1-piperidinyl and phenyl which is unsubstituted or monosubstituted by halogen or $C_1$-$C_4$alkoxy, or, if X is $CR_{17}R_{18}$, $R_{17}$ and $R_{18}$ and together form an oxo group or a group HO-C(O)-CH=, and
$R_{23}$, $R_{24}$, $R_{25}$ and $R_{26}$ are independently selected from hydrogen and $C_1$-$C_4$alkyl;
(c) a radical of substructure Id
which is bound to the molecule via the nitrogen atom, wherein
k is 0 or 1, A is $CH_2$ or a bond, B is $CH_2$ or carbonyl, D is $CH_2$ or carbonyl, E is $CH_2$ or $NR_{22}$, G is $CH_2$ or a bond, Q is $CH_2$ or carbonyl, T is $CH_2$ or $NR_{29}$, $R_{19}$ represents hydrogen, $C_1$-$C_4$alkyl, phenyl-$C_1$-$C_4$alkyl, $C_1$-$C_4$alkylcarbonyl or $C_1$-$C_4$alkyl-$SO_2$-, $R_{22}$ is hydrogen and $R_{29}$ is phenyl;
(d) a radical of substructure Ie
which is bound to the molecule via the nitrogen atom, wherein
$R_{27}$ is $C_1$-$C_4$alkyl or $C_1$-$C_4$alkylcarbonyl and $R_{28}$ is hydrogen, $C_1$-$C_4$alkoxy or halogen; or
(e) $NR_{20}R_{21}$, wherein $R_{20}$ and $R_{21}$ are independently selected from hydrogen, $C_1$-$C_4$alkyl, $C_3$-$C_7$cycloalkyl which is unsubstituted or monosubstituted by hydroxy; and phenyl which is unsubstituted or monosubstituted by 1,2,3-thiadiazol-4-yl, under the proviso that not both $R_{20}$ and $R_{21}$ can represent hydrogen at the same time; and

$R_2$ denotes $C_1$-$C_8$alkyl, which is unsubstituted or substituted by $C_3$-$C_7$cycloalkyl which is unsubstituted or disubstituted by halogen; phenyl, which is mono- or disubstituted by halogen;

under the proviso that $R_2$ does not represent -1, 1 -dimethylethyl, if

(a) $R_1$ is benzo[b]imidazol-1-yl, 1-imidazolyl, 4,5-dichloro-1-imidazolyl, 2-($C_1$-$C_4$alkyl)-1-imidazolyl, imidazolidin-2,5-dion-1-yl, 5,5-dimethyl-oxazolidin-2,4-dion-3-yl, 1 H-1,2,3-triazol-1-yl, 2H-1,2,3-triazol-2-yl, 3-nitro-1H-1,2,4-triazol-1-yl, 2H-tetrazol-2-yl or 1H-tetrazol-1-yl, or if $R_1$ is a radical of substructure Ic, $R_{23}$ to $R_{26}$ are hydrogen, X is $NR_{18}$ and $R_{18}$ is hydrogen, methyl, ethyl, acetyl, 4-pyridyl, 1-piperidinyl, phenyl, methoxyphenyl, ethoxyphenyl, fluorophenyl or chlorophenyl;
(b) $R_1$ is a radical of substructure Ic, $R_{23}$ to $R_{26}$ are hydrogen, X is $-(CH_2)_s-CR_{17}R_{18}-$, s is 0, and $R_{17}$ and $R_{18}$ are selected from hydroxyl and phenyl which is monosubstituted by chloro or $R_{17}$ and $R_{18}$ are selected from hydrogen, methoxyphenyl and N-phenyl-carbamoyl; or
(c) $R_1$ is a radical of substructure Id, k is 1, A is a bond, E is $NR_{22}$, $R_{22}$ is hydrogen, G, Q and T are $CH_2$, B and D are carbonyl and $R_{19}$ is methyl, n-propyl or iso-butyl;

under the proviso that $R_2$ does not represent 2-methylpropyl, if $R_1$ is a radical of substructure Id, k is 1, A is a bond, E is $NR_{22}$, $R_{22}$ is hydrogen, G, Q and T are $CH_2$, B and D are carbonyl and $R_{19}$ is methyl, or if $R_1$ is a radical of substructure Ic, $R_{23}$ to $R_{26}$ are hydrogen, X is $-(CH_2)_s-CR_{17}R_{18}-$, s is 0, and $R_{17}$ and $R_{18}$ are selected from hydrogen and phenyl which is monosubstituted by methoxy;
and under the proviso that $R_2$ does not represent 1-methylethyl, if $R_1$ is a radical of substructure Ic, $R_{23}$ to $R_{26}$ are hydrogen, X is $NR_{18}$ and $R_{18}$ is methoxyphenyl or ethoxyphenyl, or X is $CR_{17}R_{18}$ and $R_{17}$ and $R_{18}$ are selected from hydrogen and methoxyphenyl;
or a tautomer thereof,
or a salt of such pyrrolo pyrimidine or its tautomer.

Accordingly in further aspects the invention provides:

a compound of formula I for use as a pharmaceutical;
a pharmaceutical composition comprising a compound of formula I as an active ingredient; the use of a compound of formula I for the preparation of a medicament for therapeutic or prophylactic treatment of a disease or medical condition in which cathepsin S is implicated.

A compounds of formula I wherein Y represents $-(CH_2)_t-O-$ or $(CH_2)_r-S-$ and t, r, $R_1$, $R_2$ and p have the meanings as provided above for a compound of formula I, can be prepared, e.g., by alkylating an alcohol or thiol of formula II,

$$R_1-(Y)_p-H \quad (II)$$

wherein Y represents $-(CH_2)_t-O-$ or $(CH_2)_r-S-$ and t, r and $R_1$ have the meanings as provided above for a compound of formula I, with a pyrrolo pyrimidine of formula III

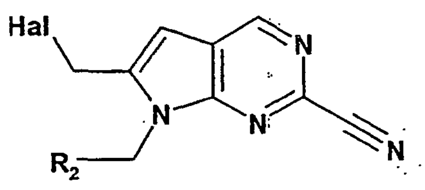

(III)

wherein $R_2$ has the meaning as provided above for a compound of formula I and Hal denotes halo, preferably bromo, wherein the starting compounds of formula II and III may also be present with functional groups in protected form, if necessary, and/or in the form of salts, provided a salt-forming group is present and the reaction in salt form is possible; wherein any protecting groups in a protected derivative of a compound of the formula I are removed;
and, if so desired, an obtainable compound of formula I is converted into another compound of formula I or a N-oxide thereof, a free compound of formula I is converted into a salt, an obtainable salt of a compound of formula I is converted into the free compound or another salt, and/or a mixture of isomeric compounds of formula I is separated into the individual isomers.

Detailed description of the alkylation:

In the more detailed description of the process below, t, r, $R_1$ and $R_2$ are as defined for compounds of formula

I, unless otherwise indicated.

The alkylation of an alcohol or thiol of formula II with an alkylhalide of formula III can be accomplished by standard procedures known in the art, e.g., by reacting both compounds in a suitable solvent, e.g. dimethylacetamide or dimethylformamide, by the addition of a suitable base, e.g. a carbonate such as potassium carbonate, at a temperature between 0 ˚C and reflux temperature of the solvent used, preferably a temperature about between 10 ˚C and about 35 ˚C, for a period of between about 15 minutes and 48 hours, preferably between 2 hours and 12 hours.

Protecting groups

If one or more other functional groups, for example carboxy, hydroxy, amino, or mercapto, are or need to be protected in a compound of formulae II or III, because they should not take part in the reaction, these are such groups as are usually used in the synthesis of peptide compounds, and also of cephalosporins and penicillins, as well as nucleic acid derivatives and sugars.

The protecting groups may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end-products. The specialist knows, or can easily establish, which protecting groups are suitable with the reactions mentioned hereinabove and hereinafter.

The protection of such functional groups by such protecting groups, the protecting groups themselves, and their removal reactions are described for example in standard reference works, such as J. F. W. McOmie, "Protective Groups in Organic Chemistry", Plenum Press, London and New York 1973, in T. W. Greene, "Protective Groups in Organic Synthesis", Wiley, New York 1981, in "The Peptides"; Volume 3 (editors: E. Gross and J. Meienhofer), Academic Press, London and New York 1981, in "Methoden der organischen Chemie" (Methods of organic chemistry), Houben Weyl, 4th edition, Volume 15/l, Georg Thieme Verlag, Stuttgart 1974, in H.-D. Jakubke and H. Jescheit, "Aminosäuren, Peptide, Proteine" (Amino acids, peptides, proteins), Verlag Chemie, Weinheim, Deerfield Beach, and Basel 1982, and in Jochen Lehmann, "Chemie der Kohlenhydrate: Monosaccharide und Derivate" (Chemistry of carbohydrates: monosaccharides and derivatives), Georg Thieme Verlag, Stuttgart 1974.

Additional process steps

Salts of a compound of formula I with a salt-forming group may be prepared in a manner known *per se.* Acid addition salts of compounds of formula I may thus be obtained by treatment with an acid or with a suitable anion exchange reagent. A salt with two acid molecules (for example a dihalogenide of a compound of formula I) may also be converted into a salt with one acid molecule per compound (for example a monohalogenide); this may be done by heating to a melt, or for example by heating as a solid under a high vacuum at elevated temperature, for example from 130 to 170˚C, one molecule of the acid being expelled per molecule of a compound of formula I.

Salts can usually be converted to free compounds, e.g. by treating with suitable basic agents, for example with alkali metal carbonates, alkali metal hydrogencarbonates, or alkali metal hydroxides, typically potassium carbonate or sodium hydroxide.

General process conditions

All process steps described here can be carried out under known reaction conditions, preferably under those specifically mentioned, in the absence of or usually in the presence of solvents or diluents, preferably such as are inert to the reagents used and able to dissolve these, in the absence or presence of catalysts, condensing agents or neutralisiing agents, for example ion exchangers, typically cation exchangers, for example in the $H^+$ form, depending on the type of reaction and/or reactants at reduced, normal, or elevated temperature, for example in the range from -100˚C to about 190˚C, preferably from about -80˚C to about 150˚C, for example at -80 to -60˚C, at room temperature, at - 20 to 40˚C or at the boiling point of the solvent used, under atmospheric pressure or in a closed vessel, where appropriate under pressure, and/or in an inert atmosphere, for example under argon or nitrogen.

Salts may be present in all starting compounds and transients, if these contain salt-forming groups. Salts may also be present during the reaction of such compounds, provided the reaction is not thereby disturbed.

The solvents from which those can be selected which are suitable for the reaction in question include for example water, esters, typically lower alkyl-lower alkanoates, e.g diethyl acetate, ethers, typically aliphatic ethers, e.g. diethylether, or cyclic ethers, e.g. tetrahydrofuran, liquid aromatic hydrocarbons, typically benzene or toluene, alcohols, typically methanol, ethanol or 1- or 2-propanol, nitriles, typically acetonitrile, halogenated hydrocarbons, typically dichloromethane, acid amides, typically dimethylformamide, bases, typically heterocyclic nitrogen bases, e.g. pyridine, carboxylic acids, typically lower alkanecarboxylic acids, e.g. acetic acid, carboxylic acid anhydrides, typically lower alkane acid anhydrides, e.g. acetic anhydride, cyclic, linear, or branched hydrocarbons; typically cyclohexane, hexane; or isopentane, or mixtures of these solvents, e.g. aqueous solutions, unless otherwise stated in the description of the process. Such solvent mixtures may also be used in processing, for example through chromatography or distribution.

The compounds of formula I, including their salts, are also obtainable in the form of hydrates, or their crystals can include for example the solvent used for crystallization (present as solvates).

In the preferred embodiment, a compound of formula I is prepared according to or in analogy to the processes and process steps defined in the Examples.

The dosage of the active ingredient depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy without toxicity requires a regimen based on the kinetics of the drug's availability to target sites. This involves a consideration of the distribution, equilibrium, and elimination of a drug.

The dose of a compound of the formula I or a pharmaceutically acceptable salt thereof to be administered to warm-blooded animals, for example humans of approximately 70 kg body weight, is preferably from approximately 3 mg to approximately 5 g, more preferably from approximately 10 mg to approximately 1.5 g, most preferably from about 100 mg to about 1000 mg per person per day, divided preferably into 1 to 3 single doses which may, for example, be of the same size. Usually, children receive half of the adult dose.

The invention relates also to pharmaceutical compositions comprising an effective amount, especially an amount effective in the treatment of one of the above-mentioned disorders, of compound of the formula I or an N-oxide or a tautomer thereof together with pharmaceutically acceptable carriers that are suitable for topical, enteral, for example oral or rectal, or parenteral administration and that may be inorganic or organic, solid or liquid. There are used for oral administration especially tablets or gelatin capsules that comprise the active ingredient together with diluents, for example lactose, dextrose, mannitol, and/or glycerol, and/or lubricants and/or polyethylene glycol. Tablets may also comprise binders, for example magnesium aluminum silicate, starches, such as corn, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavorings and sweeteners. It is also possible to use the pharmacologically active compounds of the present invention in the form of parenterally administrable compositions or in the form of infusion solutions. The pharmaceutical compositions may be sterilized and/or may comprise excipients, for example preservatives, stabilisers, wetting agents and/or emulsifiers, solubilisers, salts for regulating the osmotic pressure and/or buffers. The present pharmaceutical compositions, which may, if desired, comprise other pharmacologically active substances are prepared in a manner known per se, for example by means of conventional mixing, granulating, confectioning, dissolving or lyophilising processes, and comprise approximately from 1% to 95%, especially from approximately 1 % to approximately 20%, active ingredient(s).

Starting materials

New starting materials and/or intermediates, as well as processes for the preparation thereof, are likewise the subject of this invention. In the preferred embodiment, such starting materiats are used and reaction conditions so selected as to enable the preferred compounds to be obtained.

Starting materials of the formula II and III are known, commercially available, or can be synthesized in analogy to or according to methods that are known in the art or described in the Examples.

In particular, a pyrrolo pyrimidine of formula III

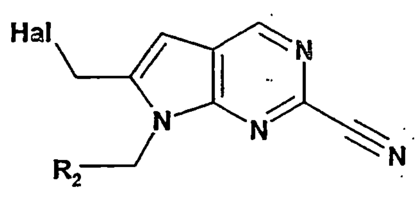

(III)

wherein $R_2$ has the meaning as provided above for a compound of formula I and Hal denotes halo, can be prepared by the following reaction sequence.

In a first step, a pyrimidine of formula IV

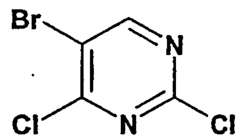

(IV)

is reacted with an amine of formula V,

$R_2$-$CH_2$-$NH_2$ (V)

wherein $R_2$ has the meaning as provided above for a compound of formula I, in a manner known as such, e.g. by adding at a temperature between about -10 °C and about + 10 °C, e.g. about 0 °C, the amine of formula V dropwise to a solution of the pyrimidine of formula IV in a suitable solvent, e.g. a $C_1$-$C_3$alcohol, and allowing the solution to react a temperature between about 15 °C and about 30 °C, e.g. about 20 °C, for a period of about 3 to 12 hours, providing a pyrimidine of formula VI

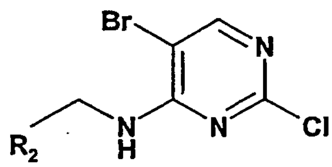

(VI)

wherein $R_2$ has the meaning as provided above for a compound of formula I.

In a second step, the pyrimidine of formula VI, wherein $R_2$ has the meaning as provided above for a compound of formula I, is reacted with a cyanide, e.g. potassium or sodium cyanide, in a suitable solvent in a manner known per se, providing the 2-cyano-pyrimidine derivative of formula VII,

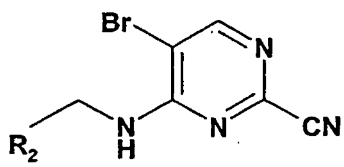

(VII)

wherein $R_2$ has the meaning as provided above for a compound of formula I.

In a second step, the 2-cyano-pyrimidine derivative of formula VII, wherein $R_2$ has the meaning as provided above for a compound of formula I, is reacted with the compound of formula VIII

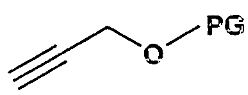

(VIII)

wherein PG denotes a suitable protecting group, which is stable under the conditions of the coupling reaction, in a suitable solent, e.g. dimethylformamide, e.g. in the presence of a palladium-(II) catalyst, cupper-(I) iodide and a suitable base, e.g. a trialkyl amine like triethylamine, furnishing the 2-cyano-pyrimidine derivative of formula IX,

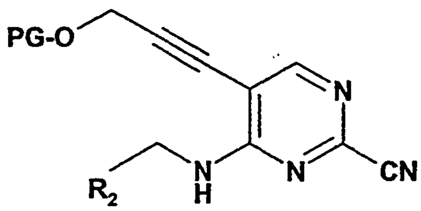

(IX)

wherein $R_2$ has the meaning as provided above for a compound of formula I and PG denotes a protecting group.

Cyclisation of the 2-cyano-pyrimidine derivative of formula IX, wherein $R_2$ has the meaning as provided above for a compound of formula I and PG denotes a protecting group, can be achieved, e.g., by adding 1,8-diazabicyclo[5.4.0] undec-7-ene at a temperature of between about 80 °C and about 120 °C, e.g. about 100 °C, to a solution of the 2-cyano-pyrimidine derivative of formula IX in a suitable solvent, such as dimethylformamide, and maintaining the mixture at about that temperature for a period of about 0.5 to 2 hours, e.g. 1 hour, furnishing a protected hydroxymethyl pyrrolo pyrimidine of formula X,

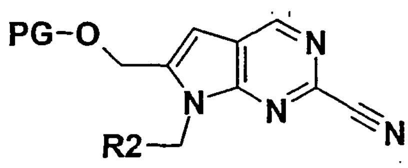

(X)

wherein $R_2$ has the meaning as provided above for a compound of formula I and PG denotes a protecting group.

The protection group PG can be detached under conditions known per se to furnish the unprotected hydroxymethyl pyrrolo pyrimidine of formula XI,

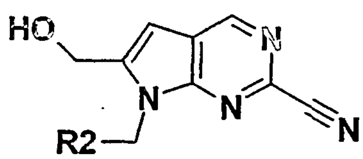

(XI)

wherein $R_2$ has the meaning as provided above for a compound of formula I. Said hydroxymethyl pyrrolo pyrimidine of formula XI can be converted into the desired pyrrolo pyrimidine of formula III by standard substitution reactions replacing the hydroxyl group by a halo group.

Alternatively, the 2-cyano-pyrimidine derivative of formula VII,

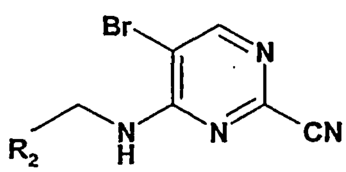

(VII)

wherein $R_2$ has the meaning as provided above for a compound of formula I, can be reacted under suitable conditions known per se, e.g. those conditions for the preparation of a compound of formula IX mentioned above, with a compound of formula XII

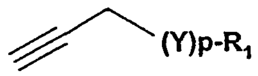

(XII)

wherein $R_1$, Y and p have the meanings as provided above for a compound of formula I, furnishing a compound of formula XIII,

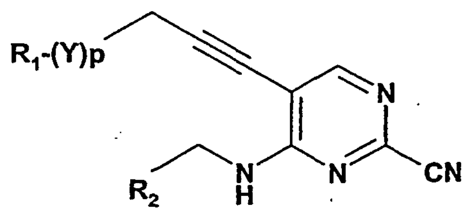

(XIII)

wherein $R_1$, $R_2$, Y and p have the meanings as provided above for a compound of formula I,

Cyclisation of the 2-cyano-pyrimidine derivative of formula XIII, wherein $R_1$, $R_2$, Y and p have the meanings as provided above for a compound of formula I, can be achieved, e.g., by adding 1,8-diazabicyclo[5.4.0]undec-7-ene at a temperature of between about 80˚C and about 120 ˚C, e.g. about 100 ˚C, to a solution of the 2-cyano-pyrimidine derivative of formula XIII in a suitable solvent, such as dimethylformamide, and maintaining the mixture at about that temperature for a period of about 0.5 to 2 hours, e.g. 1 hour, furnishing directly a protected hydroxymethyl pyrrolo pyrimidine of formula I.

Particularly preferred compounds of the invention are the compounds of the Examples.

The present invention relates to methods of using compound of formula I and their pharmaceutically acceptable salts, or pharmaceutical compositions thereof, in mammals for inhibiting cathepsin S, and for the treatment of cathepsin S dependent conditions, such as the cathepsin S dependent conditions, described herein, e.g. chronic inflammatory or neuropathic pain.

Particularly the present invention relates to a method of selectively inhibiting cathepsin S activity in a mammal which comprises administering to a mammal in need thereof an effective cathepsin S inhibiting amount of a compound of formula I.

More specifically such relates to a method of treating chronic inflammatory or neuropathic pain. (and other diseases as identified above) in mammals comprises administering to a mammal in need thereof a correspondingly effective amount of a compound of formula I.

## EXAMPLES

The Examples which follow serve to illustrate the invention without limiting the scope thereof.

Temperatures are measured in degrees Celsius. Unless indicated otherwise, reactions are carried out at room temperature. The structure of final products, intermediates and starting materials is confirmed by standard analytical methods, e.g. microanalysis and spectroscopic characteristics (e.g. MS, IR, NMR).

### Abbreviations

Abbreviations used are those conventional in the art and, in particular, have the meanings provided below.

| | |
|---|---|
| Ac | acetyl |
| aq. | Aqueous |
| Boc | tert-butoxycarbanyl |
| conc. | concentrated |
| DABCO | 1,4-diazabicyclo[2.2.2]octane |
| DEAD | diethyl azodicarboxylate |
| DMF | dimethylformamide |
| DMSO | dimethylsulfoxide |
| Et | ethyl |

| | |
|---|---|
| FC | flash chromatography |
| Me | methyl |
| min | minutes |
| MS | mass spectrometry |
| NMR | nuclear magnetic resonance |
| Ph | phenyl |
| RP-HPLC | reversed phase high pressure liquid chromatography |
| rt | room temperature |
| sat. | saturated |
| soln. | Solution |
| TFA | trifluoroacetic acid |
| THF | tetrahydrofurane |
| TsOH | toluene sulphonic acid |

Example A: 6-Bromomethyl-2-cyano-7-(2-cyclohexyl-ethyl)-7H-pyrrolo[2,3-d]pyrimidin

At 0˚C, a soln. of $CBr_4$ (56.1 g, 0.17 mol) in dry $CH_2Cl_2$ (150 ml) is added dropwise over 15 min to a soln. of step A.5 (20.65 g, 84.5 mmol) and $Ph_3P$ (44.2 g, 0.17 mol) in dry $CH_2Cl_2$ (150 ml). After stirring for 30 min at 0˚C, the mixture is warmed to rt, stirred for 3 h. The mixture is diluted with $CH_2Cl_2$ (300 ml), washed with sat. aq. $NaHCO_3$ soln. (150 ml) and brine (150 ml), and dried ($MgSO_4$). The org. layer is treated with $SiO_2$ (70 g), evaporated, and the residue is loaded on a silica gel column. FC (800 g of silica gel; hexane/EtOAc 7:4) gives the title compound as a yellow solid; $^1$H-NMR (400 MHz, $CDCl_3$) δ 0.98-1.11 (m, 2H), 1.18-1.45 (m, 5H), 1.64-1.89 (m, 6H), 4.40 (t, 2H), 4.68 (s, 2H), 6.70 (s, 1H), 8.95 (s, 1H).

Step A.1: (5-Bromo-2-chloro-pyrimidin-4-yl)-(2-cyclohexyl-ethyl)-amine

2-Cyclohexyl-ethylamine (40.3g, 320 mmol) is added dropwise at 0˚C over 20 min to a soln. of 5-bromo-2,4-dichloropyrimidine (51 g, 224 mmol) in MeOH (200 ml). After stirring for 20 min at 0˚C, the mixture is warmed to rt, stirred for 11 h, and evaporated. The residue is suspended in 200 ml of $CH_2Cl_2$, washed with water and brine, dried ($MgSO_4$), and evaporated. The residue is chromatographed on silica gel column (hexane/EtOAc 5:1) to give the title product; $^1$H-NMR (400 MHz, $CDCl_3$) δ 0.90-1.01 (m, 2H), 1.10-1.41 (m, 5H), 1.55 (q, 2H), 1.61-1.80(m, 4H), 3.52 (q, 2H), 5.43 (brs, 1H), 8.09 (s, 1H).

Step A.2: 5-Bromo-4-(2-cyclohexyl-ethylamino)-pyrimidine-2-carbonitrile

At rt, to an aqueous soln. (5 ml) of NaCN (1.27 g, 25.9 mmol) is added successively DMSO (50 ml), DABCO (0.24 g, 2.16 mmol), and the product of step A.1 (6.9 g, 21.6 mmol). The mixture is stirred for 11 h at 60˚C, poured into ice water, extracted with EtOAc, dried ($MgSO_4$), and evaporated. The residue is chromatographed on a silica gel column. (hexane/EtOAc 4:1) to give the title product.

Step A.3: 2-Cyano-4-(2-cyclohexyl-ethyl)amino-5-[3-(tetrahydro-2*H*-pyran-2-yloxy)-prop-1-ynyl]-pyrimidine

At rt, a soln. of the product of step A.2 (25.0 g, 89.9 mmol) and 2-prop-2-ynyloxy-tetrahydropyran (13.6 ml, 97.02 mmol) in dry DMF (420 ml) is treated with $Et_3N$ (56.5 ml, 40.5 mmol), CuI (0.78 mg, 4.05 mmol), and $(Ph_3P)_2PdCl_2$ (1.4 g, 2.02 mmol). The mixture is stirred for 3 h at 70˚C, poured into ice water, extracted with EtOAc, washed with brine, dried ($MgSO_4$), and evaporated. The residue is chromatographed on a silica gel column (1800 g of silica gel; hexane/EtOAc 2:1) to give the title compound; $^1$H-NMR (400 MHz, $CDCl_3$) δ 0.90-1.02 (m, 2H), 1.10-1.40 (m, 5H), 1.48-1.91 (m, 12H), 3.49 - 3.60 (m, 3H), 3.84 - 3.92 (m, 1H), 4.54 (s, 2H), 4.86 (t, 1H), 5.88 (brt, 1H), 8.19 (s, 1H).

Step A.4: 7-(2-cyclohexyl-ethyl)-6-hydroxymethyl-7H-pyrrolo[2,3-d]pyrimidin-2-ol

At rt, a soln. of the product of step A.3 (23.1 g, 62.69 mmol) in dry DMF (400 ml) is treated with DBU (11.3 ml, 75.23 mmol), stirred for 1 h at 100˚C, poured into ice water, extracted with EtOAc, washed with $H_2O$, dried ($MgSO_4$), and evaporated. The residue is chromatographed on silica gel.column (hexane/EtOAc 5:1) to give the title compound; $^1$H-NMR (400 MHz, $CDCl_3$) δ 0.93-1.08 (m, 2H), 1.12-1.40 (m, 5H), 1.48-1.91 (m, 12H), 3.54-3.62 (m, 1H), 3.82 - 3.91 (m, 1H), 4.38 (t, 2H), 4.70 (d, 1H), 4.73 (t, 1H), 4.94 (d, 1H), 6.61 (s, 1H), 8.91 (s, 1H).

Step A.5: 7-(2-cyclohexyl-ethyl)-6-hydroxymethyl-7H-pyrrolo[2,3-d]pyrimidin-2-ol

At rt, a soln. of step A.4 (21.4 g, 58.08 mmol) in MeOH (200 ml) is treated with TsOH•$H_2O$ (1.1 g, 5.78 mmol), stirred for 11 h and evaporated. The residue is diluted with $CH_2Cl_2$ and washed with water and sat. $NaHCO_3$ aq. The organic extract is dried ($MgSO_4$) and concentrated. The residue is chromatographed on a silica gel column to give the title compound.

Example B:

By repeating the procedures described under Example A using appropriate starting materials and conditions the following compounds of formula 1 are obtained as identified below in Table 1.

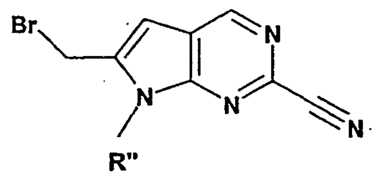

(1)

Table 1

| Ex. | R" | Rf (solvent) | NMR(400MHz, δ) |
|---|---|---|---|
| B1 | 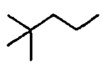 | 0.20<br>(n-hexane: AcOEt=4:1) | $CDCl_3$<br>1.09(s, 9H), 1.70-1.78(m, 2H), 4.35- 4.42 (m, 2H), 4.64(s, 2H), 6.70(s, 1H), 8.96(s, 1H) |
| B2 | 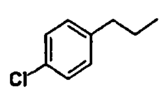 | 0.15<br>(*n*-hexane: AcOEt=4:1) | $CDCl_3$<br>3.18(t, 2H), 4.25(s, 2H), 4.58(t, 2H), 6.64(s, 1H), 7.00 (d, 2H), 7.25(d, 2H), 8.97(s, 1H) |

Example C: Phenol Derivatives

Example C.1: 3-Fluoro-4-hydroxy-N-propyl-benzamide

To the solution of 3-fluoro-4-hydroxybenzoic acid (5 g, 32 mmol) and propylamine (3.1 ml, 38 ml) in DMF (250 ml), HOAt (5.2 g, 38 mmol) and WSCl.HCl (7.2 g, 38 mmol) are added at 0˚C. The reaction mixture is stirred at rt for 15 h and quenched with saturated ammonium chloride and extracted with ethyl acetate. The combined extracts are washed with $H_2O$, brine and dried over magnesium sulfate. Chromatography on silica gel (eluent; dichloromethane and 3 % MeOH in dichloromethane) gives 4.8 g of desired product; Rf=0.76 (dichloromethane : MeOH = 8:2).

Example C.2: 3-Hydroxy-N-propyl-benzamide

To a solution of 3-hydroxy-benzoic acid (430 mg, 3.6 mmol) in THF (5 ml) are added $SOCl_2$ (0.4 ml) and DMF (2 drops). The reaction mixture is stirred at rt overnight. The mixture is divided in half, and to this mixture are added $Et_3N$ (0.42 ml) and the corresponding amine. After the mixture is stirred at rt for overnight, it is diluted with water. The mixture is extracted with EtOAc, and the combined organic extracts are washed with brine, and dried over $Na_2SO_4$, filtered, and concentrated to give the product.

Example D

Example D.1: (4-Hydroxy-phenyl)-piperidin-1-yl-methanone

To a solution of toluene (6mL) is added trimethylaluminium (1M in hexane, 3mL), piperidine (3mmol) at rt. The mixture is stirred for 0.3 h at rt. 4-Hydroxybenzoic acid ethyl ester is added and stirred for 1h at 100˚C. The reaction

mixture is diluted with water and 8N KOH aq. is added. Then the reaction mixture is acidified with conc. HCl aq. and extracted with dichloromethane (3 times). The combined organic layer is washed with water and brine, dried over $MgSO_4$, and concentrated in vacuo to give the title compound.

Example D.2: 4-Hydroxy-N-pyridin-3-ylmethyl-benzamide

To a solution of DMF/$H_2O$ (20mL/7mL) is added 4-benzyloxy-benzoic acid (1g), 3-methyl-amino pyridine (710mg), HOAt (715mg), WSCD HCl (1g). The mixture is stirred for 3h at rt, diluted with ice water. The white precipitate is collected by filtration. To a solution of the above product in methanol is added Pd/C, and the mixture is stirred for 12 h under $H_2$ atmospheres. The reaction mixture is filtrated through a pad of Celite and concentrated to give the title compound.

Example E: Azepin Derivatives

Example E.1: 7-Methoxy-2,3,4,5-tetrahydro-benzo[c]azepin-1-one and 7-Methoxy-1,3,4,5-tetrahydro-benzo[b]azepin-2-one

To a heated solution of 6-methoxy-1-tetalone (1g) in trichloroacetic acid (10g) is added sodium azide (553mg) at 70˚C, and the mixture is maintaining with stirring for 4h. The reaction mixture is diluted with ice water and neutralized with potassium carbonate, and extracted with ethyl acetate. The organic layer is successively washed with water and saturated NaCl aq, dried over $MgSO_4$, concentrated in vacuo. The crude product is purified by silica gel column chromatography to give 7-methoxy-2,3,4,5-tetrahydro-benzo[c] azepin-1-one (later) in 49% yield and 7-methoxy-1,3,4,5-tetrahydro-benzo[b] azepin-2-one in 27% yield.

Example E.2: 7-Hydroxy-2,3,4,5-tetrahydro-benzo[c]azepin-1-one

To a solution of 7-methoxy-2,3,4,5-tetrahydro-benzo[c]azepin-1-one (520 mg) in dichloromethane (3mL) is added boron tribromide in dichloromethane (1M in dichloromethane) at 0˚C and stirred for 2.5 h at rt. The reaction mixture is diluted with water and neutralized with aq. sodium hydrogen carbonate. White precipitate in the mixture is collected by filtration. The precipitate is dried in vacuo providing the title compound.

Example F: Synthesis of 4-pyrrolidinyl-phenol derivative

A mixture of 4-amino-2-fluoro-phenol (3.4mmol) and γ-butyrolactone (3.57mmol) with 90 ml of conc. HCl is heated to 190˚C and stirred for 1.5 h. After cooling down to rt the reaction mixture is diluted with THF and NaHCO3 aq, extracted with AcOEt, and dried over Na2SO4. Flush chromatography on silica gel using AcOEt-Hexane (3:1) gives 1-(3-fluoro-4-hydroxyphenyl)-pyrrolidin-2-one.

Example G: Synthesis of 4-pyrrolidinyl-phenol derivative

4-Iodophenol (1.0mmol) is dissolved in 3ml of dioxane. To the solution is added 3,3-dimethyl-pyrrolidin-2-one (1.2mmol), K2CO3 (2.0mmol), and N,N'-dimethylethylene diamine at rt. The reaction mixture is heated and stirred for 14 h at 110˚C under N2, and then filtered through celite. The resulting mixture is diluted with AcOEt and NaHCO3 aq, extracted with AcOEt, dried over Na2SO4. Flush chromatography on silica gel using AcOEt-Hexane (3:1) gives 1-(4-hydroxy-phenyl)-3,3-dimethyl-pyrrolidin-2-one as brown solid.

Example H: 4-(4-Hydroxy-phenyl)-piperidine-1-carboxylic acid tert-butyl ester

A solution of the product of Step H.1 (2g) in 1M HCl in EtOAc is stirred for 0.5 h under reflux, and concentrated in vacuo. The residue is suspended in diethyl ether, and white powder is collected by filtration. To a solution of the powder in methanol (100mL) is added Pd/C (10%w/w, 200mg) and stirred for 18 h under H2 atmosphere. The reaction mixture is filtrated through celite pad, and the filtrate is concentrated in vacuo to give the crude product. To a solution of the crude product (300mg) in DMF is added $Boc_2O$ (305mg) and Et3N, and stirred for 5 h at rt. The reaction mixture is diluted with H2O and extracted with EtOAc (twice). The organic layer are combined, successively washed with H2O, aq. NaCl, dried over MgSO4, and concentrated in vacuo. The residue is purified by column chromatography to give the pure product; Rf= 0.56 (n-hexane; EtOAc= 1:1).

Step H.1: 1-Benzyl-4-(4-benzyloxy-phenyl)-piperidin-4-ol

To a solution of 1-Benzyloxy-4-bromo-benzene (5g) in tetrahydrofurane (100mL) is added n-butyllithium (1.6M in hexane, 13mL) at -78˚C and stirred for 0.5 h at -78˚C. To the mixture is added 1-benzyl-piperidin-4-one in tetrahydrofurane (3.6g in 20mL) at -78˚C, and maintaining with stirring at -78˚C for 1.5 h. The reaction mixture is diluted with aq. NH4Cl, then extracted with EtOAc. The organic layer is successively washed with H2O and aq. NaCl, dried over MgSO4, and concentrated in vacuo. The crude product is purified by column chromatography to give the pure product; Rf= 0.15 (n-hexane; EtOAc= 1:1).

Example I: 4-(4-Hydroxy-phenyl)-3,6-dihydro-2H-pyridine-1-carboxylic acid tert-butyl ester

To a solution of the product of Example H (2g) in methanol (50ml) is added Pd/C (10%w/w, 200mg) and stirred for 9 h under $H_2$ atmosphere. The reaction mixture is filtrated through a celite pad. To the filtrate is added HCl (1M in EtOH, 50ml) and stirred under reflux. The reaction mixture is concentrated in vacuo to give crude product. To a solution of the crude product in MeOH/THF/H2O (10ml/ 5ml/ 10ml) is added NaHCO3 (until pH=9), $Boc_2O$ at 0˚C and maintaining with stirring for 1 h at 0˚C. The reaction mixture is evaporated, neutralized with aq. citric acid, and extracted with EtOAc (3 times). The organic layers are combined, successively washed with H2O, aq. NaCl, dried over MgSO4, and concentrated. The residue is purified by column chromatograph to give the pure product; Rf = 0.60 (n-hexane; EtOAc = 1:1).

Example J: 1-[4-(3-Fluoro-4-hydroxy-phenyl)-piperazin-1-yl]-ethanone

To a solution of the product of Step J.1 (1g) in methanol (100mL) is added Pd/C (10% w/w on activated carbon, 0.1 g), and stirred for 11 h under H2 atmosphere. The reaction mixture is filtrated through a celite pad. The filtrate is concentrated to give the title compound; Rf = 0.23 (dichloromethane : methanol= 9:1).

Step J.1: 1-[4-(4-Benzyloxy-3-fluoro-phenyl)-piperazin-1-yl]-ethanone

To a solution of 1-benzyloxy-4-bromo-2-fluoro-benzene (1g), 1-acetyl piperazine (0.55 g) and sodium tert-butoxide (0.51 g) in toluene (70mL) is added tri-o-tolyl-phosphane (0.05 g) and $Pd_2(dba)_3$ (0.16 g) under N2 atmosphere, stirred for 4 h under reflux. The reaction mixture is diluted with H2O, extracted with EtOAc. The organic layer is successively washed with H2O and aq. sodium chloride, dried over MgSO4, and concentrated in vacuo. The crude product is purified by silica gel column chromatography to give the pure product; Rf = 0.29 (dichloromethane:methanol= 9:1).

Example K: 4-(3-Fluoro-4-hydroxy-phenyl)-piperazine-1-carboxylic acid tert-butyl ester

To a solution of the product of Step K.1 (2.8g) in methanol (100mL) is added Pd/C (10% w/w on activated carbon), and stirred for 12 h under $H_2$ atmosphere. The reaction mixture is filtrated through a pad of Celite. The filtrate is concentrated to give the title product; Rf=0.13 (n-hexane:EtOAc= 4:1).

Step K.1: 4-(4-Benzyloxy-3-fluoro-phenyl)-piperazine-1-carboxylic acid tert-butyl ester

To a solution of 1-Benzyloxy-4-bromo-2-fluoro-benzene (3g), piperazine-1-carboxylic acid tert-butyl ester (2.36g) and sodium tert-butoxide (1.54g) in toluene (210ml) is added tri-o-tolyl-phosplane (0.163g) and $Pd_2(dba)_3$ (0.49g) under $N_2$ atmosphere, stirred for 11 h at 80˚C. The reaction mixture is diluted with $H_2O$, extracted with EtOAc. The organic layer is successively washed with $H_2O$ and aq. sodium chloride, dried over $MgSO_4$, and concentrated in vacuo. The crude product is purified by silica gel column chromatography to give the pure title product; Rf=0.19 (n-hexane: EtOAc = 4:1).

Example L: (4-Prop-2-ynyl-phenyl)-methanol

To a suspension of Mg powder (19.3 mmol) and one piece of iodine in THF (10 mL) is added (4-bromo-benzyloxy)-trimethyl-silane (16.0 mmol) in THF (20 mL) at rt and the mixture is stirred at 85 ˚C for 0.5 h. Copper(I) bromide (1.60 mmol) is added at rt, then methoxyallene (16.0 mmol) in THF (10 mL) is added at 0 ˚C and the mixture is stirred at rt for 5 h. The mixture is poured into saturated ammonium chloride, extracted with AcOEt. The organic layer is washed with 1*N* HCl solution, $H_2O$, and brine, dried over $MgSO_4$ and concentrated. Chromatography on silica gel (*n*-hexane:AcOEt=1:9) gives the title compound; Rf=0.4 ($CH_2Cl_2$:AcOEt =3:2).

Example M: 1-Chloro-4-prop-2-ynyl-benzene

A mixture of methyl propargylether (50.0 g, 714mmol) and *t*-BuOK (4.0 g, 36mmol) is refluxed under $N_2$ for 1 h. The mixture is distilled to produce a colorless oil of methoxyallen (50 g, quant.). To a solution of said methoxyallen (42 mL, 50 mmol) and CuBr (720 mg, 5 mmol) in 200 mL of diethylether is added dropwise a 1*M* solution of *p*-chlorophenyl magnesium bromide in diethylether (50 mL, 50 mmol) at 0 ˚C under $N_2$. After being stirred for 1 h at rt, 150 mL of *sat*.$NH_4Cl$ solution is added, and the mixture is extracted with ether and washed with *sat*.$NaHCO_3$ solution. The organic layer is dried over $Na_2SO_4$ and concentrated. Purification of the residue by column chromatography eluting hexane only to give 1-chloro-4-prop-2-ynyl-benzene as a yellow oil.

Example N: 1-Fluoro-4-prop-2-ynyl-benzene

1-Fluoro-4-prop-2-ynyl-benzene is synthesized from *p*-fluorophenyl magnesium bromide and methoxyallen by the procedure as described under Example M.

Example O: 1-(4-Prop-2-ynyl-phenyl)-pyrrolidin-2-one

4-Prop-2-ynyl-phenylamine (2.0 mmol) and g-butyrolactone (2.0 mmol) in *conc.* HCl is heated to 190 ˚C and stirred for 1 h. After cooling down to rt the reaction mixture is diluted with $NaHCO_3$ *aq*, extracted with AcOEt, and dried over $Na_2SO_4$. Flush chromatography on silica gel using AcOEt-Hexane (1:1) gives 1-(4-prop-2-ynyl-phenyl)-pyrrolidin-2-one.

Example P: 6-(4-Chloro-benzyl)-7H-pyrrolo[2,3-d]pyrimidine-2-carbonitrile

5-Bromo-2,4-dichloropyrimidine is dissolved in $NH_3$/MeOH and stirred at rt, and the solvent is removed under reduced pressure. The resulting solid is washed with $H_2O$ and dried in vacuo to give the white solid of 5-bromo-2-chloro-pyrimidin-4-ylamine in quantitative yield. The white solid is dissolved in DMSO/$H_2O$. To the solution are added DABCO and NaCN, then the resulting mixture is heated to 60 ˚C. The reaction mixture is diluted with water, and extracted with AcOEt. The combined organic extracts are dried over $Na_2SO_4$. Flush chromatography on silica gel using AcOEt-Hexane gives 4-amino-5-bromo-pyrimidine-2-carbonitrile as white solid. To a solution of the above product in DMF are added 1-chloro-4-prop-2-ynyl-benzene, $(PPh_3)_2PdCl_2$ and CuI under $N_2$. The resulting solution is stirred at 80˚C, and then sat $NH_4Cl$ aq is added into the mixture. After stirred for an additional 1 h, the mixture is extracted with AcOEt twice. The combined organic extracts are washed with $NaHCO_3$ aq, and dried over $Na_2SO_4$. Flash chromatography on silica gel using AcOEt-Hexane gives the title compound.

Example Q: 5-Iodo-3,3-dimethyl-pent-1-ene

3,3-Dimethyl-pent-4-en-1-ol (0.77 mmol) is dissolved in 10 ml of $CH_2Cl_2$, and then the solution is cooled down to 0 ˚C. To the cooled solution are added $PPh_3$ (0.92 mmol), pyridine (0.85 mmol), and iodine (0.92 mmol) and then stirred at 0 ˚C to rt for 16 h. After addition of aq. $Na_2SO_3$ solution, the mixture is extracted with $Et_2O$ twice. The combined organic extracts are washed with $H_2O$, and dried over $Na_2SO_4$. Flash chromatography on silica gel using *n*-hexane gives the iodide as a colorless oil.

Example R: 1-(2-Bromo-ethyl)-4-methyl-benzene

To a solution of 2-p-tolyl-ethanol (1 g, 7.30 mmol) in $CH_2Cl_2$ (20 mL) are added $PPh_3$ (1.94 g, 7.40 mmol) and NBS (1.32 g, 7.40 mmol) at -15 ˚C. The reaction mixture is stirred at -15 ˚C to room temperature for overnight. The reaction is quenched by the addition of saturated aqueous $NaHCO_3$, and the resulting mixture is extracted with $CH_2Cl_2$. The combined organic extracts are washed with brine, and dried over $Na_2SO_4$, filtered, and concentrated in vacuo. The residue is purified by silica gel column chromatography (*n*-hexane : AcOEt=1:1) to give the title compound.

Example S: (3-Bromo-propyl)-cyclopropane

To a solution of 3-cyclopropyl-propan-1-ol (530 mg, 5.30 mmol) in $CH_2Cl_2$ (10 mL) are added $PPh_3$ (1.42 g, 5.40 mmol) and NBS (960 mg, 5.40 mmol) at -20 ˚C. The reaction mixture is stirred at -20 ˚C to rt for overnight. The reaction is quenched by the addition of water, and the resulting mixture is extracted with $CH_2Cl_2$. The combined organic extracts are washed with brine, and dried over $Na_2SO_4$, filtered, and concentrated in vacuo. The residue is purified by silica gel column chromatography ($Et_2O$) to give title compound.

Example T: 2-hydroxymethyl-indan

To a solution of indan-2-carboxylic acid (1 g, 6.20 mmol) in THF (10 mL) is added portionwise $LiAlH_4$ (266 mg, 7 mmol) at 0 ˚C. The reaction mixture is stirred at 0 ˚C to room temperature for 3.5 h. The reaction is quenched by the addition of water, and the resulting mixture is extracted with $Et_2O$. The combined organic extracts are washed with brine, and dried over $Na_2SO_4$, filtered, and concentrated in vac. to give the title compound.

Example U: 1-Piperidin-1-yl-pent-4-yn-1-one

To a solution of 4-pentynoic acid (512 mg, 0.53 mmol) in benzene (10 mL) is added $(COCl)_2$ (1 mL). After being stirred at rt for 5.5 h, the reaction mixture is concentrated in vacuo to give the corresponding acid chloride, which is used for the next reaction without further purification. To a solution of piperidine (890 mg, 10.5 mmol) in benzene (3 mL) is added a solution of said acid chloride in benzene (2 mL). The reaction mixture is stirred at rt for 2h, and the diluted with EtOAc. The mixture is washed with 1*M* aq. $KHSO_4$, water, saturated aq. $NaHCO_3$, water, and brine. The organic layer is dried over $Na_2SO_4$, filtered, and concentrated in vacuo to give the title compound.

Example V: 2-But-3-ynyl-thiazole

To a suspension of NaH (60%, 424 mg, 10.6 mmol) in THF (5 mL) is added a solution of $(EtO)_2P(O)CH_2CO_2Et$ (2.6 g, 11.6 mmol) in THF (8 mL) at 0 ˚C. After being stirred at 0 ˚C for 30 min, to this solution is added a solution of 2-formylthiazole (1 g, 8.84 mmol) in THF (8 mL). The reaction mixture is stirred at 0 ˚C to rt for 13 h. After the bulk of solvent is removed in vacuo, the residue is diluted with ether, washed with 1*M* aqueous $KHSO_4$, water, and brine. The organic layer is dried over $MgSO_4$, filtered, and concentrated in vacuo. The residue is purified by silica gel column chromatography (*n*-hexane:EtOAc=5:1) to give the unsaturated ester.
To a solution of said unsaturated ester (1.16 g, 6.33 mmol) in EtOH (15 mL) is added 10% Pd on carbon (100 mg). The black slurry is stirred at room temperature under 1 atm $H_2$ for 22 h. The reaction mixture is filtered through a celite pad (EtOH rinse) and the filtrate is concentrated in vacuo to give the saturated ester.
To a solution of the above saturated ester (1.15 g, 6.21 mmol) in $CH_2Cl_2$ (10 mL) is added dropwise DIBAL (0.95 *M* in hexane, 6.6 mL, 6.27 mmol) at -78 ˚C. After being stirred at -78 ˚C for 20 min, the reaction is quenched by the addition of 1*M* aqueous $KHSO_4$. The resulting mixture is extracted with $CH_2Cl_2$ (x3). The combined organic extracts are washed with saturated aqueous $NaHCO_3$, water, and brine, and dried over $Na_2SO_4$, filtered, and concentrated in vacuo. The residue is purified by silica gel column chromatography (*n*-hexane:EtOAc=2:1) to give the corresponding alcohol.

To a solution of the above alcohol (211 mg, 1.47 mmol) in $CH_2Cl_2$ (5 mL) is added Dess-Martin periodinane (750 mg, 1.76 mmol). The reaction mixture is stirred at rt for 30 min, and the reaction is quenched by the addition of aqueous $Na_2S_2O_3$. The mixture is extracted with ether, and the organic layer is washed with water and saturated aqueous $NaHCO_3$, and dried over $MgSO_4$, filtered, and concentrated in vacuo to give the corresponding aldehyde, which is used for the next step without further purification.
To a solution of $TMSCHN_2$ (2.0 *M* in hexane, 0.6 mL, 1.20 mmol) in THF (3 mL) is added dropwise *n*-BuLi (1.58 *M* in hexane, 0.76 mL, 1.20 mmol) at -78 ˚C. After being stirred at -78 ˚C for 30 min, to this solution is added a solution of the above aldehyde (140 mg, 0.99 mmol) in THF (2 mL). The reaction mixture is stirred at -78 ˚C to rt for 2.5 h. After dilution with ether, the mixture is washed with saturated aqueous $NH_4Cl$, water, and brine. The organic layer is dried over $MgSO_4$, filtered, and concentrated in vacuo. The residue is purified by silica gel column chromatography (*n*-hexane: EtOAc=5:1) to give the title compound.

Example W: 1-(3-Bromo-benzyl)-pyrrolidin-2-one

To a solution of pyrrolidin-2-one (1.03 g, 12.1 mmol) in DMF (30 mL) is added NaH (60 %, 540 mg, 13.5 mmol) at 0 ˚C. The reaction mixture is stirred at 0 ˚C for 20 min, and then warmed up to room temperature for 40 min. To this solution is added 1-bromo-3-bromomethyl-benzene (2.45 g, 9.8 mmol) at 0 ˚C. The reaction mixture is stirred at 0 ˚C for 15 min, and then warmed up to room temperature for 13 h. After dilution with ether, the mixture is washed with 1 *M* aqueous $KHSO_4$, water, saturated aqueous $NaHCO_3$, water, and brine. The organic layer is dried over $MgSO_4$, filtered, and concentrated in vacuo. The residue is purified by silica gel column chromatography (*n*-hexane:EtOAc=1:1 to 1:2) to give the title compound.

Example X: 1-Bromo-3-methoxymethyl-benzene

To a solution of (3-bromo-phenyl)-methanol (1 g, 5.35 mmol) in THF (10 mL) is added NaH (60 %, 257 mg, 6.43 mmol) at 0 ˚C. After 13 min, to this mixture is added MeI (1 mL, 16.1 mmol). The reaction mixture is stirred at 0 ˚C

for 10 min, and then warmed up to room temperature for 50 min. The reaction is quenched by the addition of 1 *M* aqueous $KHSO_4$, and the mixtute is diluted with ether. After the resulting two phase is separated, the organic layer is washed with brine. The organic layer is dried over $MgSO_4$, filtered, and concentrated in vacuo. The residue is purified by silica gel column chromat.ography (*n*-hexane:EtOAc = 10:1) to give the title compound

Example YA:-4-Oxo-1-phenyl-1,3,8-triaza-spiro[4,5]decane-8-carboxylic acid tert-butyl ester

To a suspension of 1-Phenyl-1,3,8-triaza-spiro[4.5]decan-4-one(1.0 g, 4.32 mmol) in dichloromethane(10 ml), saturated sodium bicarbonate solution(10 ml) and di-*t*-butyldicarbonate(1.04 g, 4.76 mmol) in dichloromethane(5 ml) are added at ambient temperature. The reaction mixture is stirred for 1 h and quenched with $H_2O$ and extracted with ethyl acetate. The combined extracts are washed with $H_2O$ and brine, dried over sodium sulfate and evaporated down to the title compound; Rf=0.90($CH_2Cl_2$:MeOH = 20:1) $^1$H-NMR(400MHz, $CDCl_3$) δ: 1.51 (s, 9H), 1.63-1.71 (m, 2H), 2.50-2.65(m, 2H), 3.50-3.65(m, 2H), 3.97-4.10(m, 2H), 4.75(s, 2H), 6.74-6.76(m, 2H), 6.84-6.88(m, 1H), 7.01 (brs, 1H), 7.23-7.27(m, 2H).

Example YB: 3-[2-Cyano-7-(2-cyclohexyl-ethyl)-7H-pyrrolo[2,3-d]pyrimidin-6-ylmethyl]-4-oxo-1-phenyl-1,3,8-triaza-spiro[4.5]decane-8-carboxylic acid tert-butyl ester

To a solution of 6-chloromethyl-7-(2-cyclohexyl-ethyl)-7H-pyrrolo[2,3-d]pyrimidine-2-carbonitrile(600 mg, 1.98 mmol) in DMF(7 ml), 4-Oxo-1-phenyl-1,3,8-triaza -spiro[4.5] decane-8-carboxylic acid tert-butyl ester(657 mg, 1.98 mmol) and sodium hydride (101 mg, 2.53 mmol) are added. The mixture is stirred at room temperature under nitrogen atomosphere for 14 h. The reaction mixture is diluted with water and extracted with AcOEt(twice). The combined organic layer is washed with water and brine, dried over $MgSO_4$, and concentrated in vacuo. The residue is purified by silica gel column chromatography (n-hexane:AcOEt=1:1) to the title compound; Rf=0.25(*n*-hexane:AcOEt = 1:1). $^1$H-NMR (400MHz, $CDCl_3$) δ : 0.97-1.49(m, 7H), 1.50(s, 9H), 1.56-1.82(m, 8H), 2.45-2.60(m, 2H), 3.50-3.65(m, 2H), 4.09-4.14 (m, 2H), 4.33-4.36(m, 2H), 4.64(s, 2H), 4.87(s, 2H), 6.72-6.74(m, 2H), 6.86-6.90(m, 1H), 7.20-7.24(m, 2H), 8.94(s, 1H).

Example YC: 7-(2-Cyclohexyl-ethyl)-6-(4-oxo-1-phenyl-1,3,8-triaza-spiro[4.5]dec-3-ylmethyl)-7H-pyrrolo[2,3-d]pyrimidine-2-carbonitrile trifluoroacetic acid salt

To a solution of 3-[2-Cyano-7-(2-cyclohexyl-ethyl)-7H-pyrrolo[2,3-d]pyrimidin-6- ylmethyl]-4-oxo-1-phenyl-1,3,8-triaza-spiro[4.5]decane-8-carboxylic acid tert-butyl ester (340 mg, 0.56 mmol) in dichloromethane(5 ml), trifluoroacetic acid(5 ml) is added. After stirring for 1 h at room temperature, solvent is evaporated down to give the title compound; Rf=0.10 ($CH_2Cl_2$:MeOH = 20:1) $^1$H-NMR(400MHz, $CDCl_3$) δ : 0.98-1.38(m, 5H),1.65-1.83(m, 8H), 1.98-2.09(m, 2H), 2.71-2.80(m, 2H), 3.53-3.56(m, 2H), 3.94-4.02(m, 2H), 4.38-4.42(m, 2H), 4.73(s, 2H), 4.91 (s, 2H), 6.71 (s, 1H), 6.88-6.90 (m, 2H), 7.01-7.04(m, 1H), 7.28-7.32(m, 2H), 7.85(brs, 1H), 8.25(brs, 1H), 9.08(s, 1H).

Example YD: 4-{7-[2-(4-Chloro-phenyl)-ethyl]-2-cyano-7H-pyrrolo[2,3-d]pyrimidin-6-ylmethyl}-piperazine-1-carboxylic acid tert-butyl ester

To a solution of 6-Bromomethyl-7-[2-(4-chloro-phenyl)-ethyl]-7H-pyrrolo[2,3-d] pyrimidirie-2-carbonitrile(1.0 g, 2.66 mmol) in DMF(10ml), Piperazine-1- carboxylic acid tert-butyl ester(545 mg, 2.93 mmol) and potassium carbonate (515 mg, 3.72 mmol) are added. The mixture is stirred at room temperature under nitrogen atomosphere for 14 h. The reaction mixture is diluted with water and extracted with AcOEt (twice). The combined organic layer is washed with water and brine, dried over $MgSO_4$, and concentrated in vacuo. The residue is purified by silica gel column chromatography (n-hexane : AcOEt=1:1) to give the title compound; Rf=0.20(*n*-hexane:AcOEt = 2:1). $^1$H-NMR(400MHz, $CDCl_3$) δ :1.45 (s, 9H), 2.36-2.38(m, 4H), 3.12-3.15(m, 2H), 3.39-3.43(m, 6H), 4.58-4.62(m, 2H), 6.48(s, 1H), 7.01-7.03(m, 2H), 7.24-7.26 (m, 2H), 8.90(s, 1H).

Example YE: 5-(3-Azepan-1-yl-prop-1-ynyl)-4-(2-cyclohexyl-ethylamino)-pyrimidine-2-carbonitrile

At room temperature, a soln, of B (0.49 mmol) and C (0.73 mmol) in DMF(5 ml) is treated with $Et_3N$(2.18 mmol), CuI(0.05 mmol), and$(Ph_3P)_2PdCl_2$(0.03 mmol). The mixture is stirred for 2 h at 80°C, poured into an ice water, extracted with EtOAc, washed with brine, and dried($MgSO_4$). The residue is purified by silica gel column chromatography(AcOEt) to give the title compound as an orange solid; $^1$H-NMR(400 MHz, $CDCl_3$) δ 0.91-1.04(m, 2H), 1.12-1.38(m, 3H), 1.49-1.79 (m, 16H), 2.74(t, 4H), 3.54(t, 2H), 3.67(s, 1H), 5.77(brs. 1H), 8.18(s, 1H). Rf 0.12(hexane/EtOAc 1:3).

Example ZA. 8-Benzyl-2,8-diaza-spiro[4.5]decane-1,3-dione

To a solution of 1-benzyl-piperidin-4-one(75.1 g, 0.40 mol) in toluene(400 ml), cyano-acetic acid ethyl ester (50.6 ml, 0.48 mol) and acetic acid(18.2 ml, 0.32 mol) are added at ambient temperature. The reaction mixture is refluxed for 4h, quenched with ice-water and extracted with diethyl ether. The combined extracts are washed with $H_2O$, brine and dried over sodium sulphate to give(1-benzyl-piperidin-4-ylidene) -cyano-acetic acid ethyl ester in quant yield. Rf=0.53 (*n*-hexane:AcOEt = 1:1). $^1$H-NMR(400MHz, $CDCl_3$) $\delta$ : 1.30-1.37(m, 3H), 2.58(dd, 2H), 2.64(dd, 2H), 2.79(dd, 2H), 3.15 (dd, 2H), 3.55(s, 2H), 4.23-4.32(m, 2H), 7.21-7.36(m, 5H).

To a solution of(1-benzyl-piperidin-4-ylidene)-cyano-acetic acid ethyl ester(112.9 g, 0.40 mol) in EtOH(500 ml) and $H_2O$(100 ml), potassium cyanide(64.6 g, 0.99 mol) is added at ambient temperature. The reaction mixture is stirred at 65 C˚ for 24h. After removal of EtOH, $H_2O$ is added to the residue. The waster phase is extracted with diethyl ether. The combined extracts are washed with $H_2O$ and brine, dried over sodium sulfate and evaporated down to give 1-benzyl-4-cyanomethyl- piperidine -4-carbonitrile; Rf=0.38(*n*-hexane:AcOEt = 1:1).$^1$H-NMR (400MHz, $CDCl_3$) $\delta$ : 1.76-1.81 (m, 2H), 2.10-2.05(m, 2H), 2.23-2.39(m, 2H), 2.69(s, 2H), 2.90-2.94(m, 2H), 3.56(s, 2H), 7.21-7.38(m, 5H).

Acetic acid(56.8 ml) and sulfuric acid(11.8 ml) are added to 1-benzyl-4-cyanomethyl-piperidine-4-carbonitrile (27.2 g, 0.114 mmol) at ambient temperature. The reaction mixture is stirred at 125 C˚ for 1 h, cooled down to the room temperature and added to saturated NaOH aq. to adjust to pH 6.0. The mixture is extracted with dichloromethane. The combined extracts are washed with $H_2O$ and brine, dried over sodium sulfate and evaporated down to provide the title compound; Rf=0.40($CH_2C)_2$:MeOH = 10:1). $^1$H-NMR(400MHz, $CDCl_3$) $\delta$ : 1.52-1.57(m, 2H), 2.02-2.17(m, 4H), 2.59(s, 2H), 2.86-2.90(m, 2H), 3.52(s, 2H), 7.21-7.28(m, 2H), 7.30-7.37(m, 3H), 7.92(brs, 1H).

Example ZB: 8-Benzyl-2,8-diaza-spiro[4.5]decane

To a solution of lithium aluminium hydride(3.63g, 95.6 mmol) in THF(100 ml), a solution of the product of Example ZA (8.23 g, 31.8 mol) in THF (60 ml) are slowly added at ambient temperature. The reaction mixture is refluxed for 6h, quenched with $Na_2SO_410H_2O$ at 0˚C.

Inorganic materials are removed by filtration and THF is evaporated down to to provide the title compound; Rf=0.10(ethyl acetate only).

Example ZC. 2,8-Diaza-spiro[4.5]decane-1,3-dione hydrochloride

To a solution of the product of Example ZA (1.04 g, 4.02 mol) and $Pd(OH)_2$(8.5 g) in 200 ml of flusk, EtOH (80.5 ml) is added at ambient temperature. The reaction mixture was stirred under $H_2$ at room temperature for 15 h. The catalysts were removed by filtration and EtOH was evaporated down to give 2,8-Diaza-spiro[4.5]decane-1,3-dione in the quant yield.

To a solution of 2,8-Diaza-spiro[4.5]decane-1,3-dione in EtOH(20 ml), a 1 M dioxane solution of HCl(10 ml). After stirring for 1h at room temperature, solvent is evaporated down to to provide the title compound; $^1$H-NMR(400MHz, DMSO-$d_6$) $\delta$ : 1.76-1.79(m, 2H), 1.90-2.00(m, 2H), 2.68(s, 2H), 2.88-2.96(m, 2H), 3.20-3.28(m, 2H), 8.76(brs, 1H), 9.01 (brs, 1H), 11.25(brs, 1H).

Example ZD: 8-Benzyl-2,8-diaza-spiro[4.5]decane-2-carboxylic acid tert-butyl ester

To a suspension of the product of Example ZB (5.06g, 21.9 mmol) in dichloromethane(50 ml), 1 N NaOH(50 ml) and di-*t*-butyldicarbonate(6.14 g, 28.1 mmol) in dichloromethane(10 ml) are added at ambient temperature. The reaction mixture is stirred for 5h and quenched with $H_2O$ and extracted with ethyl acetate. The combined extracts are washed with $H_2O$ and brine, dried over sodium sulfate and evaporated down to provide the title compound; $^1$H-NMR (400MHz, $CDCl_3$) $\delta$ : 1.49(s, 9H), 1.50-1.70(m, 6H), 2.25-2.40(m, 2H), 2.45-2.55(m, 2H), 3.10-3.40(m, 4H), 3.50(s, 2H), 7.24-7.31 (m, 5H).

Example ZE. 2,8-Diaza-spiro[4.5]decane-2-carboxylic acid tert-butyl ester

To a solution of 8the product of Example ZD (7.95 g, 24.0 mol) and $Pd(OH)_2$(2.4 g) in 200 ml of flusk, EtOH (96 ml) and acetic acid(1.2 ml) are added at ambient temperature. The reaction mixture was stirred under $H_2$ at room temperature for 15 h. The catalysts were removed by filtration and EtOH was evaporated down to to provide the title compound; Rf= 0.05 (ethyl acetate only).

Example ZF: 8-Methanesulfonyl-2,8-diaza-spiro[4.5]decane hydrochloride

To a solution of the product of Example ZE (1.12 g, 4.66 mol) in dichloromethane(10 ml), triethylamine(3.88 ml) and methanesulfonylchloride (1.08 ml, 14 mmol) are added at 0˚C. The reaction mixture is stirred for over night, quenched with ice-water and extracted with dichloromethane. The combined extracts are washed with $H_2O$, brine and dried over sodium sulphate to crude 8-methanesulfonyl-2,8-diaza-spiro[4.5]decane-2-carboxylic acid tert-butyl ester; Rf=0.7($CH_2Cl_2$:MeOH = 10:1).

To a solution of said ester (1.32 g) in ethyl acetate(10 ml), a 1 M ethyl acetate solution of HCl(20 ml). After stirring for 2h at room temperature, solvent is evaporated down to provide the title compound; $^1$H-NMR(400MHz, DMSO-$d_6$) δ : 1.62-1.68(m, 4H), 1.78-1.82(m, 2H), 2.87(s, 3H), 2.98-3.12(m, 6H), 3.20-3.23(m, 2H), 9.49(brs, 1H), 9.59(brs, 1H).

Example ZG: 1-(2,8-Diaza-spiro[4.5]dec-8-yl)-ethanone hydrochloride

To a solution of the product of Example ZE (1.12 g, 4.66 mol) in dichloromethane(10 ml), triethylamine(3.88 ml) and acetic anhydride (1.32 ml, 14 mmol) are added at 0˚C. The reaction mixture is stirred for over night, quenched with ice-water and extracted with dichloromethane. The combined extracts are washed with $H_2O$, brine and dried over sodium sulphate to crude 8-acetyl-2,8- diaza -spiro[4.5]decane-2-carboxylic acid tert-butyl ester; Rf=0.6($CH_2Cl_2$:MeOH = 10:1).

To a solution of said ester (1.34 g) in ethyl acetate(10 ml), a 1 M ethyl acetate solution of HCl(20 ml). After stirring for 2h at room temperature, solvent is evaporated down to to provide the title compound as a solid; $^1$H-NMR (400MHz, DMSO-$d_6$) δ : 1.44-1.59(m, 4H), 1.76-1.83(m, 2H), 2.07(s, 3H), 2.96-3.06(m, 2H), 3.16-3.24(m, 4H), 3.38-3.56 (m, 2H), 9.55(brs, 1H), 9.67(brs, 1H).

Example ZH: 5-Fluoro-9,3-dihydro-indol-2-one

To a solution of 2,4-difluoronitro-benzene(127 g, 0.79 mol) and dimethyl malonate (210.9 g, 1.59 mol) in DMF (800 ml), potassium carbonate(220.6 g, 1.59 mol) is added at ambient temperature. The reaction mixture is stirred at 70 C˚ for 12 h. The reaction mixture is added to toluene (639 ml) and 12 N HCl(1200 ml) and extracted with ethyl acetate. The combined extracts are washed with $H_2O$ and brine, dried over sodium sulfate and evaporated down to give 2-(5-fluoro-2-nitro-phenyl)-malonic acid dimethyl ester; Rf=0.5(*n*-hexane:AcOEt = 2:1).

To the crude ester and 5 % Pd-C(10.8 g) in 2 1 of flask, MeOH(600 ml) is added at ambient temperature. The reaction mixture is stirred under $H_2$ at room temperature for 15 h. The catalysts are removed by filtration and MeOH is evaporated down to give 5-fluoro-2-oxo-2,3-dihydro-1H- indole-3-carboxylic acid' methyl ester; Rf=0.10(*n*-hexane:ethyl acetate = 1:1).

To a solution of said crude 5-fluoro-2-oxo-2,3-dihydro-1H-indole-3-carboxylic acid methyl ester in MeOH(800 ml), 6N HCl(415 ml, 1.92 mol) is added at ambient temperature. The reaction mixture is stirred at 80 C˚ for 5 h. After cooling down to room temperature, 8 N KOH(438 ml, 1.82 mol) is added to reaction mixture. The reaction mixture is stirred at 40 C˚ for 30 min. 12 N HCl(66.5 ml) is added to reaction mixture. MeOH is evaporated down and the white powder is filtrated; Rf=0.25(*n*-hexane:AcOEt = 1:1). $^1$H-NMR(400MHz, $CDCl_3$) δ : 3.54(s, 2H), 6.78-6.81 (m, 1H), 6.90-6.98(m, 2H), 8.34(brs, 1H).

Example ZI

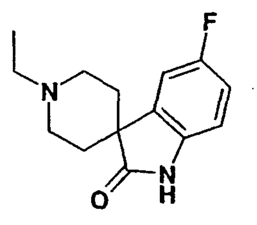

To a solution of the product of Example ZH (1.5 g, 10 mmol) in THF(160 ml), a solution of NaHMDS(1 M THF solution)(50 ml, 50 mmol) is added at -78˚C. After stirring for 30 min at -78˚C, ethyl-bis-(2-chloro-ethyl)-amine(47.3 g, 0.18 mol) in THF(176 ml) is added and the reaction mixture is stirred for 15 h at room temperature, quenched with saturated ammonium chloride and ice-water and extracted with ethyl acetate. The combined extracts are washed with

brine, dried over sodium sulphate and evaporated down. Ethyl ether is added to the residue to give the powder, which is filtrated; Rf=0.10($CH_2Cl_2$:MeOH = 30:1.

Example ZJ. 2-Fluoro-4-methoxy-1-nitro-benzene

To a solution of 3-fluoro-4-nitro-phenol(25.3 g, 0.16 mol) in acetone(160 ml), potassium carbonate(41.7 g, 0.30 mol) and methyl iodide(20.0 ml, 0.32 mol) are added at ambient temperature. The reaction mixture is stirred at 40 C˚ for 3 h. After cooling down to room temperature, dichloromethane is added to the reaction mixture, which is filtrated and evaporated. Dichloromethane is added to the residue and the combined extracts are washed with $H_2O$ and brine, dried over sodium sulfate and evaporated down to provide the title compound; $^1$H-NMR(400MHz, $CDCl_3$) $\delta$ : 3.90(s, 3H), 6.72-6.79(m, 2H), 8.06-8.13(m, 1H).

Example ZK. 5-Methoxy-1,3-dihydro-indol-2-one

To a solution of 2-fluoro-4-methoxy-1-nitro-benzene (84.1 g, 0.49 mol) and dimethyl malonate (129.9 g, 0.98 mol) in DMF(490 ml), potassium carbonate(135.9 g, 0.98 mol) is added at ambient temperature. The reaction mixture is stirred at 70 C˚ for 12 h. The reaction mixture is added to toluene (393 ml) and 12 N HCl(123 ml) and extracted with ethyl acetate. The combined extracts are washed with $H_2O$ and brine, dried over sodium sulfate and evaporated down to give 2-(5-methoxy-2-nitro-phenyl)- malonic acid dimethyl ester;
Rf=0.8(*n*-hexane:AcOEt = 1:1).

To said ester and 5 % Pd-C(7.0 g) in 1 l of flask, MeOH(490 ml) is added at ambient temperature. The reaction mixture is stirred under $H_2$ at room temperature for 15 h. The catalysts are removed by filtration and MeOH is evaporated down to give 5-methoxy-2-oxo-2,3-dihydro-1H -indole-3-carboxylic acid methyl ester; Rf=0.10(*n*-hexane:ethyl acetate = 1:1).

To a solution of crude 5-methoxy-2-oxo-2,3-dihydro-1 H-indole-3-carboxylic acid methyl ester in MeOH(320 ml), 6N HCl(255 ml, 1.92 mol) is added at ambient temperature. The reaction mixture is stirred at 70 C˚ for 3 h. After cooling down to room temperature, 8 N KOH(269 ml, 1.82 mol) is added to reaction mixture. The reaction mixture is stirred at 40 C˚ for 30 min. 12 N HCl(41 ml) is added to reaction mixture. MeOH is evaporated down and the white powder is filtrated to provide the title compound; Rf=0.25(n-hexane:AcOEt = 1:1); $^1$H-NMR(400MHz, $CDCl_3$) $\delta$ : 3.51 (s, 2H), 3.78(s, 3H), 6.72-6.85(m, 3H), 7.60(brs, 1H).

Example ZL

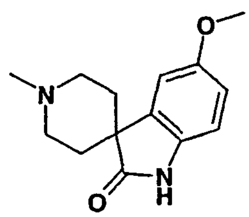

To a solution of the product of Example ZK (1.06 g, 6.49 mmol) in THF(13 ml), a solution of NaHMDS (1 M THF solution) (32.5 ml, 32.5 mmol) is added at -78˚C. After stirring for 30 min at -78˚C, methyl-bis-(2-chloro-ethyl)-amine hydrochloride (1.37g, 7.14 mol) is added and the reaction mixture is stirred for 13.5 h at room temperature, quenched with saturated ammonium chloride and ice-water and extracted with ethyl acetate. The combined extracts are washed with brine, dried over sodium sulphate and evaporated down. Ethyl ether is added to the residue to give the powder, which is filtrated; Rf=0.10($CH_2Cl_2$:MeOH = 30:1) $^1$H-NMR(400MHz, DMSO-$d_6$) $\delta$ : 1.66-1.78(m, 4H), 2.28(s, 3H), 2.44-2.47(m, 2H), 2.71-2.77(m, 2H), 3.70(s, 3H), 6.74(s, 2H), 7.01(s, 1H), 10.15(brs, 1H).

Example ZM

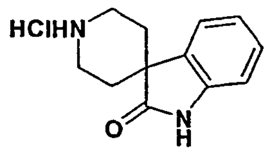

To a solution of 1,3-Dihydro-indol-2-one(8.79 g, 66 mmol) in THF(50 ml), a solution of LiHMDS(1 M THF solution)(200 ml, 200 mmol) is added at -78˚C. After stirring for 30 min at-78˚C, Bis-(2-chloro-ethyl)-carbamic acid tert-butyl ester(17.5g, 72.6 mol) is added and the reaction mixture is stirred for 21 h at room temperature, quenched with saturated ammonium chloride and ice-water and extracted with ethyl acetate. The combined extracts are washed with brine, dried over sodium sulphate and evaporated down to give crude product. Rf=0.25($CH_2Cl_2$:MeOH = 30:1) $^1$H-NMR (4.00MHz, DMSO-$d_6$) δ : 1.43(s, 9H), 1.63-1.70(m, 4H), 3.57-3.71 (m, 4H), 6.84-6.86(m, 1H), 6.95-6.97(m, 1H), 7.17-7.19 (m, 1H), 7.42-7.44(m, 1H), 10.40(brs, 1H).

To a solution of the crude product in ethyl acetate(20 ml), a 1 M ethyl acetate solution of HCl(20 ml). After stirring for 2h at room temperature, solvent is evaporated down to. Ethyl ether is added to the residue to give the powder, which is filtrated; Rf=0.05 (ethyl acetate only); $^1$H-NMR(400MHz, DMSO-$d_6$) δ :1.87-1.90(m, 2H), 2.04-2.11(m, 2H), 3.24-3.27(m, 2H), 3.45-3.49(m, 2H), 6.88-6.89(m, 1H), 7.00-7.04(m, 1H), 7.21-7.29(m, 2H), 9.04(brs, 1H), 10.57(brs, 1H).

Example ZN

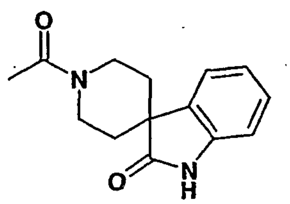

To a solution of the product of Example ZM (422 mg, 1.76 mol) in dichloromethane(5 ml), triethylamine(1.2 ml) and acetic anhydride(0.33 ml, 3.53 mmol) are added at 0˚C. The reaction mixture is stirred for 2h, quenched with ice-water and extracted with dichloromethane. The combined organic layer is washed with water and brine, dried over $MgSO_4$, and concentrated in vacuo. The residue is purified by silica gel column chromatography(n-hexane : AcOEt=5: 1) to give the product; Rf=0.6($CH_2Cl_2$:MeOH = 10:1); $^1$H-NMR(400MHz, $CDCl_3$) δ :1.79-1.95(m, 4H), 2.20(s, 3H), 3.68-3.74(m, 1H), 3.80-3.87(m, 1H), 3.98-4.22(m, 2H), 6.90-6.92(m, 1H), 7.03-7.07(m, 1H), 7.22-7.26(m, 2H), 8.06(brs, 1H).

Example ZO

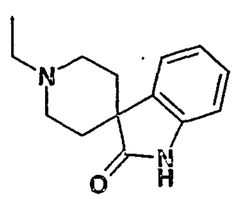

To a solution of 1,3-dihydro-indol-2-one(2.66 g, 20 mmol) in THF(40 ml), a solution of NaHMDS (1 M THF solution)(100 ml, 100 mmol) is added at -78˚C. After stirring for 30 min at -78˚C, ethyl-bis-(2-chloro-ethyl)-amine hydrochloride(4.54 g, 22 mol) is added and the reaction mixture is stirred for 18 h at room temperature, quenched with saturated ammonium chloride and ice-water and extracted with ethyl acetate. The combined organic layer is washed with water

and brine, dried over $MgSO_4$, and concentrated in vacuo. The residue is purified by silica gel column chromatography (n-hexane : AcOEt=5:1) to give the product; Rf=0.25($CH_2Cl_2$:MeOH = 30:1).

Example ZP. 4,4-Difluoro piperidine hydrochloride

To a solution of 4-oxo-piperidine-1-carboxylic acid tert-butyl ester(1g) in CH2Cl2 (10mL) is added [bis(2-methoxyethyl)amino]sulfer trifluoride(1.85mL) at 0˚C, and stirred for 1.5hr at rt. The reaction mixture is poured in aqueous NaHCO3 and extracted with dichloromethane.
The organic layer is successively washed with H2O and aqueous NaCl, dried over MgSO4, and concentrated in vacuo. The residue is purified by column chromatography to give a colorless oil.
To a solution of the oil in Et2O(10mL) was added HCl in EtOAc(4N, 5mL) and stirred for 1hr at rt. White precipitate in the reaction mixture is collected by filtration to give the pure product; 1H NMR(DMSO-d6, δ(ppm)); 2.23-2.2.36(m, 4H), 3.17-3.28(m, 4H), 9.54(brs, 2H).

Example ZQ. 3-(S)-fluoro-pyrrolidine hydrochloride

To a solution of 3-(R)-hydroxy-pyrrolidine-1-carboxylic acid tert-butyl ester(200mg) in CH2Cl2(10mL)is added [bis(2-methoxyethyl)amino]sulfer trifluoride(236uL) at 0˚C, and stirred for 1 hr at room temperature. The reaction mixture is poured in aqueous NaHCO3 and extracted with Et2O. The organic layer is successively washed with H2O and aqueous NaCl, dried over MgSO4, and concentrated in vacuo. The residue is purified by column chromatography to give a colorless oil.
The oil is dissolved in 4N HCl in dioxane(5mL) and stirred for 1.5hr at rt. The reaction mixture is concentrated in vacuo to provide the title compound.

Example ZR. 3,3-Difluoro piperidine hydrochloride

To a solution of 1-benzyl-piperidin-3-one(1g) in CH2Cl2(10mL)is added [bis(2-methoxyethyl)amino]sulfer trifluoride(1.84mL) at 0˚C, and stirred for 1.5hr at room temperature. The reaction mixture is poured in aqueous NaHCO3 and extracted with ethyl acetate. The organic layer is successively washed with H2O and aqueous NaCl, dried over MgSO4, and concentrated in vacuo. The residue is purified by column chromatography to give a colorless oil. The oil and Pd/C (5% w/w on activated carbon, 100mg) in HCl in EtOH/MeOH (50mL) is stirred for 22hr under H2 atmosphere. The reaction mixture is filtrated through celite pad. The filtrate is added HCl in EtOAc, then concentrated in vacuo to provide the title compound.

Example ZS. 3,3-Difluoro-pyrrolidine

To a solution of 3-oxo-pyrrolidine-1-carboxylic acid tert-butyl ester(1g) in CH2Cl2(10mL)is added [bis(2-methoxyethyl)amino]sulfer trifluoride(2mL) at 0˚C, and stirred for 11 hr at room temperature. The reaction mixture is poured in aqueous NaHCO3 and extracted with Et2O. The organic layer is successively washed with H2O and aqueous NaCl, dried over MgSO4, and concentrated in vacuo. The residue is purified by column chromatography to give a colorless oil. To a solution of the oil in Et20(10mL) was added HCl in EtOAc (4N, 5mL) and stirred for 3hr at room temperature. The reaction mixture is concentrated in vacuo, and the residue is suspended in Et2O. White precipitate in the Et2O is collected by filtration to to provide the title compound.

Example ZT. 3-(R)-fluoro-pyrrolidine hydrochloride

To a solution of 3-(R)-hydroxy-pyrrolidine-1-carboxylic acid tert-butyl ester(200mg) in CH2Cl2(10mL)is added [bis(2-methoxyethyl)amino]sulfer trifluoride(236uL) at 0˚C, and stirred for 1 hr at room temperature. The reaction mixture is poured in aqueous NaHCO3 and extracted with Et20. The organic layer is successively washed with H2O and aqueous NaCl, dried over MgSO4, and concentrated in vacuo. The residue is purified by column chromatography to give a colorless oil. The oil is dissolved in 4N HCl in dioxane (5mL) and stirred for 1.5hr at room temperature. The reaction mixture is concentrated in vacuoto provide the title compound.

Example ZU. 7-Methoxy-3,4-dihydro-2H-isoquinolin-1-one

To a heated solution of 6-methoxy-indan-1-one (3g) in trichloroacetic acid (30g) is added sodium azide(1.8g) at 70˚C and, and the mixture is maintaining with stirring for 12hr. The reaction mixture is diluted with ice water and neutrized with potassium carbonate, and extracted with ethyl acetate(twice). The organic layer is successively washed

with water and saturated NaClaq, dried over MgSO4, concentrated in vacuo. The crude product is purified by column chromatography to provide the title compound; 1 H NMR(CDCl3, δ(ppm)); 2.86(dd, 2H), 3.45-3.50(m, 2H), 3.78(s, 3H), 6.36(brs, 1H), 6.92-6.95(m, 1H), 7.06(d, 1H), 7.52(d, 1H).

Example ZV. 7-Methoxy-1,2,3,4-tetrahydro-isoquinoline hydrochloride

To a solution of 7-Methoxy-3,4-dihydro-2H-isoquinolin-1-one(200mg) in THF(8mL) is added LiAlH4 (76mg) and stirred for 3hr under reflux, and diluted with THF. The reaction mixture is added sodium sulfate decahydrate and filtration through celite pad. The filtrate is concentrated in vacuo. The residue is dissolved in Et2O, then HCl in EtOAc is added the Et2O. White precipitate is collected by filtration to provide the title compound; 1H NMR(CDCl3, δ(ppm)); 2.92(dd, 2H), 3.30-3.35(m, 2H), 3.72(s, 3H), 4.18-4.23(m, 2H), 6.81-6.86(m, 2H), 7.12(d, 1H), 9.45(brs, 2H).

Example ZW: 4-(2-Oxo-2,3-dihydro-benzoimidazol-1-yl)-piperidine-1-carboxylic acid tert-butyl ester

To a suspension of 1-Piperidin-4-yl-1,3-dihydro-benzoimidazol-2-one(1.0 g, 4.6 mmol) in dichloromethane(10 ml), saturated sodium bicarbonate solution(10 ml) and di-*t*-butyldicarbonate(1.1 g, 5.06 mmol) in dichloromethane(5 ml) are added at ambient temperature. The reaction mixture is stirred for 1 h and quenched with $H_2O$ and extracted with ethyl acetate. The combined extracts are washed with $H_2O$ and brine, dried over sodium sulfate and evaporated down to give the title compound; Rf=0.90($CH_2Cl_2$:MeOH = 20:1) $^1$H-NMR(400MHz, $CDCl_3$) δ : 1.60(s, 9H), 1.82-1.85(m, 2H), 2.31-2.36(m, 2H), 2.84-2.90(m, 2H), 4.25-4.45(m, 2H), 4.47-4.51(m, 2H), 7.04-7.14(m, 4H), 9.43(brs, 1H).

Example ZX: 4-{3-[2-Cyano-7-(2-cyclohexyl-ethyl)-7H-pyrrolo[2,3-d]pyrimidin-6-ylmethyl]-2-oxo-2,3-dihydro-benzoimidazol-1-yl}-piperidine-1-carboxylic acid tert-butyl ester

To a solution of 6-chloromethyl-7-(2-cyclohexyl-ethyl)-7H-pyrrolo[2,3-d]pyrimidine-2-carbonitrile(600 mg, 1.98 mmol) in DMF(7 ml), 4-(2-oxo-2,3-dihydro-benzoimidazol-1-yl)-piperidine-1-carboxylic acid tert-butyl ester(628 mg, 1.98 mmol) and sodium hydride(106 mg, 2.65 mmol) are added. The mixture is stirred at room temperature under nitrogen atomosphere for 14 h. The reaction mixture is diluted with water and extracted with AcOEt(twice). The combined organic layer is washed with water and brine, dried over $MgSO_4$, and concentrated in vacuo. The residue is purified by silica gel column chromatography(n-hexane : AcOEt=1:1) to give the title compound; Rf=0.30 (*n*-hexane:AcOEt = 1:1); $^1$H-NMR(400MHz, $CDCl_3$) δ : 0.92-0.97(m, 2H), 1.00-1.34(m, 3H), 1.50(s, 9H), 1.53-1.85(m, 10H), 2.30-2.41 (m, 2H), 2.85-2.91 (m, 2H), 4.31-4.54(m, 5H), 5.29(s, 2H), 6.54(s, 1H), 6.96-6.98(m, 1H), 7.02-7.12(m, 2H), 7.17-7.19(m, 1H), 8.88(s, 1H).

Example ZY: 7-(2-Cyclohexyl-ethyl)-6-(2-oxo-3-piperidin-4-yl-2,3-dihydro-benzoimidazol-1-ylmethyl)-7H-pyrrolo [2,3-d]pyrimidine-2-carbonitrile trifluoroacetic acid salt

To a solution of 4-{3-[2-cyano-7-(2-cyclohexyl-ethyl)-7H-pyrrolo[2,3-d]pyrimidin- 6-ylmethyl]-2-oxo-2,3-dihydro-benzoimidazol-1-yl}-piperidine-1-carboxylic acid tert-butyl ester(512 mg) in dichloromethane(5 ml), trifluoroacetic acid(5 ml) is added. After stirring for 1 h at room temperature, solvent is evaporated down to the title compound; Rf=0.10 ($CH_2Cl_2$:MeOH = 20:1) $^1$H-NMR(400MHz, $CDCl_3$) δ : 0.95-1.03(m, 2H), 1.17-1.35(m, 4H), 1.59-1.79(m, 7H), 2.14-2.17 (m, 2H), 2.86-3.01 (m, 2H), 3.29-3.32(m, 2H), 3.77-3.80(m, 2H), 4.43-4.47(m, 2H), 4.79-4.85(m, 1H), 5.36(s, 2H), 6.55 (s, 1H), 7.03-7.23(m, 3H), 7.46-7.47(m, 1H), 8.27(brs, 1H), 8.36(brs, 1H), 8.99(s, 1H).

Example 1: 7-[2-(4-Chloro-phenyl)-ethyl]-6-(2,4-difluoro-phenoxymethyl)-7H-pyrrolo[2,3-d]pyrimidine-2-carbonitrile

6-Bromomethyl-7-[2-(4-chloro-phenyl)-ethyl]-7H-pyrrolo[2,3-d]pyrimidine-2-carbonitrile (Example A; 0.1 g, 0.27 mmol) and 2,4-difluorophenol (35 mg, 0.27 mmol) are dissolved in DMF (10 ml) and potassium carbonate (75 mg, 0.54 mmol) is added to the solution. The reaction mixture is stirred at rt for 15 h and quenched with saturated ammonium chloride and extracted with ethyl acetate. The combined extracts are washed with brine, dried over $MgSO_4$ (or $Na_2SO_4$) and concentrated. Chromatography on silica gel gives the desired product; $R_f$=0.30 (*n*-hexane : ethyl acetate = 1:1). $^1$H-NMR (4.00MHz, $CDCl_3$) δ: 3.18 (t, 2H), 4.63 (t, 2H), 4.93 (s, 2H), 6.63 (s, 1H), 6.67-6.95 (m, 3H), 7.02 (d, 2H), 7.22 (d, 2H), 8.97 (s, 1H).

Examples 2 - 49

By repeating the procedure described in Example 1 using appropriate starting materials (including those of

Example A and B) and conditions the following compounds of formula 2 are obtained as identified below in Table 2.

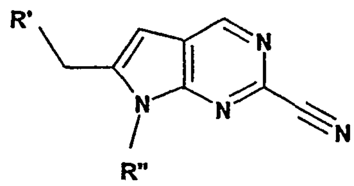

(2)

Table 2

| Ex. | R' | R" | $R_f$ (solvent) | NMR ($CDCl_3$, 400MHz, δ) |
|---|---|---|---|---|
| 2 | 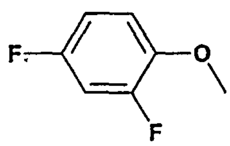 | 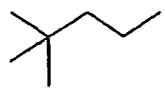 | 0.50 (n-hexane: AcOEt=1:1) | 0.97(s, 9H), 1.66-1.70 (m, 2H), 4.34-4.38(m, 2H), 5.19(s, 2H), 6.61 (s, 1H), 6.72-6.77(m, 1H), 6.81-6.86(m, 1H), 6.91-6.97(m, 1H), 8.89 (s, 1H) |
| 3 | 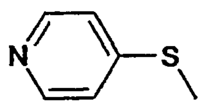 | 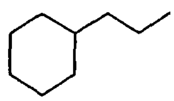 | 0.10 (*n*-hexane: AcOEt=1:1) | 0.97-1.06(m, 2H), 1.15-1.39(m, 4H), 1.66-1.83(m, 7H), 4.37 (s, 2H), 4.38-4.41 (m, 2H), 6.60(s, 1H), 7.16 (dd, 2H), 8.47(d, 2H), 8.89(s, 1H) |
| 4 | 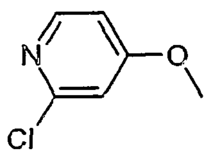 | 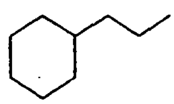 | 0.31 (n-hexane: AcOEt=1:1) | 0.96-1.02(m, 2H), 1.15-1.34(m, 4H), 1.66-1.79(m, 7H), 4.34-4.38(m, 2H), 5.30 (s, 2H), 6.77(s, 1H), 6.85-6.87(m, 1H), 5.97 (d, 1H), 8.29(d, 1H), 9.00 (s, 1H) |
| 5 | 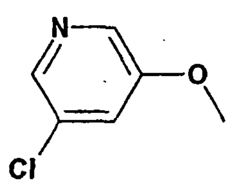 | 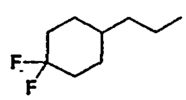 | 0.24 (n-hexane: AcOEt=1:1) | 1.28-1.45(m, 3H), 1.58-1.84(m, 6H), 2.02-2.05(m, 2H), 4.32-4.35(m, 2H), 5.23 (s, 2H), 6.72(s, 1H), 7.27-7.28(m, 1H), 8.24 (brs, 2H), 8.95(s, 1H) |
| 6 | 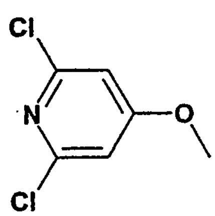 | 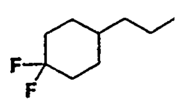 | 0.29 (n-hexane: AcOEt=1:1) | 1.17-1.43(m, 3H), 1.68-1.75(m, 4H), 1.81-1.84(m, 2H), 2.03-2.08(m, 2H), 4.29-4.32(m, 2H), 5.23 (s, 2H), 6.73(s, 1H), 6.83 (s, 2H), 8.97(s, 1H) |

(continued)

| Ex. | R' | R" | $R_f$ (solvent) | NMR ($CDCl_3$, 400MHz, δ) |
|---|---|---|---|---|
| 7 | 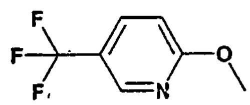 | 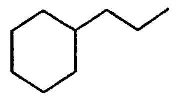 | 0.37 (n-hexane: AcOEt=1:1) | 0.93-1.02(m, 2H), 1.17-1.38(m, 4H), 1.54-1.79(m, 7H), 4.33-4.37(m, 2H), 5.40 (s, 2H), 6.55(s, 1H), 6.74 (d, 1H), 7.51-7.54(m, 1H), 7.68(s, 1H), 8.96(s, 1H) |
| 8 | 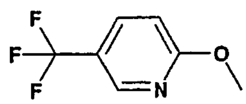 | 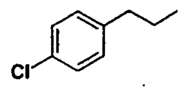 | 0.27 (n-hexane: AcOEt=1:1) | 3.09(dd, 2H), 4.58(dd, 2H), 4.89 (s, 2H), 6.42(s, 1H), 6.70(d, 1H), 6.95-6.97(m, 2H), 7.23-7.27(m, 2H), 7.48-7.51 (m, 2H), 8.97 (s, 1H) |
| 9 | 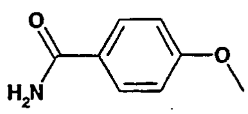 | 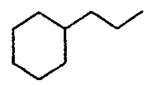 | 0.05 (*n*-hexane: AcOEt=1:1) | 0.92-1.01(m, 2H), 1.13-1.37(m, 4H), 1.65-1.79(m, 7H), 4.36-4.40(m, 2H), 5.29 (s, 2H), 6.75(s, 1H), 7.30 (d, 2H), 7.83(d, 2H), 8.98 (s, 1H) |
| 10 | 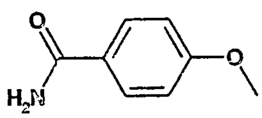 | 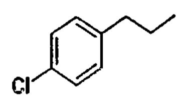 | 0.61 ($CH_2Cl_2$:MeO H=9:1) | 3.16(t, 2H), 4.57(dd, 2H), 4.91 (s, 2H), 6.69(s, 1H), 6.93-6.95 (m, 4H), 7.22 (d, 2H), 7.82(d, 2H), 9.00 (s, 1H) |
| 11 | 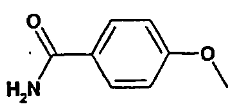 | 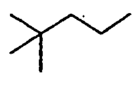 | 0.09 (n-hexane: AcOEt=1:1) | 0.93(s, 9H), 1.65-1.69 (m, 2H), 4.31 (m, 2H), 5.23(s, 2H), 6.68(s, 1H), 6.98(d, 2H), 7.77(d, 2H), 8.91(s, 1H) |
| 12 | 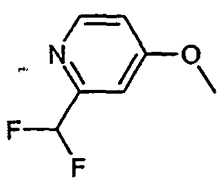 | 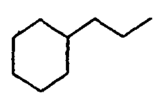 | 0.20 (n-hexane: AcOEt=1:1) | 0.93-1.02(m, 2H), 1.14-1.36(m, 4H), 1.65-1.79(m, 7H), 4.37 (dd, 2H), 5.35(s, 2H), 6.63(t, 1H), 6.78(s, 1H), 7.01 (dd, 1H), 7.26-7.27 (m, 1H), 8.55(d, 1H), 9.00 (s, 1H) |
| 13 | 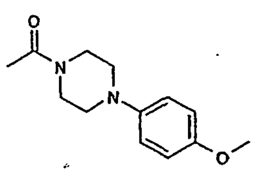 | 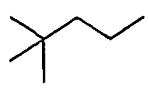 | 0.17 (n-hexane: AcOEt=1:1) | 0.94(s, 9H), 1.64-1.69 (m, 2H), 2.07(s, 3H), 3.01 (brs, 4H), 3.57 (brs, 2H), 3.72(brs, 2H), 4.32 (m, 2H), 5.12(s, 2H), 6.62(s, 1H), 6.87(brs, 4H), 8.88(s, 1H) |

(continued)

| Ex. | R' | R" | $R_f$ (solvent) | NMR ($CDCl_3$, 400MHz, δ) |
|---|---|---|---|---|
| 14 | 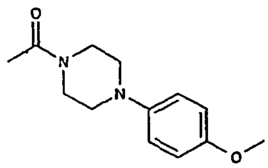 | 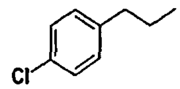 | 0.56 ($CH_2Cl_2$ :MeOH=9:1 | 2.14(s, 3H), 3.07-3.18 (m, 4H), 3.64(brs, 2H), 3.79(brs, 2H), 4.57(dd, 2H), 4.86(s, 2H), 6.64 (s, 1H), 6.85(d, 2H), 6.92-6.98 (m, 4H), 7.22 (d, 2H), 8.97(s, 1H) |
| 15 | 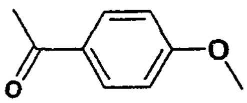 | 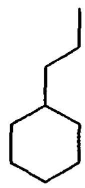 | 0.44 (*n*-hexane: AcOEt=1:2) | 0.93-1.01(m, 2H), 1.10-1.37(m, 4H), 1.65-1.79(m, 7H), 2.58 (s, 3H), 4.36-4.40(m, 2H), 5.31 (s, 2H), 6.75(s, 1H), 7.03-7.06(m, 2H), 7.98- 8.00(m, 2H), 8.98 (s, 1H) |
| 16 | 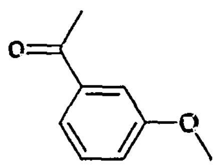 | 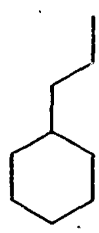 | | 0.93- 1.01 (m, 2H), 1.10-1.38 (m, 4H), 1.64-1.76(m, 7H), 2.62 (s, 3H), 4.37-4.41 (m, 2H), 5.30 (s, 2H), 6.76(s, 1H), 7.18- 7.21 (m, 1H), 7.44 (t, 1H), 7.62- 7.63 (m, 2H), 8.97(s, 1H) |
| 17 | 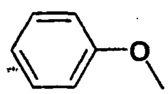 | 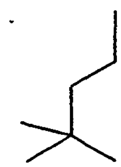 | 0.3 (*n*-hexane: AcOEt=2:1) | (CDCl3) 1.00 (s, 9H), 1.72-1.77 (m, 2H), 4.37-4.41 (m, 2H), 5.24 (s, 2H), 6.72 (s, 1H), 6.99 - 7.06 (m, 3H), 7.32 -7.36 (m, 2H), 8.96 (s, 1H) |
| 18 | 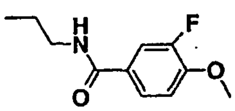 | 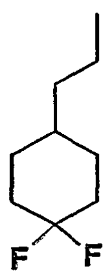 | 0.01 (*n*-hexane: AcOEt=2:1) | 1.21 (t, 3H), 1.25 -1.46 (m, 3H), 1.61-1.78 (m, 4H), 1.82-1.90 (m, 4H), 2.04-2.13 (m, 2H), 3.39-3.44 (m, 2H), 4.41-4.45 (m, 2H), 5.34 (s, 2H), 6.01 (br s, 1H), 6.75 (s, 1H), 7.10-7.14 (m, 1H), 7.54-7.58 (m, 2H), 8.99(s, 1H) |
| 19 | 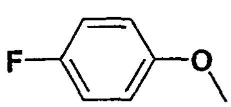 | 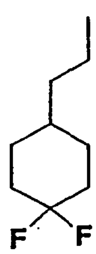 | 0.23 (*n*-hexane: AcOEt=2:1) | 1.33-1.44 (m, 3H), 1.61-1.89 (m, 6H), 2.08-2.10 (m, 2H), 4.38- 4.42 (m, 2H), 5.20 (s, 2H), 6.73 (s, 1H), 6.91- 6.94 (m, 2H), 7.02- 7.06 (m, 2H), 8.98 (s, 1H) |

(continued)

| Ex. | R' | R" | MS (M+) | NMR(400MHz, δ, $CDCl_3$) |
|---|---|---|---|---|
| 20 | 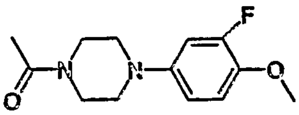 | 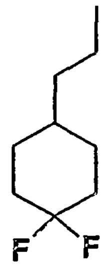 | 541 | 1.31-1.47(m, 3H), 1.66-1.78(m, 2H), 1.83-1.92(m, 4H), 2.04-2.15(m, 5H), 3.07-3.12 (m, 4H), 3.61 (t, 2H), 3.75(t, 2H), 4.46 (t, 2H), 5.22(s, 2H), 6.60-6.73(m, 3H), 6.97(t, 1H), 8.96 (s, 1H) |
| 21 | 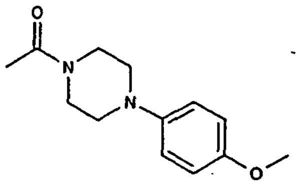 | 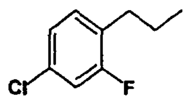 | 533.1 | 2.14(s, 3H), 3.07-3.10 (m, 4H), 3.21 (t, 2H), 3.63 (brs, 2H), 3.78(brs, 2H), 4.60(t, 2H), 4.98(s, 2H), 6.66(s, 1H), 6.86-7.04 (m, 7H), 8.95(s, 1H) |
| 22 | 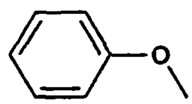 | 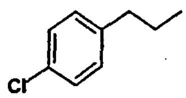 | 389.0 | 3.16(t, 2H), 4.57(t, 2H), 4.91 (s, 2H), 6.68(s, 1H), 6.91-6.93(m, 2H), 6.96 (d, 2H), 7.03-7.06(m, 1H), 7.21(d, 2H), 7.32-7.36(m, 2H), 8.98 (s, 1H) |
| 23 | 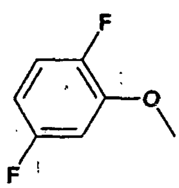 | 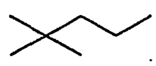 | 371.1 | 1.02(s, 9H), 4.43-4.39 (m, 2H), 4.41 (t, 2H), 5.29 (s, 2H), 6.67-6.73(m, 1H), 6.74(s, 1H), 6.81-6.85(m, 1H), 7.05-7.11 (m, 1H), 8.98 (s, 1H) |
| 24 | 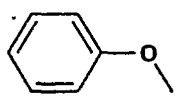 | 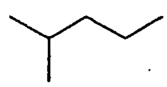 | 321.0 | 0.97(d, 6H), 1.62-1.71 (m, 1H), 1.72-1.78(m, 2H), 4.38(t, 2H), 5.25(s, 2H), 6.73(s, 1H), 6.99-7.01 (m, 2H), 7.02-7.06(m, 1H), 7.32-7.34(m, 2H), 8.96 (s, 1H) |
| 25 | 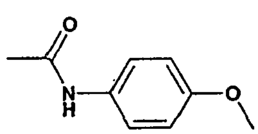 | 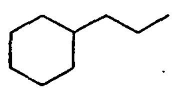 | 417.1 | 0.93-1.02(m, 2H), 1.11-1.28(m, 3H), 1.28-1.37(m, 1H), 1.64-1.79(m, 7H), 2.19 (s, 3H), 4.38(t, 2H), 5.21 (s, 2H), 6.72(s, 1H), 6.95 (d, 2H), 7.16(br, 1H), : 7.45(d, 2H), 8.97(s, 1H), |

(continued)

| Ex. | R' | R" | MS (M+) | NMR(400MHz, δ, $CDCl_3$) |
|---|---|---|---|---|
| 26 | 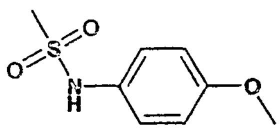 | 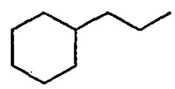 | 454.0 | 0.93-1.02(m, 2H), 1.14-1.27(m, 3H), 1.29-1.39(m, 1H), 1.65-1.80(m, 7H), 2.98 (s, 3H), 4.39(t, 2H), 5.23 (s, 2H), 6.30(s, 1H), 6.71 (s, 1H), 6.99(d, 2H), 7.24 (d, 2H), 8.98(s, 1H), |
| 27 | 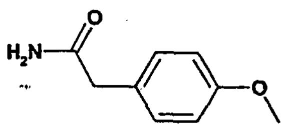 | 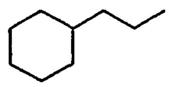 | 418.1 | 0.93-1.02(m, 2H), 1.13-1.28(m, 3H), 1.29-1.37(m, 1H), 1.69-1.80(m, 7H), 3.58 (s, 2H), 4.39(t, 2H), 5.24 (s, 2H), 5.51 (br, 1H), 6.03(br, 1H), 6.73(s, 1H), 6.99 (d, 2H), 7.25(d, 2H), 8.97(s, 1H) |
| 28 | 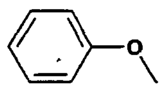 | 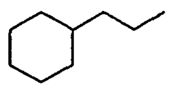 | 361.1 | 0.93-1.01 (m,2H), 1.13-1.27(m, 3H), 1.29-1.37(m, 1H), 1.66-1.79(m, 7H), 4.39(t, 2H), 5.24(s, 2H), 6.72(s, 1H), 6.98-7.01 (m, 2H), 7.03-7.06(m, 1H), 7.32-7.36(m, 2H), 8.96 (s, 1H) |
| 29 | 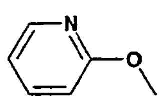 | 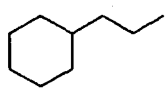 | 391.9 | 0.92-1.00(m, 2H), 1.14-1.26(m, 3H), 1.29-1.37(m, 1H), 1.48-1.54 (m, 2H), 1.65-1.78(m, 5H), 4.35 (s, 2H), 5.40(s, 2H), 6.26 (t, 1H), 6.52(s, 1H), 6.69 (d, 1H), 7.28(dd, 1H), 7.41 (m, 1H), 8.92(s, 1H) |
| 30 | 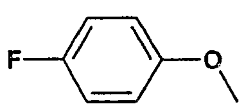 | 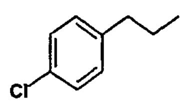 | 407.0 | 3.16(t, 2H), 4.57(t, 2H), 4.84(s, 2H), 6.65(s, 1H), 6.83-6.85(m, 2H), 6.95 (d, 2H), 7.00-7.04(m, 2H), 7.21(d, 2H), 8.98(s, 1H) |
| 31 | 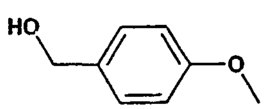 | 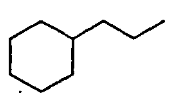 | 391.0 | 0.93-1.02(m, 2H), 1.11-1.24(m, 3H), 1.29-1.37(m, 1H), 1.69-1.79 (m, 7H), 4.39 (t, 2H), 4.66(s, 2H), 5.24 (s, 2H), 6.72(s, 1H), 6.98 (d, 2H), 7.35(d, 2H), 8.96 (s, 1H) |

(continued)

| Ex. | R' | R" | MS (M+) | NMR(400MHz, δ, $CDCl_3$) |
|---|---|---|---|---|
| 32 | 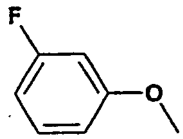 | 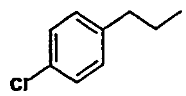 | 406.9 | 3.16(t, 2H), 4.56(t, 2H), 4.87(s, 2H), 6.62-6.70 (m, 3H), 6.73-6.79 (m, 1H), 6.95(d, 2H), 7.21(d, 2H), 7.26-7.31(m, 1H), 8.99(s, 1H) |
| 33 | 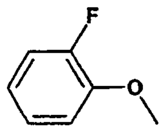 | 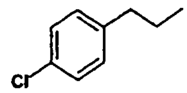 | 406.9 | 3.19(t, 2H), 4.62(t, 2H), 4.98(s, 2H), 6.67(s, 1H), 6.95-7.04(m, 4H), 7.07-7.16(m, 2H), 7.21 (d, 2H), 8.98(s, 1H) |
| 34 | 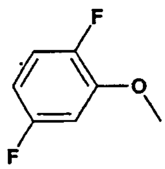 | 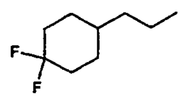 | 433.0 | 1.30-1.46(m, 2H), 1.61-1.78(m, 2H), 1.82-1.90(m, 4H), 2.05-2.13(m, 2H), 4.43(t, 2H), 5.28(s, 2H), 6.68-6.74(m, 1H), 6.76 (s, 1H), 6.81-6.86(m, 1H), 7.06-7.12(m, 1H), 9.00(s, 1H) |
| 35 | 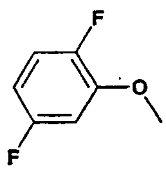 | 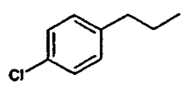 | 424.9 | 3.18(t, H), 4.60(t, 2H), 4.94(s, 2H), 6.68-6.74 (m, 3H), 7.00(d, 2H), 7.05-7.10(m, 1H), 7.22 (d, 2H), 8.99(s, 1H) |
| 36 | 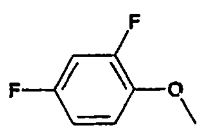 | 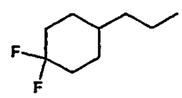 | 433.0 | 1.32-1.49(m, 2H), 1.62-1.79(m, 2H), 1.83-1.92(m, 4H), 2.06-2.13(m, 2H), 4.46(t, 2H), 5.25(s, 2H), 6.71(s, 1H), 6.80-6.86(m, 1H), 6.88-6.94(m, 1H), 7.00-7.06(m, 1H), 8.99 (s, 1H) |
| 37 | 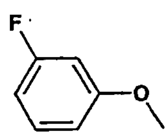 | 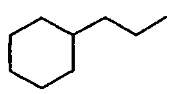 | 379.1 | 0.93-1.02(m, 2H), 1.13-1.26(m, 3H), 1.27-1.37(m, 1H), 1.64-1.79(m, 7H), 4.38(t, 2H), 5.23(s, 2H), 6.70-6.80(m, 4H), 7.28-7.32(m, 1H), 8.97 (s, 1H), |
| 38 | 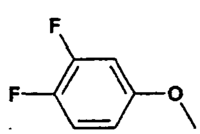 | 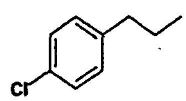 | 424.8 | 3.16(t, 2H), 4.56(t, 2H), 4.80(s, 2H), 6.57-6.61 (m, 1H), 6.66(s, 1H), 6.71-6.76(m, 1H), 6.94 (d, 2H), 7.08-7.15(m, 1H), 7.22(d, 2H), 8.99(s, 1H), |

(continued)

| Ex. | R' | R" | MS (M+) | NMR(400MHz, δ, $CDCl_3$) |
|---|---|---|---|---|
| 39 | 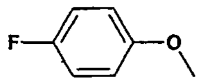 | 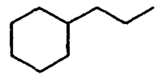 | 379.1 | 0.93-1.02(m, 2H), 1.13-1.26(m, 3H), 1.29-1.37(m, 1H), 1.67-1.79 (m, 7H), 4.39 (t, 2H), 5.20(s, 2H), 6.71 (s, 1H), 6.91-6.95(m, 2H), 7.01-7.05(m,2H), 8.96(s, 1H), |
| 40 | 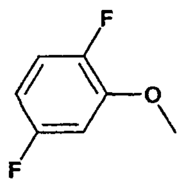 | 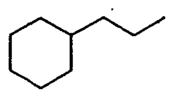 | 397.0 | 0.94-1.04(m, 2H), 1.11-1.26(m, 3H), 1.28-1.40(m, 1H), 1.66-1.81 (m, 7H), 4.41 (t, 2H), 5.28(s, 2H), 6.67-6.73(m, 1H), 6.74 (s, 1H), 6.80-6.85(m, 1H), 7.05-7.11 (m, 1H), 8.97(s, 1H), |
| 41 | 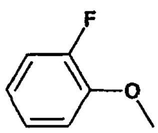 | 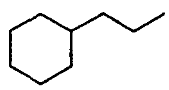 | 379.1 | 0.94-1.03(m, 2H), 1.11-1.29(m, 3H), 1.30-1.40(m, 1H), 1.63-1.81 (m, 7H), 4.44 (t, 2H), 5.30(s, 2H), 6.73 (s, 1H), 6.98-7.16(m, 4H), 8.98(s, 1H), |
| 42 | 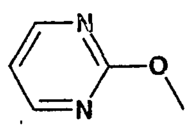 | 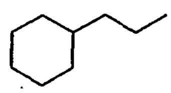 | 362.9 | 0.93-1.02(m, 2H), 1.14-1.29(m, 3H), 1.30-1.39(m, 1H), 1.52-1.57(m, 2H), 1.65-1.79(m, 5H), 4.36(t, 2H), 5.40(s, 2H), 6.48(br, 1H), 6.61 (s, 1H), 7.70(d, 1H), 8.73(br, 1H), 8.97(s, 1H) |
| 43 | 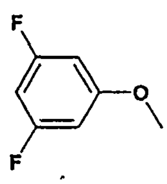 | 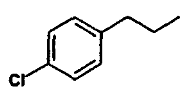 | 425.0 | 3.15(t, 2H), 4.56(t, 2H), 4.82(s, 2H), 6.43-6.46 (m, 2H), 6.49-6.54(m, 1H), 6.69(s, 1H), 6.94 (d, 2H), 7.22(d, 2H), 9.00(s, 1H), |
| 44 | 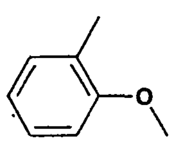 | 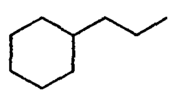 | 375.0 | 0.92-1.01 (m, 2H), 1.13-1.27(m, 3H), 1.28-1.38(m, 1H), 1.64-1.79(m, 7H), 2.24 (s, 3H), 4.41 (t, 2H), 5.24 (s, 2H), 6.73(s, 1H), 6.93-6.97(m,2H), 7.18-7.21(m, 2H), 8.96 (s, 1H) |

(continued)

| Ex. | R' | R" | MS (M+) | NMR(400MHz, δ, $CDCl_3$) |
|---|---|---|---|---|
| 45 | 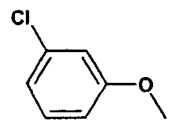 | 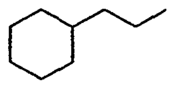 | 395.0 | 0.93-1.02(m, 2H), 1.14-1.25(m, 3H), 1.30-1.38(m, 1H), 1.64-1.79(m, 7H), 4.37(t, 2H), 5.22(s, 2H), 6.73(s, 1H), 6.88(dd, 1H), 7.00-7.05(m, 2H), 7.24-7.28(m, 1H), 8.97 (s, 1H) |
| 46 | 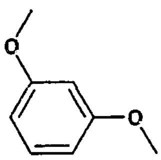 | 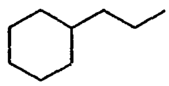 | 391.1 | 1.01-1.10(m, 2H), 1.18-1.35(m, 3H), 1.36-1.45(m, 1H), 1.72-1.87(m, 7H), 3.88 (s, 3H), 4.46(t, 2H), 5.30 (s, 2H), 6.62(t, 1H), 6.67 (d, 2H), 6.81 (s, 1H), 7.31 (t, 1H), 7.33(s, 1H), 9.05 (s, 1H) |
| 47 | 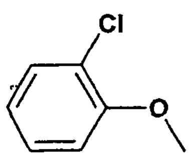 | 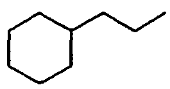 | 395.0 | 0.93-1.02(m, 2H), 1.13-1.25(m, 3H), 1.30-1.38(m, 1H), 1.63-1.80(m, 7H), 4.45(t, 2H), 5.30(s, 2H), 6.75(s, 1H), 6.98-7.06(m, 2H), 7.24-7.28(m, 1H), 7.41 (dd, 1H), 8.97(s, 1H) |
| 48 | 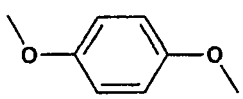 | 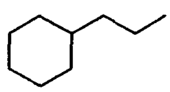 | 391.0 | 0.93-0.99(m, 2H), 1.13-1.28(m, 3H), 1.29-1.38(m, 1H), 1.64-1.80(m, 7H), 3.79 (s, 3H), 4.40(t, 2H), 5.19 (s, 2H), 6.71(s, 1H), 6.85-6.93(m, 4H), 8.97 (s, 1H) |
| 49 | 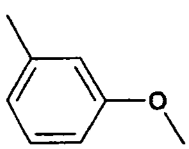 | 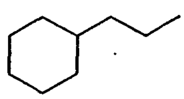 | 375.0 | 0.93-0.99(m, 2H), 1.13-1.27(m, 3H), 1.29-1.35(m, 1H), 1.64-1.77(m, 7H), 2.36 (s, 3H), 4.38(t, 2H), 5.22 (s, 2H), 6.71(s, 1H), 6.78-6.81(m, 2H), 6.85 (d, 1H), 7.21(t, 1H), 8.96 (s, 1H) |

Example 50: 4-{7-[2-(4-Chloro-phenyl)-ethyl]-2-cyano-7H-pyrrolo[2,3-d]pyrimidin-6-ylmethoxy}-3-fluoro-N-propyl-benzamide

6-Bromomethyl-7-[2-(4-chloro-phenyl)-ethyl]-7H-pyrrolo[2,3-d]pyrimidine-2-carbonitrile (Example A, 3.8 g, 10.1 mmol) and 3-fluoro-4-hydroxy-N-propyl-benzamide (2.0 g, 10.1 mmol) are dissolved in DMF (220 ml) and potassium carbonate (2.8 g, 20.2 mmol) is added to the solution. The reaction mixture is stirred at rt for 3 h and quenched with saturated ammonium chloride and extracted with ethyl acetate. The combined extracts are washed with brine, dried

over magnesium sulfate and evaporated down. Chromatography on silica gel (eluent; n-hexane : ethyl acetate = 4 :1, 2 :1, 1 :1, 1 :2) gives yellow product, which is recrystallized from acetonitrile to afford a pale yellow powder; Rf=0.30 (*n*-hexane : ethyl acetate = 1:1); $^1$H-NMR (400MHz, $CDCl_3$) δ : 0.99 (s, 3H), 1.65 (q, 2H), 3.18 (t, 2H), 3.41 (q, 2H), 4.60 (t, 2H), 4.97 (s, 2H), 5.94-6.05 (br, 1H), 6.77 (s, 1H), 6.97-6.99 (m, 3H), 7.26-7.31 (m, 2H), 7.50-7.58 (m, 2H), 8.97 (s, 1H).

<u>Examples 51 to 68</u>

By repeating the procedures described in Example 50 using appropriate starting materials (including those prepared in Example C) and conditions the following compounds of formula 2 are obtained as identified below in Table 3

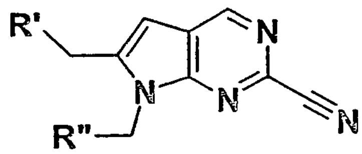

(2)

Table 3

| Ex. | R' | R" | $R_f$ (solvent) | NMR (400MHz, δ) |
|---|---|---|---|---|
| 51 | 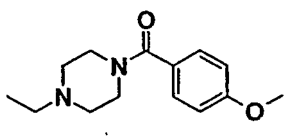 | 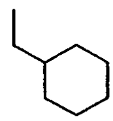 | 0.45 ($CH_2Cl_2$: MeOH=9:1) | ($CDCl_3$) 0.89-1.01 (m, 2H), 1.10 (t, 3H), 1.13-1.19 (m, 4H), 1.54 (brs, 3H), 1.67-1.93 (m, 6H), 2.43-2.47 (m, 6H), 3.4-3.9 (br, 4H), 4.36-4.40 (m, 2H), 5.20 (s, 2H), 6.73 (s, 1H), 6.95 (d, 2H), 7.43 (d, 2H), 8.97 (s, 1H) |
| 52 | 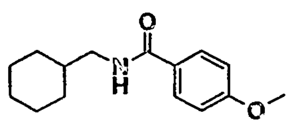 | 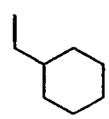 | 0.12 (*n*-hexane: AcOEt=1:1) | ($CDCl_3$) 0.93-1.10 (m, 4H), 1.13-1.40 (m, 4H), 1.56-1.79 (m, 14H), 2.25-2.45 (br, 6H), 3.29-3.32 (m, 2H), 4.36-4.41 (m, 2H), 5.28 (s, 2H), 6.10 (br, 1H), 6.79 (s, 1H), 7.00 (d, 2H), 7.76 (d, 2H), 8.98 (s, 1H) |
| 53 | 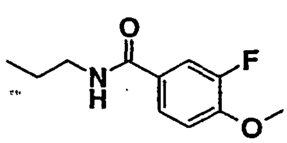 | 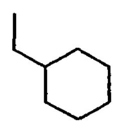 | 0.30 (*n*-hexane: AcOEt=1:2) | ($CDCl_3$) 0.94-0.99 (m, 5H), 1.11-1.43 (m, 4H), 1.61-1.80 (m, 9H), 3.40 (q, 2H), 4.39-4.43 (m, 2H), 5.34 (s, 2H), 5.94-6.05 (br, 1H), 6.73 (s, 1H), 7.10 (t, 1H), 7.53-7.68 (m, 2H), 8.97 (s, 1H) |

(continued)

| Ex. | R' | R" | $R_f$ (solvent) | NMR (400MHz, δ) |
|---|---|---|---|---|
| 54 | 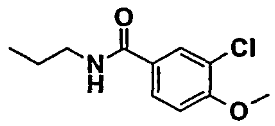 | 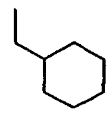 | 0.42 (*n*-hexane: AcOEt=1:2) | ($CDCl_3$) 0.94-0.99(m, 5H), 1.11-1.43 (m, 4H), 1.61-1.80(m, 9H), 3.41 (q, 2H), 4.41-4.45 (m, 2H), 5.34 (s, 2H), 5.94-6.05 (br, 1H), 6.73 (s, 1H), 7.09 (d, 1H), 7.70-7.77 (m, 1H), 7.81 (d, 1H), 8.98(s, 1H) |
| 55 | 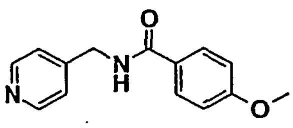 | 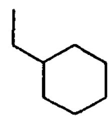 | 0.52 ($CH_2Cl_2$: MeOH=9:1) | ($CDCl_3$) 0.94-1.05(m, 2H), 1.11-1.43 (m, 4H), 1.61-1.80(m, 7H), 4.30-4.40 (m, 2H), 4.66 (d, 2H), 5.28 (s, 2H), 6.45-6.52 (br, 1H), 6.75 (s, 1H), 7.06 (d, 2H), 7.86 (d, 2H), 8.57 (d, 2H), 8.97 (s, 1H) |
| 56 | 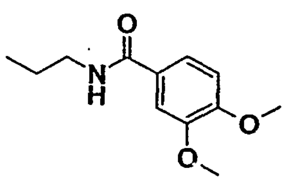 | 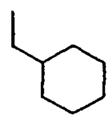 | 0.42 (*n*-hexane: AcOEt=1:1) | ($CDCl_3$) 0.94-0.99(m, 5H), 1.11-1.43 (m, 4H), 1.61-1.80(m, 9H), 3.41 (q, 2H), 3.91 (s, 3H), 4.30-4.44(m, 2H), 5.31 (s, 2H), 5.94-6.05(br, 1H), 6.70 (s, 1H), 6.96(d, 1H), 7.20 (dd, 1H), 7.46(s, 1H), 8.97 (s, 1H) |
| 57 | 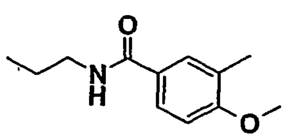 | 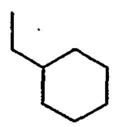 | 0.28 (n-hexane: AcOEt=1:1) | ($CDCl_3$) 0.94-0.99(m, 5H), 1.11-1.43 (m, 4H), 1.61-1.80 (m, 9H), 2.31 (s, 3H), 3.41 (q, 2H), 4.31-4.42 (m, 2H), 5.28 (s, 2H), 5.94-6.05 (br, 1H), 6.78 (s, 1H), 6.98 (d, 1H), 7.60-7.66 (m, 2H), 8.97 (s, 1H) |
| 58 | 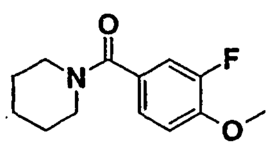 | 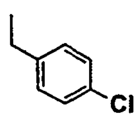 | 0.12 (*n*-hexane: AcOEt=1:2) | ($CDCl_3$) 1.67-1.88 (m, 6H), 3.18 (t, 2H), 3.39-3.63 (m, 4H), 4.61 (t, 2H), 4.96 (s, 2H), 6.68 (s, 1H), 6.90-7.01 (m, 3H), 7.15-7.36 (m, 4H), 8.96 (s, 1H) |
| 59 | 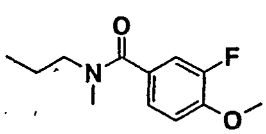 | 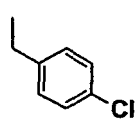 | 0.45 (*n*-hexane: AcOEt=1:5) | ($CDCl_3$) 0.86-0.97 (br, 3H), 1.64-1.73 (br, 2H), 3.01-3.47 (br, 7H), 4.61 (t, 2H), 4.95 (s, 2H), 6.67 (s, 1H), 6.90-7.01 (m, 3H), 7.15-7.26 (m, 4H), 8.96 (s, 1H) |

(continued)

| Ex. | R' | R" | $R_f$ (solvent) | NMR (400MHz, δ) |
|---|---|---|---|---|
| 60 | 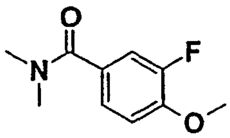 | 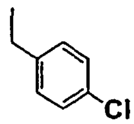 | 0.18 (*n*-hexane: AcOEt=1:5) | ($CDCl_3$) 2.96-3.06 (br, 6H), 3.18 (t, 2H), 4.61 (t, 2H), 4.95 (s, 2H), 6.67 (s, 1H), 6.94-7.01 (m, 3H), 7.19-7.26 (m, 4H), 8.96 (s, 1H) |
| 61 | 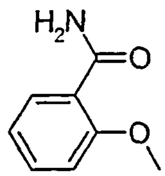 | 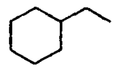 | 0.24 (*n*-hexane: AcOEt=1:1) | (DMSO-$d_6$) 0.89-0.95(m, 2H), 1.12-1.27 (m, 4H), 1.59-1.67(m, 5H), 1.73(d, 2H), 4.40(t, 2H), 5.58 (s, 2H), 7.02(s, 1H), 7.07(td, 1H), 7.35(d, 1H), 7.45-7.51 (m, 3H), 7.70(d, 1H), 9.17(s, 1H) |
| 62 | 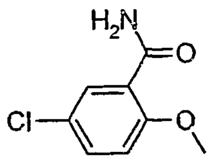 | 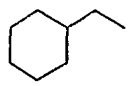 | 0.88 ($CH_2Cl_2$: MeOH=9:1) | (DMSO-$d_6$) 0.90-0.96(m, 2H), 1.12-1.19 (m, 3H), 1.23-1.25(m, 1H), 1.61-1.66 (m, 5H), 1.74(d, 2H), 4.39(t, 2H), 5.59(s, 2H), 7.01 (s, 1H), 7.40(d, 1H), 7.53 (dd, 1H), 7.61 (br, 2H), 7.63 (d, 1H), 9.17(s, 1H) |
| 63 | 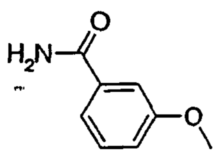 | 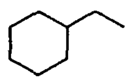 | 0.5 ($CH_2Cl_2$: MeOH=9:1) | (DMSO-$d_6$) 0.91-0.95(m, 2H), 1.10-1.20 (m, 3H), 1.21-1.35(m, 1H), 1.65-1.80 (m, 7H), 4.37(t, 2H), 5.51 (s, 2H), 7.02(s, 1H), 7s.24(dd, 1H), 7.39-7.43(m, 2H), 7.52(d, 1H), 7.61(t, 1H), 7.97(s, 1H), 9.18(s, 1H) |
| 64 | 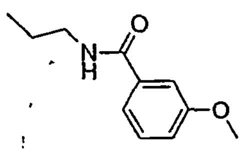 | 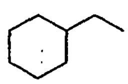 | 0.38 (n-hexane: AcOEt=1:1) | ($CDCl_3$) 0.95-1.05(m, 2H), 1.00(t, 3H), 1.15-1.30(m, 3H), 1.30-1.40 (m, 1H), 1.63-1.79(m, 9H), 3.44(q, 2H), 4.36-4.41 (m, 2H), 5.30(s, 2H), 6.13(br, 1H), 6.76(s, 1H), 7.10(ddd, 1H), 7.32(dd, 1H), 7.38 (t, 1H), 7.54(dd, 1H), 8.97 (s, 1H) |
| 65 | 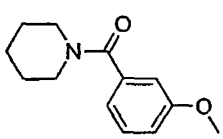 | 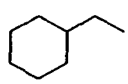 | 0.25 (n-hexane: AcOEt=1:1) | ($CDCl_3$) 0.93-0.99(m, 2H), 1.13-1.25 (m, 3H), 1.28-1.38(m, 1H), 1.45-1.60(br, 2H), 1.69-1.80 (m, 11H), 3.34(br, 2H), 3.71 (br, 2H), 4.36-4.40(m, 2H), 5.25(s, 1H), 6.73(s, 1H), 7.01-7.05(m, 3H), 7.36(t, 1H), 8.97(s, 1H) |

(continued)

| Ex. | R' | R" | $R_f$ (solvent) | NMR (400MHz, δ) |
|---|---|---|---|---|
| 66 | 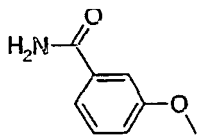 | 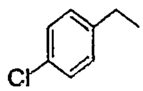 | 0.59 ($CH_2Cl_2$: MeOH=9:1) | ($DMSO-d_6$) 3.13(t, 2H), 4.60(t, 2H), 5.39 (s, 2H), 6.97(s, 1H), 7.09(dd, 2H), 7.21-7.22(m, 1H), 7.24 (dd, 2H), 7.41(t, 2H), 7.53(d, 1H), 7.60 (dd, 1H), 7.98(br, 1H), 9.15(s, 1H) |
| 67 | 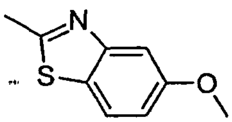 | 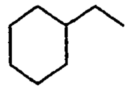 | 0.22 (n-hexane: AcOEt=1:1) | ($CDCl_3$) 0.95-1.03(m, 2H), 1.15-1.25 (m, 3H), 1.30-1.40(m, 1H), 1.66-1.79(m, 7H), 2.84(s, 3H), 4.39-4.43(m, 2H), 5.31 (s, 2H), 6.77(s, 1H), 7.05(dd, 1H), 7.56(d, 1H), 7.72(d, 1H), 8.96(s, 1H) |
| 68 | 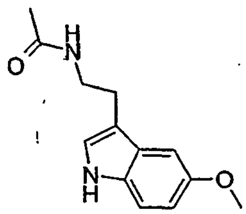 | 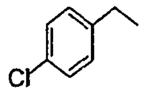 | 0.52 ($CH_2Cl_2$: MeOH=9:1) | (CDCl3) 1.96(s, 3H), 2.95(t, 2H), 3.18 (t, 2H), 3.58(dd, 2H), 4.62(t, 2H), 5.00(s, 2H), 5.56(br, 1H), 6.74 (s, 1H), 6.86(dd, 1H), 7.00(dd, 2H), 7.07(d, 1H), 7.15(d, 1H), 7.19(dd, 2H), 7.30(d, 1H), 8.02 (br, 1H), 8.97(s, 1H) |

Examples 69 to 81

By repeating the procedures described in Example 50 using appropriate starting materials (including those prepared in Example D) and conditions the following compounds of formula 2 are obtained as identified below in Table 4

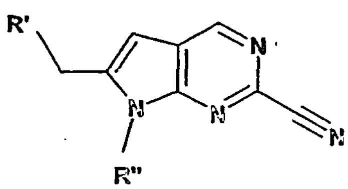

(2)

Table 4.

| Ex. | R' | R" I | $R_f$(solvent) | NMR (400MHz, δ) |
|---|---|---|---|---|
| 69 | 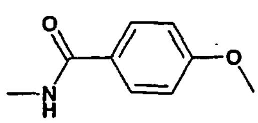 | 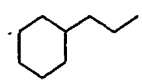 | 0.08 (n-hexane: AcOEt=1:1) | CDCl3 0.92-1.01 (m, 2H), 1.12-1.35 (m, 4H), 1.64-1.78(m, 7H), 3.02(d, 3H), 4.36-4.40(m, 2H), 5.28(s, 2H), 6.04(brs, 1H), 6.74(s, 1H), 7.02(d, 2H), 7.77 (d, 2H), 8.97(s, 1H) |

(continued)

| Ex. | R' | R" I | $R_f$(solvent) | NMR (400MHz, δ) |
|---|---|---|---|---|
| 70 | 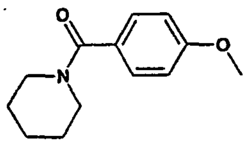 | 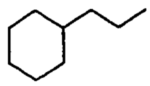 | 0.13 (n-hexane: AcOEt=1:1) | CDCl3 0.92-1.01(m, 2H), 1.13-1.25 (m, 3H), 1.30-1.36(m, 1H), 1.58-1.79(m, 13H), 3.54 (brs, 4H), 4.36-4.40(m, 2H), 5.26 (s, 2H), 6.74(s, 1H), 7.00(d, 2H), 7.41 (d, 2H), 8.97(s, 1H) |
| 71 | 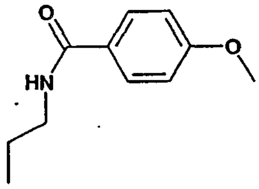 | 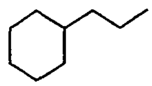 | 0.15 (n-hexane: AcOEt=1:1) | 0.92-1.01 (m, 5H), 1.13-1.35 (m, 4H), 1.61-1.78(m, 9H), 3.42(m, 2H), 4.36-4.40(m, 2H), 5.28(s, 2H), 6.07(br, 1H), 6.74(s, 1H), 7.00-7.04 (m, 2H), 7.76-7.79(m, 2H), 8.97(s, 1H) |
| 72 | 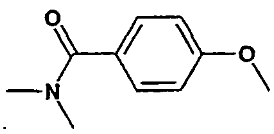 | 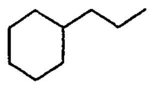 | 0.58 ($CH_2Cl_2$: MeOH=9:1) | CDCl3 0.92-1.01(m, 2H), 1.12-1.36(m, 4H), 1.62-1.79(m, 7H), 3.06 (brs, 6H), 4.36-4.40(m, 2H), 5.26(s, 2H), 6.74(s, 1H), 7.01(d, 2H), 7.45(d, 2H), 8.97(s, 1H) |
| 73 | 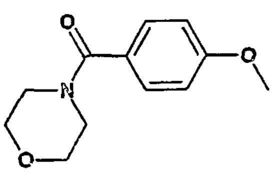 | 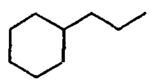 | 0.54 ($GH_2Cl_2$: MeOH=9:1) | CDCl3 0.92-1.01(m, 2H), 1.10-1.36 (m, 4H), 1.63-1.79(m, 7H), 3.55-3.70(m, 8H), 4.36-4.4.0(m, 2H), 5.27(s, 2H), 6.74(s, 1H), 7.02(d, 2H), 7.44(d, 2H), 8.98 (s, 1H) |
| 74 | 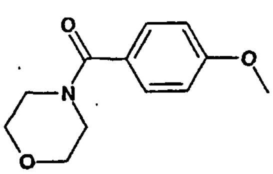 | 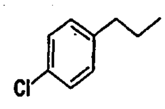 | 0.20 (AcOEt) | CDCl3 3.16(dd, 2H), 3.51-3.83 (m, 8H), 4.57(dd, 2H), 4.89(s, 2H), 6.68(s, 1H), 6.91-6.96(m, 4H), 7.21 (dd, 2H), 7.43(dd, 2H), 8.99(s, 1H) |
| 75 | 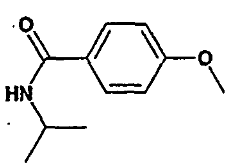 | 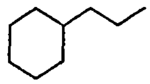 | 0.37 (AcOEt) | CDCl3 0.93-1.01 (m, 2H), 1.13-1.36 (m, 10H), 1.64-1.78(m, 7H), 4.25-4.32(m, 1H), 4.36-4.40 (m, 2H), 5.28(s, 2H), 5.82(brd, 1H), 6.74(s, 1H), 7.02(d, 2H), 7.77(d, 2H), 8.97(s, 1H) |

(continued)

| Ex. | R' | R" I | $R_f$(solvent) | NMR (400MHz, δ) |
|---|---|---|---|---|
| 76 | 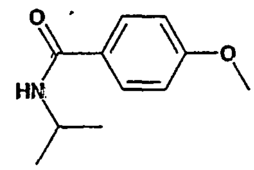 | 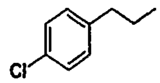 | 0.53 (AcOEt) | CDCl3 1.27(d, 6H), 3.16(dd, 2H), 4.26-4.31 (m, 1H), 4.57 (dd, 2H), 4.90(s, 2H), 5.80-5.83 (m, 1H), 6.68(s, 1H), 6.90-6.95(m, 4H), 7.19-7.23(m, 2H), 7.74-7.77(m, 2H), 8.99 (s, 1H) |
| 77 | 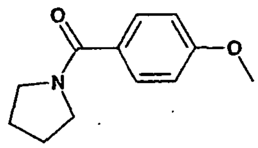 | 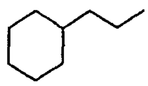 | 0.36 (AcOEt) | CDCl3 0.92-1.01(m, 2H), 1.12-1.36 (m, 4H), 1.64-1.79(m, 7H), 1.92 (brs, 4H), 3.55(brs, 4H), 4.36-4.40(m, 2H), 5.26(s, 2H), 6.74(s, 1H), 6.99-7.01 (m, 2H), 7.56(d, 2H), 8.97(s, 1H) |
| 78 | 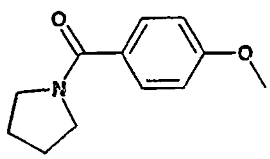 | 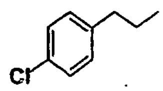 | 0.23 (AcOEt) | CDCl3 1.87-1.99(m, 4H), 3.16 (dd, 2H), 3.46-3.49(m, 2H), 3.63-3.66(m, 2H), 4.57(dd, 2H), 4.90(s, 2H), 6.68(s, 1H), 6.90-6.97(m, 4H), 7.19-7.22(m, 2H), 7.54-7.57(m, 2H), 8.99(s, 1H) |
| 79 | 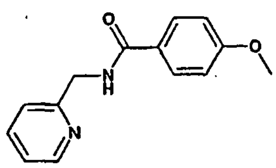 HCl | 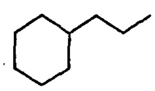 | Free salt 0.68 ($CH_2Cl_2$: MeOH = 9: 1) | (DMSO-$d_6$) 0.91-0.97(m, 2H), 1.09-1.30 (m, 4H), 1.60-1.76(m, 7H), 4.37 (dd, 2H), 4.76(d, 2H), 5.56 (s, 2H), 7.04(s, 1H), 7.22 (d, 2H), 7.79-7.85(m, 2H), 7.96(d, 2H), 8.37(dd, 1H), 8.77(d, 1H), 9.19(s, 1H), 9.30 (dd, 1H) |
| 80 | 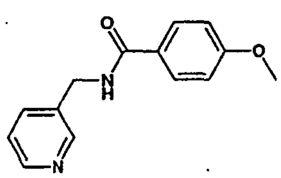 HCl | 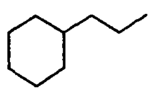 | Free salt 0.48 ($CH_2Cl_2$: MeOH = 9: 1) | (DMSO-$d_6$) 0.88-0.97(m, 2H), 1.09-1.30 (m, 4H), 1.59-1.76(m, 7H), 4.37 (dd, 2H), 4.61 (d, 2H), 5.54 (s, 2H), 7.04(s, 1H), 7.20 (d, 2H), 7.86-7.93(m, 3H), 8.32(d, 1H), 8.74(d, 1H), 8.80 (brs, 1H), 9.14-9.18 (m, 2H) |

(continued)

| Ex. | R' | R" I | $R_f$(solvent) | NMR (400MHz, δ) |
|---|---|---|---|---|
| 81 | 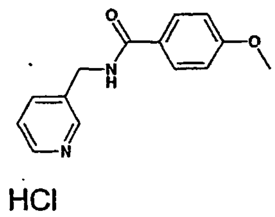 | 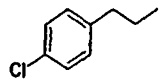 | Free salt<br>0.48 ($CH_2Cl_2$: MeOH = 9: 1) | (DMSO-$d_6$)<br>3.12(dd, 2H), 4.57-4.64 (m, 4H), 5.42(s, 2H), 6.98 (s, 1H), 7.07-7.10(m, 2H), 7.18 (d, 2H), 7.23-7.26(m, 2H), 7.93(d, 2H), 7.98-8.02(m, 1H), 8.48(d, 1H), 8:86-8.89(m, 1H), 9.15(s, 1H), 9.25 |

Examples 82 to 87

By repeating the procedures described in Example 50 using appropriate starting materials (including those prepared in Example E) and conditions the following compounds of formula 2 are obtained as identified below in Table 5

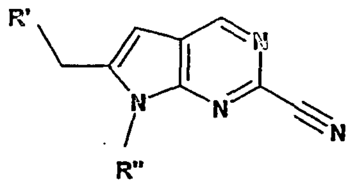

(2)

Table 5

| Ex. | R' | R" | Rf (solvent) | NMR (DMSO-$d_6$, 400MHz, δ) |
|---|---|---|---|---|
| 82 | 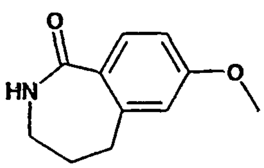 | 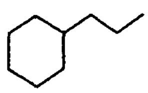 | 0.10<br>(n-hexane: AcOEt=1:1) | 0.90-0.96(m, 2H), 1.12-1.30 (m, 4H), 1.60-1.76(m, 7H), 1.83-1.92(m, 2H), 2.74(dd, 2H), 2.88-2.93(m, 2H), 4.36 (dd, 2H), 5.50(s, 2H), 6.99-9.07(m, 3H), 7.49(d, 1H), 7.91 (brt, 1H), 9.18(s, 1H) |
| 83 | 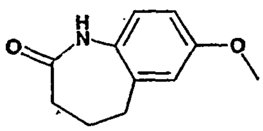 | 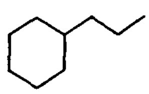 | 0.46<br>($CH_2Cl_2$: MeOH=9:1) | 0.89-0.95(m, 2H), 1.11-1.17 (m, 4H), 1.56-1.74(m, 7H), 2.06-2.09(m, 4H), 2.64-2.67 (m, 2H), 4.35(dd, 2H), 5.41 (s, 2H), 6.90-7.01 (m, 4H), 9.16(s, 1H), 9.32(s, 1H) |
| 84 | 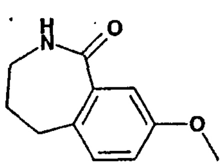 | 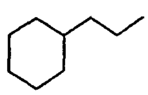 | 0.09<br>(n-hexane: AcOEt=1:1) | 0.90-0.95(m, 2H), 1.12-1.28 (m, 4H), 1.60-1.75(m, 7H), 1.83 -1.88(m, 2H), 2.69 (dd, 2H), 2.88-2.93(m, 2H), 4.36 (dd, 2H), 5.47(s, 2H), 7.00(s, 1H), 7.12-7.15(m, 1H), 7.21-7.23 (m, 2H), 8.06(t, 1H), 9.17(s, 1H) |

(continued)

| Ex. | R' | R" | Rf (solvent) | NMR (DMSO-$d_6$, 400MHz, δ) |
|---|---|---|---|---|
| 85 | 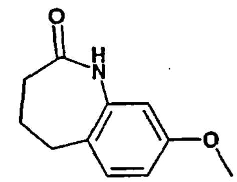 | 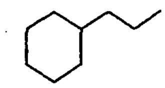 | 0.1 (n-hexane: AcOEt=1:1) | 0.91-0.96(m, 2H), 1.12-1.27 (m, 4H), 1.61-1.76(m, 7H), 2.04 -2.16(m, 2H), 2.13-2.16 (m, 2H), 2.62(dd, 2H), 4.35(m, 2H), 5.41(s, 2H), 6.67(d, 1H), 6.84-6.87(m, 1H), 7.00(s, 1H), 7.19 d, 1H), 9.17(s, 1H), 9.52 (brs, 1H) |
| 86 | 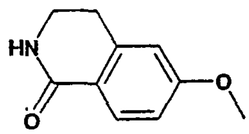 | 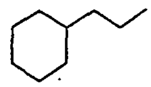 | 0.54 ($CH_2Cl_2$: MeOH=9:1) | 0.88-0.96(m, 2H), 1.09-1.29 (m, 4H), 1.60-1.91 (m, 7H), 2.89(dd, 2H), 3.34-3.38(m, 2H), 4.36(dd, 2H), 5.52(s, 2H), 7.03-7.06(m, 3H), 7.76-7.82 (m, 2H), 9.18(s, 1H) |
| 87 | 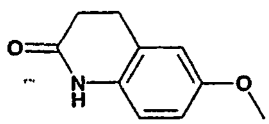 | 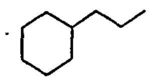 | 0.54 ($CH_2Cl_2$: MeOH=9:1) | 0.88-0.97(m, 2H), 1.10-1.29 (m, 4H), 1.60-1.76(m, 7H), 2.39-2.43(m, 2H), 2.85(dd, 2H), 4.35(dd, 2H), 5.38(s, 2H), 6.79(d, 1H), 6.87-6.90(m, 1H), 6.96-6.98(m, 2H), 9.16(s, 1H) |

Example 88: 7-[2-(4-Chloro-phenyl)-ethyl]-6-(2-fluoro-4-formyl-phenoxymethyl)-7H-pyrrolo[2,3-d]pyrimidine-2-carbonitrile

To a solution of 6-bromomethyl-7-[2-(4-chloro-phenyl)-ethyl]-7H-pyrrolo[2,3-d]pyrimidine-2-carbonitrile (Example B2, 500mg) in DMF (5mL) is added 3-fluoro-4-hydroxybenzaldehyde (224mg), potassium carbonate (276mg), stirred for 2h. The reaction mixture is diluted with water and extracted with EtOAc. The organic layer is successively washed with water and aqueous sodium chloride, dried over magnesium sulfate, and concentrated in vacuo. The crude product is purified by silica gel column chromatography to give the title compound; Rf = 0.25 (n-hexane; EtOAc=1:1); $^1$H NMR (DMSO-d6, δ (ppm); 3.19(dd, 2H), 4.60(dd, 2H), 5.00(s, 2H), 6.72)s, 1H), 6.98(dd, 2H), 7.06(dd, 1H), 7.22(d, 2H), 7.65-7.69(m, 2H), 9.01 (s, 1H), 9.90(s, 1H)

Example 89: 4-{7-[2-(4-Chloro-phenyl)-ethyl]-2-cyano-7H-pyrrolo[2,3-d]pyrimidin-6-ylmethoxy}-3-fluoro-benzoic acid

To a solution of 7-[2-(4-chloro-phenyl)-ethyl]-6-(2-fluoro-4-formyl-phenoxy methyl)-7H-pyrrolo[2,3-d]pyrimidine-2-carbonitrile (Example 88, 480mg), $NaClO_4$ (298mg) in tetrahydrofurane (10mL) is added $NH_2SO_3H$ (160mg) in $H_2O$ at 0˚, stirred for 3h at rt. The reaction mixture is diluted with $H_2O$ and extracted with EtOAc. The organic layer is successively washed with $H_2O$ and aq. sodium chloride, dried over $MgSO_4$, and concentrated in vacuo. The crude product is washed with $Et_2O$ to give the title compound; Rf = 0.08 (n-hexane; EtOAc=1:1); $^1$H NMR (DMSO-d6, δ (ppm): 3.13(dd, 2H), 4.61(dd, 2H), 5.54(s, 2H), 7.01(s, 1H), 7.08-7.10(m, 2H), 7.22-7.25(m, 2H), 7.45-7.49(m, 1H), 7.71-7.74 (m, 1H), 7.80-7.82(m, 1H), 9.16(s, 1H), 13.00(brs, 1H).

Example 90: 4-{7-[2-(4-Chloro-phenyl)-ethyl]-2-cyano-7H-pyrrolo[2,3-d]pyrimidin-6-ylmethoxy}-3-fluoro-N,N-dipropylbenzamide

To a solution of 4-{7-[2-(4-chloro-phenyl)-ethyl]-2-cyano-7H-pyrrolo[2,3-d]pyrimidin-6-ylmethoxy}-3-fluorobenzoic acid (60mg) in pyridine (1 mL) is added $POCl_3$ (15uL) at 0˚C and continuing with stirring at 0˚C for 1 h. To the reaction mixture is added di-n-propylamine (17uL) and stirred for 1 hr at 0˚C, diluted with $H_2O$ and extracted with EtOAc. The organic layer is successively washed with $H_2O$ and aqueous sodium chloride, dried over MgSO4, and concentrated in vacuo. The crude product is purified by silica gel column chromatography to give the title compound; Rf = 0.13(n-hexane; EtOAc=1:1); $^1$H NMR ($CDCl_3$, δ (ppm)); 0.88-1.04(m, 6H), 1.65-1.85 (m, 4H), 3.18-3.61 (m, 6H), 4.70(dd, 2H), 5.05(s, 2H), 6.76(s, 1H), 7.02-7.10(m, 3H), 7.20-7.31 (m, 4H), 9.08(s, 1H).

Example 91: 6-[4-(5,5-Dimethyl-2,4-dioxo-oxazolidin-3-ylmethyl)-phenoxymethyl]-7-(3-ethyl-heptyl)-7H-pyrrolo[2,3-d]pyrimidine-2-carbonitrile

To a solution of 7-(3-ethyl-heptyl)-6-(4-formyl-phenoxymethyl)-7H-pyrrolo[2,3-d]pyrimidine-2-carbonitrile (720 mg, 1.90 mmol) in MeOH (30 ml) and THF (30 ml) is added portionwise $NaBH_4$ (100 mg, 2.60 mmol). The reaction mixture is stirred at rt for 4 h, and the bulk of solvents are removed in vacuo. The residue is diluted with water, and extracted with $CH_2Cl_2$. The combined organic extracts are washed with brine, and dried over $Na_2SO_4$, filtered, and concentrated in vacuo. The residue is purified by silica gel column chromatography to give the alcohol 7-(3-ethyl-heptyl)-6-(4-hydroxy methyl-phenoxymethyl)-7H-pyrrolo[2,3-d]pyrimidine-2-carbonitrile. To a solution of said alcohol (140 mg, 0.36 mmol), 5,5-dimethyl-oxazolidinedione (46 mg, 360 mmol), and $Ph_3P$ (105 mg, 0.40 mmol) in THF (2 mL) is added DEAD (0.25 ml, 0.46 mmol). The reaction mixture is stirred at rt for overnight. After concentration, the residue is purified by RP-HPLC to give the title compound; Rf 0.38 (n-Hexane:EtOAc=1:1); $^1$H-HMR (400 MHz) $\delta$ 0.92-1.00(m, 2H), 1.18-1.25(m, 3H), 1.30-1.40(m, 1H), 1.58(s, 6H), 1.68-1.78(m, 7H), 4.35-4.39(m, 2H), 4.62(s, 2H), 5.22(s, 2H), 6.71 (s, 1H), 6.95(dd, 2H), 7.37(dd, 2H), 8.96(s, 1H).

Example 92:

6-Bromomethyl-7-(2-cyclohexyl-ethyl)-7H-pyrrolo[2,3-d]pyrimidine-2-carbonitrile (Example A, 0.23mmol) is dissolved in 2ml of DMF. To the solution is added 1-(4-hydroxy-phenyl)-3,3-dimethyl-pyrrolidin-2-one (0.25mmol) and K2CO3 (0.27mmol) at rt. After 1.5 h the reaction mixture is diluted with H2O, extracted with AcOEt twice, and dried over Na2SO4. Flash chromatography on silica gel using AcOEt-Hexane (1:2) provides the product (for physical data see Table 6 below).

Examples 93 to 96

By repeating the procedures described in Example 92 using appropriate starting materials (including those prepared in Examples F and G) and conditions the following compounds of formula 2 are obtained as identified below in Table 6.

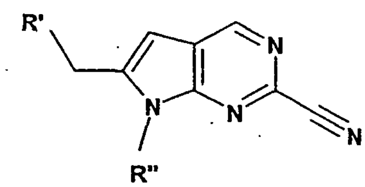

(2)

Table 6

| Ex. | R' | R" | Rf (solvent) | NMR($CDCl_3$, 400MHz, $\delta$) |
|---|---|---|---|---|
| 92 | 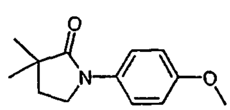 | 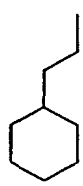 | | 0.93-1.02(m, 2H), 1.13-1.38 (m, 10H), 1.64-1.80(m, 7H), 2.01 (t, 2H), 3.73(t, 2H), 4.37-4.40(m, 2H), 5.23(s, 2H), 6.71 (s, 1H), 6.98-7.00(m, 2H), 7.60-7.62(m, 2H), 8.95(s, 1H) |
| 93 | 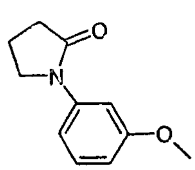 | 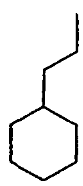 | MS m/z 444 (M+H$^+$) | 0.93-1.02(m, 2H), 1.10-1.38 (m, 4H), 1.60-1.80(m, 7H), 1.14-2.21(m, 2H), 2.64(t, 2H), 3.86(t, 2H), 4.37-4.41 (m, 2H), 5.27(s, 2H), 6.75-6.78(m, 2H), 7.00-7.04(m, 1H), 7.32 (t, 1H), 7.75-7.76(m, 1H), 8.96 (s, 1H) |

(continued)

| Ex. | R' | R" | Rf (solvent) | NMR($CDCl_3$, 400MHz, δ) |
|---|---|---|---|---|
| 94 | 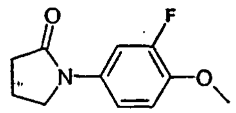 | 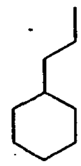 | 0.11 (*n*-hexane: AcOEt = 2:1) | 0.95-1.04(m, 2H), 1.14-1.29 (m, 3H), 1.31- 1.41(m, 1H), 1.65-1.82(m, 7H), 2.13- 2.21 (m, 2H), 2.61 (t, 2H), 3.81 (t, 2H), 4.42 - 4.46(m, 2H), 5.28 (s, 2H), 6.69(s, 1H), 7.03(t, 1H), 7.26- 7.29 (m, 2H), 7.57-7.61 (m, 1H), 8.95(s, 1H) |
| 95 | 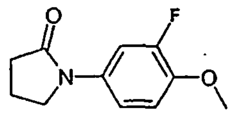 | 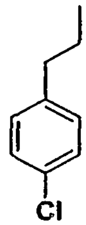 | 0.17 (*n*-hexane: AcOEt=2 :1) | 2.14-2.21 (m, 2H), 2.62(t, 2H), 3.19(t, 2H), 3.82(t, 2H), 4.62 (t, 2H), 4.94(s, 2H), 6.64(s, 1H), 6.93(t, 1H), 6.99-7.02 (m, 2H), 7.20-7.23(m, 2H), 7.58-7.62 (m, 1H), 8.97(s, 1H) |
| 96 | 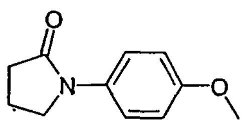 | 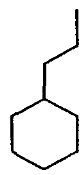 | MS m/z 444 ($M+H^+$) | 0.93-1.02 (m, 2H), 1.14-1.35 (m, 4H), 1.64 - 1.80 (m, 7H), 2.13 - 2.21 (m, 2H), 2.61 (t, 2H), 3.84 (t, 2H), 4.36-4.40 (m, 2H), 5.23 (s, 2H), 6.71 (s, 1H), 6.98-7.00 (m, 2H), 7.55 -7.58 (m, 2H), 8.96 (s, 1H) |

Example 97: 2,2,2-Trifluoro-ethanesulfonic acid {4-[2-cyano-7-(2-cyclohexyl-ethyl)-7H-pyrrolo[2,3-d]pyrimidin-6-yl-methoxy]-phenyl}-amide

6-(4-Amino-phenoxymethyl)-7-(2-cyclohexyl-ethyl)-7H-pyrrolo[2,3-d]pyrimidine-2-carbonitrile (0.21 mmol) is dissolved in 2ml of CH2Cl2. To the solution is added 2,2,2-trifluoroethanesulfonyl chloride (0.25mmol) and pyridine (0.25mmol) at rt. After 0.5 h the reaction mixture is diluted with H2O, extracted with Et2O twice, and dried over Na2SO4. Flash chromatography on silica gel using AcOEt-Hexane (1:1) gives the title product; Rf 0.18 (*n*-Hexane: AcOEt=2: 1); $^1$H-HMR (400 MHz, CDCl3) δ : 0.93 -1.02 (m, 2H), 1.10 - 1.28 (m, 3H), 1.29 - 1.39 (m, 1H), 1.65 - 1.79 (m, 5H), 3.73 - 3.79 (q, 2H), 4.37 - 4.41 (m, 2H), 5.24 (s, 2H), 6.63 (br s, 1H), 6.74 (s, 1H), 7.01 - 7.03 (m, 2H), 7.27 (d, 2H), 8.98 (s, 1H).

Example 98: 6-[4-(4-Acetyl-piperazin-1-yl)-phenoxymethyl]-7-(2-cyclopentyl-ethyl)-7H-pyrrolo[2,3-d]pyrimidine-2-carbonitrile

5-Bromo-4-(2-cyclopentyl-ethylamino)-pyrimidine-2-carbonitrile (0.46mmol) and 1-[4-(4-prop-2-ynyloxy-phenyl)-piperazin-1-yl]-ethanone (0.41 mmol) are dissolved in 4ml of DMF. The mixture is degassed by evaporation and purging with nitrogen under stirring a few times. $(Ph_3P)_2PdCl_2$ (0.021 mmol), CuI (0.041 mmol), and $Et_3N$ (0.82mmol) are added and the reaction is heated under nitrogen at 80˚C for 9 h. After the mixture is cooled to rt, the mixture is extracted twice with AcOEt, and the combined organic layer are washed with brine several times, dried over Na2SO4, and concentrated under reduced pressure. Flush chromatography on silica gel provides a solid. The solid is dissolved in 3ml of DMF. To the solution is added DBU (60ml) and then heated at 100˚C for 1.5h. After the mixture is cooled to rt., the mixture is concentrated under reduced pressure. Flush chromatography on silica gel using gives the title compound as a yellow solid; Rf 0.13 (AcOEt); $^1$H-HMR (400 MHz, CDCl3) δ : 1.14-1.16(m, 2H), 1.49-1.65 (m, 4H), 1.78-1.88(m, 5H), 2.14(s, 3H), 3.04-3.10 (m, 4H), 3.62(t, 2H), 3.77(t, 2H), 4.39 (t, 2H), 5.20(s, 2H), 6.70(s, 1H), 6.74(d, 2H), 7.03(d, 2H), 8.95(s, 1H).

Examples 99 to 103

By repeating the procedures described in Example 98 using appropriate starting materials (including some of those prepared in Examples A to G) and conditions the following compounds of formula 2 are obtained as identified below in Table 7.

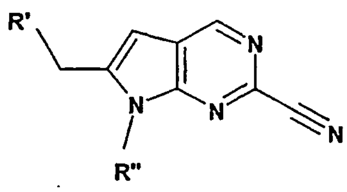

(2)

Table 7

| Ex. | R' | R" | $R_f$ (solvent) | NMR (400MHz, CDCl3, δ) |
|---|---|---|---|---|
| 99 | 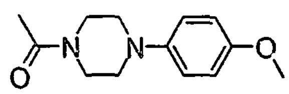 | 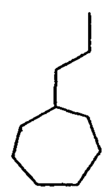 | 0.16 (AcOEt) | 0.97-1.09(m, 2H), 1.29-1.38 (m, 4H), 1.42-1.81 (m, 4H), 1.64-1.75(m, 3H), 1.80-1.90 (m, 2H), 2.14(s, 3H), 3.04-3.10(m, 4H), 3.62(t, 2H), 3.77 (t, 2H), 4.39(t, 2H), 5.20(s, 2H), 6.70(s, 1H), 6.70(d, 2H), 6.92(d, 2H), 8.97(s, 1H) |
| 100 | 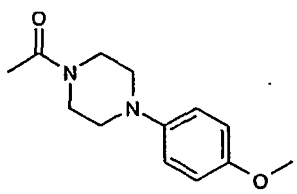 | 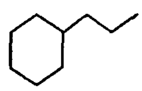 | 0.55 ($CH_2Cl_2$: MeOH=$_9$ :1) | 0.96-1.02(m, 2H), 1.14-1.36 (m, 4H), 1.65-1.79(m, 7H), 2.14(s, 3H), 3.04-3.10(m, 4H), 3.62(dd, 2H), 3.77(dd, 2H), 4.38(dd, 2H), 5.19(s, 2H), 6.69 (s, 1H), 6.92(s, 4H), 8.95(s, 1H) |
| 101 | 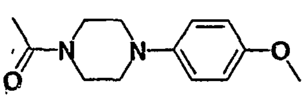 | 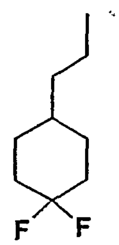 | 0.13 (AcOEt) | 1.32-1.44(m, 3H), 1.59-1.75 (m, 2H), 1.80-1.88(m, 4H), 2.06 -2.09(m, 2H), 2.14(s, 3H), 3.04-3.10(m, 4H), 3.62(t, 2H), 3.77(t, 2H), 4.38-4.42(m, 2H), 5.19(s, 2H), 6.71 (s, 1H), 6.92 (s, 4H), 8.97(s, 1H) |
| 102 | 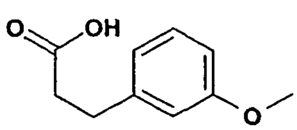 | 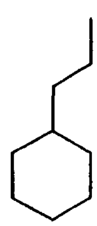 | 0.40 (AcOEt) | 0.91-1.02 (m, 2H), 1.12-1.39 (m, 3H), 1.61-1.82 (m, 8H), 2.69 (t, 2H), 2.96 (t, 2H), 4.39(t, 2H), 5.23 (s, 2H), 6.72 (s, 1H), 6.84 (d, 1H), 6.89 (s, 1H), 6.90 (d, 1H), 7.26 (t, 1H), 8.96 (s, 1H) |
| 103 | 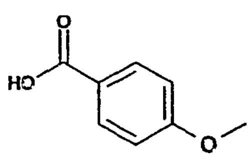 | 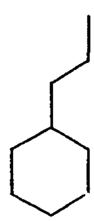 | 0.10 ($CH_2Cl_2$: MeOH = 8:2) | (DMSO-d6); 0.90-0.96 (m, 2H), 1.12-1.26 (m, 4H), 1.61-1.75 (m, 7H), 4.34-4.38 (m, 2H), 5.55(s, 2H), 7.04(s, 1H), 7.19 (d, 2H), 7.93(d, 2H), 9.18 (s, 1H) |

Example 104: 4-[2-Cyano-7-(2-cyclohexyl-ethyl)-7H-pyrrolo[2,3-d]pyrimidin-6-ylmethoxy]-N-(2,2,2-trifluoro-ethyl)-benzamide

To the solution of 4-[2-cyano-7-(2-cyclohexyl-ethyl)-7H-pyrrolo[2,3-d]pyrimidin -6-ylmethoxy]-benzoic acid (51 mg, 0.13 mmol) and 2,2,2-trifluoroethylamine (25 mg, 0.25 mmol) in DMF (3 ml), HOAt (26 mg, 0.19 mmol) and WSCl.HCl (36 mg, 0.19 mmol) are added at 0°C. The reaction mixture is stirred at rt for 15 h and quenched with saturated ammonium chloride and extracted with ethyl acetate. The combined extracts are washed with $H_2O$, brine and dried over magnesium sulfate. The crude product is purified by reverse phase HPLC and fraction are collected and evaporated down. Saturated sodium bicarbonate is added and neutralized and the water phase is extracted with ethyl acetate. The combined extracts are washed with brine, dried over magnesium sulfate and evaporated down to give the desired product; Rf=0.76 (*n*-hexane : ethyl acetate = 1:2); $^1$H-NMR (400MHz, $CDCl_3$) δ : 0.94-0.99 (m, 2H), 1.11-1.39 (m, 4H), 1.61-1.84 (m, 7H), 4.09-4.17 (m, 2H), 4.37-4.40 (m, 2H), 5.39 (s, 2H), 6.27-6.30 (br, 1H), 6.76 (s, 1H), 7.07 (d, 2H), 7.82 (d, 2H), 8.97 (s, 1H).

Examples 105 to 106

By repeating the procedures described in Example 104 using appropriate starting materials (including some of those prepared in Examples A to G) and conditions the following compounds of formula 2 are obtained as identified below in Table 8.

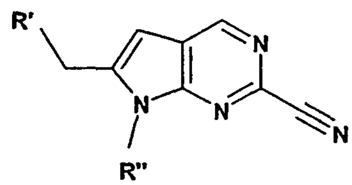

(2)

Table 8

| Ex. | R' | R" | Rf (solvent) | NMR (400MHz, CDCl3, δ) |
|---|---|---|---|---|
| 105 | 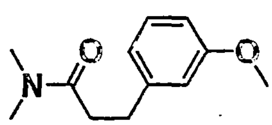 | 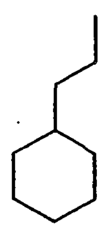 | 0.12 (AcOEt) | 0.92-1.04(m, 2H), 1.10-1.39 (m, 4H), 1.52-1.71(m, 9H), 2.61 (t, 2H), 2.95(s, 6H), 2.97(t, 2H), 4.83(t, 2H), 5.23(s, 2H), 6.73(s, 1H), 6.83(dd, 1H), 6.89 (s, 1H), 6.90(dd, 1H), 7.26(t, 1H), 8.97(s, 1H) |
| 106 | 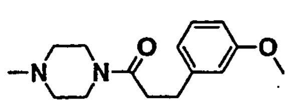 | 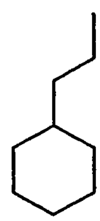 | 0.04 (AcOEt) | 0.91-1.02(m, 2H), 1.11-1.38 (m, 3H), 1.51-1.81 (m, 8H), 2.61 (t, 2H), 2.28(s, 3H), 2.30(t, 2H), 2.35(t, 2H), 2.62(t, 2H), 2.97(t, 2H), 3.41 (t, 2H), 3.63(t, 2H), 4.39(t, 2H), 5.22 (s, 2H), 6.73(s, 1H), 6.83(dd, 1H), 6.87 (s, 1H), 6.90(dd, 1H), 7.26(t, 1H), 8.98(s, 1H) |

Example 107A: {4-[2-cyano-7-(2-cyclohexyl-ethyl)-7H-pyrrolo[2,3-d]pyrimidin-6-yl-methoxy]-phenyl}-carbamic acid tert-butyl ester

6-Chloromethyl-7-(2-cyclohexyl-ethyl)-7H-pyrrolo[2,3-d]pyrimidine-2-carbonitrile and (4-hydroxy-phenyl)-carbamic acid tert-butyl ester are reacted by the procedure described in Example 104 in order to give the title compound; $R_f$ = 0.16 (*n*-hexane:AcOEt = 3:1). NMR (400MHz, $CDCl_3$, δ) 0.92-1.05 (m, 2H), 1.15-1.40 (m, 4H), 1.51 (s, 9H), 1.60-1.84 (m, 7H), 4.38 (t, 2H), 5.30 (s, 2H), 6.38 (br s, 2H), 6.70 (s, 2H), 6.92 (d, 2H), 7.31 (d, 2H), 8.95 (s, 1H).

Examples 107B: N-{4-[2-Cyano-7-(2-cyclohexyl-ethyl)-7H-pyrrolo[2,3-d]pyrimidin-6-ylmethoxy]-phenyl}-propionamide

The compound of Example 107A is treated with TFA in methylene chloride providing the amine 6-(4-amino-phenoxymethyl)-7-(2-cyclohexyl-ethyl)-7H-pyrrolo[2,3-d]pyrimidine-2-carbonitrile. To a solution of said amine (0.18 mmol) in methylene chloride (10 ml) are added propionyl chloride (0.62 mmol) and triethylamine (0.97 mmol) dropwise at 0 ˚C. The mixture is stirred at rt for 4h. The reaction mixture is diluted with water and extracted with AcOEt. The organic layer is washed with water and brine, dried over $MgSO_4$, and concentrated in vacuo. The residue is purified by HPLC with reverse phase column (0.1%TFA in $H_2O$ and 0.1%TFA in MeCN) to give the title compound; Rf (*n*-hexane: AcOEt=1:1): 0.15; $^1$H-HMR (400 MHz) δ: 0.92-1.04 (m, 2H), 1.11-1.40 (m, 7H), 1.62-1.81 (m, 7H), 2.38 (q, 2H), 4.38 (t, 2H), 5.21 (s, 2H), 6.71 (s, 2H), 6.94 (d, 2H), 7.05 (br s, 1H), 7.47 (d, 2H), 8.86 (s, 1H).

Examples 108 and 109

By repeating the procedures described in Example 107A and 107B using appropriate starting materials (including some of those prepared in Examples A to G) and conditions the following compounds of formula 2 are obtained as identified below in Table 9.

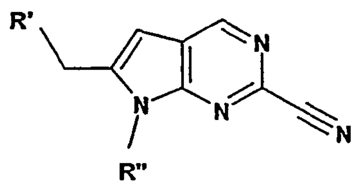

(2)

Table 9

| Ex. | R' | R" | Rf (solvent) | NMR (400MHz, $CDCl_3$, δ) |
|---|---|---|---|---|
| 108 | 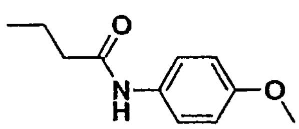 | 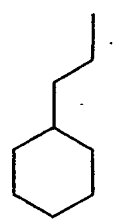 | 0.31 (*n*-hexane: AcOEt = 1:1) | 0.92-1.05 (m, 5H), 1.12-1.42 (m, 7H), 1.60-1.85 (m, 9H), 2.33 (t, 2H), 4.40 (t, 2H), 5.21 (s, 2H), 6.71 (s, 2H), 6.94 (d, 2H), 7.03 (br s, 1H), 7.47 (d, 2H), 8.95 (s, 1H) |
| 109 | 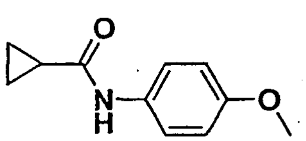 | 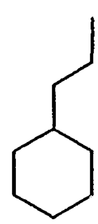 | 0.26 (*n*-hexane: AcOEt = 1:1) | 0.81-0.89 (m, 2H), 0.91-1.04 (m, 2H), 1.07-1.21 (m, 2H), 1.15-1.40 (m, 4H), 1.45-1.55 (m, 1H), 1.62-1.82 (m, 7H), 4.38 (t, 2H), 5.21 (s, 2H), 6.70 (s, 1H), 6.94 (d, 2H), 7.25 (br s, 1H), 7.47 (d, 2H), 8.95 (s, 1H) |

Example 110: 7-[2-(4-Chloro-phenyl)-ethyl]-6-(3-fluoro-4-nitro-phenoxymethyl)-7H-pyrrolo[2,3-d]pyrimidine-2-carbonitrile

6-Bromomethyl-7-[2-(4-chloro-phenyl)-ethyl]-7H-pyrrolo[2,3-d]pyrimidine-2-carbonitrile and 3-fluoro-4-nitrophenol are reacted by the same procedures described in Example 104 to give the title compound; Rf 0.43 (*n*-hexane: AcOEt=1:1); $^1$H-HMR (400 MHz) δ: 3.16 (t, 2H), 4.56 (t, 2H), 4.84 (s, 2H), 6.71 (s, 1H), 6.74 (s, 1H), 6.76 (s, 1H), 6.91 (d, 2H), 7.22 (d, 2H), 8.14 (t, 1H), 9.03 (s, 1H).

Example 111: N-(4-{7-[2-(4-Chloro-phenyl)-ethyl]-2-cyano-7H-pyrrolo[2,3-d]pyrimidin-6-ylmethoxy}-2-fluoro-phenyl)-acetamide

The compound of Example 110 is reduced by hydrogenation over 10% Pd-C under hydrogen atmosphere to the amine 7-[2-(4-chloro-phenyl)-ethyl]-6-(3-fluoro-4-amino-phenoxymethyl)-7H-pyrrolo[2,3-d] pyrimidine-2-carbonitrile. Said amine is acylated with acetyl chloride by the same procedures described above to give the title compound; Rf 0.14 (*n*-hexane: AcOEt = 1:1); $^1$H-HMR ($CDCl_3$, 400 MHz) δ: 2.22 (s, 3H), 3.15 (t, 2H), 4.56 (t, 2H), 4.83 (s, 2H), 6.65-6.71 (m, 3H), 6.95 (d, 2H), 7.18 (br s, 1H), 7.21 (d, 2H), 8.18 (t, 1H), 8.99 (s, 1H).

Examples 112 to 119

By repeating the procedures described in Example 110 and 111 using appropriate starting materials (including some of those prepared in Examples A to G) and conditions the following compounds of formula 2 are obtained as identified below in Table 10.

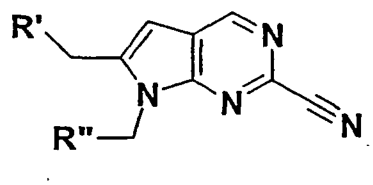

(2)

Table 10

| Ex. | R' | R" | Rf (solvent) | NMR(400MHz, $CDCl_3$, δ) |
|---|---|---|---|---|
| 112 | 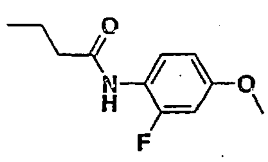 | 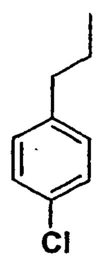 | 0.34 (*n*-hexane: AcOEt = 1:1) | 1.03 (t, 3H), 1.71-1.84 (m, 2H), 2.38 (t, 2H), 3.15 (t, 2H), 4.56 (t, 2H), 4.83 (s, 2H), 6.65-6.71 (m, 3H), 6.94 (d, 2H), 7.15 (br s, 1H), 7.21 (d, 2H), 8.23 (t, 1H), 8.99 (s, 1H) |
| 113 | 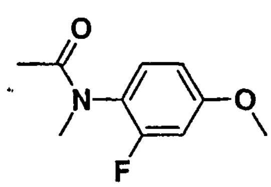 | 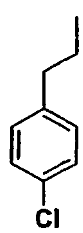 | 0.26 (*n*-hexane: AcOEt = 1:2) | 1.87 (s, 3H), 3.17 (t, 2H), 3.20 (s, 3H), 4.57 (t, 2H), 4.84 (s, 2H), 6.68-6.75 (m, 3H), 6.95 (d, 2H), 7.20 (t, 1H), 7.24 (d, 2H), 9.01 (s, 1H) |
| 114 | 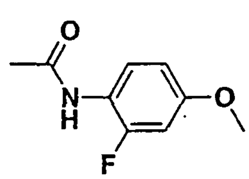 | 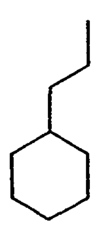 | 0.22 (*n*-hexane: AcOEt = 1:1) | 0.92-1.04 (m, 2H), 1.10-1.40 (m, 4H), 1.60-1.82 (m, 7H), 2.21 (s, 3H), 4.37 (t, 2H), 5.20 (s, 2H), 6.72 (s, 2H), 6.73-6.80 (m, 2H), 7.18 (br s, 1H), 8.18 (t, 1H), 8.97 (s, 1H) |

(continued)

| Ex. | R' | R" | Rf (solvent) | NMR(400MHz, $CDCl_3$, δ) |
|---|---|---|---|---|
| 115 | 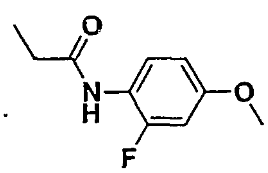 | 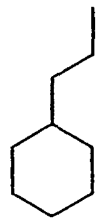 | 0.22 (*n*-hexane: AcOEt = 1:1) | 0.91-1.03 (m, 2H), 1.12-1.40 (m, 3H), 1.65-1.81 (m, 7H), 2.43 (q, 2H), 4.37 (q, 2H), 5.20 (s, 2H), 6.71 (s, 2H), 6.72-6.82 (m, 2H), 7.17 (br s, 1H), 8.23 (t, 1H), 8.97 (s, 1H) |
| 116 | 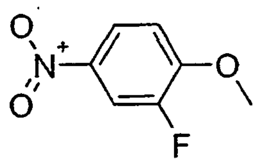 | 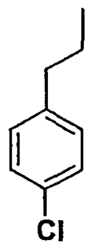 | 0.29 (*n*-hexane: AcOEt = 1:1) | 3.19 (t, 2H), 4.60 (t, 2H), 4.98 (s, 2H), 6.72 (s, 1H), 6.96 (d, 2H), 7.02 (t, 1H), 7.21 (d, 2H), 8.02-8.11 (m, 2H), 9.02 (s, 1H) |
| 117 | 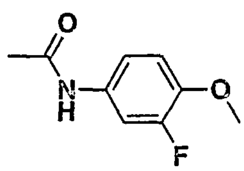 | 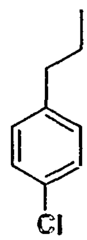 | 0.20 (*n*-hexane: AcOEt = 1:2) | 2.17 (s, 3H), 3.18 (t, 2H), 4.62 (t, 2H), 4.92 (s, 2H), 6.63 (s, 1H), 6.89 (t, 1H), 7.00 (d, 2H), 7.05-7.12 (m, 2H), 7.22 (dd, 2H), 7.48-7.54 (m, 1H), 9.00 (s, 1H) |
| 118 | 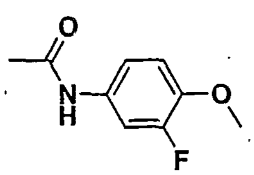 | 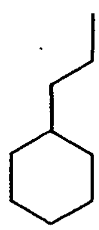 | 0.11 (*n*-hexane: AcOEt = 1:1) | 0.93-1.05 (m, 2H), 1.15-1.43 (m, 4H), 1.61-1.85 (m, 7H), 2.17 (s, 3H), 4.43 (t, 2H), 5.26 (s, 2H), 6.68 (s, 2H), 6.98 (t, 1H), 7.08-7.12 (m, 2H), 7.51 (dd, 1H), 8.95 (s, 1H) |
| 119 | 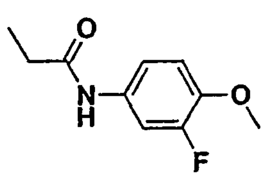 | 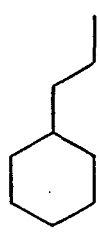 | 0.21 (*n*-hexane: AcOEt = 1:1) | 0.92-1.04 (m, 2H), 1.12-1.45 (m, 4H), 1.61-1.84 (m, 7H), 2.38 (q, 2H), 4.43 (t, 2H), 5.26 (s, 2H), 6.68 (s, 2H), 6.98 (t, 1H), 7.05-7.14 (m, 2H), 7.54 (dd, 1H), 8.95 (s, 1H) |

Example 120: 7-[2-(4-Chloro-phenyl)-ethyl]-6-(2-fluoro-4-propylaminomethylphenoxymethyl)-7H-pyrrolo[2,3-d]pyrimidine-2-carbonitrile

6-(4-Chloromethyl-2-fluoro-phenoxymethyl)-7-[2-(4-chloro-phenyl)-ethyl]-7H-pyrrolo[2,3-d]pyrimidine-2-carbonitrile and propylamine are treated with potassium carbonate in DMF to give 7-[2-(4-chloro-phenyl)-ethyl]-6-(2-fluoro-4-propylaminomethyl-phenoxymethyl)-7H-pyrrolo[2,3-d]pyrimidine-2-carbonitrile; Rf 0.10 (*n*-hexane: AcOEt = 1:2); $^1$H-HMR ($CDCl_3$, 400 MHz) δ: 0.92 (t, 3H), 1.49-1.59 (m, 2H), 2.62 (t, 2H), 3.18 (t, 2H), 3.77 (s, 2H), 4.61 (t, 2H), 4.95 (s, 2H), 6.65 (s, 1H), 6.91 (t, 1H), 6.98-7.08 (m, 3H), 7.11-7.21 (m, 3H), 8.97 (s, 1H).

Examples 121 to 130

By repeating the procedures described in Example 120 using appropriate starting materials (including some

of those prepared in Examples A to G) and conditions the following compounds of formula 2 are obtained as identified below in Table 11.

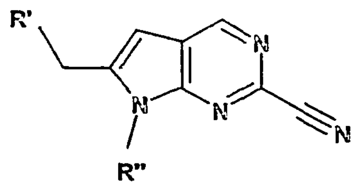

(2)

Table 11

| Ex. | R' | R" | Rf (solvent) | NMR(400MHz, $CDCl_3$, δ) |
|---|---|---|---|---|
| 121 | 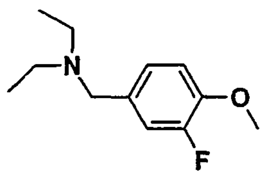 | 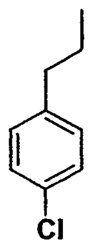 | 0.11 ($CH_2Cl_2$: MeOH= 95:5) | 1.06 (br s, 6H), 2.55 (br s, 4H), 3.19 (t, 2H), 3.53 (br, 2H), 4.62 (t, 2H), 4.96 (s, 2H), 6.66 (s, 1H), 6.89 (t, 1H), 7.01 (d, 2H), 7.02-7.08 (br, 1H), 7.13-7.22 (m, 3H), 8.97 (s, 1H) |
| 122 | 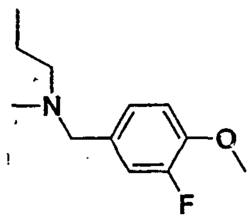 | 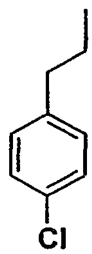 | 0.13 (*n*-hexane: AcOEt = 1:2) | 0.91 (t, 3H), 1.48-1.61 (br, 2H), 2.20 (br s, 3H), 2.35 (br s, 2H), 3.19 (t, 2H), 3.44 (br s, 2H), 4.61 (t, 2H), 4.96 (s, 2H), 6.66 (s, 1H), 6.90 (t, 1H), 7.03-7.08 (m, 3H), 7.12 (d, 1H), 7.21 (d, 2H), 8.97 (s, 1H) |
| 123 | 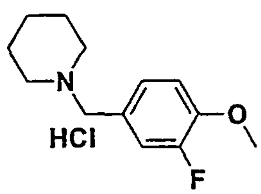 | 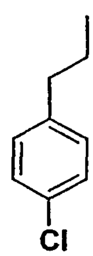 | 0.09 (*n*-hexane: AcOEt = 1:1) | DMSO-d6; 1.22-1.41 (m, 2H), 1.60-1.83 (m, 4H), 2.75-2.88(m, 2H), 3.13 (t, 2H), 3.22-3.37(m, 2H), 4.21 (s, 2H), 4.60(t, 2H), 5.48(s, 2H), 7.01(s, 1H), 7.08 (d, 2H), 7.24 (d, 2H), 7.37(d, 1H), 7.46(t, 1H), 7.53(d, 1H), 9.16(s, 1H), 9.96(s, 1H) |
| 124 | 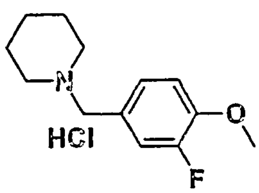 | 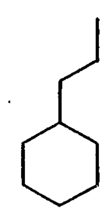 | 0.08 (*n*-hexane: AcOEt = 1:1) | 0.91-1.97(m, 17H), 2.12-2.38 (m, 2H), 2.51-2.65(m, 2H), 3.38-3.52(m, 2H), 4.04(s, 2H), 4.41 (t, 2H), 5.33(s, 2H), 6.75(s, 1H), 7.10-7.21 (m, 1H), 7.41 (d, 1H), 7.58-7.72 (m, 1H), 8.99(s, 1H), 12.41 (s, 1H) |

(continued)

| Ex. | R' | R" | Rf (solvent) | NMR(400MHz, $CDCl_3$, δ) |
|---|---|---|---|---|
| 125 | 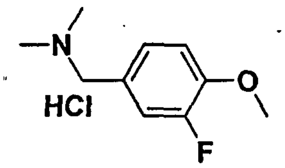 | 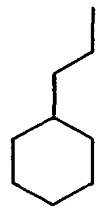 | 0.06 (*n*-hexane: AcOEt = 1:1) | 0.91-1.07(m, 2H), 1.12-1.41 (m, 4H), 1.57-1.85(m, 7H), 2.76(s, 6H), 4.08(s, 2H), 4.41 (t, 2H), 5.33(s, 2H), 6.75(s, 1H), 7.19(t, 1H), 7.38(d, 1H), 7.60(d, 1H), 8.89(s, 1H), 13.00(s, 1H) |
| 126 | 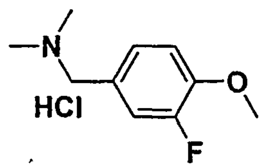 | 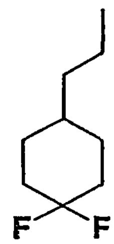 | 0.09 Salt free (n-hexane: AcOEt = 1:1 | 1.18-1.63(m, 9H), 2.01-2.16 (m, 2H), 2.76(s, 6H), 4.09(s, 2H), 4.42(t, 2H), 5.33(s, 2H), 6.78(s, 1H), 7.21 (t, 1H), 7.38 (d, 1H), 7.63(d, 1H), 9.00(s, 1H), 12.97 (s, 1H) |
| 127 | 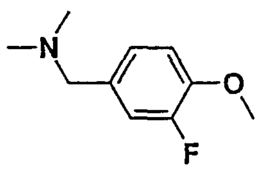 | 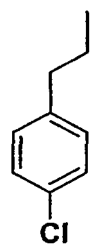 | MS (M+H) 464.3 | DMSO-d6 2.74(s, 6H), 3.19(t, 2H), 4.27 (s, 2H), 4.67(t, 2H), 5.54(s, 2H), 7.07(s, 1H), 7.15(d, 2H), 7.30(d, 2H), 7.42(d, 1H), 7.51 (d, 1H), 7.57(d, 1H), 9.22(s, 1H) |
| 128 | 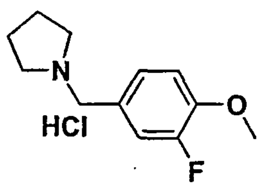 | 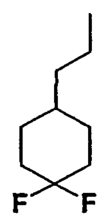 | MS (M+H) 498.3 | 1.22-1.55(m, 4H), 1.57-2.13 (m, 9H), 2.18-2.32(m, 2H), 2.76-2.89(m, 2H), 3.59-3.75 (m, 2H), 4.13(s, 2H), 4.43(t, 2H), 5.33(s, 2H), 6.78(s, 1H), 7.16-7.25(m, 1H), 7.39(d, 1H), 7.62-7.72(m, 1H), 9.01 (s, 1H), 12.74(s, 1H) |
| 129 | 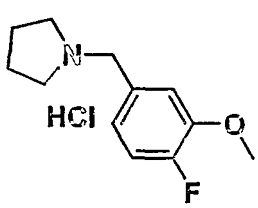 | 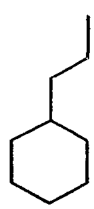 | MS (M+H) 462.2 | 0.91-1.06(m, 2H), 1.08-1.41 (m, 6H), 1.59-2.36(m, 9H), 2.77-2.89(m, 2H), 3.51-3.72 (m, 2H), 4.15(s, 2H), 4.43(t, 2H), 5.58(s, 2H), 6.85(s, 1H), 7.07(d, 1H), 7.10(s, 1H), 8.34 (d, 1H), 8.96(s, 1H), 12.71 (s, 1H) |
| 130 | 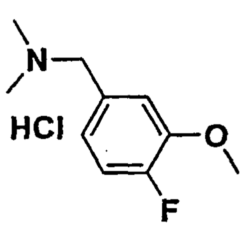 | 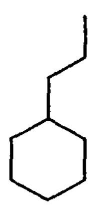 | 0.04 Salt free (n-hexane: AcOEt = 1:1 | 0.92-1.42(m, 5H), 1.59-1.85 (m, 8H), 2.79(m, 6H), 4.15(s, 2H), 4.43(t, 2H), 5.58(s, 2H), 6.85(s, 1H), 7.06-7.14(m, 2H), 8.29(d, 1H), 8.98(s, 1H), 12.76(s, 1H) |

Example 131: 7-(2-Cyclohexyl-ethyl)-6-[4-(4-propionyl-piperazin-1-yl)-phenoxymethyl]-7H-pyrrolo[2,3-d]pyrimidine-2-carbonitrile

6-Chloromethyl-7-(2-cyclohexyl-ethyl)-7H-pyrrolo[2,3-d]pyrimidine-2-carbonitrile and 4-(4-hydroxy-phenyl)-piperazine-1-carboxylic acid tert-butyl ester are reacted by the procedure described in Example 107A to give 4-{4-[2-cyano-7-(2-cyclohexyl-ethyl)-7H-pyrrolo[2,3-d]pyrimidin-6-ylmethoxy]-phenyl}-piperazine-1-carboxylic acid tert-butyl ester. Such ester is treated with TFA for deprotection of the Boc group and the deprotected piperazine derivative is acylated with propionyl chloride according to the same procedures described in Example 107B to furnish the title compound; $^1$H-HMR ($CDCl_3$, 400 MHz) δ: 0.58 ($CH_2Cl_2$:MeOH=9:1); 0.93-1.03(m, 2H), 1.15-1.43(m, 7H), 1.59-1.81 (m, 7H), 2.39(q, 2H), 3.07(brs, 4H), 3.62(brt, 2H), 3.78(brt, 2H), 4.39(t, 2H), 5.19(s, 2H), 6.69(s, 1H), 6.92(s, 4H), 8.95(s, 1H).

Examples 132 to 139

By repeating the procedures described in Example 131 using appropriate starting materials (including some of those prepared in Examples A to G) and conditions the following compounds of formula 2 are obtained as identified below in Table 12.

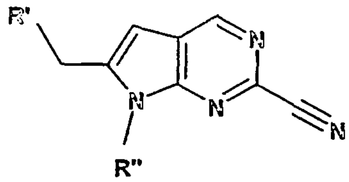

(2)

Table 12

| (R" represents cyclohexylethyl) | | | |
|---|---|---|---|
| Ex. | R' | Rf (solvent) | NMR(400MHz, $CDCl_3$, δ) |
| 132 | 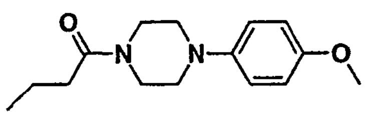 | 0.75 ($CH_2Cl_2$: MeOH = 9:1) | 0.93-1.05(m, 5H), 1.12-1.39(m, 7H), 1.61-1.82(m, 9H), 2.34(t, 2H), 3.07 (brs, 4H), 3.63(brs, 2H), 3.78(brs, 2H), 4.39(t, 2H), 5.19(s, 2H), 6.69(s, 1H), 6.92 (s, 4H), 8.95(s, 1H) |
| 133 | 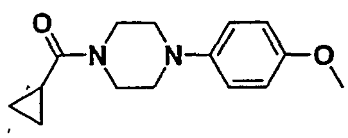 | 0.57 ($CH_2Cl_2$: MeOH = 9:1) | 0.77-0.85(m, 2H), 0.92-1.05(m, 4H), 1.11-1.40(m, 4H), 1.65-1.82(m, 8H), 3.10(brd, 4H), 3.83 (brs, 4H), 4.39(t, 2H), 5.19(s, 2H), 6.69(s, 1H), 6.93(s, 4H), 8.95(s, 1H) |
| 134 | 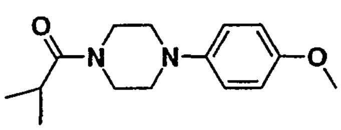 | 0.50 ($CH_2Cl_2$: MeOH= 9:1) | 0.89-1.02(m, 2H), 1.12-1.42(m, 10H), 1.60-1.85(m, 7H), 2.79-2.89(m, 1H), 3.07(brs, 4H), 3.68 (brs, 2H), 3.79(brs, 2H), 4.40 (t, 2H), 5.19(s, 2H), 6.69(s, 1H), 6.94(s, 4H), 8.95(s, 1H) |
| 135 | 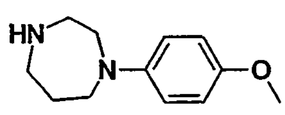 | 0.16 ($CH_2Cl_2$: MeOH = 9:1) | 0.92-1.05(m, 2H), 1.12-1.40(m, 4H), 1.60-1.84(m, 8H), 1.89-1.95 (m, 2H), 2.87(t, 2H), 3.05(t, 2H), 3.52-3.58(m, 4H), 4.40(t, 2H), 5.15(s, 2H), 6.65(d, 2H), 6.66(s, 1H), 6.87(d, 2H), 8.94(s, 1H) |

(continued)

| (R" represents cyclohexylethyl) | | | |
|---|---|---|---|
| Ex. | R' | Rf (solvent) | NMR(400MHz, $CDCl_3$, δ) |
| 136 | 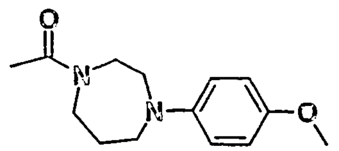 | 0.57 ($CH_2Cl_2$: MeOH = 9:1) | 0.93-1.06(m, 2H), 1.11-1.41(m, 4H), 1.60-1.35(m, 7H), 1.95-2.05(m, 2H), 3.39(t, 1H), 3.45-3.62(m, 4H), 3.76(t, 1H), 4.40 (t, 2H), 5.16 (s, 2H), 6.65-6.69 (m, 3H), 6.96 (d, 2H), 8.94 (s, 1H) |
| 137 | 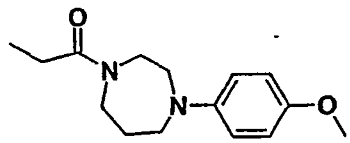 | 0.76 ($CH_2Cl_2$: MeOH = 9:1) | 0.92-1.40(m, 9H), 1.60-1.83(m, 7H), 1.92-2.01(m, 2H), 2.23(q, 1H), 2.34(q, 1H), 3.38(t, 1H), 3.45 -3.63(m, 6H), 3.79(t, 1H), 4.40(t, 2H), 5.15(s, 2H), 6.63-6.70(m, 3H), 6.89(d, 2H), 8.94 (s, 1H) |
| 138 | 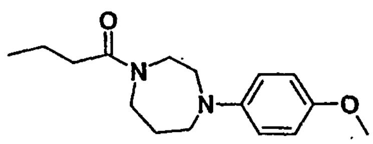 HCl salt | 0.71 (*n*-hexane: AcOEt = 9:1) | DMSO-$d_6$: 0.70-2.28 (m, 22H), 3.45-3.65 (m, 8H), 4.35 (t, 2H), 5.32 (s, 2H), 6.68-7.01 (m, 6H), 9.16 (s, 1H) |
| 139 | 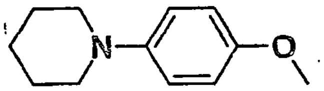 HCl salt | 0.55 (*n*-hexane: AcOEt = 1:1) | 0.89-1.07 (m, 2H), 1.11-1.40 (m, 4H), 1.58-2.15 (m, 13H), 3.40-3.65 (m, 4H), 4.41 (t, 2H), 5.56 (br s, 2H), 7.08 (s, 1H), 7.30 (br s, 2H), 7.70-7.95 (br, 2H), 9.23 (s, 1H), 12.00-12.20 (br, 1H) |

Examples 140 to 147

By repeating the procedures described in Example 131 and 107 B (for removal of the boc group) using appropriate starting materials (including some of those prepared in Examples H to K) the following compounds of formula 2 are obtained as identified below in Table 13.

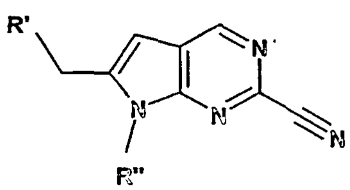

(2)

Table 13

| Ex. | R' | R" | Rf (solvent) | NMR(400MHz, δ) |
|---|---|---|---|---|
| 140 | 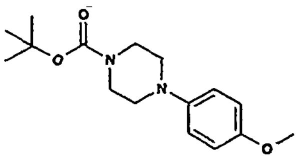 | 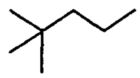 | 0.41 (n-hexane: AcOEt = 1:1) | CDCl3 1.01(s, 9H), 1.48(s, 9H), 1.71-1.76(m, 2H), 3.04 (brs, 4H), 3.58(brs, 4H), 4.36-4.41 (m, 2H), 5.19(s, 2H), 6.69(s, 1H), 6.92(brs, 4H), 8.95(s, 1H) |
| 141 | 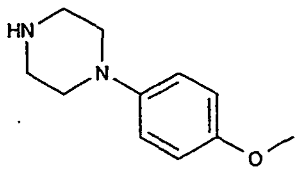 HCl | 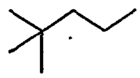 | 0.17 Salt Free (n-hexane: AcOEt = 1:1) | DMSO-d6 0.95(s, 9H), 1.65-1.70(m, 2H), 3.21-3.25(m, 8H), 4.32-4.36 (m, 2H), 5.38(s, 2H), 6.96-7.03(m, 4H), 9.08(brs, 2H), 9.15(s, 1H) |
| 142 | 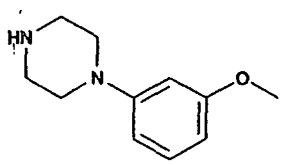 HCl | 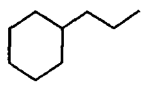 | 0.17 Salt Free (n-hexane: AcOEt = 1:1) | DMSO-d6 0.86-0.95(m, 2H), 1.08-1.28 (m, 4H), 1.59-1.74(m, 7H), 3.18 (brs, 4H), 3.36-3.39(m, 4H), 4.33-4.37(m, 2H), 5.42(s, 2H), 6.59-5.66(m, 3H), 6.99(s, 1H), 7.19(dd, 1H), 9.16(s, 1H), 9.24(brs, 2H) |
| 143 | 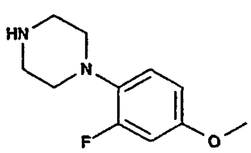 HCl | 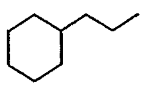 | ($CH_2Cl_2$: MeOH = 9:1) | DMSO-d6 0.88-0.97(m, 2H), 1.08-1.29 (m, 4H), 1.63-1.76(m, 7H), 3.13-3.16(m, 4H), 3.22 (brs, 4H), 4.33-4.37(m, 2H), 5.44(s, 2H), 6.88-6.91 (m, 1H), 7.01(s, 1H), 7.05-7.12(m, 2H), 9.12-9.18(m, 3H) |
| 144 | 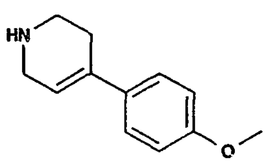 HCl | 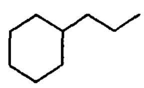 | ($CH_2Cl_2$: MeOH = 9:1) | DMSO-d6 0.87-0.95(m, 2H), 1.08-1.30 (m, 4H), 1.60-1.68(m, 7H), 2.64 (brs, 2H), 3.28-3.29(m, 2H), 3.71(brs, 2H), 4.33-4.37 (m, 2H), 5.47(s, 2H), 6.10(brs, 1H), 7.00(s, 1H), 7.10(d, 2H), 7.45(d, 2H), 9.10-9.16(m, 3H) |

(continued)

| Ex. | R' | R" | Rf (solvent) | NMR(400MHz, δ) |
|---|---|---|---|---|
| 145 | 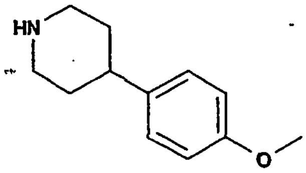 HCl | 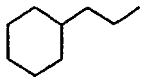 | ($CH_2Cl_2$: MeOH = 9:1) | DMSO-d6 1.28-1.37(m, 1H), 1.68-1.79 (m, 5H), 4.46-4.72(m, 4H), 3.35-3.38(m, 2H), 4.46-4.47 (m, 2H), 4.70 (dd, 2H), 7.03(d, 2H), 7.20 (d, 2H), 7.28(s, 1H), 9.20 (s, 1H), 10.96(brs, 1H) |
| 146 | 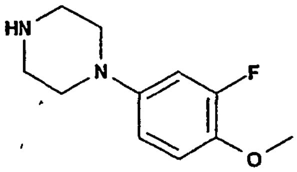 HCl | 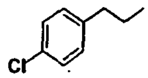 | ($CH_2Cl_2$: MeOH = 9:1) | DMSO-d6 3.11-3.19(m, 6H), 3.29-3.32 (m, 4H), 4.60 (dd, 2H), 5.31 (s, 2H), 6.74-6.77(m, 1H), 6.93-6.99(m, 2H), 7.09(d, 2H), 7.19 -7.26(m, 3H), 9.13-9.18(m, 3H) |
| 147 | 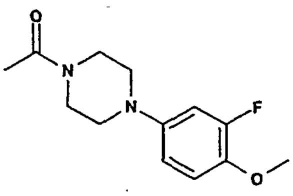 | 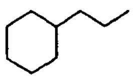 | 0.06 (n-hexane; EtOAc = 1:1) | CDCl3 0.94-1.04(m, 2H), 1.14-1.40 (m, 4H), 1.62-1.83(m, 7H), 2.13 (s, 3H), 3.06-3.12(m, 4H), 3.61 (dd, 2H), 3.76(dd, 2H), 4.42-4.46(m, 2H), 5.22(s, 2H), 6.59-6.62(m, 1H), 6.66(s, 1H), 6.69-6.73 (m, 1H), 6.95(dd, 1H), 8.94 (s, 1H) |

Example 148: 6-[4-(4-Acetyl-piperazin-1-yl)-2-fluoro-phenoxymethyl]-7-[2-(4-chloro-phenyl)-ethyl]-7H-pyrrolo[2,3-d] pyrimidine-2-carbonitrile.

To a solution of 7-[2-(4-Chloro-phenyl)-ethyl]-6-(2-fluoro-4-piperazin-1-yl-phenoxymethyl)-7H-pyrrolo[2,3-d] pyrimidine-2-carbonitrile (80mg) in CH2Cl2 is added Et3N (55uL), acetyl chloride (11.3uL) at 0˚C, and stirred for 2 h at rt. The reaction mixture is concentrated in vacuo. The crude product is purified column chromatography to give the product; Rf= 0.27 (dichloromethane:methanol=10:1); $^1$H NMR(CDCl3, δ(ppm)): 2.14 (s, 3H), 3.06-3.12(m, 4H), 3.18(dd, 2H), 3.61 (dd, 2H), 3.76(dd, 2H), 4.63(dd, 2H), 4.90(s, 2H), 6.58-6.62(m, 2H), 6.70(dd, 1H), 6.87(t, 1H), 6.99-7.02(m, 2H), 7.19-7.22(m, 2H), 8.96(s, 1H).

Examples 149 to 156

By repeating the procedures described in Example 148 using appropriate starting materials (including some of those prepared in Examples A to K) the following compounds of formula 2 are obtained as identified below in Table 14.

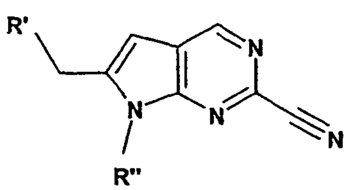

(2)

Table 14

| Ex. | R' | R" | Rf (solvent) | NMR(400MHz, δ) |
|---|---|---|---|---|
| 149 | 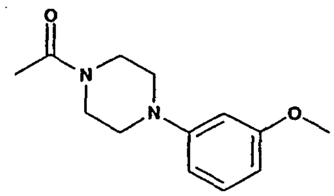 HCl | 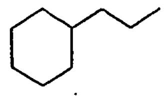 | 0.74 Salt Free ($CH_2Cl_2$: MeOHI= 9:1) | DMSO-d6 0.83-0.91 (m, 2H), 1.02-1.23 (m, 4H), 1.56-1.70(m, 7H), 1.98 (s, 3H), 3.06-3.09(m, 2H), 3.13-3.15(m, 2H), 3.53(brs, 4H), 4.28-4.32(m, 2H), 5.37 (s, 2H), 6.54-6.63(m, 3H), 6.94(s, 1H), 7.13 (dd, 1H), 9.11 (s, 1H) |
| 150 | 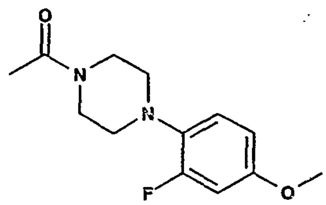 HCl | 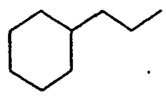 | 0.41 Salt Free (n-hexane: AcOEt= 1:1) | DMSO-d6 0.91-0.97(m, 2H), 1.13-1.28 (m, 4H), 1.61-1.76(m, 7H), 2.03 (s, 3H), 2.85-2.88(m, 2H), 2.92-2.94(m, 2H), 3.56-3.59 (m, 4H), 4.33-4.37(m, 2H), 5.43 (s, 2H), 6.87-6.89(m, 1H), 7.00(s, 1H), 7.02-7.07(m, 2H), 9.17 (s, 1H) |
| 151 | 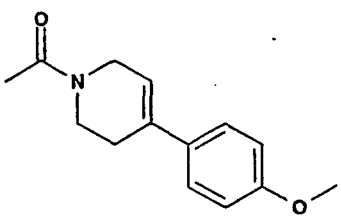 | 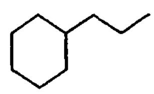 | ($CH_2Cl_2$: MeOH = 9:1) | CDCl3 0.95-1.02(m, 2H), 1.17-1.35 (m, 4H), 1.64-1.79(m, 7H), 2.16 (d, 3H), 2.52-2.57(m, 2H), 3.67(dd, 1H), 3.82 (dd, 1H), 4.10-4.15(m, 1H), 4.22-4.25 (m, 1H), 4.37-4.41 (m, 2H), 5.24 (s, 2H), 5.95-6.03(m, 1H), 6.72(s, 1H), 6.95-6.98(m, 2H), 7.33-7.37(m, 2H), 8.96 (s, 1H) |
| 152 | 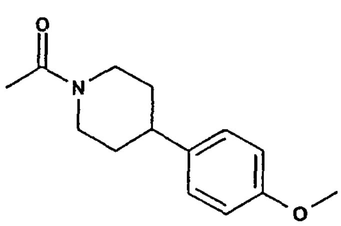 | 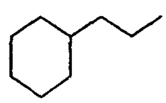 | ($OH_2Cl_2$: MeOH = 9:1) | CDCl3 0.92-1.01(m, 2H), 1.13-1.35(m, 4H), 1.59-1.88(m, 11H), 2.13(s, 3H), 2.59-2.74 (m, 2H), 3.13-3.20(m, 1H), 3.91-3.95(m, 1H), 4.36-4.40(m, 2H), 4.77-4.81 (m, 1H), 5.22 (s, 2H), 6.71(s, 1H), 6.92-6.96(m, 2H), 7.16 (dd, 2H), 8.95(s, 1H) |

(continued)

| Ex. | R' | R" | Rf (solvent) | NMR(400MHz, δ) |
|---|---|---|---|---|
| 153 | 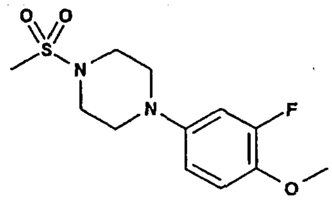 | 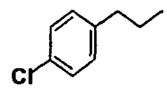 | 0.08 (AcOEt) | CDCl3 2.81-2.85(m, 3H), 3.17-3.22 (m, 6H), 3.37-3.40(m, 4H), 4.63 (dd, 2H), 4.90(s, 2H), 6.60-6.63(m, 2H), 6.74 (dd, 1H), 6.87 (t, 1H), 7.00-7.03(m, 2H), 7.20-7.22(m, 2H), 8.96 (s, 1H) |
| 154 | 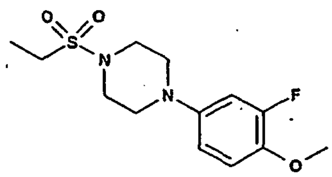 | 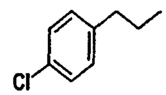 | 0.10 (AcOEt) | CDCl3 1.31-1.35(m, 3H), 2.91-2.96 (m, 2H), 3.09-3.12(m, 6H), 3.37-3.39(m, 4H), 4.56 (dd, 2H), 4.83(s, 2H), 6.52-6.55(m, 2H), 6.62-6.67(m, 1H), 6.80 (t, 1H), 6.94(dd, 2H), 7.13-7.15 (m, 2H), 8.89 (s, 1H) |
| 155 | 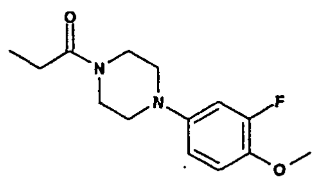 | 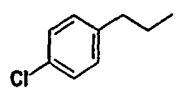 | 0.33 (AcOEt) | CDCl3 1.15-1.20(m, 3H), 2.39 (dt, 2H), 3.07-3.10(m, 4H), 3.18 (dd, 2H), 3.60-3.63(m, 2H), 3.76-3.78(m, 2H), 4.63 (dd, 2H), 4.90(s, 2H), 6.56-6.61 (m, 2H), 6.70 (dd, 1H), 6.87(t, 1H), 6.99-7.03(m, 2H), 7.19-7.23 (m, 2H), 8.96 (s, 1H) |
| 156 | 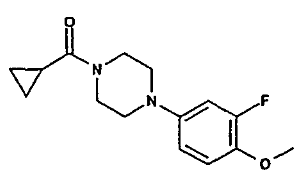 | 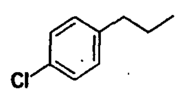 | 0.4 (AcOEt) | CDCl3 0.71-0.76(m, 2H), 0.93-0.97(m, 2H), 1.66-1.72(m, 1H), 3.06-3.14(m, 6H), 3.73-3.82(m, 4H), 4.56 (dd, 2H), 4.84(s, 2H), 6.55-6.59(m, 2H), 6.65-6.69(m, 1H), 6.81 (t, 1H), 6.94(d, 2H), 7.13-7.16(m, 2H), 8.89 (s, 1H) |

Example 157: 7-[2-(4-Chloro-phenyl)-ethyl]-6-[4-(4-ethyl-piperazin-1-yl)-2-fluorophenoxymethyl]-7H-pyrrolo[2,3-d]pyrimidine-2-carbonitrile

To a solution of 7-[2-(4-chloro-phenyl)-ethyl]-6-(2-fluoro-4-piperazin-1-yl-phenoxymethyl)-7H-pyrrolo[2,3-d]pyrimidine-2-carbonitrile (80mg) in DMF is added iodoethane (12.8uL), potassium carbonate (55mg), and stirred for 11 h at 60˚C. A solid in the reaction mixture is removed by filtration. The filtrate is loaded on HPLC, and pure product is

obtained; Rf= 0.15 (n-hexane:EtOAc=1:1) HCl salt; $^1H$ NMR (DMSO-d6), δ (ppm): 1.28(t, 3H), 3.04-3.17(m, 8H), 3.50-3.53(m, 2H), 3.65-3.75(m, 2H), 4.60(dd, 2H), 5.31 (s, 2H), 6.75-6.78(m, 1H), 6.93(s, 1H), 6.99(dd, 1H), 7.09(d, 2H), 7.20-7.26(m, 3H), 9.14(s, 1H), 10.70(brs, 1H).

Examples 158 to 160

By repeating the procedures described in Example 157 using appropriate starting materials (including some of those prepared in Examples A to K) the following compounds of formula 2 are obtained as identified below in Table 15.

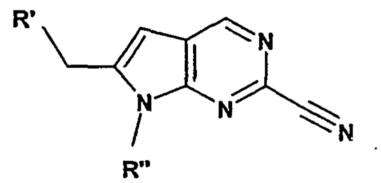

(2)

Table 15

| Ex. | R' | R" | Rf (solvent) | NMR (400MHz, δ) |
|---|---|---|---|---|
| 158 | 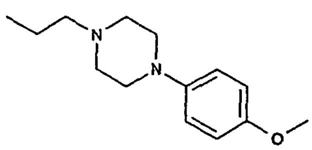 | 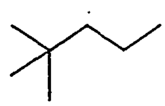 | 0.63 ($CH_2Cl_2$: MeOH = 9:1) | CDCl3<br>0.95(t, 3H), 1.01 (s, 9H), 1.55 (brs, 2H), 1.71-1.76 (m, 2H), 2.43 (brs, 2H), 2.67(brs, 4H), 3.19(brs, 4H), 4.36-4.41 (m, 2H), 5.18(s, 2H), 6.69(s, 1H), 6.91 (s, 4H), 8.94(s, 1H) |
| 159 | 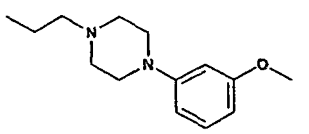<br>HCl | 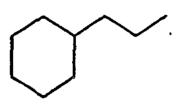 | ($CH_2Cl_2$: MeOH = 9:1) | DMSO-d6<br>0.89-0.96(m, 5H), 1.08-1.27(m, 4H), 1.59-1.77(m, 9H), 3.01-3.18(m, 6H), 3.50-3.53(m, 2H), 3.80-3.83(m, 2H), 4.33-4.37(m, 2H), 5.42 (s, 2H), 6.62(dd, 2H), 6.67(s, 1H), 6.99(s, 1H), 7.19 (dd, 1H), 9.16(s, 1H), 10.86 (brs, 1H) |
| 160 | 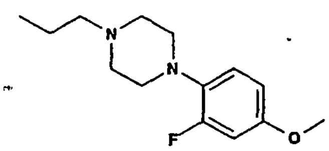<br>HCl | 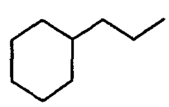 | ($CH_2Cl_2$: MeOH = 9:1) | DMSO-d6<br>0.87-0.95(m, 5H), 1.08-1.27(m, 4H), 1.63-1.72(m, 9H), 3.06-3.18 (m, 6H), 3.33-3.50(4H, m), 4.32-4.36(m, 2H), 5.43 (s, 2H), 6.89 (dd, 1H), 6.99(s, 1H), 7.05-7.11 (m, 2H), 9.16(s, 1H), 10.11 (brs, 1H) |

Example 161: 7-[2-(4-Chloro-phenyl)-ethyl]-6-(4-methoxy-benzyl)-7H-pyrrolo[2,3-d]pyrimidine-2-carbonitrile

To a solution of 6-bromomethyl-7-[2-(4-chloro-phenyl)-ethyl]-7H-pyrrolo[2,3-d]pyrimidine-2-carbonitrile (110 mg, 0.293 mmol) and p-methoxyphenyl boronic acid (98 mg, 0.645 mmol) in THF (1.5 mL) are added $Cs_2CO_3$ (143 mg, 0.439 mmol) and Pd(dppf)$Cl_2$.$CH_2Cl_2$ (24 mg, 0.029 mmol). The reaction mixture is stirred at 60 ˚C under nitrogen atomosphere for 1 h. The mixture is filtered through celite pad, and the filtrate is concentrated in vacuo. The residue is purified by silica gel column chromatography (*n*-hexane:EtOAc = 4:1 to 3:1) to give the product; Rf 0.46 (*n*-hexane: AcOEt=1:1); $^1$H NMR ($CDCl_3$), δ (ppm): 0.95(t, 2H), 3.75(s, 3H), 3.80(s, 3H), 4.36(t, 2H), 6.23(s, 1H), 6.87(d, 2H), 6.89 (d, 2H), 6.98(d, 2H), 7.24(d, 2H), 8.85(s, 1H).

Examples 162 to 170

By repeating the procedures described in Example 161 using appropriate starting materials (including some of those prepared in Examples A to K) the following compounds of formula 2 are obtained as identified below in Table 16.

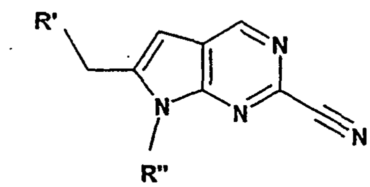

(2)

Table 16

| Ex. | R' | R" | Rf (solvent) | NMR (400MHz, δ) |
|---|---|---|---|---|
| 162 | 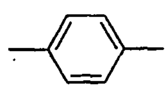 | 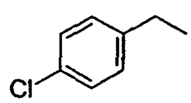 | 0.58 (*n*-hexane: AcOEt=1:1) | ($CDCl_3$) 2.35(s, 3H), 2.93(t, 2H), 3.78 (s, 2H), 4.34(t, 2H), 6.25(s, 1H), 6.87(d, 2H), 6.95(d, 2H), 7.14(d, 2H), 7.23(d, 2H), 8.85 (s, 1H) |
| 162 | 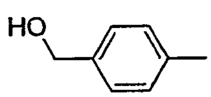 | 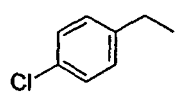 | 0.18 (*n*-hexane: AcOEt=1:1) | ($CDCl_3$) 1.69(t, 1H), 2.97(t, 2H), 3.79 (s, 2H), 4.36(t, 2H), 4.70(d, 2H), 6.23(s, 1H), 6.88(d, 2H), 7.06(d, 2H), 7.23(d, 2H), 7.35 (d, 2H), 8.86(s, 1H) |
| 164 | 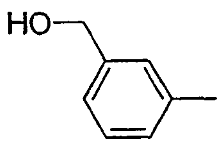 | 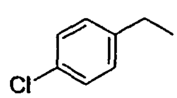 | 0.15 (*n*-hexane: AcOEt=1:1) | ($CDCl_3$) 1.70(t, 1H), 2.95(t, 2H), 3.80 (s, 2H), 4.36(t, 2H), 4.69(d, 2H), 6.24(s, 1H), 6.87(d, 2H), 7.00(d, 1H), 7.08(s, 1H), 7.23 (d, 2H), 7.28-7.36(m, 2H), 8.86(s, 1H) |
| 165 | 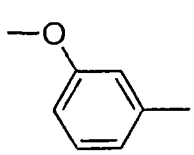 | 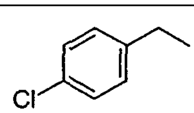 | 0.47 (*n*-hexane: AcOEt=1:1) | ($CDCl_3$) 2.92(t, 2H), 3.78(s, 2H), 3.78 (s, 3H), 4.35(t, 2H), 6.29(s, 1H), 6.61(d, 1H), 6.67(d, 1H), 6.83(dd, 1H), 6.88(d, 2H), 7.22-7.28(m, 3H), 8.87(s, 1H) |

(continued)

| Ex. | R' | R" | Rf (solvent) | NMR (400MHz, δ) |
|---|---|---|---|---|
| 166 | 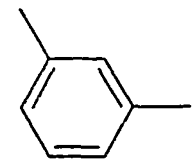 | 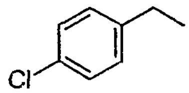 | 0.54 (*n*-hexane: AcOEt=1:1) | ($CDCl_3$) 2.33(s, 3H), 2.91(t, 2H), 3.78 (s, 2H), 4.35(t, 2H), 6.26(s, 1H), 6.86-6.89(m, 4H), 7.10 (d, 1H), 7.21-7.25(m, 3H), 8.86(s, 1H) |
| 167 | 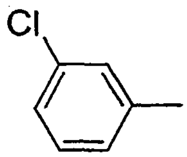 | 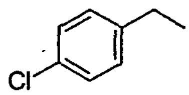 | 0.47 (n-hexane: AcOEt=1:1) | ($CDCl_3$) 2.96(t, 2H), 3.75(s, 2H), 4.36 (t, 2H), 6.25(s, 1H), 6.88(d, 2H), 6.93-6.96(m, 1H), 7.06 (s, 1H), 7.23-7.29(m, 4H), 8.88(s, 1H) |
| 168 | 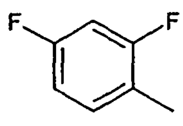 | 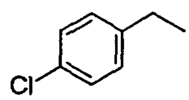 | 0.63 (*n*-hexane: AcOEt=1:1) | ($CDCl_3$) 2.98(dd, 2H), 3.68(s, 2H), 4.36(dd, 2H), 6.09(s, 1H), 6.77-6.91(m, 5H), 7.156-7.19(m, 2H), 8.78(s, 1H) |
| 169 | 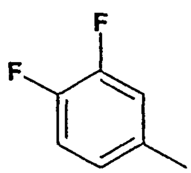 | 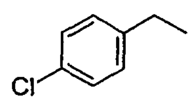 | ($CH_2Cl_2$:MeO H=9:1) | ($CDCl_3$) 2.95(dd, 2H), 3.60(s, 2H), 4.30(dd, 2H), 6.12(s, 1H), 6.68-6.71 (m, 1H), 6.75-6.82 (m, 3H), 7.03-7.10(m, 1H), 7.16-7.18(m, 2H), 8.81(s, 1H) |
| 170 | 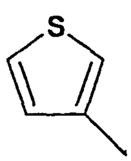 | 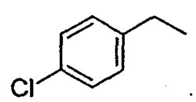 | 0.28 (*n*-hexane: AcOEt=2:1) | ($CDCl_3$) 2.95 (t, 2H), 3.82 (s, 2H), 4.37 (t, 2H), 6.30(s, 1H), 6.82 - 6.84 (m, 1H), 6.89 - 6.91 (m, 2H), 6.94 - 6.95 (m, 1H), 7.23 - 7.25 (m, 2H), 7.33 - 7.35 (m, 1H), 8.87 (s, 1H) |

Example 171: 6-[4-(4-Acetyl-piperazin-1-yl)-benzyl]-7-(2-cyclohexyl-ethyl)-7H-pyrrolo[2,3-d]pyrimidine-2-carbonitrile

To a solution of palladium acetate(6.7 mg), (di-*t*-butylphosphino)biphenyl (18mg), $Cs_2CO_3$ (120 mg) and $Et_3N$ (51.4 mL) in degassed dioxane (1.3 mL) are added 6-(4-chloro-benzyl)-7-(2-cyclohexyl-ethyl)-7H-pyrrolo[2,3-d]pyrimidine-2-carbonitrile (100 mg) and 1-acetyl piperazine (40,4 mg), and stirred for 18 hr under reflux. The reaction mixture is diluted with $H_2O$, extracted with EtOAc. The organic layer is successively washed with $H_2O$, aqueous NaCl, and concentrated in vacuo. The residue is purified by HPLC to give the pure product; Rf = 0.47 (dichloromethane : methanol= 9 : 1); $^1H$ NMR (400 MHz, $CDCl_3$) δ 0.88-0.98(m, 2H), 1.14-1.32(m, 4H), 1.49-1.73(m, 7H), 2.14(s, 3H), 3.14-3.20(m, 4H), 3.61-3.67(m, 2H), 3.77-3.82(m, 2H), 4.10(s, 2H), 4.17-4.21 (m, 2H), 6.30(s, 1H), 6.91-6.95(m, 2H), 7.11 (d, 2H), 8.83(s, 1H).

Example 172: 7-(2-Cyclohexyl-ethyl)-6-(4-hydroxymethyl-benzyl)-7H-pyrrolo[2,3-d] pyrimidine-2-carbonitrile

5-Bromo-4-(2-cyclohexyl-ethylamino)-pyrimidine-2-carbonitrile (1.03 mmol), (4-prop-2-ynyl-phenyl)-methanol (4.10 mmol), dichlorobis(triphenylphosphine) palladium(II) (0.05 mmol), copper (I) iodide (0.10 mmol) and triethylamine (5.15 mmol) in DMF (20 mL) is stirred at 75 ˚C for 3 h. After the reaction mixture is treated with saturated ammonium chloride, the mixture is extracted with AcOEt. The organic layer is washed with brine, dried over magnesium sulfate and evaporated down. The crude product is applied to a column chromatography on silica gel, which is eluted with following solvents: *n*-hexane:AcOEt=3:7 (v/v). The solvent of the latter effluent is removed by evaporation and dried in vacuo to

afford the title compound, Rf = 0.22 (*n*-hexane: AcOEt=1:1); $^1$H NMR ($PDCl_3$), δ (ppm): 0.89-1.32(m, 6H), 1.50-1.58(m, 3H), 1.64 (t, 1H), 1.62-1.78(m, 4H), 4.18(s, 2H), 4.19(t, 2H), 4.72(d, 2H), 6.31 (s, 1H), 7.20(d, 2H), 7.36(d, 2H), 8.84(s, 1H).

Example 173: 4-[2-Cyano-7-(2-cyclohexyl-ethyl)-7H-pyrrolo[2,3-d]pyrimidin-6-ylmethyl]-benzoic acid

7-(2-Cyclohexyl-ethyl)-6-(4-hydroxymethyl-benzyl)-7H-pyrrolo[2,3-d]pyrimidine -2-carbonitrile (0.88 mmol), TMPO (0.088mmol), and sodium phosphate buffer (pH 6.8) (3 mL) are dissolved in MeCN (10 mL). To the solution, $NaClO_2$ (3.52mmol) in water (3 ml) and 8.5 % NaOCl aq. (0.04 mmol) are added. The mixture is stirred at 35 ˚C temperature under nitrogen atomosphere for 2 days. The reaction mixture is diluted with $CH_2Cl_2$ and water and extracted with $CH_2Cl_2$ (twice). The combined organic layer is washed with water and brine, dried over $MgSO_4$, and concentrated in vacuo. The residue is purified by silica gel column chromatography (AcOEt) to give the title compound; Rf = 0.17 (*n*-hexane: AcOEt=2:3); $^1$H NMR ($CDCl_3$), δ (ppm): 0.87-1.32(m, 6H), 1.54-1.77(m, 7H), 4.20(t, 2H), 4.26(s, 2H), 6.34 (s, 1H), 7.32(d, 2H), 8.09(d, 2H), 8.87(s, 1H).

Example 174: 4-[2-Cyano-7-(2-cyclohexyl-ethyl)-7H-pyrrolo[2,3-d]pyrimidin-6-ylmethyl]-benzamide

4-[2-Cyano-7-(2-cyclohexyl-ethyl)-7H-pyrrolo[2,3-d]pyrimidin-6-ylmethyl]-benzoic acid (0.23 mmol) is dissolved in $CH_2Cl_2$ (15 mL). To the solution, $(COCl)_2$ (2.27mmol) and DMF (1 drop) are added at 0 ˚C. The mixture is stirred at room temperature under nitrogen atomosphere for 30 min. The reaction mixture is evapolated and residue is dissolved in $Et_2O$ (2 mL) - AcOEt (5 mL). To the solution, $NH_4OH$ (5 mL) is added at 0 ˚C. The mixture is stirred at room temperature under nitrogen atomosphere for 11 h, and the reaction mixture is diluted with AcOEt and water and extracted with AcOEt (twice). The combined organic layer is washed with water and *sat.* $NaHCO_3$ *aq.*, then dried over $MgSO_4$, and concentrated in vacuo. The residue is purified by silica gel column chromatography (AcOEt) to give the title compound; Rf = 0.16 (*n*-hexane: AcOEt=2:3); $^1$H NMR ($CDCl_3$), δ (ppm): 0.87-1.33(m, 6H), 1.54-1.79(m, 7H), 4.19(t, 2H), 4.24(s, 2H), 5.71 (brs, 1H), 6.02(brs, 1H), 6.32(s, 1H), 7.30(d, 2H), 7.82(d, 2H), 8.86(s, 1H).

Examples 175 to 178

By repeating the procedures described in Examples 172 to 174 using appropriate starting materials (including some of those prepared in Examples A to K) the following compounds of formula 2 are obtained as identified below in Table 17.

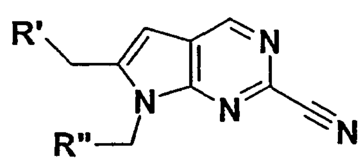

(2)

Table 17

| Ex. | R' | R" | Rf (solvent) | NMR (400MHz, δ) |
|---|---|---|---|---|
| 175 | 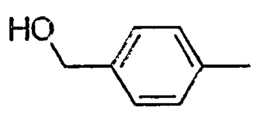 | 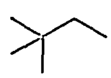 | 0.17 (*n*-hexane: AcOEt=3:2) | ($CDCl_3$) 0.99(s, 9H), 1.50-1.57(m, 2H), 1.69(t, 1H), 4.18(s, 2H), 4.18(t, 1H), 4.71 (d, 2H), 6.33(s, 1H), 7.19(d, 2H), 7.39(d, 2H), 8.84(s, 1H) |
| 176 | 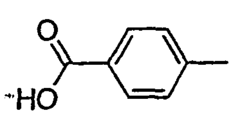 | 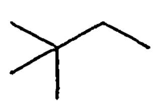 | 0.20 (*n*-hexane: AcOEt=1:1) | (DMSO-$d_6$) 0.91 (s, 9H), 1.39(t, 2H), 4.19 (t, 2H), 4.43(s, 2H), 6.53(s, 1H), 7.41 (d, 2H), 7.93(d, 2H), 9.05(s, 1H) |

(continued)

| Ex. | R' | R" | Rf (solvent) | NMR (400MHz, δ) |
|---|---|---|---|---|
| 177 | 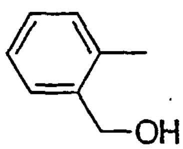 | 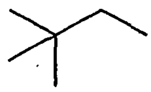 | 0.29 (*n*-hexane: AcOEt=1:1) | ($CDCl_3$) 1.00(s, 9H), 1.56(t, 1H), 1.61 (t, 2H), 4.25(t, 2H), 4.31 (s, 2H), 4.69(d, 2H), 6.10(s, 1H), 7.10(dd, 1H), 7.28-7.38 (m, 2H), 7.45(dd, 1H), 8.79 (s, 1H) |
| 178 | 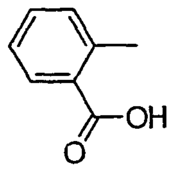 | 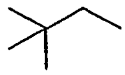 | 0.14 (*n*-hexane: AcOEt=1:1) | ($CDCl_3$) 1.02(s, 9H), 1.65(t, 1H), 4.31 (t, 2H), 4.62(s, 2H), 5.94(s, 1H), 7.27(dd, 1H), 7.47(dt, 1H), 7.59(dt, 1H), 8.17(dd, 1H), 8.74(s, 1H) |

Example 179: 7-(2-Cyclohexyl-ethyl)-6-[4-(2-oxo-pyrrolidin-1-yl)-benzyl]-7H-pyrrolo [2,3-d]pyrimidine-2-carbonitrile

5-Bromo-4-(2-cyclohexyl-ethylamino)-pyrimidine-2-carbonitrile (0.3 mmol) and 1-(4-prop-2-ynyl-phenyl)-pyrrolidin-2-one (0.3 mmol) are dissolved in 3 mL of DMF. The mixture is degassed by evaporation and purging with nitrogen under stirring a few times. $(Ph_3P)PdCl_2$ (0.015 mmol), CuI (0.03 mmol), and $Et_3N$ (0.6 mmol) are added and the reaction is heated under nitrogen at 80 °C for 16 h. After the mixture is cooled to rt, the aqueous layer is extracted twice with AcOEt, and the combined organic extracts are washed with brine several times, dried over $Na_2SO_4$, and concentrated under reduced pressure. Flush chromatography on silica gel using AcOEt-Hexane (1:1) gives the title compound as a yellow solid; $^1H$ NMR ($CDCl_3$), δ (ppm): 0.91 - 1.01 (m, 2H), 1.14 - 1.28 (m, 4H), 1.66 - 1.76 (m, 7H), 2.14 - 2.22 (m, 2H), 2,63 (t, 2H), 3.86 (t, 2H), 4.15 (s, 2H), 4.17 - 4.21 (m, 2H), 6.30 (s, 1H), 7.20 (d, 2H), 7.61 - 7.63 (m, 2H), 8.84 (s, 1H).

Examples 180 to 193

By repeating the procedures described in Example 179 using appropriate starting materials (including some of those prepared in Examples M to O) the following compounds of formula 2 are obtained as identified below in Table 18.

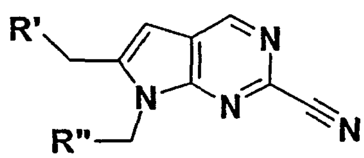

(2)

Table 18

| Ex. | R' | R" | NMR(400MHz, δ), ($CDCl_3$) |
|---|---|---|---|
| 180 | 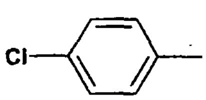 | 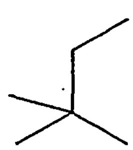 | 1.00 (s, 9H), 1.51 - 1.55 (m, 2H), 4.15 - 4.19 (m, 4H), 6.31 (s, 1H), 7.14 (d, 2H), 7.33-7.35 (m, 2H), 8.85 (s, 1H) |

(continued)

| Ex. | R' | R" | NMR(400MHz, δ), ($CDCl_3$) |
|---|---|---|---|
| 181 | 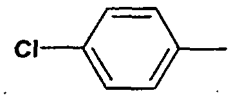 | 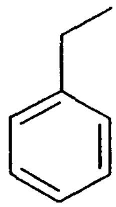 | 3.03 (t, 2H), 3.62 (s, 2H), 4.40 (t, 2H), 6.13 (s, 1H), 6.92-6.97 (m, 4H), (m, 2H), 8.85 (s, 1H) |
| 182 | 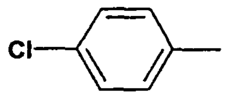 | 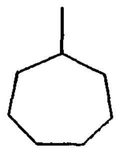 | 1.17 - 1.39 (m, 4H), 1.47 - 1.70 (m, 10H), (m, 1H), 4.01 (d, 2H), 4.15 (s, 2H), 6.27 (s, 1H), 7.12 - 7.15 (m, 2H), 7.32 - 7.35 (m, 2H), 8.85 (s, 1H) |
| 183 | 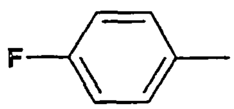 | 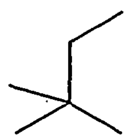 | 1.09 (s, 9H), 1.66 - 1.71 (m, 2H), 4.42 - 4.46 (m, 2H), 6.87 (s, 1H), 7.11 (d, 1H), 7.37 - 7.46 (m, 4H), 7.54 - 7.56 (m, 2H), 8.90 (s, 1H) |
| 184 | 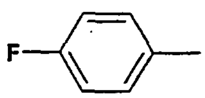 | 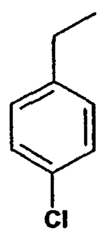 | 2.99 (t, 2H), 3.73 (s, 2H), 4.37 (t, 2H), 6.18 (s, 1H), 6.87 (d, 2H), 7.01 - 7.04 (m, 4H), 7.23 - 7.26 (m, 2H), 8.87 (s, 1H) |
| 185 | 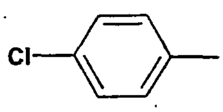 | 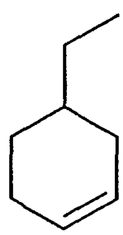 | 1.23 - 1.33 (m, 1H), 1.56 - 1.81 (m, 5H), 2.04 - 2.10 (m, 3H), 4.16 (s, 2H), 4.21 - 4.25 (m, 2H), 5.61 - 5.70 (m, 2H), 6.32 (s, 1H), 7.13 - 7.16 (m, 2H), 7.32 - 7.35 (m, 2H), 8.86 (s, 1H) |
| 186 | 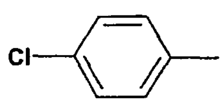 | 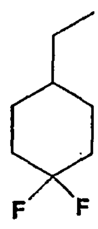 | 1.26-1.34 (m, 3H), 1.58 - 1.83 (m, 6H), 2.04 - 2.14 (m, 2H), 4.16 (s, 2H) 4.19 - 4.21 (m, 2H), 6.35 (s, 1H), 7.13 (d, 2H), 7.33 - 7.35 (m, 2H), 8.88 (s, 1H) |
| 187 | 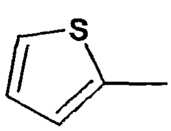 | 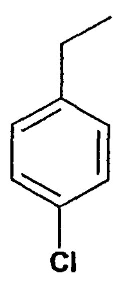 | 2.96 (t, 2H), 3.99 (s, 2H), 4.40 (t, 2H), 6.39 (s, 1H), 6.77 - 6.79 (m, 1H), 6.90 - 6.92 (m, 2H), 6.97-6.99 (m, 1H), 7.23 - 7.26 (m, 3H), 8.89 (s, 1H) |

(continued)

| Ex. | R' | R" | NMR(400MHz, δ), ($CDCl_3$) |
|---|---|---|---|
| 188 | 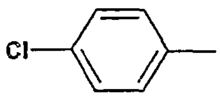 | 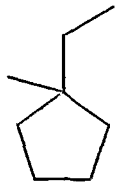 | 1.05 (s, 3H), 1.40 - 1.43 (m, 4.H), 1.62 - 1.68 (m, 6H), 4.16 - 4.20 (m, 4H), 6.31 (s, 1H), 7.14 (d, 2H), 7.33 (d, 2H), 8.85 (s, 1H) |
| 189 | 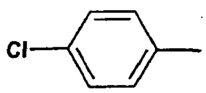 | 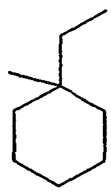 | 0.80 - 0.96 (m, 2H), 1.00 (s, 3H), 1.30 - 1.61 (m, 10H), 4.14 - 4.18 (m, 4H), 6.31 (s, 1H), 7.14 (d, 2H), 7.33 (d, 2H), 8.86 (s, 1H) |
| 190 | 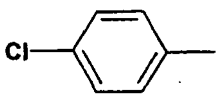 | 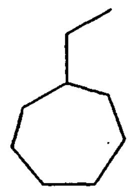 | 1.23-1.35 (m, 2H), 1.38-1.73(m, 15H), 4.16 (s, 2H), 4.18 (t, 2H), 6.31 (s, 1H), 7.17 (d, 2H), 7.34 (d, 2H), 8.85 (s, 1H) |
| 191 | 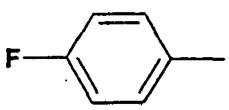 | 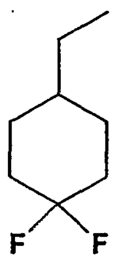 | 1.24 - 1.34 (m, 3H), 1.57 - 1.83 (m, 6H), 2.05 - 2.10 (m, 2H), 4.16 (s, 2H), 4.18 - 4.22 (m, 2H), 6.34 (s, 1H), 7.04 - 7.08 (m, 2H), 7.15 - 7.18 (m, 2H), 8.87 (s, 1H) |
| | | | |
| Ex. | R' | R" | NMR(400MHz, δ), |
| 192 | 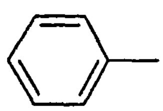 | 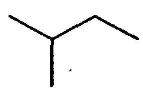 | ($CDCl_3$) 0.93(d, 6H), 1.48-1.63(m, 3H), 4.17(m, 4H), 6.34(s, 1H), 7.20(d, 2H), 7.28-7.38(m, 3H), 8.85(s, 1H) |
| 193 | 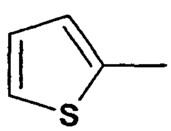 | 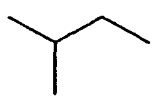 | ($CDCl_3$) 0.96(d, 6H), 1.51-1.65(m, 3H), 4.23(t, 2H), 4.39(s, 2H), 6.47 (s, 1H), 6.88(m, 1H), 6.98(dd, 1H), 7.24(dd, 1H), 8.89(s, 1H) |

Example 194: 6-(4-Chloro-benzyl)-7-(2-p-tolyl-ethyl)-7H-pyrrolo[2,3-d]pyrimidine-2-carbonitrile

To a solution of 6-(4-chloro-benzyl)-7H-pyrrolo[2,3-d]pyrimidine-2-carbonitrile (68 mg, 250 mmol) in DMF (3 mL) are added $K_2CO_3$ (40 mg, 0.29 mmol) and 1-(2-bromo-ethyl)-4-methyl-benzene (100 mg, 0.50 mmol). The reaction mixture is stirred at rt for overnight. After water is poured, the resulting mixture is extracted with EtOAc. The combined organic extracts are washed with brine, and dried over $Na_2SO_4$, filtered, and concentrated in vacuo.

The residue is purified by silica gel column chromatography (*n*-hexane : AcOEt=3:1) to give the title compound; $^1H$ NMR ($CDCl_3$), δ (ppm): 2.33(s, 3H), 2.99(t, 2H), 3.63(s, 2H), 4.37(t, 2H), 6.12(s, 1H), 6.81 (d, 2H), 6.95 (d, 2H), 7.07(d, 2H), 7.30(d, 2H), 8.84(s, 1H).

Examples 195 to 200

By repeating the procedures described in Example 194 using appropriate starting materials (including some of those prepared in Examples P to S) the following compounds of formula 2 are obtained as identified below in Table 19.

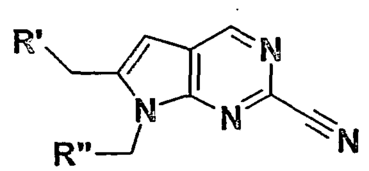

(2)

Table 19

| Ex. | R' | R" | NMR (400MHz, δ) |
|---|---|---|---|
| 195 | 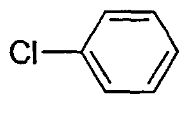 | 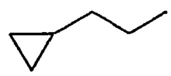 | ($CDCl_3$) -0.02- -0.01 (m, 2H), 0.41-0.45(m, 2H), 1.18-1.24(m, 2H), 1.73-1.80(m, 2H), 4.17(s, 2H), 4.18-4.22(m, 2H), 6.32(s, 1H), 7.15(d, 2H), 7.33(d, 2H), 8.85(s, 1H) |
| 196 | 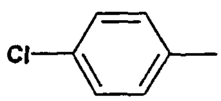 | 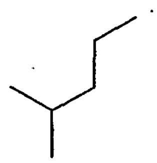 | ($CDCl_3$) 0.85 (d, 6H), 1.13 - 1.18 (m, 2H), (m, 3H), 4.13 - 4.16 (m, 4H), 6.32 (s, 1H), 7.14 (d, 2H), 7.33 (d, 2H), 8.86 (s, 1H) |
| 197 | 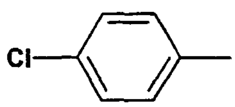 | 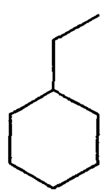 | ($CDCl_3$) 0.90 - 0.99 (m, 2H), 1.14 - 1.30 (m, 4H), 1.50 -1.56 (m, 2H), 1.66 -1.75 (m, 5H), 4.15 (s, 2H), 4.17 -4.20 (m, 2H), 6.30 (s, 1H), 7.12 - 7.16 (m, 2H), 7.32 - 7.34 (m, 2H), 8.85 (s, 1H) |
| 198 | 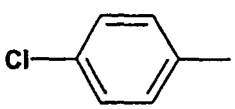 | 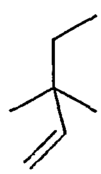 | ($CDCl_3$) 1.08 (s, 6H), 1.61 - 1.65 (m, 2H), 4.07 - 4.12 (m, 4H), 5.01 - 5.07 (m, 2H), 5.74 - 5.81 (dd, 1H), 6.31 (s, 1H), 7.13 (d, 2H), 7.32 - 7.34 (d, 2H), 8.85 (s, 1H) |
| 199 | 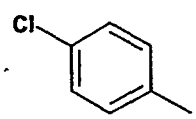 | 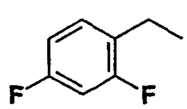 | ($CDCl_3$) 2.98(dd, 2H), 3.81 (s, 2H), 4.31 (dd, 2H), 6.16(s, 1H), 6.66-6.75(m, 2H), 6.79-6.85(m, 1H), 6.97(d, 2H), 7.24(dd, 2H), 8.78(s, 1H) |
| 200 | 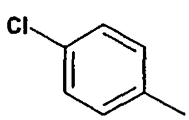 | 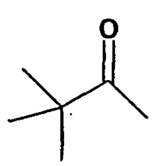 | ($CDCl_3$) 1.28(s, 9H), 3.99(s, 2H), 5.14(s, 2H), 6.37(s, 1H), 7.10-7.12(m, 2H), 7.32-7.34(m, 2H), 8.88(s, 1H) |

Example 201: 6-(4-Chloro-benzyl)-7-(2-cyclopentyl-ethyl)-7H-pyrrolo[2,3-d]pyrimidine-2-carbonitrile

To a solution of 6-(4-chloro-benzyl)-7H-pyrrolo[2,3-d]pyrimidine-2-carbonitrile (270 mg, 1.00 mmol), 2-cyclopentylethanol (140 mg, 1.20 mmol), and $Ph_3P$ (310 mg, 1.20 mmol) in THF (3 mL) is added dropwise DEAD (190

mg, 1.10 mmol). The reaction mixture is stirred at room temperature under nitrogen atomosphere for overnight. After concentration, the residue is purified by silica gel column chromatography (*n*-hexane : AcOEt=3:1) followed by RP-HPLC purification to give the title compound; $^1$H NMR ($CDCl_3$), δ (ppm): 1.09-1.12(m, 2H), 1.24-1.70(m, 7H), 1.74-1.80(m, 2H), 4.16(s, 2H), 4.17(t, 2H), 6.30(s, 1H), 7.14(d, 2H), 7.33(d, 2H), 8.85(s, 1H).

Examples 202 to 210

By repeating the procedures described in Example 201 using appropriate starting materials (including some of those prepared in Examples P to T) the following compounds of formula 2 are obtained as identified below in Table 20.

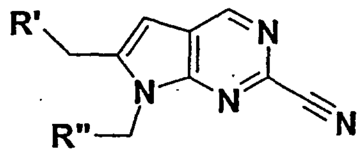

(2)

Table 20

| Ex. | R' | R" | NMR(400MHz, δ) |
|---|---|---|---|
| 202 | 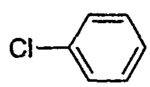 | 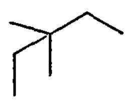 | ($CDCl_3$) 0.88(t, 3H), 0.94(s, 6H), 1.25-1.34(m, 2H), 1.49-1.53 (m, 2H), 4.14(t, 2H), 4.15(s, 2H), 6.31 (s, 1H), 7.14(d, 1H), 7.33(d, 1H), 8.86(s, 1H) |
| 203 | 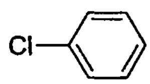 | 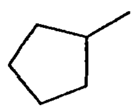 | ($CDCl_3$) 1.26-1.34(m, 2H), 1.53-1.65(m, 4H), 1.68-1.74(m, 2H), 2.36-2.42(m, 1H), 4.15(s, 2H), 4.18(s, 2H), 6.25(s, 1H), 7.13(d, 2H), 7.33(d, 2H), 8.84(s, 1H) |
| 204 | 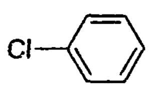 | 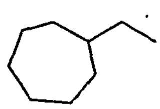 | ($CDCl_3$) 1.20-1.26(m, 2H), 1.4.0-1.73(m, 13H), 4.16(s, 2H), 4.17-4.20(m, 2H), 6.30(s, 1H), 7.14(d, 2H), 7.33(d, 2H), 8.85(s, 1H) |
| 205 | 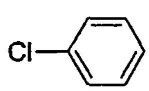 | 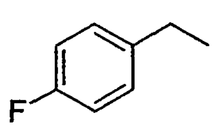 | ($CDCl_3$) 2.99(t, 2H), 3.72(s, 2H), 4.36(t, 2H), 6.20(s, 1H), 6.88-7.00(m, 6H), 7.31 (d, 2H), 8.87(s, 1H) |
| 206 | 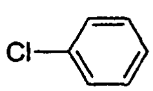 | 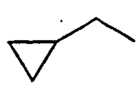 | ($CDCl_3$) -0.073-0.008(m, 2H), 0.38-0.42(m, 2H), 0.58-0.64(m, 1H), 1.62(q, 2H), 4.19(s, 2H), 4.28(t, 2H), 6.29(s, 1H), 7.13(d, 2H), 7.33(d, 2H), 8.85(s, 1H) |
| 207 | 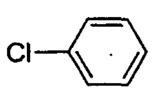 | 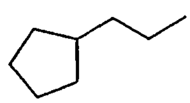 | ($CDCl_3$) 0.98-1.02(m, 2H), 1.27-1.30(m, 2H), 1.48-1.60(m, 4H), 1.62-1.74(m, 5H), 4.16(t, 2H), 4.16(s, 2H), 6.31 (s, 1H), 7.14(d, 2H), 7.33(d, 2H), 8.86(s, 1H) |
| 208 | 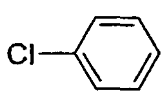 | 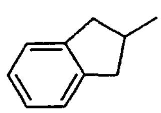 | ($CDCl_3$) 2.68(dd, 2H), 2.95(dd, 2H), 3.14-3.18(m, 1H), 4.08(s, 2H), 4.19(d, 2H), 6.31 (s, 1H), 6.99(dd, 2H), 7.18-7.22 (m, 4H), 7.28(dd, 2H), 8.88(s, 1H) |

(continued)

| Ex. | R' | R" | NMR(400MHz, δ) |
|---|---|---|---|
| 209 | 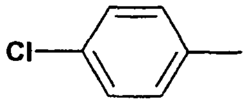 | 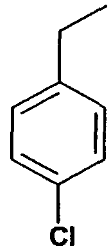 | ($CDCl_3$) 3.00 (t, 2H), 3.72 (s, 2H), 4.36 (t, 2H), 6.19 (s, 1H), 6.85 - 6.87 (m, 2H), 6.98 (d, 2H), 7.23 - 7.25 (m, 2H), 7.31 - 7.33 (m, 2H), 8.87 (s, 1H) |
| 210 | 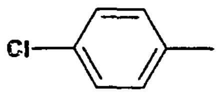 | 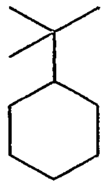 | ($CDCl_3$) 0.89 (s, 6H), 1.05 - 1.32 (m, 6H), 1.70 - 1.73 (m, 1H), 1.84. - 1.92 (m, 4H), 4.12 (s, 2H), 4.17 (s, 2H), 6.23 (s, 1H), 7.09 - 7.11 (m, 2H), 7.32 - 7.34 (m, 2H), 8.83 (s, 1H) |

Example 211: 7-(3,3-Dimethyl-butyl)-6-styryl-7H-pyrrolo[2,3-d]pyrimidine-2-carbonitrile

7-(3,3-Dimethyl-butyl)-6-hydroxymethyl-7H-pyrrolo[2,3-d]pyrimidine-2-carbonitrile is dissolved in $CH_2Cl_2$. To the solution is added Dess-Martin periodinane at 0 °C, and resulting solution is stirred. After dilution with $H_2O$, the mixture is extracted twice with AcOEt, and washed with brine. Flash chromatography on silica gel using AcOEt-Hexane gives 7-(3,3-dimethyl-butyl)-6-formyl-7H-pyrrolo[2,3-d]pyrimidine-2-carbonitrile. The aldehyde is dissolved in THF. To the solution are added benzyl-phosphonic acid diethyl ester and sodium hydride and the resulting solution is stirred. The reaction is quenched by the addition with $H_2O$, and the mixture is extracted twice with AcOEt. The combined organic extracts are washed with brine and dried over $Na_2SO_4$, filtered, and concentrated in vacuo. Flash chromatography on silica gel using AcOEt-Hexane gives the title compound. $^1$H NMR ($CDCl_3$), δ (ppm): 0.99 (s, 9H), (m, 2H), 3.80 (q, 2H), 4.16-4.21 (m, 4H), 6.34 (s, 1H), 7.24 - 7.26 (m, 4H), 8.87 (s, 1H).

Example 212: 7-(3,3-Dimethyl-butyl)-6-phenethyl-7H-pyrrolo[2,3-d]pyrimidine-2-carbonitrile

The product of Example 211 is dissolved in MeOH. The solution is degassed by evaporation and purging with nitrogen under stirring a few times. Pd/C (mmol) is added and the mixture is degassed by evaporation and purging with hydrogen under stirring a few times. The suspension is vigorously stirred under hydrogen. After 2 h, the mixture is filtered through celite and the filtrate is concentrated. Flash chromatography on silica gel using AcOEt-Hexane gives the title compound. $^1$H NMR ($CDCl_3$), δ (ppm): 1.03 (s, 9H), 1.55 - 1.59 (m, 2H), 3.12 (s, 4H), 4.15 - 4.20 (m, 2H), 6.43 (s, 1H), 7.19 - 7.21 (m, 1H), (m, 2H), 8.85 (s, 1H).

Examples 213

By repeating the procedures des,cribed in Example 211 and 212 using appropriate starting materials (including some of those prepared in Examples P to T) the following compounds of formula 2 are obtained as identified below in Table 21.

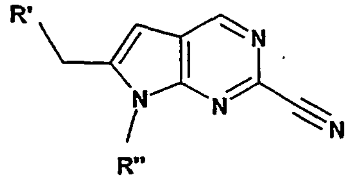

(2)

Table 21

| (R" represents 3,3-dimethyl-1-butyl) | | |
|---|---|---|
| Ex. | R' | NMR(400MHz, δ) |
| 213 | 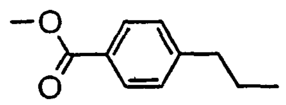 | ($CDCl_3$) 1.03 (s, 9H), 1.56 - 1.60 (m, 2H), 3.14 - 3.17 (m, 4H), 3.92 (s, 3H), 4.19 - 4.23 (m, 2H), 6.40 (s, 1H), 7.47 (d, 2H), 7.99 - 8.01 (m, 2H), 8.85 (s, 1H). |

Example'214: 6-(4-Amino-benzyl)-7-(3,3-dimethyl-butyl)-7H-pyrrolo[2,3-d]pyrimidine-2-carbonitrile

5-Bromo-4-(3,3-dimethyl-butylamino)-pyrimidine-2-carbonitrile and (4-prop-2-ynyl-phenyl)-carbamic acid *tert*-butyl ester are dissolved in DMF. The mixture is degassed by evaporation and purging with nitrogen under stirring a few times. $(Ph_3P)PdCl_2$, CuI, and $Et_3N$ are added and the reaction is heated under nitrogen at 80 ˚C. After the mixture is cooled to rt, the aqueous layer is extracted twice with AcOEt, and the combined organic extracts are washed with brine several times, dried over $Na_2SO_4$, and concentrated under reduced pressure.
The yellow solid is dissolved in $CH_2Cl_2$. To the solution is added dropwise TFA, and the resulting solution is stirred for some h. After dilution with $H_2O$, the mixture is extracted twice with AcOEt, and the combined organic extracts are washed with brine. Flash chromatography on silica gel using AcOEt-Hexane gives the title compound as a yellow solid; $^1H$ NMR ($CDCl_3$), δ (ppm): 1.00 (s, 9H), 3.67 (s, 2H), 4.06 (s, 2H), 4.16 - 4.20 (m, 2H), 6.31 (s, 1H), 6.65-6.67 (m, 2H), 6.97 (d, 2H), 8.83 (s, 1H).

Example 215: 6-(4-Amino-benzyl)-7-(3,3-dimethyl-butyl)-7H-pyrrolo[2,3-d]pyrimidine-2-carbonitrile

The amine of Example 214 is dissolved in $CH_2Cl_2$. To the solution are added 2-methoxy-ethanesulfonyl chloride and pyridine at rt. After stirred at rt for some h, the reaction mixture is diluted with $H_2O$. The mixture is extracted with AcOEt twice, and the combined organic extracts are dried over $Na_2SO_4$. Flash chromatography on silica gel using AcOEt-Hexane gives the title compound; $^1H$ NMR ($CDCl_3$), δ (ppm): 0.99 (s, 9H), 3.22 (t, 2H), 3.43 (s, 3H), 3.84 (t, 2H), 4.16 - 4.21 (m, 4H), 6.33 (s, 1H), 6.44 (s, 1H), 7.17 (d, 2H), 7.23 - 7.26 (m, 2H), 8.86 (s, 1H).

Examples 216 to 218

By repeating the procedures described in Example 214 and 215 using appropriate starting materials (including some of those prepared in Examples A to T) the following compounds of formula 3 are obtained as identified below in Table 22.

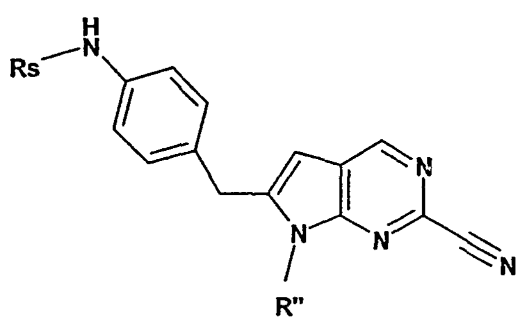

(3)

Table 22

| Ex. | $R^s$ | R" | NMR(400MHz, δ) |
|---|---|---|---|
| 216 | 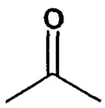 | 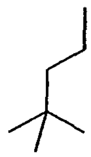 | ($CDCl_3$) 0.99 (s, 9H), 2.19 (s, 2H), 4.14 (s, 2H), 4.15 - 4.20 (m, 2H), 6.32 (s, 1H), 7.15 (d, 2H), 7.49 (d, 2H), 8.84 (s, 1H) |
| 217 | 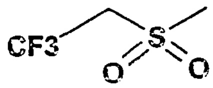 | 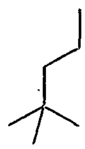 | ($CDCl_3$) 0.99 (s, 9H), (m, 2H), 3.80 (q, 2H), 4.16 - 4.21 (m, 4H), 6.34 (s, 1H), 7.24 - 7.26 (m, 4H), 8.87 (s, 1H) |
| 218 | 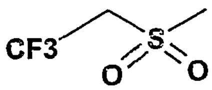 | 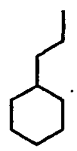 | ($CDCl_3$) 0.89 - 0.99 (m, 2H), 1.11 - 1.31 (m, 4H), 1.51 - 1.76 (m, 7H), 3.80 (q, 2H), 4.18 - 4.22 (m, 4H), 6.32 (s, 1H), 7.22 - 7.27 (m, 4H), 8.87 (s, 1H) |

Example 219: 3-[2-Cyano-7-(3,3-dimethyl-butyl)-7H-pyrrolo[2,3-d]pyrimidin-6-ylmethyl]-benzoic acid

7-(3,3-Dimethyl-butyl)-6-(3-formyl-benzyl)-7H-pyrrolo[2,3-d]pyrimidine-2-carbonitrile is dissolved in THF/$H_2O$. and reacted with $NaClO_2$ and $NH_2SO_3H$ in THF/$H_2O$ at a temperature of 0˚C; $^1H$ NMR ($CDCl_3$), δ (ppm): 0.98 (s, 9H), 1.40- 1.45 (m, 2H), 4.26 - 4.30 (m, 2H), 4.50 (s, 2H), 6.65 (s, 1H), 7.57 - 7.64 (m, 2H), 7.95 - 7.96 (m, 2H), 9.13 (s, 1H).

Example 220: 7-[2-(4-Chloro-phenyl)-ethyl]-6-[3-(2,5-dioxo-imidazolidin-1-ylmethyl)-benzyl]-7H-pyrrolo[2,3-d]pyrimidine-2-carbonitrile

To a solution of 7-[2-(4-chloro-phenyl)-ethyl]-6-(3-hydroxymethyl-benzyl)-7H-pyrrolo[2,3-d]pyrimidine-2-carbonitrile (31 mg, 0.077 mmol) in $CH_2Cl_2$ (0.5 mL) are added $Ph_3P$ (24 mg, 0.092 mmol) and $CBr_4$ (33 mg, 0.093 mmol). After being stirred at rt for 30 min, the additional $Ph_3P$ (29 mg, 0.11 mmol) and $CBr_4$ (40 mg, 0.12 mmol) are added. The reaction mixture is stirred at rt for 20 min, and concentrated in vacuo. The residue is purified by silica gel column chromatography (*n*-hexane:EtOAc=5:1) to give the corresponding bromide.
To a solution of said bromide (14 mg, 0.030 mmol) in DMF (0.3 mL) are added hydantoin (4 mg, 0.040 mmol) and $K_2CO_3$ (5 mg, 0.036 mmol). The reaction mixture is stirred at rt for 16 h. After dilution with EtOAc, the mixture is washed with water and brine. The organic layer is dried over $Na_2SO_4$, filtered, and concentrated in vacuo. The residue is purified by silica gel column chromatography (n-hexane:EtOAc=2:3 to 1:2) to give the title compound; $^1H$ NMR ($CDCl_3$), δ (ppm): 2.95(t, 2H), 3.79(s, 2H), 3.96(s, 2H), 4.34(t, 2H), 4.65(s, 2H), 5.23(s, 1H), 6.24(s, 1H), 6.87(d, 2H), 6.97(d, 1H), 7.17(s, 1H), 7.18(d, 2H), 7.28-7.35(m, 2H), 8.87(s, 1H).

Examples 221 to 225

By repeating the procedures described in Example 220 using appropriate starting materials (including some of those prepared in Examples P to T) the following compounds of formula 2 are obtained as identified below in Table 23.

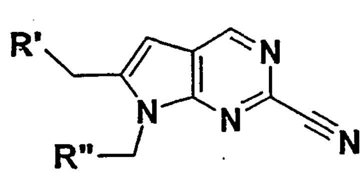

(2)

Table 23

| Ex. | R' | R" | NMR (400MHz, δ) |
|---|---|---|---|
| 221 | 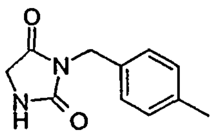 | 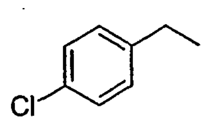 | ($CDCl_3$) 2.93(t, 2H), 3.76(s, 2H), 3.95(d, 2H), 4.33(t, 2H), 4.66 (s, 2H), 5.29(br, 1H), 6.23(s, 1H), 6.85(s, 2H), 7.02 (d, 2H), 7.24(d, 2H), 7.39(d, 2H), 8.86(s, 1H) |
| 222 | 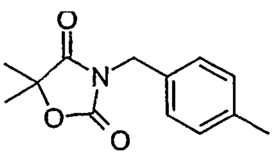 | 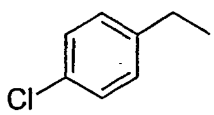 | ($CDCl_3$) 1.53(s, 3H), 1.56(s, 3H), 2.93(t, 2H), 3.76(s, 2H), 4.34(t, 2H), 4.66(s, 2H), 6.22(s, 1H), 6.85(d, 2H), 7.04(d, 2H), 7.23(d, 2H), 7.35(d, 2H), 8.87(s, 1H) |
| 223 | 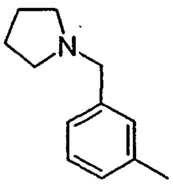 | 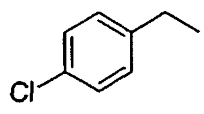 | ($CDCl_3$) 1.83(br, 4H), 2.55(br, 4H), 2.94(t, 2H), 3.66(s, 2H), 3.82(s, 2H), 4.36(t, 2H), 6.25(s, 1H), 6.88(d, 2H), 6.97(br, 1H), 7.16(br, 1H), 7.22(d, 2H), 7.29-7.30(m, 2H), 8.86(s, 1H) |
| 224 | 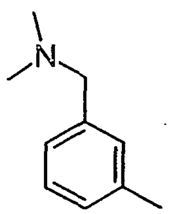 | 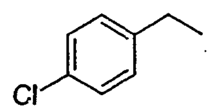 | ($CDCl_3$, HCl salt) 2.74(s, 6H), 3.05(t, 2H), 3.77(s, 2H), 4.10(s, 2H), 4.43(t, 2H), 6.20(s, 1H), 6.90(d, 2H), 7.13(d, 1H), 7.13(d, 2H), 7.39-7.56(m, 3H), 8.86(s, 1H), 13.0(br, 1H) |
| 225 | 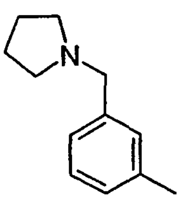 | 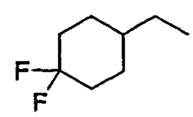 | (DMSO-$d_6$, HCl salt) 1.07-1.17(m, 2H), 1.36(br, 1H), 1.44-1.50(m, 2H), 1.66-2.00(m, 10H), 3.00-3.06(m, 2H), 3.29-3.35(m, 2H), 4.26(t, 2H), 4.28(d, 2H), 4.37(s, 2H), 6.52(s, 1H), 7.39(d, 1H), 7.44-7.48(m, 2H), 7.52(d, 1H), 9.06 (s, 1H), 10.58(br, 1H) |

Example 226: 6-(4-Chloro-3-hydroxymethyl-benzyl)-7-[2-(4-chloro-phenyl)-ethyl]-7H-p yrrolo[2,3-d]pyrimidine-2-carbonitrile

To a solution of (5-bromo-2-chloro-phenyl)-methanol (272 mg, 1.23 mmol) and bis-(pinacolate)diboron (343 mg, 1.35 mmol) in DMSO (7 mL) are added KOAc (362 mg, 3.69 mmol) and Pd(dppf)$Cl_2$.$CH_2Cl_2$ (50 mg, 0.062 mmol). The reaction mixture is stirred at 80 ˚C under nitrogen atomosphere for 9 h. After dilution with ether, the mixture is washed with water (x2) and brine. The organic layer is dried over $MgSO_4$, filtered, and concentrated in vacuo. The residue is purified by silica gel column chromatography (*n*-hexane:EtOAc=5:1) to give the corresponding boron ester. To a solution of 6-bromomethyl-7-[2-(4-chloro-phenyl)-ethyl]-7H-pyrrolo[2,3-d]pyrimidine-2-carbonitrile (88 mg, 0.234 mmol) and said boron ester (126 mg, 0.469 mmol) in THF (1.5 mL) are added $Cs_2CO_3$ (115 mg, 0.353 mmol) and Pd (dppf)$Cl_2$.$CH_2Cl_2$ (19 mg, 0.023 mmol). The reaction mixture is stirred at 60˚C under nitrogen atomosphere for 1 h. The mixture is filtered through celite pad, and the filtrate is concentrated in vacuo. The residue is purified by silica gel column chromatography (*n*-hexane:EtOAc=2:1) to give the title compound; Rf = 0.25 (*n*-hexane: AcOEt=1:1); $^1$H NMR ($CDCl_3$), δ (ppm): 1.98(t, 1H), 3.00(t, 2H), 3.73(s, 2H), 4.38(t, 2H), 4.77(d, 2H), 6.19(s, 1H), 6.87(dd, 2H), 6.92(dd, 1H), 7.22-7.25 (m, 3H), 7.32(d, 1H), 8.86(s, 1H).

Examples 227 to 228

By repeating the procedures described in Example 226 using appropriate starting materials (including some of those prepared in Examples A to T) the following compounds of formula 2 are obtained as identified below in Table 24.

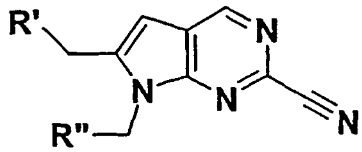

(2)

Table 24

| Ex. | R' | R" | Rf (solvent) | NMR(400MHz, δ), ($CDCl_3$) |
|---|---|---|---|---|
| 227 | 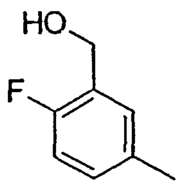 | 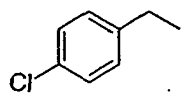 | 0.27 (*n*-hexane: AcOEt=1:1) | 1.86(t, 1H), 3.00(t, 2H), 3.73(s, 2H), 4.38(t, 2H), 4.75(d, 2H), 6.18 (s, 1H), 6.87(d, 2H), 6.94-6.97(m, 1H), 7.03(t, 1H), 7.14(dd, 1H), 7.24 (d, 2H), 8.86(s, 1H) |
| 228 | 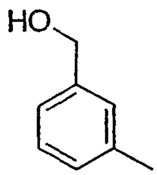 | 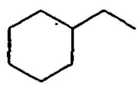 | 0.28 (*n*-hexane: AcOEt=1:1) | 0.88-0.98(m, 2H), 1.13-1.30(m, 4H), 1.49-1.54(m, 2H), 1.67-1.74 (m, 5H), 4.17-4.21 (m, 4H), 4.69(s, 2H), 6.33(s, 1H), 7.13(d, 1H), 7.23 (s, 1H), 7.31-7.37(m, 2H), 8.84(s, 1H) |

Example 229: 7-(2-Cyclohexyl-ethyl)-6-(3-oxo-3-piperidin-1-yl-propyl)-7H-pyrrolo[2,3-d]pyrimidine-2-carbonitrile

To a solution of the alkyne (71 mg, 0.43 mmol) and the cyanopyrimidine (110 mg, 0.36 mmol) in DMF (2 mL) are added $Et_3N$ (0.15 mL, 1.08 mmol), CuI (6.8 mg, 0.036 mmol), and $Pd(Ph_3P)_2Cl_2$ (13 mg, 0.019 mmol). The flask is evacuated and backfilled with nitrogen, and then stirred at 80 ˚C for 2 h. After dilution with EtOAc, the mixture is washed with water (x2) and brine. The organic layer is dried over $Na_2SO_4$, filtered, and concentrated in vacuo. The residue is purified by silica gel column chromatography (*n*-hexane:EtOAc=2:1) to give the coupling product.

To a solution of the above product (133 mg) in DMF (1 mL) is added 2 drops of DMF. The reaction mixture is stirred at 100 ˚C for 2.5 h. After dilution with EtOAc, the mixture is washed with 1 *M* aqueous $KHSO_4$ and brine. The organic layer is dried over $Na_2SO_4$, filtered, and concentrated in vacuo. The residue is purified by silica gel column chromatography (*n*-hexane:EtOAc=2:1 to 1:1) followed by RP-HPLC to give the title compound; $^1H$ NMR ($CDCl_3$), δ (ppm): 0.95-1.05(m, 2H), 1.14-1.26(m, 3H), 1.29-1.37(m, 1H), 1.54-1.75(m, 11H), 1.80-1.83(m, 2H), 2.81 (t, 2H), 3.19 (t, 2H), 3.45(t, 2h), 3.59(t, 2H), 4.32(t, 2H), 6.37(s, 1H), 8.83(s, 1H).

Example 230

By repeating the procedures described in Example 229 using appropriate starting materials (including some of those prepared in Examples A to V) the following compounds of formula 2 are obtained as identified below in Table 24.

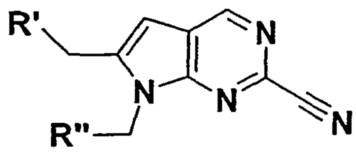

(2)

Table 24

| Ex. | R' | R" | Rf (solvent) | NMR (400MHz, δ, $CDCl_3$) |
|---|---|---|---|---|
| 230 | 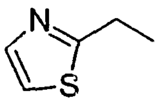 | 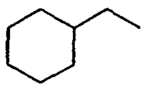 | 0.18 (*n*-hexane: AcOEt=1:1) | 0.95-1.04(m, 2H), 1.15-1.26(m, 3H), 1.29-1.37(m, 1H), 1.60-1.83(m, 5H), 3.37 (t, 2H), 3.53(t, 2H), 4.28(t, 2H), 6.4.5(s, 1H), 7.24(d, 1H), 7.73(d, 1H), 8.85(s, 1H) |

Example 231: 7-[2-(4-Chloro-phenyl)-ethyl]-6-(3-methoxymethyl-benzyl)-7H-pyrrolo[2,3-d]pyrimidine-2-carbonitrile

To a solution of 1-bromo-3-methoxymethyl-benzene (286 mg, 1.42 mmol) and bis-(pinacolate)diboron (397 mg, 1.56 mmol) in DMSO (8 mL) are added KOAc (419 mg, 4.27 mmol) and $Pd(dppf)Cl_2.CH_2Cl_2$ (58 mg, 0.071 mmol). The reaction mixture is stirred at 80 ˚C under nitrogen atomosphere for 1 h. After dilution with ether, the mixture is washed with water (x2) and brine. The organic layer is dried over $MgSO_4$, filtered, and concentrated in vacuo. The residue is purified by silica gel column chromatography (*n*-hexane:EtOAc=10:1) to give the corresponding boron ester. To a solution of 6-bromomethyl-7-[2-(4-chloro-phenyl)-ethyl]-7H-pyrrolo[2,3-d]pyrimidine-2-carbonitrile (83 mg, 0.221 mmol) and the above boron ester (110 mg, 0.443 mmol) in THF (1.6 mL) are added $Cs_2CO_3$ (108 mg, 0.331 mmol), benzyl alcohol (0.046 mmol, 0.445 mmol), and $Pd(dppf)Cl_2.CH_2Cl_2$ (18 mg, 0.022 mmol). The reaction mixture is stirred at 60 ˚C under nitrogen atomosphere for 1 h. The mixture is filtered through celite pad, and the filtrate is concentrated in vacuo. The residue is purified by silica gel column chromatography (*n*-hexane:EtOAc=4:1) followed by RP-HPLC to give the title compound; $^1$H NMR ($CDCl_3$), δ (ppm): 1.94-2.01 (m, 2H), 2.42(t, 2H), 2.96(t, 2H), 3.24(t, 2H), 3.77(s, 2H), 4.36(t, 2H), 4.42(s, 2H), 6.20(s, 1H), 6.88(d, 2H), 6.97-6.98(m, 2H), 7.17(d, 1H), 7.17(d, 2H), 7.31(t, 1H), 8.86(s, 1H).

Example 232

By repeating the procedures described in Example 230 using appropriate starting materials (including some of those prepared in Examples A to X) the following compounds of formula 2 are obtained as identified below in Table 25.

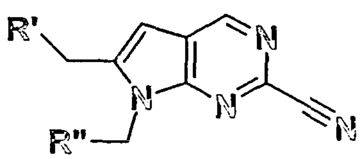

(2)

Table 25

| Ex. | R' | R" | Rf (solvent) | NMR (400MHz, δ) |
|---|---|---|---|---|
| 232 | 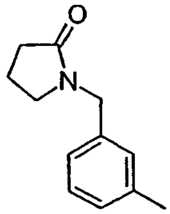 | 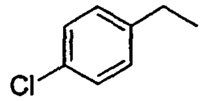 | 0.17 (*n*-hexane: AcOEt=1:3) | ($CDCl_3$) 1.94-2.01 (m, 2H), 2.42(t, 2H), 2.96 (t, 2H), 3.24(t, 2H), 3.77(s, 2H), 4.36 (t, 2H), 4.42(s, 2H), 6.20(s, 1H), 6.88 (d, 2H), 6.97-6.98(m, 2H), 7.17(d, 1H), 7.17(d, 2H), 7.31 (t, 1H), 8.86(s, 1H) |

Example 233: 7-(2-Cyclohexyl-ethyl)-6-(4-hydroxy-4-phenyl-piperidin-1-ylmethyl)-7H-pyrrolo[2,3-d]pyrimidine-2-carbonitrile

6-Bromomethyl-7-(2-cyclohexyl-ethyl)-7H-pyrrolo[2,3-d]pyrimidine-2-carbonitrile(80 mg, 0.23 mmol) and 4-phenyl-piperidin-4-ol (41.8 mg, 0.23 mmol) are dissolved in DMF (2 ml) and potassium carbonate (63.6 mg, 0.46 mmol) is added to the solution. The reaction mixture is stirred at rt for 3 h and quenched with saturated ammonium chlroride and extracted with ethyl acetate. The combined extracts are washed with brine, dried over magnesium sulfate and evaporated down. The crude product is purified by reverse phase HPLC and fraction are collected and evaporated down. Saturated sodium bicarbonate is added and neutralized and the water phase is extracted with ethyl acetate. The combined extracts are washed with brine, dried over magnesium sulfate and evaporated down to give the title compound; Rf=0.30 (*n*-hexane : ethyl acetate = 1:1); $^1$H-NMR(400MHz, $CDCl_3$) δ : 1.02-1.05(m, 2H), 1.23-1.40(m, 3H), 1.71-1.86(m, 9H), 2.10-2.19(m, 2H), 2.61(t, 2H), 2.77-2.79(m, 2H), 3.78(s, 2H), 4.43-4.47(m, 2H), 6.54(s, 1H), 7.28(t, 1H), 7.37(t, 2H), 7.49(d, 2H), 8.88(s, 1H).

Examples 234 to 296

By repeating the procedures described in Example 233 using appropriate starting materials (including some of those prepared in Examples A to X) the following compounds of formula 2 are obtained as identified below in Table 26.

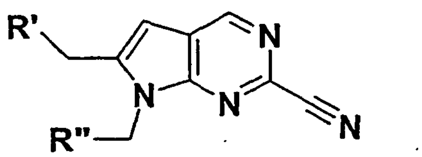

(2)

Table 26

| Ex. | R' | R" | Rf (solvent) | NMR (400MHz, δ; CDCl3) |
|---|---|---|---|---|
| 234 | 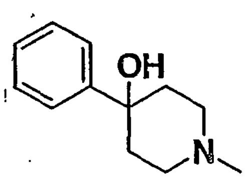 | 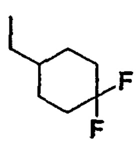 | 0.15 (*n*-hexane: AcOEt = 1:1) | 1.39-1.51 (m, 2H), 1.69-1.82(m, 6H), 1.84-1.92(m, 1H), 2.04-2.14(m, 4H), 2.62 (t, 2H), 2.75-2.78(m, 2H), 3.77(s, 2H), 4.45-4.49(m, 2H), 6.60 (s, 1H), 7.28(t, 1H), 7.38 (t, 2H), 7.49(d, 2H), 8.90 (s, 1H). |

(continued)

| Ex. | R' | R" | Rf (solvent) | NMR (400MHz, δ; CDCl3) |
|---|---|---|---|---|
| 235 | 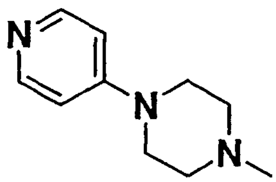 | 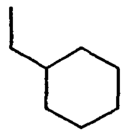 | 0.12 (dichlorom ethane: MeOH= 1:1) | 0.85-1.42(m, 7H), 1.60-1.97(m, 6H), 2.61-2.64(m, 4H), 3.34-3.36(m, 4H), 3.74 (s, 2H), 4.41-4.94(m, 2H), 6.55(s, 1H), 6.66(d, 2H), 8.28(d, 2H), 8.96(s, 1H) |
| 236 | 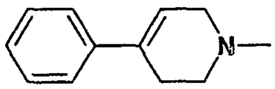 | 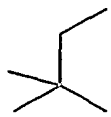 | 0.38 (n-hexane: AcOEt= 2:1) | 1.03(s, 9H), 1.69-1.73 (m, 2H), 2.56-2.57(m, 2H), 2.77(t, 2H), 3.19-3.21(m, 2H), 3.82 (s, 2H), 4.42-4.46(m, 2H), 6.04-6.05(s, 1H), 6.56(s, 1H), 7.25-7.38 (m, 2H), 8.89(s, 1H) |
| 237 | 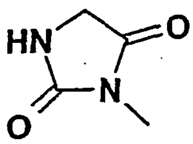 | 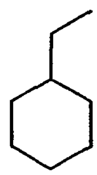 | 0.37 (AcOEt) | 0.98-1.07(m, 2H), 1.14-1.31 (m, 3H), 1.33-1.42(m, 1H), 1.62-1.77(m, 5H), 1.84 (m, 2H), 4.05(s, 2H), 4.47-4.51 (m, 2H), 4.90 (s, 2H), 6.71 (s, 1H), 8.91 (s, 1H) |
| 238 | 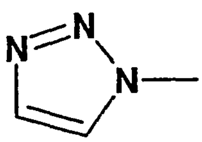 | 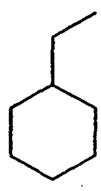 | 0.03 (n-hexane: AcOEt= 2:1) | 0.91-1.00(m, 2H), 1.44-1.35(m, 4H), 1.39-1.45(m, 2H), 1.61-1.77(m, 5H), 4.27-4.31 (m, 2H), 5.83 (s, 2H), 6.68(s, 1H), 7.57 (s, 1H), 7.78(s, 1H), 8.98 (s, 1H |
| 239 | 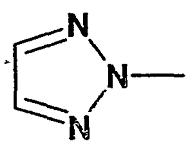 | 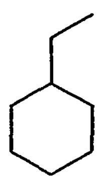 | 0.13 (n-hexane: AcOEt= 2:1) | 0.93-1.02(m, 2H), 1.11-1.36(m, 4H), 1.43-1.49(m, 2H), 1.62-1.79(m, 5H), 4.33-4.37(m, 2H), 5.84 (s, 2H), 6.72(s, 1H), 7.68 (s, 2H), 8.95(s, 1H) |
| 240 | 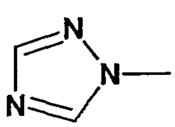 | 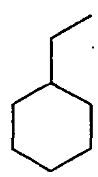 | 0.09 (n-hexane: AcOEt= 2:1) | 0.94-1.02(m, 2H), 1.15-1.34(m, 4H), 1.47-1.53(m, 2H), 1.62-1.79(m, 5H), 4.33-4.37(m, 2H), 5.61 (s, 2H), 6.62(s, 1H), 8.02 (s, 1H), 8.16(s, 1H), 8.96 (s, 1H) |

(continued)

| Ex. | R' | R" | Rf (solvent) | NMR (400MHz, δ; CDCl3) |
|---|---|---|---|---|
| 241 | 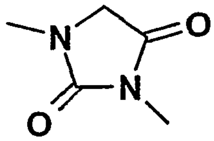 | 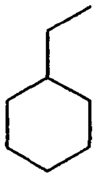 | 0.17 (n-hexane: AcOEt= 2:1) | 0.97-1.07(m, 2H), 1.16-1.31 (m, 3H), 1.33-1.40(m, 1H), 1.61-1.76(m, 5H), 1.82-1.86(m, 2H), 3.03 (s, 3H), 3.94(s, 2H), 4.47-4.51 (m, 2H), 4.88 (s, 2H), 6.71(s, 1H), 8.90 (s,1H) |
| 242 | 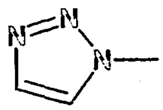 | 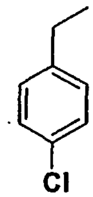 | 0.32 (AcOEt) | 2.90(t, 2H), 4.48(t, 2H), 5.36(s, 2H), 6.60(s, 1H), 6.97(d, 2H), 7.27(d, 2H), 7.44(s, 1H), 7.76(s, 1H), 9.01 (s, 1H) |
| 243 | 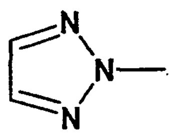 | 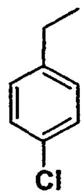 | 0.43 (n-hexane: AcOEt= 1:2) | 2.93(t, 2H), 4.54(t, 2H), 5.46(s, 2H), 6.73(s, 1H), 6.99-7.01 (m, 2H), 7.25-7.27(m, 2H), 7.66 (s, 2H), 8.87(s, 1H) |
| 244 | 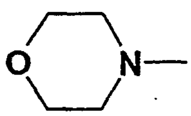 | 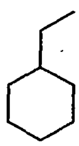 | 0.28 (n-hexane: AcOEt= 2:1) | 0.97- 1.06(m, 2H), 1.13-1.43(m, 4H), 1.68-1.77(m, 5H), 1.81-1.84 (m, 2H), 2.49(t, 4H), 3.69-3.72(m, 6H), 4.40-4.44(m, 2H), 6.52(s, 1H), 8.88(s, 1H) |
| 245 | 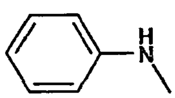 | 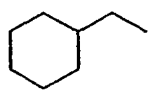 | (MS) 360.1 | (DMSO-d6) 0.90-0.99(m, 2H), 1.11-1.19(m, 3H), 1.23-1.30 (m, 1H), 1.62-1.79(m, 7H), 4.37(t, 2H), 4.60(s, 2H), 6.57(t, 1H), 6.67(d, 2H), 6.70(s, 1H), 7.08 (dd, 2H), 9.05(s, 1H), |
| 246 | 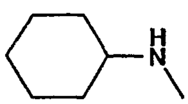 | 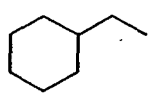 | (MS) 366.0 | 8.95(s, 1H), 6.90(s,1H), 4.35(t, 2H), 4.29(s, 2H), 2.99(br,1H), 1.96-1.93 (m,2H), 1.79-1.69(m, 8H), 1.54(dd,2H), 1.30-1.14 (m,8H), 1.03-0.93(m,3H) |

(continued)

| Ex. | R' | R" | Rf (solvent) | NMR (400MHz, δ; CDCl3) |
|---|---|---|---|---|
| 247 | 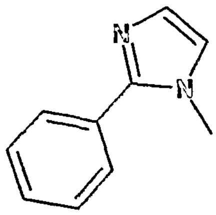 | 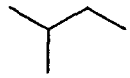 | (MS) 371.0 | 0.85(d, 6H), 1.37-1.46 (m, 3H), 4.08(t, 2H), 5.73(s, 2H), 6.59(s, 1H), 7.20(d, 1H), 7.42(d, 1H), 7.56(t, 2H), 7.65(t, 1H), 7.79(d, 2H), 8.99(s, 1H) |
| 248 | 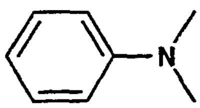 | 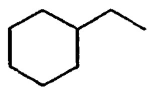 | (MS) 374.1 | 0.90-0.98(m, 2H), 1.14-1.28(m, 4H), 1.53-1.58(m, 2H), 1.64-1.75(m, 5H), 3.17 (s, 3H), 4.14(t, 2H), 4.73 (s, 2H), 6.74(s, 1H), 7.09-7.12(m, 3H), 7.33-7.39(m, 2H), 8.92 (s,1H) |
| 249 | 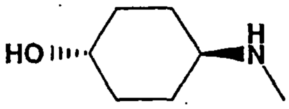 | 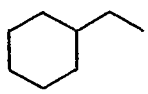 | (MS) 382.1 | 0.96-1.04(m, 2H), 1.54 (dd, 2H), 1.15-1.82(m, 15H), 2.10(dd, 2H), 2.58 (br, 1H), 3.65(br, 1H), 4.02(s, 2H), 4.38(t, 2H), 6.56(s, 1H), 8.88(s, 1H), |
| 250 | 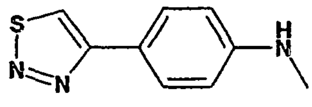 | 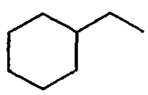 | (MS) 444.0 | 0.99-1.05(m, 2H), 1.18-1.25(m, 3H), 1.31-1.37(m, 1H), 1.66-1.83(m, 7H), 4.37 (t, 2H), 4.65(s, 2H), 6.64 (s, 1H), 6.79(d, 2H), 7.92 (d, 2H), 8.46(s, 1H), 8.90 (s, 1H), |
| 251 | 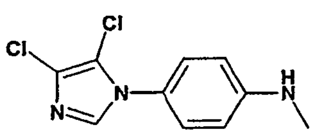 | 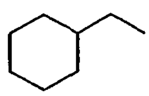 | (MS) 494.0 | 0.99-1.05(m, 2H), 1.15-1.25(m, 3H), 1.31-1.38(m, 1H), 1.68-1.82(m, 7H), 4.37 (t, 2H), 4.62(s, 2H), 6.63 (s, 1H), 6.75(d, 2H), 7.18 (d, 2H), 7.48(s, 1H), 8.90 (s, 1H) |
| 252 | 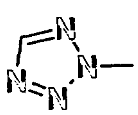 | 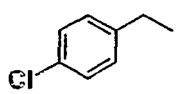 | 0.65 (*n*-hexane: AcOEt= 1:1) | 3.02(t, 2H), 4.61 (t, 2H), 5.56(s, 2H), 6.77(s, 1H), 6.97(d, 2H), 7.28(d, 2H), 8.54(s, 1H), 9.02(s, 1H) |
| 253 | 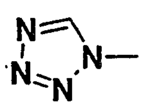 | 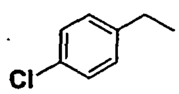 | 0.17 (*n*-hexane: AcOEt= 1:1) | 3.05(t, 2H), 4.54(t, 2H), 5.24(s, 2H), 6.55(s, 1H), 6.93(d, 2H), 7.29(d, 2H), 8.48(s, 1H), 9.04(s, 1H) |

(continued)

| Ex. | R' | R" | Rf (solvent) | NMR (400MHz, δ; CDCl3) |
|---|---|---|---|---|
| 254 | 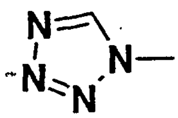 | 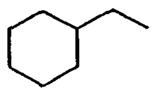 | 0.16 (n-hexane: AcOEt= 1:1) | 0.92-1.05(m, 2H), 1.11-1.40(m, 4H), 1.52-1.82(m, 7H), 4.41 (t, 2H), 6.05(s, 2H), 6.81 (s, 1H), 8.57(s, 1H), 9.00 (s, 1H) |
| 255 | 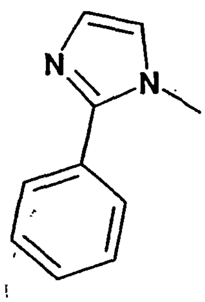 | 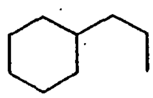 | 0.15 (diethyl ether: AcOEt = 1:1) | 0.85-0.90(m, 2H), 1.10-1.18(m, 4H), 1.38-1.44(m, 2H), 1.63-1.69(m, 5H), 4.08 (dd, 2H), 5.42(s, 2H), 6.44(s, 1H), 7.00(d, 1H), 7.24(d, 1H), 7.43-7.46 (m, 3H), 7.54-7.55(m, 2H), 8.92(s, 1H) |
| 256 | 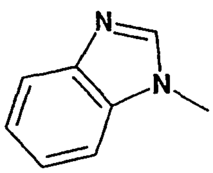 | 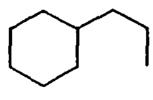 | 0.21 (dichlorom ethane: MeOH = 9:1) | 0.90-0.95(m, 2H), 1.15-1.25(m, 4H), 1.46-1.50(m, 2H), 1.65-1.71 (m, 5H), 4.26 (dd, 2H), 5.59(s, 2H), 6.41 (s, 1H), 7.30-7.37 (m, 3H), 7.88(dd, 1H), 7.95(s, 1H), 8.90(s, 1H) |
| 257 | 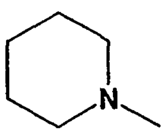 | 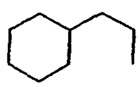 | 0.46 (n-hexane: AcOEt= 1:1) | 0.97-1.07(m, 2H), 1.17-1.29(m, 3H), 1.34-1.39(m, 1H), 1.43-1.47(m, 2H), 1.55-1.60(m, 4H), 1.68-1.76(m, 5H), 1.80-1.84 (m, 2H), 2.41 (brs, 4H), 3.62(s, 2H), 4.42(dd, 2H), 6.48(s, 1H), 8.86(s, 1H) |
| 258 | 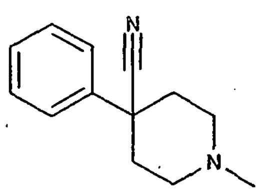 | 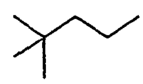 | 0.59 (n-hexane: AcOEt= 1:1) | 1.08(s, 9H), 1.69-1.74 (m, 2H), 2.07-2.17(m, 4H), 2.65(dt, 2H), 3.00 (brd, 2H), 3.81 (s, 2H), 4.41- 4.45(m, 2H), 6.56 (s, 1H), 7.35-7.37(m, 1H), 7.42(dd, 2H), 7.48 (d, 2H), 8.90(s, 1H) |
| 259 | 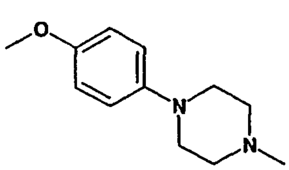 | 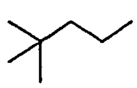 | 0.10 (n-hexane: diethyl-ether = 4:3) | 1.06(s, 9H), 1.71-1.75 (m, 2H), 2.65(dd, 4H), 3.09(dd, 4H), 3.75(s, 2H), 3.77(s, 3H), 4.43-4.47(m, 2H), 6.54 (s, 1H), 6.83-6.90(m, 4H), 8.89(s, 1H) |

(continued)

| Ex. | R' | R" | Rf (solvent) | NMR (400MHz, δ; CDCl3) |
|---|---|---|---|---|
| 260 | 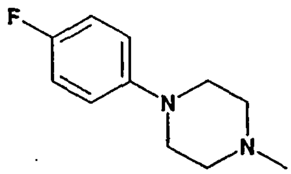 | 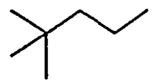 | 0.56 (n-hexane: AcOEt= 1:1) | 1.04(s, 9H), 1.66-1.70 (m, 2H), 1.91 (s, 3H), 2.02-2.08(m, 2H), 2.33-2.38(m, 2H), 2.45-2.48(m, 2H), 2.69-2.72(m, 2H), 3.65 (s, 2H), 4.40-4.45(m, 2H), 6.48(s, 1H), 7.27-7.30(m, 3H), 7.35-7.38(m, 2H), 8.87 (s, 1H) |
| 261 | 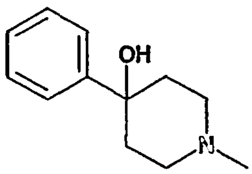 | 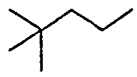 | 0.43 (n-hexane: AcOEt= 1:1) | 1.06(s, 9H), 1.70-1.75 (m, 2H), 2.65-2.67(m, 4H), 3.11-3.13(m, 4H), 3.76(s, 2H), 4.43-4.47 (m, 2H), 6.55(s, 1H), 6.85-6.88(m, 2H), 6.94-6.98(m, 2H), 8.89 (s, 1H) |
| 262 | 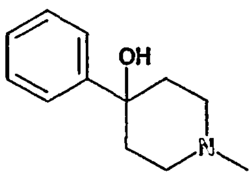 | 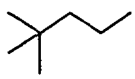 | 0.29 (n-hexane: AcOEt= 1:1) | 1.08(s, 9H), 1.72-1.82 (m, 4H), 2.08-2.16(m, 2H), 2.58-2.64(m, 2H), 2.76- 2.79(m, 2H), 3.77 (s, 2H), 4.45-449(m, 2H), 6.54(s, 1H), 7.27-7.30(m, 1H), 7.37 (ddd, 2H), 7.48- 7.51 (m, 2H), 8.87(s, 1H) |
| 263 | 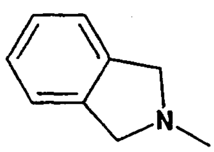 AA | 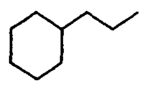 | Free salt 0.58 (n-hexane: AcOEt = 1:1) | DMSO-d6 0.89-0.98(m, 2H), 1.08-1.25(m, 4H), 1.54-1.66(m, 5H), 1.76-1.78(m, 2H), 4.46 (dd, 2H), 4.67(brs, 2H), 4.80(brs, 2H), 5.02 (brs, 2H), 36-7.41 (m, 5H), 9.27(s, 1H), 12.49(brs, 1H) |
| 264 | 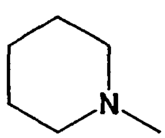 AA | 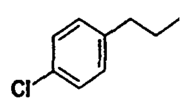 | Free salt 0.56 (n-hexane: AcOEt = 1:1) | DMSO-d6 1.28-1.37(m, 1H), 1.68-1.79(m, 5H), 4.46-4.72(m, 4H), 3.35-3.38(m, 2H), 4.46-4.47(m, 2H), 4.70 (dd, 2H), 7.03(d, 2H), 7.20(d, 2H), 7.28(s, 1H), 9.20(s, 1H), 10.96(brs, 1H) |

(continued)

| Ex. | R' | R'' | Rf (solvent) | NMR (400MHz, δ; CDCl3) |
|---|---|---|---|---|
| 265 | 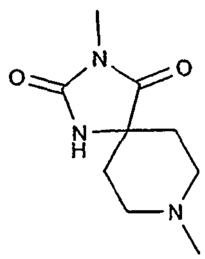 | 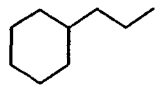 | 0.09 (n-hexane: AcOEt= 1:1) | 0.98-1.07(m, 2H), 1.18-1.41 (m, 4H), 1.68-1.84(m, 9H), 2.11-2.16(m, 2H), 2.27-2.32(m, 2H), 2.92-2.99(m, 2H), 3.03(s, 3H), 3.73-3.78(m, 2H), 4.40- 4.44(m, 2H), 5.84 (brs, 1H), 6.53(s, 1H), 8.89(s, 1H) |
| 266 | 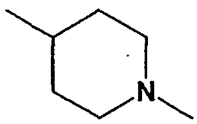 | 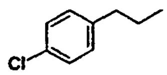 | 0.60 (n-hexane: AcOEt= 1:1) | 0.93(d, 3H), 1.15-1.28 (m, 2H), 1.34-1.46(m, 1H), 1.61-1.65(m, 2H), 1.99(brdd, 2H), 2.75-2.78 (m, 2H), 3.13 (dd, 2H), 3.41(s, 2H), 4.. 59(dd, 2H), 6.45(s, 1H), 7.06-7.09(m, 2H), 7.25-7.27(m, 2H), 8.88 (s, 1H) |
| 267 | 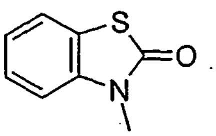 | 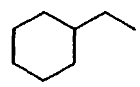 | 0.55 (n-hexane: AcOEt= 1:1) | 0.95-1.05(m, 2H), 1.15-1.30(m, 3H), 1.32-1.42(m, 1H), 1.58-1.62(m, 2H), 1.75-1.82(m, 5H), 4.40-4.44(m, 2H), 5.37 (d, 2H), 6.46(s, 1H), 7.01 (dd, 1H), 7.19-7.30(m, 2H), 7.50(dd, 1H), 8.87 (s, 1H) |
| 268 | 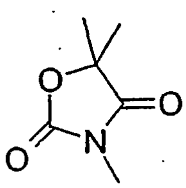 | 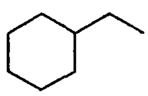 | 0.59 (n-hexane: AcOEt= 1:1) | 0.98-1.06(m, 2H), 1.20-1.28(m, 3H), 1.32-1.40(m, 1H), 1.61 (s, 6H), 1.63-1.69(m, 3H), 1.73-1.77(m, 2H), 1.84(d, 2H), 4.45-4.49 (m, 2H), 4.91 (s, 2H), 6.69(s, 1H), 8.94(s, 1H) |
| 269 | 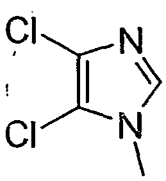 | 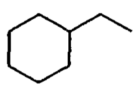 | 0.33 (n-hexane: AcOEt= 1:1) | 0.95-1.03(m, 2H), 1.18-1.26(m, 3H), 1.30-1.40(m, 1H), 1.51-1.55(m, 2H), 1.70-1.80(m, 5H), 4.26-4.30(m, 2H), 5.32(s, 2H), 6.43(s, 1H), 7.42(s, 1H), 8.96(s, 1H) |

(continued)

| Ex. | R' | R" | Rf (solvent) | NMR (400MHz, δ; CDCl3) |
|---|---|---|---|---|
| 270 | 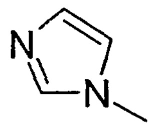 | 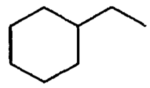 | MS 335.1 (M+H) | 0.91-1.01(m, 2H), 1.15-1.25(m, 3H), 1.27-1.34(m, 1H), 1.44-1.50(m, 2H), 1.66-1.77(m, 5H), 4.23 (t, 2H), 5.42(s, 2H), 6.51 (s, 1H), 6.96(s, 1H), 7.19 (s, 1H), 7.83(s, 1H), 8.95 (s, 1H) |
| 271 | 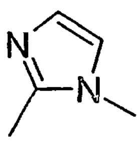 | 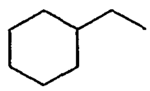 | MS 349.1 (M+H) | (CDCl3) 0.96-1.04(m, 2H), 1.16-1.26(m, 3H), 1.26-1.35(m, 1H), 1.54-1.60(m, 2H), 1.67-1.80(m, 5H), 2.44 (s, 3H), 4.26(t, 2H), 5.27 (s, 2H), 6.25(s, 1H), 6.85 (s, 1H), 7.04(s, 1H), 8.91 (s, 1H) |
| 272 | 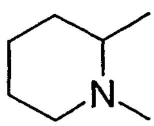 | 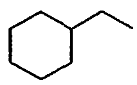 | MS 366.1 (M+H) | 0.97-1.06(m, 2H), 1.14-1.28(m, 6H), 1.32-1.45(m, 4H), 1.50-1.51 (m,2H), 1.67-1.79(m, 6H), 1.82-1.85(m, 2H), 1.99-2.03(m, 1H), 2.38 (br, 1H), 2.63(br, 1H), 3.31 (3.35)(s, 1H), 4.16 (4.20)(s, 1H), 4.34-4.42 (m, 1H), 4.46-4.53(m, 1H), 6.48(s, 1H), 8.85(s, 1H) |
| 273 | 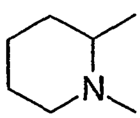 | 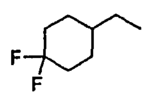 | MS 402.1 (M+H) | (DMSO-d6) 1.15(d, 3H), 1.25-1.34 (m, 5H), 1.45-1.52(m, 2H), 1.59-1.65(m, 2H), 1.72-1.90(m, 6H), 2.01-2.06(m, 3H), 2.40 (br, 1H), 2.55- 2.59(m, 1H), 3.41(3.44)(s, 1H), 4.22(4.25)(s, 1H), 4.31-4.39(m, 1H), 4.41-4.48(m, 1H), 6.75(s, 1H), 9.06(s, 1H) |
| 274 | 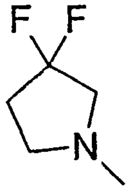 | 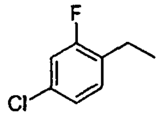 | 0.45 (n-hexane: AcOEt= 1:1) | 2.26-2.37(m, 2H), 2.79 (t, 2H), 2.92(t, 2H), 3.17 (t, 2H), 3.67(s, 2H), 4.60 (t, 2H), 6.53(s, 1H), 7.01- 7.09(m, 3H), 8.92 (s, 1H) |

(continued)

| Ex. | R' | R" | Rf (solvent) | NMR (400MHz, δ; CDCl3) |
|---|---|---|---|---|
| 275 | 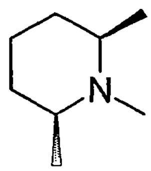 | 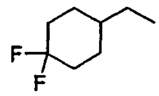 | 0.56 (n-hexane: AcOEt= 1:1) | (DMSO-d6) 1.21(d, 3H), 1.27-1.35 (m, 2H), 1.33(d, 3H), 1.41 (br, 1H), 1.61-2.00 (m, 14H), 3.58-3.66(m, 2H), 4.41 (4.47)(t, 2H), 4.57(4.83)(d, 2H), 7.55 (7.68)(s, 1H), 9.21(9.22) (s, 1H), 10.41 (10.49) (br, 1H) |
| 276 | 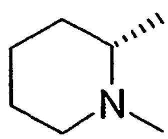 | 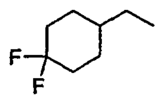 | 0.56 (n-hexane: AcOEt= 1:1) | 1.19(d, 3H), 1.34-1.50 (m, 7H), 1.65-1.84(m, 6H), 1.89-2.03(m, 3H), 2.08 -2.17(m, 2H), 2.38 (br, 1H), 2.60-2.63(m, 1H), 3.33(d, 1H), 4.18(d, 1H), 4.35- 4.43(m, 1H), 4.49-4.56(m, 1H), 6.50 (s, 1H), 8.86(s, 1H) |
| 277 | 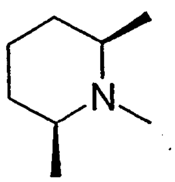 | 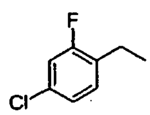 | 0.62 (n-hexane: AcOEt= 1:1) | (DMSO-d6) 1.15(1.28)(d, 6H), 1.61-1.78(m, 4H), 1.87-1.93(m, 2H), 3.07-3.14(m, 2H), 3.55-3.64(m, 2H), 4.42 (d, 1H), 4.64-4.73(m, 3H), 7.03-7.15(m, 2H), 7.23 -7.27(m, 1H), 7.51 (7.68)(s, 1H), 9.15(9.16) (s, 1H), 10.49(br, 1H) |
| 278 | 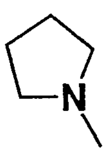 | 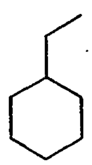 | 0.42 (*n*-hexane: AcOEt= 1:1) | 0.98-1.02(m, 2H), 1.21-1.34 (m, 4H), 1.64-1.82(m, 11H), 2.54 (br s, 4H), 3.80 (s, 2H), 4.38-4.42(m, 2H), 6.50 (s, 1H), 8.87(s, 1H) |
| 279 | 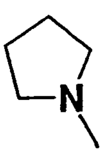 | 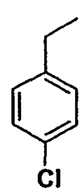 | 0.29 (*n*-hexane: AcOEt= 1:1) | 1.81 (br s, 4H), 2.50(br s, 4H), 3.12(t, 2H), 3.56 (s, 2H), 4.58(t, 2H), 6.47 (s, 1H), 7.05(d, 2H), 7.24 (d, 2H), 8.89(s, 1H) |
| 280 | 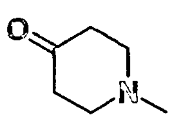 | 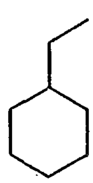 | 0.33 (*n*-hexane: AcOEt= 1:1) | 0.98-1.027(m, 2H), 1.12-1.45(m, 4H), 1.67-1.85(m, 7H), 2.47 (t, 4H), 2.82(t, 4H), 3.82 (s, 2H), 4.47(t, 2H), 6.55 (s, 1H), 8.90(s, 1H) |

(continued)

| Ex. | R' | R" | Rf (solvent) | NMR (400MHz, δ; CDCl3) |
|---|---|---|---|---|
| 281 | 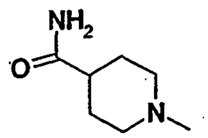 | 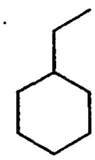 | 0.41 (Dichloromethane: MeOH= 9:1) | 0.95-1.08(m, 2H), 1.05-1.41 (m, 4H), 1.64-1.91 (m, 11H), 2.08-2.25(m, 3H), 2.92 (s, 2H), 3.68(s, 2H), 4.42 (t, 2H), 5.30(br d, 2H), 6.49(s, 1H), 8.87(s, 1H) |
| 282 | 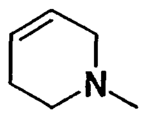 | 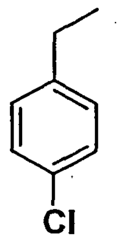 | 0.60(n-hexane: AcOEt= 1:1) | 2.10-2.25(m, 2H), 2.57 (t, 2H), 2.94-2.98(m, 2H), 3.13(t, 2H), 3.51 (s, 2H), 4.58(t, 2H), 5.61-5.70(m, 1H), 5.75-5.82(m, 1H), 6.50 (s, 1H), 7.07(d, 2H), 7.24 (d, 2H), 8.90(s, 1H) |
| 283 | 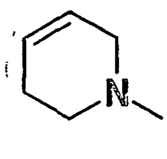 | 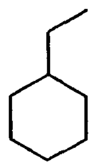 | 0.63 (n-hexane: AcOEt= 1:2) | 0.96-1.08(m, 2H), 1.12-1.40(m, 4H), 1.62-1.85(m, 7H), 2.12-2.20(m, 2H), 2.62 (t, 2H), 2.99(t, 2H), 3.76 (s, 2H), 4.39-4.44(m, 2H), 5.61-5.68(m, 1H), 5.74-5.82(m, 1H), 6.53 (s, 1H), 8.88(s, 1H) |
| 284 | 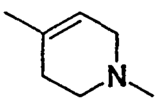 | 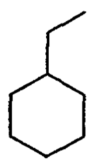 | 0.57 (n-hexane: AcOEt= 1:1) | 0.93-1.04(m, 2H), 1.17-1.41 (m, 4H), 1.63-1.84(m, 10H), 1.98-2.09(m, 2H), 2.61 (t, 2H), 2.94(br s, 2H), 3.74(s, 2H), 4.44(t, 2H), 5.33(br s, 1H), 6.52(s, 1H), 8.87(s, 1H) |
| 285 | 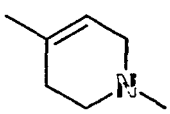 | 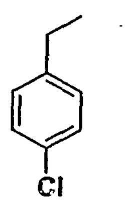 | 0.55(n-hexane: AcOEt= 1:1) | 1.70(s, 3H), 2.05(br s, 2H), 2.55(t, 2H), 2.91 (br s, 2H), 3.11 (t, 2H), 3.49 (s, 2H), 4.57(t, 2H), 5.36 (br s, 1H), 6.49(s, 1H), 7.04(d, 2H), 7.25(d, 2H), 8.90(s, 1H) |
| 286 | 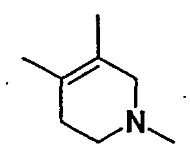 | 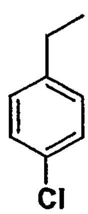 | 0.58 (*n*-hexane: AcOEt= 1:1) | 1.58(s, 3H), 1.64(s, 3H), 2.06(br s, 4H), 2.52(t, 2H), 2.79(br s, 2H), 3.11 (t, 2H), 3.48(s, 2H), 4.56 (t, 2H), 6.49(s, 1H), 7.04 (d, 2H), 7.24(d, 2H), 8.90(s, 1H) |

(continued)

| Ex. | R' | R" | Rf (solvent) | NMR (400MHz, δ; CDCl3) |
|---|---|---|---|---|
| 287 | 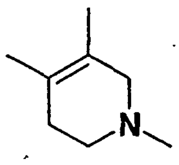 | 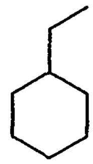 | 0.62(n-hexane: AcOEt= 1:1) | 0.95-1.04(m, 2H), 1.15-1.38 (m, 4H), 1.56 (s, 3H), 1.60-1.82(m, 10H), 2.17(br s, 2H), 2.57(t, 2H), 2.83(br s, 2H), 3.73(s, 2H), 4.41 (t, 2H), 6.52(s, 1H), 8.87(s, 1H) |
| 288 | 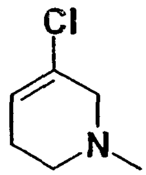 | 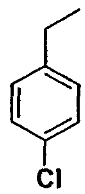 | 0.51 (n-hexane: AcOEt= 1:1) | 2.15-2.26(m, 2H), 2.58 (t, 2H), 3.05-3.18(m, 4H), 3.51 (s, 2H), 4.57(t, 2H), 5.90(br s, 1H), 6.51 (s, 1H), 7.01 (d, 2H), 7.25(d, 2H), 8.92(s, 1H) |
| 289 | 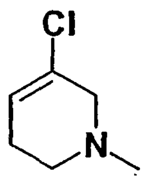 | 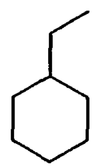 | 0.55(n-hexane: AcOEt= 1:1) | 0.93-1.05(m, 2H), 1.14-1.40(m, 4H), 1.61-1.82(m, 17H), 2.20-2.28(m, 2H), 2.65 (t, 2H), 3.13(br s, 2H), 3.81 (s, 2H), 4.40(t, 2H), 5.83-5.92(m, 1H), 6.54 (s, 1H), 8.90(s, 1H) |
| 290 | 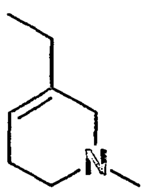 | 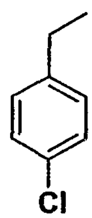 | 0.48 (*n*-hexane: AcOEt= 1:1) | 1.02(t, 3H), 1.90-1.99 (m, 2H), 2.10-2.18(m, 2H), 2.52(t, 2H), 2.86(br s, 1H), 3.12(t, 2H), 3.53 (s, 2H), 4.56(t, 2H), 5.48 (br s, 1H), 6.50(s, 1H), 7.07(d, 2H), 7.25(d, 2H), 8.91 (s, 1H) |
| 291 | 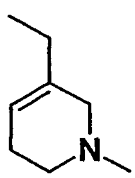 | 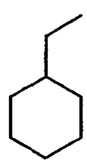 | 0.70 (*n*-hexane: AcOEt= 1:1) | 0.93-1.08(m, 5H), 1.15-1.40 (m, 4H), 1.62-1.85(m, 11H), 1.92 (br q, 2H), 2.10-2.18 (m, 2H), 2.56(s, 2H), 2.88 (br s, 2H), 3.76(s, 2H), 4.42 (t, 2H), 5.45-5.59 (m, 1H), 6.53(s, 1H), 8.88(s, 1H) |
| 292 | 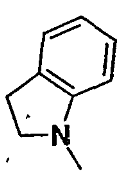 | 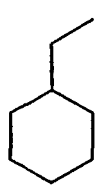 | 0.69 (*n*-hexane: AcOEt= 1:1) | 0.90-1.00(m, 2H), 1.12-1.39 (m, 4H), 1.59-1.80(m, 7H), 3.00 (t, 2H), 3.30(t, 2H), 4.39 (t, 2H), 4.42(s, 2H), 6.56 (d, 1H), 6.60(s, 1H), 6.78 (t, 1H), 7.11(t, 1H), 7.15 (d, 1H), 8.90(s, 1H) |

(continued)

| Ex. | R' | R" | Rf (solvent) | NMR (400MHz, δ; CDCl3) |
|---|---|---|---|---|
| 293 | 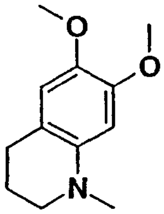 | 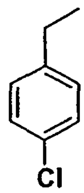 | 0.35 (*n*-hexane: AcOEt= 1:1) | 2.73(t, 2H), 2.80(t, 4H), 3.10(t, 2H), 3.53(s, 2H), 3.58(s, 2H), 3.82(s, 3H), 3.85(s, 3H), 4.61(t, 2H), 6.48(s, 1H), 6.54(s, 1H), 6.61 (s, 1H), 6.93(d, 2H), 7.17(d, 2H), 8.93(s, 1H) |
| 294 | 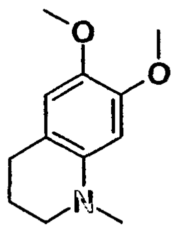 | 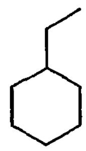 | 0.44 (*n*-hexane: AcOEt= 1:1) | 0.89-0.99(m, 2H), 1.08-1.48(m, 4H), 1.56-1.77(m, 7H), 2.76-2.87(m, 4H), 3.58 (s, 2H), 3.81 (s, 3H), 3.85(s, 3H), 3.87(s, 2H), 4.48(t, 2H), 6.47(s, 1H), 6.58(s, 1H), 6.61 (s, 1H), 8.91(s, 1H) |
| 295 | 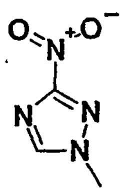 | 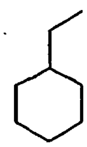 | 0.25 (*n*-hexane: AcOEt= 1:3) | 0.90-1.08(m, 2H), 1.18-1.42(m, 4H), 1.51-1.82(m, 7H), 4.39 (t, 2H), 5.73(s, 2H), 6.77 (s, 1H), 8.24(s, 1H), 9.05 (s, 1H) |
| 296 | 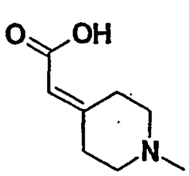 | 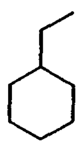 | 0.47 (*n*-hexane: AcOEt= 5:1) | 0.96-1.08(m, 2H), 1.12-1.42 (m, 4H), 1.68-1.89(m, 7H), 2.35-2.39(m, 2H), 2.57 -2.64 (m, 4H), 2.97- 3.04 (m, 2H), 3.70(s, 2H), 4.41-4.46 (m, 2H), 5.72 (s, 1H), 6.51 (s, 1H), 8.89(s, 1H) |

<u>Example 297: 7-(2-Cyclohexyl-ethyl)-6-(4-hydroxy-piperidin-1-ylmethyl)-7H-pyrrolo[2,3-d]pyrimidine-2-carbonitrile</u>

7-(2-Cyclohexyl-ethyl)-6-(4-oxo-piperidin-1-ylmethyl)-7H-pyrrolo[2,3-d]pyrimidine-2-carbonitrile is reduced in methanol by sodium borohydride to the corresponding alcohol; Rf=0.15(*n*-hexane:AcOEt=1:2). NMR (400MHz, $CDCl_3$, δ) 0.94-1.09(m, 2H), 1.15-1.42(m, 4H), 1.52-1.78(m, 11H), 1.80-1.94(m, 4H), 2.21-2.29(m, 2H), 2.74-2.78(m, 2H), 3.67 (s, 2H), 4.42(t, 2H), 6.49(s, 1H), 8.87(s, 1H).

<u>Example 298: 6-(8-Acetyl-2,8-diaza-spiro[4.5]dec-2-ylmethyl)-7-(3,3-dimethyl-butyl)-7H-pyrrolo[2,3-d]pyrimidine-2-carbonitrile</u>

To a solution of 6-Bromomethyl-7-(3,3-dimethyl-butyl)-7H-pyrrolo[2,3-d]pyrimidine -2-carbonitrile(440 mg, 1.37 mmol) in DMF(5 ml), 1-(2,8-diaza-spiro[4.5]dec-8-yl)- ethanone hydrochloride (Example ZG, 300 mg, 1.37 mmol) and $K_2CO_3$(568 mg, 4.11 mmol) and triethylamine(5 ml) are added. The mixture is stirred at room temperature under nitrogen atomosphere for 11 h. The reaction mixture is diluted with water and extracted with AcOEt(twice). The combined organic layer is washed with water and brine, dried over $MgSO_4$, and concentrated in vacuo. The residue is purified by silica gel column chromatography(n-hexane : AcOEt=1:1) to to provide the title compound; Rf=0.30(*n*-hexane:AcOEt = 1: 1); $^1$H-NMR(400MHz, $CDCl_3$) δ : 1.05(s, 9H), 1.53-1.72(m, 8H), 2.07(s, -3H), 2.40-2.48(m, 2H), 2.60-2.69(m, 2H), 3.35-3.45(m, 2H), 3.60-3.67(m, 1H), 3.74-3.82(m, 2H), 4.40-4.44(m, 2H), 6.49(s, 1H), 8.87(s, 1H).

Examples 299 to 330

By repeating the procedures described in Example 298 using appropriate starting materials (including some of those prepared in Examples A to X and ZA to ZV) the following compounds of formula 2 are obtained as identified below in Table 27.

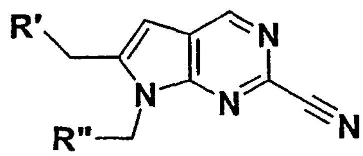

(2)

Table 27

| Ex. | R' | R" | NMR(400MHz, CDCl3, δ) |
|---|---|---|---|
| 299 | 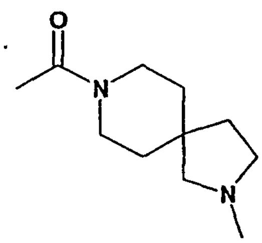 | 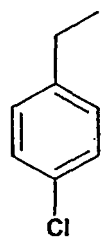 | 1.50-1.71 (m, 6H), 2.06(s, 3H), 2.32-2.41 (m, 2H), 2.48-2.65 (m, 2H), 3.10-3.14(m, 2H), 3.29-3.52(m, 5H), 3.62-3.69 (m, 1H), 4.58-4.61 (m, 2H), 6.46(s, 1H), 6.99-7.01 (m, 2H), 7.23-7.26 (m, 2H), 8.89 (s, 1H). |
| 300 | 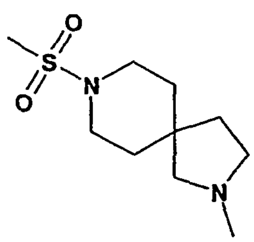 | 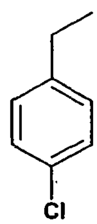 | 1.53-1.55(m, 2H), 1.63-1.70(m, 6H), 2.35(s, 2H), 2.56-2.60(m, 2H), 2.75(s, 3H), 3.05-3.13(m, 2H), 3.20-3.26(m, 2H), 3.46(s, 2H), 4.57-4.61 (m, 2H), 6.45 (s, 1H), 6.97-6.99(m, 2H), 7.22- 7.25(m, 2H), 8.90(s, 1H). |
| 301 | 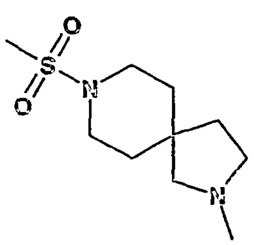 | 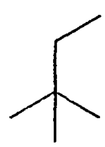 | 1.04(s, 9H), 1.66-1.70(m, 8H), 2.43(brs, 2H), 2.62-2.65(m, 2H), 2.75(s, 3H), 3.09-3.15(m, 2H), 3.20-3.25(m, 2H), 3.78(s, 2H), 4.39-4.43(m, 2H), 6.49 (s, 1H), 8.88(s, 1H). |
| 302 | 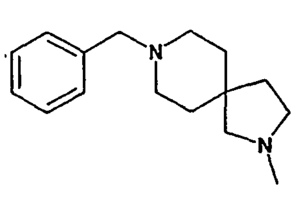 | 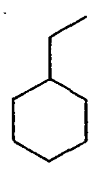 | 0.97-1.03(m, 2H), 1.15-1.34(m, 5H), 1.56-1.80(m, 12H), 2.35- 2.40(m, 6H), 2.55-2.58(m, 2H), 3.45(s, 2H), 3.75(s, 2H), 4.38-4.41 (m, 2H), 6.47(s, 1H), 7.29-7.30(m, 5H), 8.86(s, 1H). |

(continued)

| Ex. | R' | R" | NMR(400MHz, CDCl3, δ) |
|---|---|---|---|
| 303 | 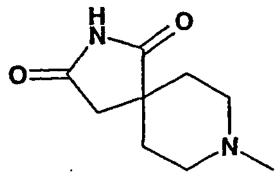 | 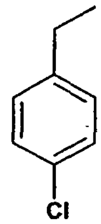 | 1.53-1.60(m, 4H), 2.09-2.16(m, 4H), 2.59(s, 2H), 2.80-2.83(m, 2H), 3.12-3.14(m, 2H), 3.37(s, 2H), 4.55-4.64(m, 2H), 6.47(s, 1H), 6.99-7.03(m, 2H), 7.23-7.26 (m, 2H), 7.75(brs, 1H), 8.90(s, 1H). |
| 304 | 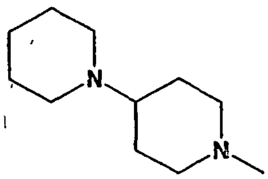 | 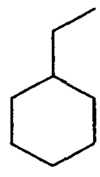 | 0.96-1.87(m, 26H), 2.06-2.08 (m, 2H), 2.55-2.65(m, 4H), 2.92- 2.95(m, 2H), 3.65(s, 2H), 4.38- 4.42 (m, 2H), 6.48(s, 1H), 8.87 (s, 1H). |
| 305 | 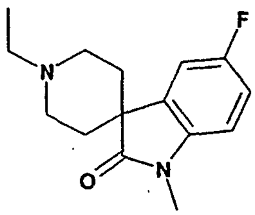 | 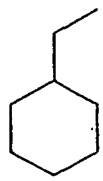 | 0.98-1.39(m, 9H), 1.65-1.82(m, 7H), 1.99-2.03(m, 4H), 2.59-2.64 (m, 2H), 2.74-2.77(m, 2H), 2.92- 2.98 (m, 2H), 4.36-4.39(m, 2H), 5.10(s, 2H), 6.40(s, 1H), 6.69 -6.72(m, 1H), 6.88-6.93(m, 1H), 7.16-7.18(m, 1H), 8.86(s, 1H). |
| 306 | 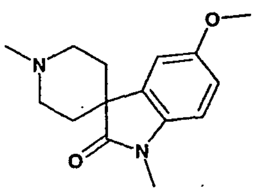 | 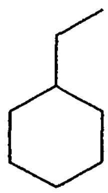 | 0.97-1.39(m, 6H), 1.60-1.82(m, 8H), 1.98-2.00(m, 3H), 2.46(s, 3H), 2.71-2.74(m, 2H), 2.92- 2.94(m, 2H), 3.77(s, 3H), 4.36- 4.40(m, 2H), 5.09(s, 2H), 6.40 (s, 1H), 6.66-6.73(m, 2H), 7.02 (d, 1H), 8.85(s, 1H). |
| 307 | 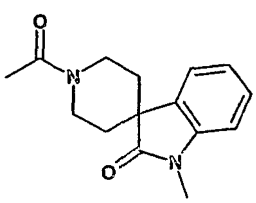 | 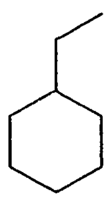 | 1.02-1.42(m, 6H), 1.68-1.95(m, 11H), 2.12(s, 3H), 3.75-3.85(m, 2H), 4.01-4.07(m, 1H), 4.24- 4.29(m, 1H), 4.40-4.44(m, 2H), 5.16(s, 2H), 6.44(s, 1H), 6.84-6.86(m, 1H), 7.11-7.15(m, 1H), 7.24-7.33(m, 2H), 8.85 (s, 1H). |
| 308 | 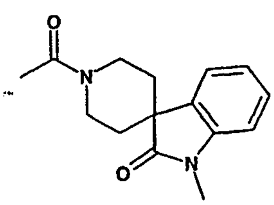 | 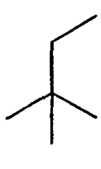 | 1.09(s, 9H), 1.70-1.74(m, 2H), 1.88-1.94(m, 4H), 2.19 (s, 3H), 3.74-3.81 (m, 2H), 4.04-4.14(m, 1H), 4.26-4.29(m, 1H), 4.38- 4.42(m, 2H), 5.13(s, 2H), 6.38 (s, 1H), 6.80(d, 1H), 7.11-7.15 (m, 1H), 7.23-7.32(m, 2H), 8.85 (s, 1H). |
| 309 | 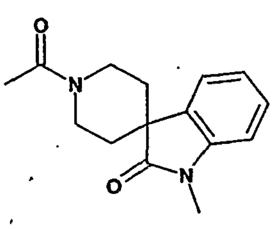 | 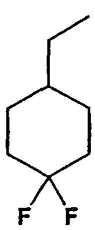 | 1.38-1.93(m, 13H), 2.08-2.17 (m, 2H), 2.19(s, 3H), 3.72-3.84 (m, 2H), 3.99-4.06(m, 1H), 4.23 -4.29(m, 1H), 4.41-4.45(m, 2H), 5.12(s, 2H), 6.48(s, 1H), 6.84-6.86(m, 1H), 7.11-7.15 (m, 1H), 7.24-7.32(m, 2H), 8.89 (s, 1H) |

(continued)

| Ex. | R' | R" | NMR(400MHz, CDCl3, δ) |
|---|---|---|---|
| 310 | 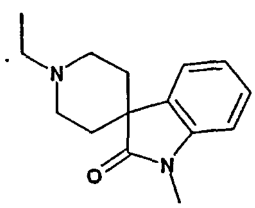 | 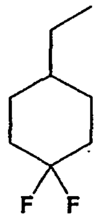 | (DMSO-$d_6$)<br>1.07(t, 3H), 1.24-1.46(m, 3H), 1.69-2.02(m, 12H), 2.60-2.75 (m, 2H), 2.80-2.90(m, 2H), 3.25 -3.36(m, 2H), 4.40-4.44(m, 2H), 5.26(s, 2H), 6.54(s, 1H), 7.04-7.09(m, 2H), 7.22-7.25(m, 1H), 7.55-7.57(m, 1H), 9.02 (s, 1H). |
| 311 | 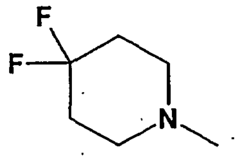 | 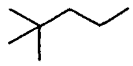 | (DMSO-$d_6$)<br>1.03(s, 9H), 1.66-1.70(m, 2H), 1.92-2.00(m, 4H), 2.55-2.59(m, 4H), 3.87(s, 2H), 4.37-4.41 (m, 2H), 6.78 (s, 1H), 9.09(s, 1H) |
| 312 | 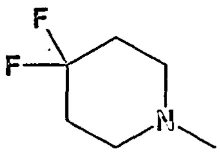 | 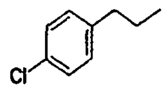 | (DMSO-$d_6$)<br>1.92-1.99(m, 4H), 2.52-2.56(m, 4H), 3.1 1 (dd, 2H), 3.66(s, 2H), 4.60(dd, 2H), 6.74(s, 1H), 7.11 (d, 2H), 7.27-7.29(m, 2H), 9.07 (s, 1H) |
| 313 | 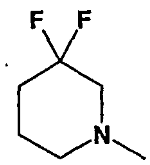<br>HCl | 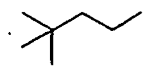 | (DMSO-$d_6$) (AA)<br>1.01 (s, 9H), 1.66-1.71 (m, 4H), 1.91-1.94(m, 2H), 2.50-2.55(m, 2H), 2.72-2.77(m, 2H), 3.93 (brs, 2H), 4.35-4.39(m, 2H), 6.82(s, 1H), 9.11 (s, 1H) |
| 314 | 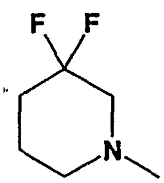 | 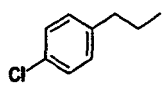 | 1.68-1.74(m, 2H), 1.82-1.92(m, 2H), 2.37-2.40(m, 2H), 2.58(dd, 2H), 3.07(dd, 2H), 3.39(s, 2H), 4.53(dd, 2H), 6.45(s, 1H), 6.98 (dd, 2H), 7.17-7.20(m, 2H), 8.85 (s, 1H) |
| 315 | 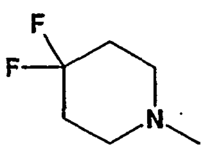 | 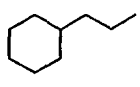 | DMSO-d6 0.96-1.02(m, 2H), 1.17-1.33(m, 4H), 1.61-1.71(m, 5H), 1.76- 1.80(m, 2H), 1.91-2.01 (m, 4H), 2.56-2.61 (m, 4H), 3.86(s, 2H), 4.35-4.39(m, 2H), 6.78(s, 1H), 9.08(s, 1H) |
| 316 | 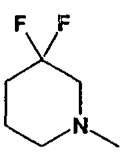 | 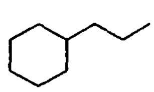 | 0.92-1.02(m, 2H), 1.16-1.39(m, 4H), 1.60-1.82(m, 9H), 1.90-1.99 (m, 2H), 2.51-2.54(m, 2H), 2.72 (t, 2H), 3.80(s, 2H), 4.39- 4.43 (m, 2H), 6.56(s, 1H), 8.90 (s, 1H) |
| 317 | 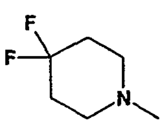 | 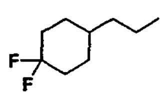 | 1.32-1.46(m, 3H), 1.64-1.81 (m, 4H), 1.89-2.04(m, 6H), 2.09- 2.16(m, 2H), 2.61-2.65(m, 4H), 3.75(s, 2H), 4.41-4.45(m, 2H), 6.55(s, 1H), 8.91 (s, 1H) |

(continued)

| Ex. | R' | R" | NMR(400MHz, CDCl3, δ) |
|---|---|---|---|
| 318 | 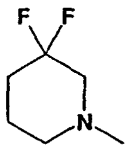 | 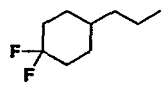 | 1.29-1.43(m, 4H), 1.69-1.75(m, 5H), 1.83-1.91 (m, 4H), 2.02- 2.08(m, 2H), 2.45-2.48(m, 2H), 2.65(t, 2H), 3.73(s, 2H), 4.34- 4.38(m, 2H), 6.50(s, 1H), 8.85 (s, 1H) |
| 319 | 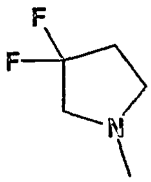 | 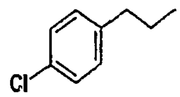 | 2.26-2.37(m, 2H), 2.75(dd, 2H), 2.90(t, 2H), 3.13(dd, 2H), 3.53 (s, 2H), 4.57(dd, 2H), 6.51(s, 1H), 7.02(dd, 2H), 7.24(d, 2H), 8.93(s, 1H) |
| 320 | 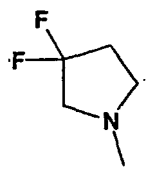 | 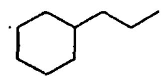 | 0.96-1.05(m, 2H), 1.16-1.39(m, 4H), 1.65-1.82(m, 7H), 2.26- 2.37(m, 2H), 2.81 (dd, 2H), 2.96 (t, 2H), 3.85(s, 2H), 4.36-4.40 (m, 2H), 6.55(s, 1H), 8.90(s, 1H) |
| 321 | 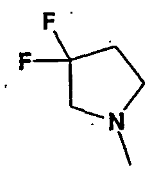 | 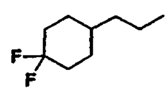 | DMSO 1.19-1.28(m, 2H), 1.43-1.52(m, 1H), 1.70-1.88(m, 6H), 1.98- 2.04(m, 2H), 2.21-2.32(m, 2H), 2.78(dd, 2H), 2.96(t, 2H), 3.97 (s, 2H), 4.33-4.37(m, 2H), 6.79 (s, 1H), 9.10(s, 1H) |
| 322 | 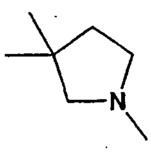 | 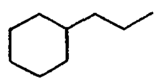 | 0.96-1.06(m, 2H), 1.08(s, 6H), 1.19-1.43(m, 4H), 1.58-1.83(m, 9H), 2.33(s, 2H), 2.63(dd, 2H), 3.77(s, 2H), 4.41-4.45(m, 2H), 6.48(s, 1H), 8.86(s, 1H) |
| 323 | 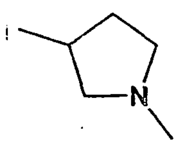 AA | 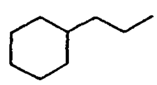 | 0.96-1.04(m, 5H), 1.13-1.41 (m, 5H), 1.66-1.76(m, 5H), 1.77- 1.83(m, 2H), 1.99-2.08(m, 1H), 2.11-2.15 (m, 1H), 2.20-2.31 (m, 1H), 2.53-2.67(m, 2H), 2.76(dd, 1H), 3.79(brs, 2H), 4.38-4.42 (m, 2H), 6.50(s, 1H), 8.86 (s, 1H) |
| 324 | 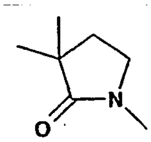 | 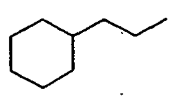 | 0.96-1.04(m, 2H), 1.18-1.39(m, 11H), 1.59-1.82(m, 6H), 1.89 (dd, 2H), 3.22(dd, 2H), 4.29- 4.33(m, 2H), 4.63(s, 2H), 6.52 (s, 1H), 8.91 (s, 1H) |
| 325 | 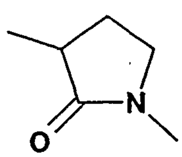 | 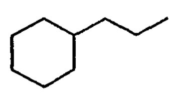 | 0.95-1.05(m, 2H), 1.15-1.42(m, 7H), 1.56-1.82(m, 8H), 2.24-2.32 (m, 1H), 2.49-2.59(m, 1H), 3.19- 3.27 (m, 2H), 4.30-4.34(m, 2H), 4.60-4.64(m, 1H), 4.74-4.78 (m, 1H), 6.53(s, 1H), 8.91 (s, 1H) |

(continued)

| Ex. | R' | R" | NMR(400MHz, CDCl3, δ) |
|---|---|---|---|
| 326 | 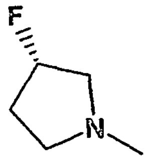 | 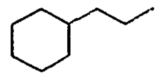 | 0.96-1.02(m, 2H), 1.04-1.37(m, 4H), 1.66-1.82(m, 6H), 2.04- 2.21 (m, 2H), 2.48-2.54(m, 1H), 2.72-2.95 (m, 3H), 3.86(s, 2H), 4.38-4.42(m, 2H), 5.08-5.12(m, 1H), 5.23-5.28(m, 1H), 6.52(s, 1H), 8.88(s, 1H) |
| 327 | 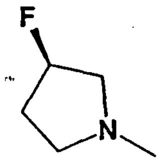 | 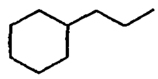 | 0.94-1.04(m, 2H), 1.15-1.39(m, 4H), 1.65-1.82(m, 6H), 2.01- 2.23(m, 2H), 2.49-2.54(m, 1H), 2.80-2.95 (m, 3H), 3.87(s, 2H), 4.38-4.42(m, 2H), 5.10-5.13(m, 1H), 5.24-5.28(m, 1H), 6.53(s, 1H), 8.88(s, 1H) |
| 328 | 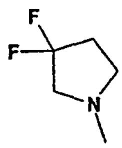 | 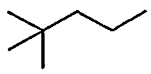 | 1.08(s, 9H), 1.56-1.60(m, 2H), 2.68-2.74(m, 2H), 3.62-3.72(m, 4H), 4.49-4.53(m, 4H), 7.31 (s, 1H), 9.04 (s, 1H) |
| 329 | 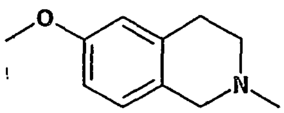 | 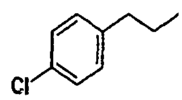 | 2.71-2.74(m, 2H), 2.84-2.89(m, 2H), 3.09(dd, 2H), 3.54-3.59(m, 4H), 3.78(s, 3H), 4.59(dd, 2H), 6.55(s, 1H), 6.55(d, 1H), 6.71 -6.74(m, 1H), 6.90-6.93(m, 3H), 7.14-7.18(m, 2H), 8.92(s, 1H) |
| 330 | 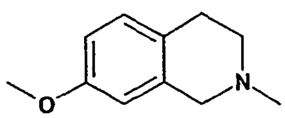 | 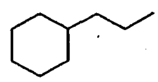 | CDCl3 0.87-0.97(m, 2H), 1.09-1.34(m, 4H), 1.59-1.75 (m, 7H), 2.76- 2.82(m, 2H), 2.86-2.90(m, 2H), 3.61 (s, 2H), 3.78(s, 3H), 3.86 (brs, 2H), 4.41-4.45(m, 2H), 6.58 (s, 1H), 6.65(d, 1H), 6.68- 6.72(m, 1H), 6.88-6.91 (m, 1H), 8.90(s, 1H) |

Example 331: 6-[3-(1-Acetyl-piperidin-4-yl)-2-oxo-2,3-dihydro-benzoimidazol-1-ylmethyl]-7-(2-cyclohexyl-ethyl)-7H-pyrrolo[2,3-d]pyrimidine-2-carbonitrile

To a solution of 7-(2-Cyclohexyl-ethyl)-6-(2-oxo-3-piperidin-4-yl-2,3-dihydro- benzoimidazol-1 -ylmethyl)-7H-pyrrolo[2,3-d]pyrimidine-2-carbonitrile trifluoroacetic acid salt(141 mg, 0.29 mol) in dichloromethane(2 ml), triethylamine (395 μl) and acetic anhydride(60 μl, 0.63 mmol) are added at 0°C. The reaction mixture is stirred for over night at room temperature, quenched with ice-water and extracted with ethyl acetate. The combined extracts are washed with $H_2O$, brine and dried over sodium sulphate. Chromatography on silica gel gives the title compound; Rf=0.30(*n*-hexane:AcOEt = 1:1); $^1$H-NMR(400MHz, $CDCl_3$) δ : 0.95-1.33(m, 5H), 1.53-1.96(m, 8H), 2.20(s, 3H), 2.32-2.41 (m, 2H), 2.66-2.72(m, 1H), 3.22-3.29(m, 1H), 4.01-4.11 (m, 1H), 4.40-4.44(m, 2H), 4.54-4.60(m, 1H), 4.87-4.91 (m, 1H), 5.29(s, 2H), 6.54(s, 1H), 6.96-7.17(m, 4H), 8.88(s, 1H).

Example 332: 7-(2-Cyclohexyl-ethyl)-6-[3-(1-methanesulfonyl-piperidin-4-yl)-2-oxo-2,3-dihydro-benzoimidazol-1-yl-methyl]-7H-pyrrolo[2,3-d]pyrimidine-2-carbonitrile

To a solution of 7-(2-Cyclohexyl-ethyl)-6-(2-oxo-3-piperidin-4-yl-2,3-dihydro- benzoimidazol-1-ylmethyl)-7H-pyrrolo[2,3-d]pyrimidine-2-carbonitrile trifluoroacetic acid salt(141 mg, 0.29 mol) in dichloromethane(2 ml), triethylamine (395 μl) and acetic anhydride(60 μl, 0.77 mmol) are added at 0°C. The reaction mixture is stirred for over night at room temperature, quenched with ice-water and extracted with ethyl acetate. The combined extracts are washed with $H_2O$, brine and dried over sodium sulphate. Chromatography on silica gel gives the title compound; Rf=0.30(*n*-hexane:AcOEt = 1:1). $^1$H-NMR(400MHz, $CDCl_3$) δ : 0.93-1.01(m, 2H), 1.13-1.33(m, 3H), 1.54-1.78(m, 8H), 1.95-2.04(m, 2H), 2.52-2.63 (m, 2H), 2.87-2.93(m, 5H), 4.03-4.06(m, 2H), 4.40-4.44(m, 2H), 4.50-4.56(m, 1H), 5.29(s, 2H), 6.53(s, 1H), 6.97-7.24 (m, 4H), 8.88(s, 1H).

Example 333: 6-(8-Acetyl-4-oxo-1-phenyl-1,3,8-triaza-spiro[4.5]dec-3-ylmethyl)-7-(2-cyclohexyl-ethyl)-7H-pyrrolo [2,3-d]pyrimidine-2-carbonitrile

To a solution of 7-(2-Cyclohexyl-ethyl)-6-(4-oxo-1-phenyl-1,3,8-triaza-spiro[4.5]dec-3-ylmethyl)-7H-pyrrolo [2,3-d]pyrimidine-2-carbonitrile trifluoroacetic acid salt(142 mg, 0.28 mol) in dichloromethane(2 ml), triethylamine(395 μl) and acetic anhydride (54 μl, 0.57 mmol) are added at 0˚C. The reaction mixture is stirred for over night at room temperature, quenched with ice-water and extracted with ethyl acetate. The combined extracts are washed with $H_2O$, brine and dried over sodium sulphate. Chromatography on silica gel gives the title compound; Rf=0.30(*n*-hexane:AcOEt = 1:1). $^1$H-NMR(400MHz, $CDCl_3$) δ : 0.97-1.40(m, 6H), 1.64-1.82(m, 9H), 2.14(s, 3H), 2.37-2.44(m, 2H), 3.40-3.48(m, 1H), 3.74-3.79(m, 1H), 3.93-4.01(m, 1H), 4.34-4.38(m, 2H), 4.56-4.66(m, 3H), 4.87(s, 2H), 6.61 (s, 1H), 6.74-6.76(m, 2H), 6.91-6.95(m, 1H), 7.23-7.25(m, 2H), 8.94(s, 1H).

Example 334: 7-[2-(4-Chloro-phenyl)-ethyl]-6-(4-phenylacetyl-piperazin-1-ylmethyl)-7H-pyrrolo[2,3-d]pyrimidine-2-carbonitrile

To a solution of 4-{7-[2-(4-Chloro-phenyl)-ethyl]-2-cyano-7H-pyrrolo[2,3-d]pyrimidin -6-ylmethyl} -piperazine-1-carboxylic acid tert-butyl ester(125 mg, 0.26 mmol) in dichloromethane (1 ml), trifluoroacetic acid(1 ml) is added. After stirring for 30 min at room temperature, solvent is evaporated down to give7-[2-(4-chloro-phenyl)-ethyl]-6- piperazin-1-ylmethyl-7H-pyrrolo[2,3-d]pyrimidine-2-carbonitrile trifluoroacetic acid salt; Rf=0.10 ($CH_2Cl_2$:MeOH = 10:1).

To a solution of 7-[2-(4-chloro-phenyl)-ethyl]-6-piperazin-1-ylmethyl-7H-pyrrolo [2,3-d]pyrimidine-2-carbonitrile trifluoroacetic acid salt in pyridine(5 ml), phenylacetyl chloride (172 μl, 1.30 mmol) are added at 0˚C. The reaction mixture is stirred for 6h at 80˚C, quenched with ice-water and extracted with ethyl acetate. The combined extracts are washed with $H_2O$, brine and dried over sodium sulphate. Chromatography on silica gel gives the title compound; Rf=0.30(*n*-hexane:AcOEt = 1:1). $^1$H-NMR(400MHz, $CDCl_3$) δ : 2.18-2.21 (m, 2H), 2.37-2.39(m, 2H), 3.09-3.13(m, 2H), 3.32(s, 2H), 3.40-3.48(m, 2H), 3.63-3.65(m, 2H), 3.72(s, 2H), 4.55-4.59(m, 2H), 6.44(s, 1H), 6.96-6.98(m, 2H), 7.21-7.33(m, 7H), 8.89(s, 1H).

Example 335: 6-(2-Acetyl-2,8-diaza-spiro[4.5]dec-8-ylmethyl)-7-(2-cyclohexyl-ethyl)-7H-pyrrolo[2,3-d]pyrimidine-2-carbonitrile

To a solution of 6-bromomethyl-7-(2-cyclohexyl-ethyl)-7H-pyrrolo[2,3-d] pyrimidine-2-carbonitrile(290 mg, 0.84 mmol) in DMF(1.7 ml), 2,8-diaza-spiro[4.5]decane-2- carboxylic acid tert-butyl ester(201 mg, 0.84 mmol) and potassium carbonate(138 mg, 1.0 mmol) are added. The mixture is stirred at room temperature under nitrogen atomosphere for 14 h. The reaction mixture is diluted with water and extracted with AcOEt(twice). The combined organic layer is washed with water and brine, dried over $MgSO_4$, and concentrated in vacuo. The residue is purified by silica gel column Chromatography (n-hexane : AcOEt=1:1) to give 8-[2-cyano-7-(2-cyclohexyl-ethyl)-7H-pyrrolo[2,3-d]pyrimidin-6-ylmethyl]-2,8-diaza-spiro[4.5]decane-2-carboxylic acid tert-butyl ester; Rf=0.45(*n*-hexane:AcOEt = 1:1).

To a solution of 8-[2-cyano-7-(2-cyclohexyl-ethyl)-7H-pyrrolo[2,3-d]pyrimidin-6- ylmethyl]-2,8-diaza-spiro[4.5] decane-2-carboxylic acid tert-butyl ester (300 mg, 0.59 mmol) in dichloromethane (5 ml), trifluoroacetic acid(3 ml) is added. After stirring for 1.5h at room temperature, solvent is evaporated down to give 7-(2-cyclohexyl-ethyl)-6-(2,8-diaza-spiro[4.5]dec-8-ylmethyl)-7H-pyrrolo[2,3-d] pyrimidine -2-carbonitrile trifluoroacetic acid salt in quant yield; Rf=0.10 ($CH_2Cl_2$:MeOH = 10:1).

To a solution of 7-(2-cyclohexyl-ethyl)-6-(2,8-diaza-spiro[4.5]dec-8-ylmethyl)-7H -pyrrolo[2,3-d]pyrimidine-2-carbonitrile trifluoroacetic acid salt in pyridine(5 ml), acetic anhydride(0.28 ml, 2.90 mmol) are added at 0˚C. The reaction mixture is stirred for over night at room temperature, quenched with ice-water and extracted with ethyl acetate. The combined extracts are washed with $H_2O$, brine and dried over sodium sulphate. Chromatography on silica gel gives the title compound; Rf=0.30(*n*-hexane:AcOEt = 1:1). $^1$H-NMR(400MHz, $CDCl_3$) δ : 1.00-1.84(m, 17H), 2.04(s, 3H), 2.33-2.56 (m, 4H), 3.25-3.35(m, 2H), 3.47-3.53(m, 2H), 3.66-3.69(m, 2H), 4.38-4.43(m, 2H), 6.49(s, 1H), 8.87(s, 1H).

Example 336: 7-(2-Cyclohexyl-ethyl)-6-indol-1-ylmethyl-7H-pyrrolo[2,3-d]pyrimidine-2-carbonitrile

To a solution of 7-(2-cyclohexyl-ethyl)-6-(2,3-dihydro-indol-1-ylmethyl)-7H- pyrrolo [2,3-d]pyrimidine-2-carbonitrile(NVP-TAC583, 167 mg, 0.481 mmol) in toluene (1.5 mL) is added MnO2 (487 mg). After 1 h, the additional MnO2 (430 mg) is added, and the mixture is atirred at room temperature for 36 h. To this mixture is added the additional MnO2 (430 mg), and the resulting mixture is heated to 50 oC for 2 h. The mixture is filtered through celite pad, and the filtrate is concentrated in vacuo. The residue is purified by silica gel column chromatography(n-hexane:EtOAc=5:1) to give the title compound; 1H NMR(400 MHz, DMSO-d6) □ 0.77-0.84(m, 2H), 1.06-1.14(m, 4H), 1.24-1.30(m, 2H), 1.60-1.63(m, 5H), 4.29(t, 2H), 5.84(s, 2H), 6.38(s, 1H), 6.56(d, 1H), 7.06(t, 1H), 7.14(t, 1H), 7.44(d, 1H), 7.55(d, 1H), 7.60(d, 1H),

9.05(s, 1H); Rf 0.47(n-hexane:EtOAc=1:1).

Example 337: 7-(2-Cyclohexyl-ethyl)-6-(6-fluoro-indol-1-ylmethyl)-7H-pyrrolo[2,3-d] pyrimidine-2-carbonitrile

-To a solution of 6-fluoro-1H-indole(147 mg, 1.09 mmol) in THF(3.7 mL) is added NaH (60 %, 48 mg, 1.20 mmol) at 0 oC. After being stirred at 0 oC for 20 min, to this mixture is added propargyl bromide(0.1 mL, 1.33 mmol), and the mixture is stirred at 0 oC rt for 11 h. The reaction is quenched by the addition of water, and the mixture is extracted with ether. The combined organic extracts are washed with water and brine. The organic layer is dried over MgSO4, filtered, and concentrated in vacuo. The residue is purified by silica gel column chromatography (n-hexane: EtOAc=10:1) to give the propargyl indole.

To a solution of the above propargyl indole(82 mg, 0.473 mmol) and the 5-bromo-4-(2-cyclohexyl-ethylamino)-pyrimidine-2-carbonitrile (140 mg, 0.453 mmol) in DMF (1.7 mL) are added Et3N(0.19 mL, 1.37 mmol), CuI(9.0 mg, 0.047 mmol), and Pd(Ph3P)2Cl2(16 mg, 0.023 mmol). The flask is evacuated and backfilled with nitrogen, and then stirred at 80 oC for 70 min. After dilution with EtOAc, the mixture is washed with water(x2) and brine. The organic layer is dried over Na2SO4, filtered, and concentrated in vacuo. The residue is purified by silica gel column chromatography (n-hexane:EtOAc=4:1) to give the coupling product.

To a solution of the said product (105 mg) in DMF (1 mL) is added 1 drop of DMF. The reaction mixture is stirred at 100 oC for 30 min. After dilution with EtOAc, the mixture is washed with 1 M aqueous KHSO4 and brine. The organic layer is dried over Na2SO4, filtered, and concentrated in vacuo. The residue is purified by silica gel column chromatography(n-hexane:EtOAc=4:1 to 1:1) followed by trituration with ether-n-hexane to give the the title compound; 1H NMR(400 MHz, DMSO-d6) δ 0.75-0.83(m, 2H), 1.04-1.14(m, 4H), 1.22-1.28(m, 2H), 1.59-1.61 (m, 5H), 4.27(t, 2H), 5.80(s, 2H), 6.39(s, 1H), 6.57(t, 1H), 6.91 (dt, 1H), 7.41 (d, 1H), 7.46(dd, 1H), 7.59(dd, 1H), 9.05(s, 1H); Rf 0.55(n-hexane:EtOAc=1:1).

Example 338: 7-[2-(4-Chloro-2-fluoro-phenyl)-ethyl]-6-(3-trifluoromethyl-2,5-dihydro-pyrrol-1-ylmethyl)-7H-pyrrolo [2,3-d]pyrimidine-2-carbonitrile

To a solution of 3-oxo-pyrrolidine-1-carboxylic acid tert-butyl ester(2.71 g, 14.7 mmol) in THF(50 mL) are successively added CF3TMS(2.4 mL, 16.2 mmol) and TBAF(1.0 M in THF, 0.8 mL, 0.8 mmol) at 0 oC. The reaction mixture is stirred at 0 oC for 20 min, and then at room temperature for 9 h. The reaction is quenched by the addition of saturated aqueous NH4Cl and TBAF. The mixture is extracted with ether, and the combined organic extracts are washed with 1 M aqueous KHSO4, water, and brine. The organic layer is dried over MgSO4, filtered, and concentrated in vacuo to give the product.

To a solution of the above product in pyridine(50 mL) is added SOCl2(5 mL). The reaction mixture is stirred at 100 oC for 15 min, and then diluted with ether. The mixture is washed with 1 M aqueous KHSO4, water, saturated aqueous NaHCO3, water, and brine. The organic layer is dried over MgSO4, filtered, and concentrated in vacuo. The residue is purified by silica gel column chromatography (n-hexane:EtOAc=20:1) to give 3-trifluoromethyl-2,5-dihydro-pyrrole-1-carboxylic acid tert-butyl ester.

The above ester (105 mg, 0.443 mmol) is treated with 4N HCl-EtOAc(1 mL), and then the mixture was concentrated in vacuo to give the hydrochloride.

To a solution of 6-bromomethyl-7-(2-cyclohexyl-ethyl)-7H-pyrrolo[2,3-d] pyrimidine-2-carbonitrile (76 mg, 0.193 mmol) in DMF(1.0 mL) are added the above hydrochloride and K2CO3 (138 mg, 1.00 mmol). The reaction mixture is stirred at room temperature for 11 h. After dilution with EtOAc, the mixture is washed with water (x2) and brine. The organic layer is dried over Na2SO4, filtered, and concentrated in vacuo. The residue is purified by silica gel column chromatography (n-hexane:EtOAc=4:1 to 3:1) to give the title compound; Rf 0.47(n-hexane: AcOEt=1:1); 1 H NMR(400 MHz, CDCl3) □3.19(t, 2H), 3.60-3.64(m, 4H), 3.81 (s, 2H), 4.62(t, 2H), 6.31 (q, 1H), 6.52(s, 1H), 6.96-7.08(m, 3H), 8.91 (s, 1H).

Example 339: 7-[2-(4-Chloro-2-fluoro-phenyl)-ethyl]-6-(3-trifluoromethyl-pyrrolidin-1-ylmethyl)-7H-pyrrolo[2,3-d]pyrimidine-2-carbonitrile

A mixture of 3-trifluoromethyl-2,5-dihydro-pyrrole-1-carboxylic acid tert-butyl ester(764 mg, 3.22 mmol) and 10 % Pd on carbon(460 mg) in EtOH(10 mL) is stirred under 1 atom H2 at room temperature for 19 h. The mixture is filtered through a celite pad, and the filtrate is concentrated in vacuo. The residue is purified by silica gel column chromatography (n-hexane:EtOAc=15:1) to give the product.

The above product is treated with 4N HCl-EtOAc at room temperature for 1 h to give the hydrochloride.

To a solution of 6-bromomethyl-7-(2-cyclohexyl-ethyl)-7H-pyrrolo[2,3-d]pyrimidine -2-carbonitrile(75 mg, 0.190 mmol) in DMF(1.0 mL) are added the above hydrochloride (62 mg, 0.353 mmol) and K2CO3(176 mg, 1.27 mmol). The

reaction mixture is stirred at room temperature for 6.5 h. After dilution with EtOAc, the mixture is washed with water(x2) and brine. The organic layer is dried over Na2SO4, filtered, and concentrated in vacuo. The residue is purified by silica gel column chromatography (n-hexane:EtOAc=4:1 to 3:1) to give the title compound; Rf 0.36(n-hexane: AcOEt=1:1); 1H NMR(400 MHz, CDCl3) δ1.91-1.99(m, 1H), 2.02-2.11 (m, 1H), 2.55-2.67(m, 3H), 2.78(t, 1H), 2.82-2.92(m, 1H), 3.16 (t, 2H), 3.63(d, 2H), 4.61(t, 2H), 6.49(s, 1H), 6.97-7.08(m, 3H), 8.90(s, 1H).

Example 340: 1-[2-Cyano-7-(3-ethyl-heptyl)-7H-pyrrolo[2,3-d]pyrimidin-6-ylmethyl]-piperidine-4-carboxylic acid phenylamide

To a solution of piperidine-4-carboxylic acid(1 g, 7.7 mmol) in 1,4-dioxane(10 mL), water(5 mL), and 1N aqueous NaOH(8 mL) is added a solution of Boc2O(1.86 g, 8.5 mmol) in 1,4-dioxane(5 mL). The reaction mixture is stirred at room temperature for overnight, and then acidified by the addition of 10 % aqueous citric acid. The mixture is extracted with EtOAc, and the combined organic extracts are washed with brine. The organic layer is dried over Na2SO4, filtered, and concentrated in vacuo to give the desired acid.

To a solution of the above acid(1.64 g, 7.2 mmol), aniline(745 mg, 8 mmol), and HOBt(990 mg, 7.3 mmol) in DMF(10 mL) is added WSCD(1.13 g, 7.3 mmol). The reaction mixture is stirred at room temperature for overnight. After water is poured, the mixture is extracted with EtOAc. The combined organic extracts are washed with brine, and dried over Na2SO4, filtered, and concentrated in vacuo. The residue is purified by HPLC(n-Hexane-EtOAc) to give the desiredamide.

To a solution of the above amide (1.63 g, 5.4 mmol) in 1,4-dioxane(5 mL) and THF(10 mL) is added 4N HCl-dioxane(5 mL). The reaction mixture is stirred at room temperature for overnight. The resulting white precipitate is collected by filtration, washed with ether, and dried to give the desired hydrochloride.

To a solution of 6-chloromethyl-7-(2-cyclohexyl-ethyl)-7H-pyrrolo[2,3-d] pyrimidine -2-carbonitrile(85 mg, 0.28 mmol) in DMF(3 mL) are added the above hydrochloride (72 mg, 0.30 mmol) and K2CO3(83 mg, 0.60 mmol). The reaction mixture is stirred at room temperature for overnight. After water is poured, the mixture is extracted with EtOAc. The combined organic extracts are washed with brine, and dried over Na2SO4, filtered, and concentrated in vacuo. The residue is purified by RP-HPLC to give the the title compound; Rf 0.53(CH2Cl2:acetone=9:1); 1H-NMR(400 MHz, CDCl3) δ 0.98-1.08(m, 2H), 1.18-1.27(m, 3H), 1.30-1.42(m, 1H), 1.67-1.78(m, 4h), 1.82-1.97(m, 7H), 2.13-2.18(m, 2H), 2.25-2.31 (m, 1H), 2.96(d, 2H), 3.70(s, 2H), 4.42-4.46(m, 2H), 6.51 (s, 1H), 7.11 (br, 2H), 7.32(t, 2H), 7.50(d, 2H), 8.88(s, 1H).

Example 341: 6-Azepan-1-ylmethyl-7-(2-cyclohexyl-ethyl)-7H-pyrrolo[2,3-d]pyrimidine-2-carbonitrile

At room temperature, a soln. of 5-(3-azepan-1-yl-prop-1-ynyl)-4-(2-cyclohexyl- ethylamino)-pyrimidine-2-carbonitrile (0.27 mmol) in DMF(10 ml) is treated with DBU (0.40 mmol), stirred for 3 h at 100˚C, poured into water extracted with EtOAc, washed with $H_2O$, dried($MgSO_4$), and evaporated. The residue is purified by silica gel column chromatography (AcOEt) to give the title compound; 1H-NMR(400 MHz, CDCl3) δ 0.97-1.06(m, 2H), 1.15-1.42 (m, 4H), 1.68(brs, 8H), 1.58- 1.85(m, 7H), 2.64(brs, 4H), 3.79 (s, 2H), 4.46 (t, 2H), 6.48 (s, 1H), 8.86(s, 1H).

Example 342

By repeating the procedures described in Example 341 using appropriate starting materials (including some of those prepared in Examples A to ZZ) the following compounds of formula 2 are obtained as identified below in Table 28.

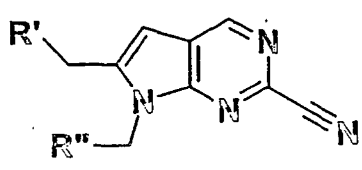

(2)

Table 28

| Ex. | R' | R" | Rf (solvent) | NMR(400MHz, CDCl3, δ) |
|---|---|---|---|---|

(continued)

| | | | | |
|---|---|---|---|---|
| 342 | 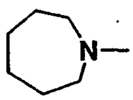 | 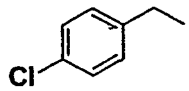 | 0.32 (AcOEt) | 1.68(brs, 8H), 2.58(brs, 4H), 3.13(t, 2H), 3.50(s, 2H), 4.66 (t, 2H), 6.42(s, 1H), 7.02(d, 2H), 7.24(d, 2H), 8.88 (s, 1H) |

Example 343: 7-(2-Cyclohexyl-ethyl)-6-((R)-3-methoxy-pyrrolidin-1-ylmethyl)-7H-pyrrolo[2,3-d]pyrimidine-2-carbonitrile

(R)-3-Methoxy-pyrrolidine.hydrochloride (Step 343.3, 46 mg, 0.34 mmol) and 6-bromomethyl-7- (2-cyclohexyl-ethyl)-7H-pyrrolo[2,3-d]pyrimidine-2-carbonitrile(117 mg, 0.34 mmol) are dissolved in DMF(2 ml) and potassium carbonate(130 mg, 1.02 mmol) is added to the solution. The reaction mixture is stirred at room temperature for 2 h and quenched with saturated ammonium chlroride and extracted with ethyl acetate. The combined extracts are washed with brine, dried over magnesium sulfate and evaporated down. Chromatography on silica gel (eluent; n-hexane : ethyl acetate = 4 : 1, 2 : 1, 1 : 1) gives the title compound; Rf=0.30 (*n*-hexane : ethyl acetate = 1:2); $^1$H-NMR(400MHz, $CDCl_3$) δ : 0.95-1.04(m, 2H), 1.16-1.38(m, 5H), 1.52-1.53(m, 2H), 1.64-1.87(m, 9H), 2.02-2.11 (m, 1H), 2.52-2.60(m, 2H), 2.67-2.71 (m, 1H), 2.79-2.83(m, 1H), 3.81-3.82(d, 2H), 3.91-3.96(m, 1H), 4.37-4.41 (m; 2H), 6.51 (s, 1H), 8.87(s, 1H).

Step 343.1: (R)-3-Hydroxy-pyrrolidine-1-carboxylic acid tert-butyl ester

To (R)-1-benzyl-pyrrolidin-3-ol (1.5 g, 8.24 mol), di-*t*-butyldicarbonate(2.2 g, 9.9 mmol) and 5 % Pd/C(0.2 g) in 100 ml of flask, MeOH : ethyl acetate(10 ml : 10 ml) is added at ambient temperature. The reaction mixture is stirred under $H_2$ at room temperature for 15 h. The catalysts are removed by filtration and MeOH and ethyl acetate are evaporated down to give crude oily product. Chromatography on silica gel (eluent; dichloromethane and 2 % MeOH in dichloromethane) gives the title compound; Rf=0.45(dichloroethane : MeOH = 9 : 1).

Step 343.2: (R)-3-Methoxy-pyrrolidine-1-carboxylic acid tert-butyl ester

To a suspension of NaH (98 mg, 2.46 mmol) in DMF(10ml), (R)-3-hydroxy-pyrrolidine-1-carboxylic acid tert-butyl ester (460 mg, 2.46 mmol) is successively added at 0 ˚C. To the mixture, methyl iodine (0.19 ml, 3.0 mmol) is added at 0 ˚C and the mixture is stirred for 2 h at ambient temperature. The reaction mixture is quenched with ice-water and extracted with AcOEt. The combined extracts are washed with brine, dried over magnesium sulphate and evaporated down to give the title compound; Rf=0.45(*n*-hexane : ethyl acetate = 2 :1).

Step 343.3: R)-3-methoxy-pyrrolidine hydrochloride

(R)-3-Methoxy-pyrrolidine-1-carboxylic acid tert-butyl ester(0.2 g, 0.99 mmol) is dissolved in 4N HCl in dioxane (0.75 ml, 3.0 mmol) at 0˚C. The mixture is stirred for overnight for 1 h at room temperature. After removal of the solvent, the oily residue is dried to give crude (R)-3-methoxy-pyrrolidine hydrochloride.

Example 344

By repeating the procedures described in Example 343 using appropriate starting materials (including some of those prepared in Examples A to ZZ) the following compounds of formula 2 are obtained as identified below in Table 29.

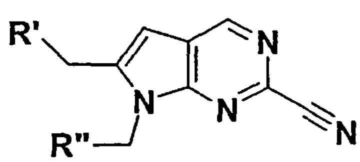

(2)

Table 29

| Ex. | R' | R" | Rf (solvent) | NMR(400MHz, CDCl3, δ) |
|---|---|---|---|---|
| 344 | 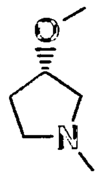 | 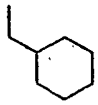 | 0.20 (*n*-hexane: ethyl acetate= 1:2) | 0.95-1.04(m, 2H), 1.16-1.38(m, 5H), 1.52-1.56(m, 2H), 1.64-1.87(m, 9H), 2.02-2.11(m, 1H), 2.52-2.60(m, 2H), 2.67-2.73(m, 1H), 2.79-2.83(m, 1H), 3.81-3.82(m, 2H), 3.91- 3.96(m, 1H), 4.37-4.41 (m, 2H), 6.51 (s, 1H), 8.87(s, 1H). |

Example 345: Soft Capsules

5000 soft gelatin capsules, each comprising as active ingredient 0.05 g of one of the compounds of formula I mentioned in the preceding Examples, are prepared as follows:

Composition

| | |
|---|---|
| Active ingredient | 250 g |
| Lauroglycol | 2 litres |

Preparation process: The pulverized active ingredient is suspended in Lauroglykol® (propylene glycol laurate, Gattefossé S.A., Saint Priest, France) and ground in a wet pulverizer to produce a particle size of about 1 to 3 μm. 0.419 g portions of the mixture are then introduced into soft gelatin capsules using a capsule-filling machine.

Example 346: Biological Activity

Some exemplary $IC_{50}$s for the inhibition of human cathepsin S for compounds of formula I as determined in the in vitro cathepsin S assay described herein are provided below:

Table 30

| Example | IC50 (μmol/l) |
|---|---|
| 54 | 0.032 |
| 169 | 0.030 |
| 175 | 0.0051 |
| 179 | 0.035 |
| 193 | 0.0041 |
| 278 | 0.033 |
| 335 | 0.048 |

**Claims**

**1.** A pyrrolo pyrimidine of formula I,

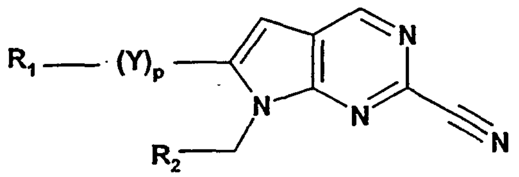

(I)

wherein
Y represents $-(CH_2)_t$-O- or $-(CH_2)_r$-S-,
p is 1 or 2,
r is 1, 2 or 3,
t is 1, 2 or 3,
$R_1$ represents

(h) phenyl which is unsubstituted or mono-, di- or trisubstituted by

(α) halogen, carboxy, alkoxy, nitro, alkyl-C(O)-NH-, cycloalkyl-C(O)-NH-, alkyl-C(O)-N(alkyl)-, formyl, alkyl-C(O)-, alkyl-$S(O)_2$-NH-, $CF_3$-alkyl-$S(O)_2$-NH-, pyrrolidinyl carbonyl, piperidinyl carbonyl, morpholinyl carbonyl, N-alkyl piperazinyl carbonyl, piperidinyl, 1-(alkyl carbonyl) piperidinyl, 1,2,3,6-tetrahydropyridyl, alkyl carbonyl 1,2,3,6-tetrahydropyridyl, piperazinyl, alkyl piperazinyl, alkyl carbonyl piperazinyl, cycloalkyl carbonyl piperazinyl, alkoxy carbonyl piperazinyl, alkyl-$SO_2$-piperazinyl, diazacycloheptyl, alkyl carbonyl diazacycloheptyl, 2-oxo-1-pyrrolidinyl, 3,3-di-alkyl-2-oxo-1-pyrrolidinyl;
(β) $R_3$-alkyl, wherein $R_3$ represents hydrogen, hydroxy, carboxy, alkyl-N(alkyl)-, alkyl-NH-, 1-pyrrolidinyl, 1-piperidyl, 4-alkyl-1-piperazinyl carbonyl, 2,4-dioxa-5,5-(di-alkyl)-oxazolidin-3-yl, $R_4R_5$N-C(O)-, wherein $R_4$ and $R_5$ independently of each other represent hydrogen or alkyl; or
(γ) $R_6R_7$N-C(O)-, wherein $R_6$ and $R_7$ independently of each other represent hydrogen, alkyl, cycloalkyl alkyl, $CF_3$-alkyl or pyridyl alkyl;

(i) pyridyl, which is unsubstituted or mono-, di- or trisubstituted by halogen or alkyl which is mono-, di- or trisubstituted by halogen;
pyrimidyl;
(k) indolyl, which is mono- or disubstituted by alkyl-C(O)-NH-alkyl;
(l) 2-(alkyl)-benzothiazolyl;
(m) a radical of subformula Ia

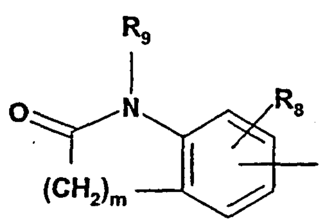

(Ia)

wherein $R_8$ is hydrogen, halogen or alkyl, $R_9$ is hydrogen or alkyl, and m is 1, 2, 3 or 4; or
(n) a radical of subformula Ib

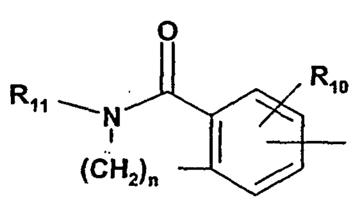

(Ib)

wherein $R_{10}$ is hydrogen, halogen or alkyl, $R_{11}$ is hydrogen or alkyl, and n is 1, 2, 3 or 4;

$R_2$ represents alkyl, which is unsubstituted or substituted by cycloalkyl, which is unsubstituted or mono- or disubstituted by halogen, or phenyl, which is mono- or disubstituted by halogen;
under the proviso that $R_2$ does not represent 1,1-dimethylethyl if Y is O and $R_1$ is selected from 3-pyridyl, 4-pyridyl, 5-chloro-3-pyridyl, 6-chloro-3-pyridyl, 2-chloro-4-pyridyl, 2-trifluoromethyl-4-pyridyl, 2-difluoromethyl-4-pyridyl, 4-acetyl-1-piperazinyl-phenyl, 4-methyl-1-piperazinyl-methyl-phenyl, and
under the proviso that $R_2$ does not represent 1,1-dimethylethyl, if Y is S and $R_1$ is 4-pyridyl; or
Y is $-(CH_2)_j-$ or -CH=CH-,
j is 1 or 2;
p is 1 or 2,
$R_1$ represents

(c) thienyl, thiazolyl, 1-piperidinyl-carbonyl, or
(d) phenyl which is unsubstituted or mono-, di- or trisubstituted by

(i) alkoxy, $H_2N$-C(O)-, 4-(alkyl carbonyl) 1-piperazinyl, 2-oxo-1-pyrrolidinyl, or halogen;
(ii) $R_{12}$-O-C(O)-, wherein $R_{12}$ is hydrogen or alkyl, or
(iii) $R_{13}$NH-, wherein $R_{13}$ represents hydrogen or a radical $R_{14}$-alkyl-Z-, wherein Z is CO, SO or $SO_2$ and $R_{14}$ denotes hydrogen, trifluoromethyl or alkoxy,
(iv) $R_{15}$-alkyl, wherein $R_{15}$ denotes hydrogen, hydroxy, lalkoxy, 1-pyrrolidinyl, 2-oxo-1-pyrrolidinyl, imidazolidin-2,5-dion-1-yl, 5,5-di-alkyl-oxazolidin-2,4-dion-3-yl or alkyl-N($R_{16}$)-, wherein $R_{16}$ represents hydrogen or alkyl; and

$R_2$ represents

(a) alkyl, which is unsubstituted or substituted by alkenyl, indanyl, cycloalkyl which is unsubstituted or mono- or disubstituted by halogen or alkyl, cycloalkenyl, phenyl, which is unsubstituted or mono- or disubstituted by halogen or by alkyl;
(b) cycloalkyl; or
(c) alkylcarbonyl;

under the proviso that, if Y is $CH_2$, $R_1$ represents 4-chlorophenyl and p is 1, $R_2$ does not denote 1,1-dimethylethyl, 1-methylethyl, cyclopropyl, cyclohexyl, 2-methyl-propyl or 2-ethyl-propyl;
under the proviso that $R_2$ does not represent 1,1-dimethylethyl, if p is 1, Y is $CH_2$ and $R_1$ represents thienyl, phenyl, methoxyphenyl, propoxyphenyl, 4-fluorophenyl, 4-methylphenyl, 4-ethylphenyl, 4-butylphenyl, hydroxymethylphenyl, 4-(5,5-dimethyloxazolidin-2,4-dion-3-yl-methyl)-phenyl, 4-(methylsulfonylamino)-phenyl, 4-(n-butylsulfonylamino)-phenyl, 4-(ethylsulfonylamino)-phenyl, 4-(n-propylsulfonylamino)-phenyl, 4-(iso-propylsulfonylamino)-phenyl, 4-aminophenyl, 4-(acetylamino)-phenyl, 4-(butanoylamino)-phenyl or 4-(diethylaminomethyl)-phenyl;
and under the proviso that that $R_2$ does not represent 1-methylethyl, if p is 1, Y is $CH_2$ and $R_1$ represents phenyl which is unsubstituted or substituted by 4-acetyl-1-piperazinyl; or
Y is $-(CH_2)_f-$,
f is 1 or 2;
p is 1,
$R_1$ represents

(a) 1,2,3,6-tetrahydropyrid-1-yl, alkyl-1,2,3,6-tetrahydropyrid-1-yl, di-alkyl-1,2,3,6-tetrahydropyrid-1-yl, halo-1,2,3,6-tetrahydropyrid-1-yl, phenyl-1,2,3,6-tetrahydropyrid-1-yl, imidazolyl, alkyl imidazolyl, di-halo imidazolyl, imidazolidin-2,5-dion-1-yl, 5,5-dilalkyloxazolidin-2,4-dion-3-yl, alkyl imidazolidin-2,5-dion-1-yl, trifluoromethyl-3,4-pyrrolin-1-yl, pyrrolidinyl, alkyl 1-pyrrolidinyl, di-alkyl) pyrrolidinyl, alkoxy pyrrolidinyl, alkyl 2-oxo-1-pyrrolidinyl, di-alkyl 2-oxo-1-pyrrolidinyl, halo 1-pyrrolidinyl, di-halo 1-pyrrolidinyl, di-halo 1-piperidinyl, triazolyl, nitro triazolyl, phenyl imidazolyl, tetrazolyl, benzo[b]imidazolyl, (1-(alkyl-$SO_2$)-4-piperidinyl)-2,3-dihydro-2-oxo-benzo [b]imidazolyl, 3-(alkyl carbonyl-4-piperidinyl)-2,3-dihydro-2-oxo-benzo[b]imidazolyl, indolyl, halo 1-indolyl, 1,3-dihydro-2-isoindolyl, 2,3-dihydro-1-indolyl, 2,3-dihydro-2-oxo-benzo[b]thiazolyl, di-alkoxy 1,2,3,4-tetrahydroquinnolin, alkoxy-1,2,3,4-tetrahydroisoquinnolin;
(b) a radical of substructure Ic

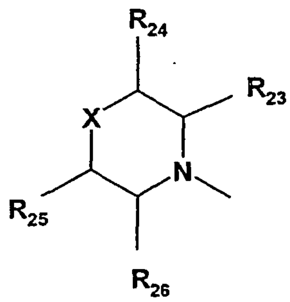

(Ic)

which is bound to the molecule via the nitrogen atom, wherein
X is -O-, $-(CH_2)_s-CR_{17}R_{18}-$ or $-NR_{18}$, wherein
s is 0, 1 or 2, $R_{17}$ and $R_{18}$ are independently selected from hydrogen, halogen, hydroxy, alkyl, phenyl alkyl carbonyl, carbamoyl, N-phenyl carbamoyl, cyano, pyridyl, piperidinyl and phenyl which is unsubstituted or mono- or disubstituted by halogen or alkoxy, or, if X is $CR_{17}R_{18}$, $R_{17}$ and $R_{18}$ and together form an oxo group or a group HO-C(O)-CH=, and $R_{23}$, $R_{24}$, $R_{25}$ and $R_{26}$ are independently selected from hydrogen and alkyl;
(c) a radical of substructure Id

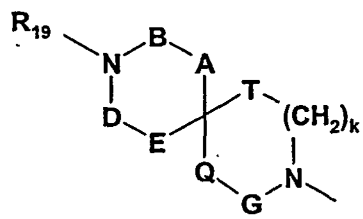

(Id)

which is bound to the molecule via the nitrogen atom, wherein
k is 0, 1 or 2, A is $CH_2$ or a bond, B is $CH_2$ or carbonyl, D is $CH_2$ or carbonyl, E is $CH_2$ or $NR_{22}$, G is $CH_2$ or a bond, Q is $CH_2$ or carbonyl, T is $CH_2$ or $NR_{29}$, $R_{19}$ represents hydrogen, alkyl, phenyl alkyl, alkyl carbonyl or alkyl-$SO_2$-, $R_{22}$ is hydrogen or alkyl and $R_{29}$ is phenyl;
(d) a radical of substructure Ie

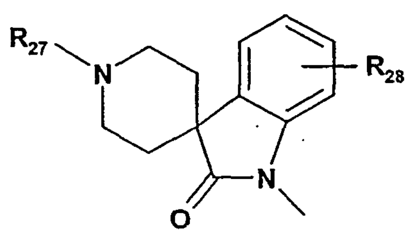

(Ie)

which is bound to the molecule via the nitrogen atom, wherein
$R_{27}$ is alkyl or alkyl carbonyl and $R_{28}$ is hydrogen, alkoxy or halogen; or
(e) $NR_{20}R_{21}$, wherein $R_{20}$ and $R_{21}$ are independently selected from hydrogen, alkyl, cycloalkyl which is unsubstituted or mono- or disubstituted by hydroxy; and phenyl which is unsubstituted or mono- or disubstituted by 1,2,3-thiadiazolyl, under the proviso that not both $R_{20}$ and $R_{21}$ can represent hydrogen at the same time; and

$R_2$ denotes alkyl, which is unsubstituted or substituted by cycloalkyl which is unsubstituted or mono- or disubstituted by halogen; or phenyl, which is mono- or disubstituted by halogen;
under the proviso that $R_2$ does not represent 1,1-dimethylethyl, if

(a) $R_1$ is benzo[b]imidazol-1-yl, 1-imidazolyl, 4,5-dichloro-1-imidazolyl, 2-($C_1$-$C_4$alkyl)-1-imidazolyl, imidazolidin-2,5-dion-1-yl, 5,5-dimethyl-oxazolidin-2,4-dion-3-yl, 1H-1,2,3-triazol-1-yl, 2H-1,2,3-triazol-2-yl, 3-nitro-1H-1,2,4-triazol-1-yl, 2H-tetrazol-2-yl or 1 H-tetrazol-1-yl, or if $R_1$ is a radical of substructure Ic, $R_{23}$ to $R_{26}$ are hydrogen, X is $NR_{18}$ and $R_{18}$ is hydrogen, methyl, ethyl, acetyl, 4-pyridyl, 1-piperidinyl, phenyl, methoxyphenyl, ethoxyphenyl, fluorophenyl or chlorophenyl;

(b) $R_1$ is a radical of substructure Ic, $R_{23}$ to $R_{26}$ are hydrogen, X is $-(CH_2)_s-CR_{17}R_{18}-$, s is 0, and $R_{17}$ and $R_{18}$ are selected from hydroxyl and phenyl which is monosubstituted by chloro or $R_{17}$ and $R_{18}$ are selected from hydrogen, methoxyphenyl and N-phenylcarbamoyl; or

(c) $R_1$ is a radical of substructure Id, k is 1, A is a bond, E is $NR_{22}$, $R_{22}$ is hydrogen, G, Q and T are $CH_2$, B and D are carbonyl and $R_{19}$ is methyl, n-propyl or iso-butyl;

under the proviso that $R_2$ does not represent 2-methylpropyl, if $R_1$ is a radical of substructure Id, k is 1, A is a bond, E is $NR_{22}$, $R_{22}$ is hydrogen, G, Q and T are $CH_2$, B and D are carbonyl and $R_{19}$ is methyl, or if $R_1$ is a radical of substructure Ic, $R_{23}$ to $R_{26}$ are hydrogen, X is $-(CH_2)_s-CR_{17}R_{18}-$, s is 0, and $R_{17}$ and $R_{18}$ are selected from hydrogen and phenyl which is monosubstituted by methoxy;
and under the proviso that $R_2$ does not represent 1-methylethyl, if $R_1$ is a radical of substructure Ic, $R_{23}$ to $R_{26}$ are hydrogen, X is $NR_{18}$ and $R_{18}$ is methoxyphenyl or ethoxyphenyl, or X is $CR_{17}R_{18}$ and $R_{17}$ and $R_{18}$ are selected from hydrogen and methoxyphenyl;
or an N-oxide or a tautomer thereof,
or a salt of such pyrrolo pyrimidine, its N-oxide or its tautomer.

**2.** A pyrrolo pyrimidine of formula I according to claim 1,
wherein
Y represents $-CH_2-O-$ or $-CH_2-S-$,
p is 1,
$R_1$ represents

(o) phenyl which is unsubstituted or mono- or disubstituted by

(α) halogen, carboxy, $C_1$-$C_4$alkoxy, nitro, $C_1$-$C_4$alkyl-C(O)-NH-, $C_3$-$C_4$cycloalkyl-C(O)-NH-, $C_1$-$C_4$alkyl-C(O)-N($C_1$-$C_4$alkyl)-, formyl, $C_1$-$C_4$alkyl-C(O)-, $C_1$-$C_4$alkyl-$S(O)_2$-NH-, $CF_3$-$C_1$-$C_3$alkyl-$S(O)_2$-NH-, 1-pyrrolidinyl-carbonyl, 1-piperidinyl-carbonyl, 4-morpholinyl-carbonyl, 4-($C_1$-$C_4$alkyl)-1-piperazinyl carbonyl, 4-piperidinyl, 1-piperidinyl, 1-($C_1$-$C_4$alkyl-carbonyl)-4-piperidinyl, 1,2,3,6-tetrahydro-4-pyridyl, 1-($C_1$-$C_4$alkyl-carbonyl)-1,2,3,6-tetrahydro-4-pyridyl, 1-piperazinyl, 4-($C_1$-$C_4$alkyl)-1-piperazinyl, 4-($C_1$-$C_4$alkyl-carbonyl)-1-piperazinyl, 4-($C_3$-$C_5$cycloalkyl-carbonyl)-1-piperazinyl, 4-($C_1$-$C_4$alkoxy-carbonyl)-1-piperazinyl, 4-($C_1$-$C_4$alkyl-$SO_2$)-1-piperazinyl, 1,4-diazacyclohept-1-yl, 4-($C_1$-$C_4$alkyl-carbonyl)-1,4-diazacyclohept-1-yl, 2-oxo-1-pyrrolidinyl, 3,3-di-($C_1$-$C_4$alkyl)-2-oxo-1-pyrrolidinyl;

(β) $R_3$-$C_1$-$C_4$alkyl, wherein $R_3$ represents hydrogen, hydroxyl, carboxy, $C_1$-$C_4$alkyl-N($C_1$-$C_4$alkyl)-, $C_1$-$C_4$alkyl-NH-, 1-pyrrolidinyl, 1-piperidyl, 4-($C_1$-$C_4$alkyl)-1-piperazinyl carbonyl, 2,4-dioxa-5,5-(di-$C_1$-$C_4$alkyl)-oxazolidin-3-yl, $R_4R_5$N-C(O)-, wherein $R_4$ and $R_5$ independently of each other represent hydrogen or $C_1$-$C_4$alkyl; or

(γ) $R_6R_7$N-C(O)-, wherein $R_6$ and $R_7$ independently of each other represent hydrogen, $C_1$-$C_4$alkyl, $C_5$-$C_7$cycloalkyl-$C_1$-$C_4$alkyl, $CF_3$-$C_1$-$C_3$alkyl or pyridyl-$C_1$-$C_4$alkyl;

(p) pyridyl, which is unsubstituted or mono- or disubstituted by halogen or $C_1$-$C_4$alkyl which is di- or trisubstituted by halogen;
(q) pyrimidyl;
(r) indolyl, which is monosubstituted by $C_1$-$C_4$alkyl-C(O)-NH-$C_1$-$C_4$alkyl;
(s) 2-($C_1$-$C_4$alkyl)-benzothiazolyl;
(t) a radical of subformula Ia
wherein $R_8$ is hydrogen, $R_9$ is hydrogen, and m is 2 or 3; or
(u) a radical of subformula Ib
wherein $R_{10}$ is hydrogen, $R_{11}$ is hydrogen, and n is 2 or 3;

$R_2$ represents $C_1$-$C_5$alkyl, which is unsubstituted or substituted by $C_5$-$C_7$cycloalkyl, which is unsubstituted or disubstituted by halogen, or phenyl, which is mono- or disubstituted by halogen;
under the proviso that $R_2$ does not represent 1,1-dimethylethyl if Y is O and $R_1$ is selected from 3-pyridyl, 4-pyridyl, 5-chloro-3-pyridyl, 6-chloro-3-pyridyl, 2-chloro-4-pyridyl, 2-trifluoromethyl-4-pyridyl, 2-difluoromethyl-4-pyridyl, 4-acetyl-1-piperazinyl-phenyl, 4-methyl-1-piperazinyl-methyl-phenyl, and
under the proviso that $R_2$ does not represent 1,1-dimethylethyl, if Y is S and $R_1$ is 4-pyridyl; or
Y is $CH_2$ or -CH=CH-,
p is 1 or 2,
$R_1$ represents

(e) thienyl, thiazolyl, 1-piperidinyl-carbonyl, or
(f) phenyl which is unsubstituted or mono- or disubstituted by

(i) $C_1$-$C_4$alkoxy, $H_2N$-C(O)-, 4-($C_1$-$C_4$alkyl-carbonyl)-1-piperazinyl, 2-oxo-1-pyrrolidinyl, or halogen;
(ii) $R_{12}$-O-C(O)-, wherein $R_{12}$ is hydrogen or $C_1$-$C_4$alkyl, or
(iii) $R_{13}$NH-, wherein $R_{13}$ represents hydrogen or a radical $R_{14}$-$C_1$-$C_4$alkyl-Z-, wherein Z is CO or $SO_2$ and $R_{14}$ denotes hydrogen, trifluoromethyl or $C_1$-$C_4$alkoxy,
(iv) $R_{15}$-$C_1$-$C_4$alkyl, wherein $R_{15}$ denotes hydrogen, hydroxy, lower alkoxy, 1-pyrrolidinyl, 2-oxo-1-pyrrolidinyl, imidazolidin-2,5-dion-1-yl, 5,5-dimethyl-oxazolidin-2,4-dion-3-yl or $C_1$-$C_4$alkyl-N($R_{16}$)-, wherein $R_{16}$ represents hydrogen or $C_1$-$C_4$alkyl; and

$R_2$ represents

(a) $C_1$-$C_7$alkyl, which is unsubstituted or substituted by $C_2$-$C_3$alkenyl, indanyl, $C_3$-$C_7$cycloalkyl which is unsubstituted or disubstituted by halogen or $C_1$-$C_4$alkyl, $C_3$-$C_7$cycloalkenyl, phenyl, which is unsubstituted or mono- or disubstituted by halogen or by $C_1$-$C_4$alkyl;
(b) $C_3$-$C_7$cycloalkyl; or
(c) $C_1$-$C_4$alkylcarbonyl;

under the proviso that, if Y is $CH_2$, $R_1$ represents 4-chlorophenyl and p is 1, $R_2$ does not denote 1,1-dimethylethyl, 1-methylethyl, cyclopropyl, cyclohexyl, 2-methyl-propyl or 2-ethyl-propyl;
under the proviso that $R_2$ does not represent 1,1-dimethylethyl, if p is 1, Y is $CH_2$ and $R_1$ represents thienyl, phenyl, methoxyphenyl, propoxyphenyl, 4-fluorophenyl, 4-methylphenyl, 4-ethylphenyl, 4-butylphenyl, hydroxymethylphenyl, 4-(5,5-dimethyloxazolidin-2,4-dion-3-yl-methyl)-phenyl, 4-(methylsulfonylamino)-phenyl, 4-(n-butylsulfonylamino)-phenyl, 4-(ethylsulfonylamino)-phenyl, 4-(n-propylsulfonylamino)-phenyl, 4-(iso-propylsulfonylamino)-phenyl, 4-aminophenyl, 4-(acetylamino)-phenyl, 4-(butanoylamino)-phenyl or 4-(diethylaminomethyl)-phenyl;
and under the proviso that that $R_2$ does not represent 1-methylethyl, if p is 1, Y is $CH_2$ and $R_1$ represents phenyl which is unsubstituted or substituted by 4-acetyl-1-piperazinyl; or
Y is $CH_2$,
p is 1,
$R_1$ represents

(a) 1,2,3,6-tetrahydropyrid-1-yl, 4-($C_1$-$C_4$alkyl)-1,2,3,6-tetrahydropyrid-1-yl, 4,5-di($C_1$-$C_4$alkyl)-1,2,3,6-tetrahydropyrid-1-yl, 5-chloro-1,2,3,6-tetrahydropyrid-1-yl, 4-phenyl-1,2,3,6-tetrahydropyrid-1-yl, 1-imidazolyl, 2-($C_1$-$C_4$alkyl)-1-imidazolyl, 4,5-dihalo-1-imidazolyl, imidazolidin-2,5-dion-1-yl, 5,5-dimethyl-oxazolidin-2,4-dion-3-yl, 3-($C_1$-$C_4$alkyl)-imidazolidin-2,5-dion-1-yl, 3-trifluoromethyl-3,4-pyrrolin-1-yl, 1-pyrrolidinyl, 3-$C_1$-$C_4$alkyl-1-pyrrolidinyl, 3,3-di-($C_1$-$C_4$alkyl)-1-pyrrolidinyl, 3-$C_1$-$C_4$alkoxy-1-pyrrolidinyl, 3-$C_1$-$C_4$alkyl-2-oxo-1-pyrrolidinyl, 3,3-di-($C_1$-$C_4$alkyl)-2-oxo-1-pyrrolidinyl, 3-halo-1-pyrrolidinyl, 3,3-di-halo-1-pyrrolidinyl, 3,3-di-halo-1-piperidinyl, 1H-1,2,3-triazol-1-yl, 2H-1,2,3-triazol-2-yl, 1H-1,2,4-triazof-1-yl, 3-nitro-1H-1,2,4-triazol-1-yl, 2-phenyl-1-imidazolyl, 2H-tetrazol-2-yl, 1H-tetrazol-1-yl, benzo[b]imidazol-1-yl, 3-(1-($C_1$-$C_4$alkyl-$SO_2$)-4-piperidinyl)-2,3-dihydro-2-oxo-benzo[b]imidazol-1-yl, 3-(1-$C_1$-$C_4$alkylcarbonyl-4-piperidinyl)-2,3-dihydro-2-oxo-benzo[b]imidazol-1-yl, 1-indolyl, 6-halo-1-indolyl, 1,3-dihydro-2-isoindolyl, 2,3-dihydro-1-indolyl, 2,3-dihydro-2-oxo-benzo[b]thiazol-3yl, 6,7-di-($C_1$-$C_4$alkoxy)-1,2,3,4-tetrahydroquinnolin, 6-$C_1$-$C_4$alkoxy-1,2,3,4-tetrahydroisoquinnolin, 7-$C_1$-$C_4$alkoxy-1,2,3,4-tetrahydroisoquinnolin;
(b) a radical of substructure Ic
which is bound to the molecule via the nitrogen atom, wherein
X is -O-, -$(CH_2)_s$-$CR_{17}R_{18}$- or -$NR_{18}$, wherein
s is 0 or 1, $R_{17}$ and $R_{18}$ are independently selected from hydrogen, halogen, hydroxy, $C_1$-$C_4$alkyl, phenyl-$C_1$-$C_4$alkyl-carbonyl, carbamoyl, N-phenyl-carbamoyl, cyano, 4-pyridyl, 1-piperidinyl and phenyl which is unsubstituted or monosubstituted by halogen or $C_1$-$C_4$alkoxy, or, if X is $CR_{17}R_{18}$, $R_{17}$ and $R_{18}$ and together form an oxo group or a group HO-C(O)-CH=, and
$R_{23}$, $R_{24}$, $R_{25}$ and $R_{26}$ are independently selected from hydrogen and $C_1$-$C_4$alkyl;

(c) a radical of substructure Id which is bound to the molecule via the nitrogen atom, wherein k is 0 or 1, A is $CH_2$ or a bond, B is $CH_2$ or carbonyl, D is $CH_2$ or carbonyl, E is $CH_2$ or $NR_{22}$, G is $CH_2$ or a bond, Q is $CH_2$ or carbonyl, T is $CH_2$ or $NR_{29}$, $R_{19}$ represents hydrogen, $C_1$-$C_4$alkyl, phenyl-$C_1$-$C_4$alkyl, $C_1$-$C_4$alkylcarbonyl or $C_1$-$C_4$alkyl-$SO_2$-, $R_{22}$ is hydrogen and $R_{29}$ is phenyl;

(d) a radical of substructure Ie which is bound to the molecule via the nitrogen atom, wherein $R_{27}$ is $C_1$-$C_4$alkyl or $C_1$-$C_4$alkylcarbonyl and $R_{28}$ is hydrogen, $C_1$-$C_4$alkoxy or halogen; or

(e) $NR_{20}R_{21}$, wherein $R_{20}$ and $R_{21}$ are independently selected from hydrogen, $C_1$-$C_4$alkyl, $C_3$-$C_7$cycloalkyl which is unsubstituted or monosubstituted by hydroxy; and phenyl which is unsubstituted or monosubstituted by 1,2,3-thiadiazol-4-yl, under the proviso that not both $R_{20}$ and $R_{21}$ can represent hydrogen at the same time; and

$R_2$ denotes $C_1$-$C_8$alkyl, which is unsubstituted or substituted by $C_3$-$C_7$cycloalkyl which is unsubstituted or disubstituted by halogen; phenyl, which is mono- or disubstituted by halogen;
under the proviso that $R_2$ does not represent 1,1-dimethylethyl, if

(a) $R_1$ is benzo[b]imidazol-1-yl, 1-imidazolyl, 4,5-dichloro-1-imidazolyl, 2-($C_1$-$C_4$alkyl)-1-imidazolyl, imidazolidin-2,5-dion-1-yl, 5,5-dimethyl-oxazolidin-2,4-dion-3-yl, 1H-1,2,3-triazol-1-yl, 2H-1,2,3-triazol-2-yl, 3-nitro-1H-1,2,4-triazol-1-yl, 2H-tetrazol-2-yl or 1 H-tetrazol-1-yl, or if $R_1$ is a radical of substructure Ic, $R_{23}$ to $R_{26}$ are hydrogen, X is $NR_{18}$ and $R_{18}$ is hydrogen, methyl, ethyl, acetyl, 4-pyridyl, 1-piperidinyl, phenyl, methoxyphenyl, ethoxyphenyl, fluorophenyl or chlorophenyl;

(b) $R_1$ is a radical of substructure Ic, $R_{23}$ to $R_{26}$ are hydrogen, X is $-(CH_2)_s-CR_{17}R_{18}-$, s is 0, and $R_{17}$ and $R_{18}$ are selected from hydroxyl and phenyl which is monosubstituted by chloro or $R_{17}$ and $R_{18}$ are selected from hydrogen, methoxyphenyl and N-phenylcarbamoyl; or

(c) $R_1$ is a radical of substructure Id, k is 1, A is a bond, E is $NR_{22}$, $R_{22}$ is hydrogen, G, Q and T are $CH_2$, B and D are carbonyl and $R_{19}$ is methyl, n-propyl or iso-butyl;

under the proviso that $R_2$ does not represent 2-methylpropyl, if $R_1$ is a radical of substructure Id, k is 1, A is a bond, E is $NR_{22}$, $R_{22}$ is hydrogen, G, Q and T are $CH_2$, B and D are carbonyl and $R_{19}$ is methyl, or if $R_1$ is a radical of substructure Ic, $R_{23}$ to $R_{26}$ are hydrogen, X is $-(CH_2)_s-CR_{17}R_{18}-$, s is 0, and $R_{17}$ and $R_{18}$ are selected from hydrogen and phenyl which is monosubstituted by methoxy;
and under the proviso that $R_2$ does not represent 1-methylethyl, if $R_1$ is a radical of substructure Ic, $R_{23}$ to $R_{26}$ are hydrogen, X is $NR_{18}$ and $R_{18}$ is methoxyphenyl or ethoxyphenyl, or X is $CR_{17}R_{18}$ and $R_{17}$ and $R_{18}$ are selected from hydrogen and methoxyphenyl;
or a tautomer thereof,
or a salt of such pyrrolo pyrimidine or its tautomer.

3. A pyrrolo pyrimidine of formula I according to claim 1 or 2, or an N-oxide or a tautomer thereof, or a pharmaceutically acceptable salt of such a compound, for use in a method for the treatment of the human or animal body.

4. Use of a pyrrolo pyrimidine of formula I according to claim 1 or 2, or an N-oxide or a tautomer thereof, or a pharmaceutically acceptable salt of such a compound, for the preparation of medicament for the treatment of neuropathic pain.

5. A pharmaceutical preparation, comprising a pyrrolo pyrimidine of formula I according to claim 1 or 2, or an N-oxide or a tautomer thereof, or a pharmaceutically acceptable salt of such a compound, or a hydrate or solvate thereof, and at least one pharmaceutically acceptable carrier.

6. A process for the preparation of a pyrrolo pyrimidine of formula I

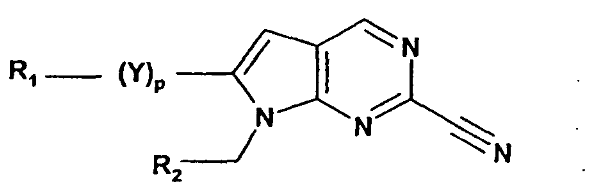

(I)

wherein Y represents $-(CH_2)_t$-O- or $(CH_2)_r$-S-
p is 1 or 2,
r is 1, 2 or 3,
t is 1, 2 or 3,
$R_1$ represents

(v) phenyl which is unsubstituted or mono-, di- or trisubstituted by

(α) halogen, carboxy, alkoxy, nitro, alkyl-C(O)-NH-, cycloalkyl-C(O)-NH-, alkyl-C(O)-N(alkyl)-, formyl, alkyl-C(O)-, alkyl-$S(O)_2$-NH-, $CF_3$-alkyl-$S(O)_2$-NH-, pyrrolidinyl carbonyl, piperidinyl carbonyl, morpholinyl carbonyl, N-alkyl piperazinyl carbonyl, piperidinyl, 1-(alkyl carbonyl) piperidinyl, 1,2,3,6-tetrahydropyridyl, alkyl carbonyl 1,2,3,6-tetrahydropyridyl, piperazinyl, alkyl piperazinyl, alkyl carbonyl piperazinyl, cycloalkyl carbonyl piperazinyl, alkoxy carbonyl piperazinyl, alkyl-$SO_2$-piperazinyl, diazacycloheptyl, alkyl carbonyl diazacycloheptyl, 2-oxo-1-pyrrolidinyl, 3,3-di-alkyl-2-oxo-1-pyrrolidinyl;
(β) $R_3$-alkyl, wherein $R_3$ represents hydrogen, hydroxy, carboxy, alkyl-N(alkyl)-, alkyl-NH-, 1-pyrrolidinyl, 1-piperidyl, 4-alkyl-1-piperazinyl carbonyl, 2,4-dioxa-5,5-(di-alkyl)-oxazolidin-3-yl, $R_4R_5$N-C(O)-, wherein $R_4$ and $R_5$ independently of each other represent hydrogen or alkyl; or
(γ) $R_6R_7$N-C(O)-, wherein $R_6$ and $R_7$ independently of each other represent hydrogen, alkyl, cycloalkyl alkyl, $CF_3$-alkyl or pyridyl alkyl;

(w) pyridyl, which is unsubstituted or mono-, di- or trisubstituted by halogen or alkyl which is mono-, di- or trisubstituted by halogen;
(x) pyrimidyl;
(y) indolyl, which is mono- or disubstituted by alkyl-C(O)-NH-alkyl;
(z) 2-(alkyl)-benzothiazolyl;

(aa) a radical of subformula Ia

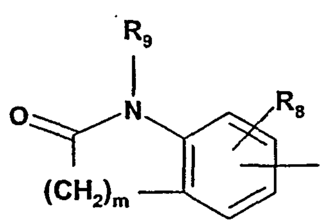

(Ia)

wherein $R_8$ is hydrogen, halogen or alkyl, $R_9$ is hydrogen or alkyl, and m is 1, 2, 3 or 4; or
(bb) a radical of subformula Ib

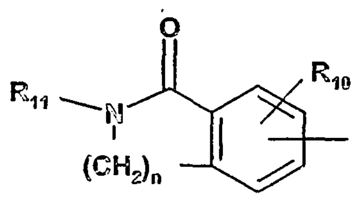

(Ib)

wherein $R_{10}$ is hydrogen, halogen or alkyl, $R_{11}$ is hydrogen or alkyl, and n is 1, 2, 3 or 4;

$R_2$ represents alkyl, which is unsubstituted or substituted by cycloalkyl, which is unsubstituted or mono- or disubstituted by halogen, or phenyl, which is mono- or disubstituted by halogen;
under the proviso that $R_2$ does not represent 1,1-dimethylethyl if Y is O and $R_1$ is selected from 3-pyridyl, 4-pyridyl, 5-chloro-3-pyridyl, 6-chloro-3-pyridyl, 2-chloro-4-pyridyl, 2-trifluoromethyl-4-pyridyl, 2-difluoromethyl-4-pyridyl, 4-acetyl-1-piperazinyl-phenyl, 4-methyl-1-piperazinyl-methyl-phenyl, and
under the proviso that $R_2$ does not represent 1,1-dimethylethyl, if Y is S and $R_1$ is 4-pyridyl; wherein an alcohol or

a thiol of formula II,

$R_1$-$(Y)_p$-H (II)

wherein Y represents $-(CH_2)_t$-O- or $(CH_2)_r$-S- and t, r and $R_1$ have the meanings as provided above for a compound of formula I, is alkylated with a pyrrolo pyrimidine of formula III

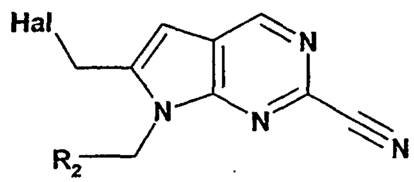

(III)

wherein $R_2$ has the meaning as provided above for a compound of formula I and Hal denotes halo, preferably bromo, wherein the starting compounds of formula II and III may also be present with functional groups in protected form, if necessary, and/or in the form of salts, provided a salt-forming group is present and the reaction in salt form is possible;
wherein any protecting groups in a protected derivative of a compound of the formula I are removed;
and, if so desired, an obtainable compound of formula I is converted into another compound of formula I or a N-oxide thereof, a free compound of formula I is converted into a salt, an obtainable salt of a compound of formula I is converted into the free compound or another salt, and/or a mixture of isomeric compounds of formula I is separated into the individual isomers.

## Patentansprüche

1. Pyrrolopyrimidin der Formel

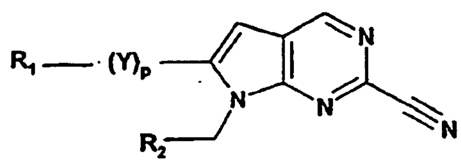

(I)

worin
Y für $-(CH_2)_t$-O- oder $-(CH_2)_r$-S- steht,
p für 1 oder 2 steht,
r für 1, 2 oder 3 steht,
t für 1, 2 oder 3 steht,
$R_1$ steht für

(h) Phenyl, das unsubstituiert oder mono-, di- oder trisubstituiert ist durch

($\alpha$) Halogen, Carboxy, Alkoxy, Nitro, Alkyl-C(O)-NH-, Cycloalkyl-C(O)-NH-, Alkyl-C(O)-N(alkyl)-, Formyl, Alkyl-C(O)-, Alkyl-$S(O)_2$-NH-, $CF_3$-Alkyl-$S(O)_2$-NH-, Pyrrolidinylcarbonyl, Piperidinylcarbonyl, Morpholinylcarbonyl, N-Alkylpiperazinylcarbonyl, Piperidinyl, 1-(Alkylcarbonyl)-piperidinyl, 1,2,3,6-Tetrahydropyridyl, Alkylcarbonyl-1,2,3,6-tetrahydropyridyl, Piperazinyl, Alkylpiperazinyl, Alkylcarbonylpiperazinyl, Cycloalkylcarbonylpiperazinyl, Alkoxycarbonylpiperazinyl, Alkyl-$SO_2$-piperazinyl, Diazacycloheptyl, Alkylcarbonyldiazacycloheptyl, 2-Oxo-1-pyrrolidinyl oder 3,3-Dialkyl-2-oxo-1-pyrrolidinyl,
($\beta$) $R_3$-Alkyl, worin $R_3$ für Wasserstoff, Hydroxy, Carboxy, Alkyl-N(alkyl)-, Alkyl-NH-, 1-Pyrrolidinyl, 1-Piperidyl, 4-Alkyl-1-piperazinylcarbonyl, 2,4-Dioxa-5,5-(dialkyl)-oxazolidin-3-yl oder $R_4R_5$N-C(O)- steht, worin $R_4$ und $R_5$ jeweils unabhängig voneinander für Wasserstoff oder Alkyl stehen, oder
($\gamma$) $R_6R_7$N-C(O)-, worin $R_6$ und $R_7$ jeweils unabhängig voneinander für Wasserstoff, Alkyl, Cycloalkylalkyl,

$CF_3$-Alkyl oder Pyridylalkyl stehen,

(i) Pyridyl, das unsubstituiert oder mono-, di- oder trisubstituiert ist durch Halogen oder Alkyl, das mono-, di- oder trisubstituiert ist durch Halogen,
(j) Pyrimidyl,
(k) Indolyl, das mono- oder disubstituiert ist durch Alkyl-C(O)-NH-alkyl,
(l) 2-(Alkyl)-benzothiazolyl,
(m) einen Rest der folgenden Subformel Ia

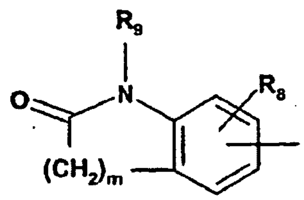
(Ia)

worin $R_8$ für Wasserstoff, Halogen oder Alkyl steht, $R_9$ für Wasserstoff oder Alkyl steht und m für 1, 2, 3 oder 4 steht, oder
(n) einen Rest der folgenden Subformel Ib

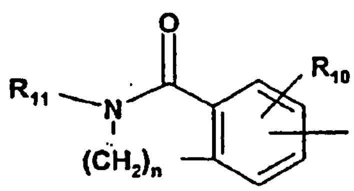
(Ib)

worin $R_{10}$ für Wasserstoff, Halogen oder Alkyl steht, $R_{11}$ für Wasserstoff oder Alkyl steht, und n für 1, 2, 3 oder 4 steht,

$R_2$ für Alkyl, das unsubstituiert oder substituiert ist durch Cycloalkyl, das unsubstituiert oder mono- oder disubstituiert ist durch Halogen, oder für Phenyl steht, das mono- oder disubstituiert ist durch Halogen,
mit der Maßgabe, dass $R_2$ nicht für 1,1-Dimethylethyl steht, wenn Y für O steht und $R_1$ ausgewählt ist aus 3-Pyridyl, 4-Pyridyl, 5-Chlor-3-pyridyl, 6-Chlor-3-pyridyl, 2-Chlor-4-pyridyl, 2-Trifluormethyl-4-pyridyl, 2-Difluormethyl-4-pyridyl, 4-Acetyl-1-piperazinylphenyl, 4-Methyl-1-piperazinylmethylphenyl, und
mit der Maßgabe, dass $R_2$ nicht für 1,1-Dimethylethyl steht, wenn Y für S steht und $R_1$ für 4-Pyridyl steht, oder
Y für $-(CH_2)_j-$ oder -CH=CH- steht,
j für 1 oder 2 steht,
p für 1 oder 2 steht,
$R_1$ steht für

(c) Thienyl, Thiazolyl oder 1-Piperidinylcarbonyl, oder
(d) Phenyl, das unsubstituiert oder mono-, di- oder trisubstituiert ist durch

(i) Alkoxy, $H_2N$-C(O)-, 4-(Alkylcarbonyl)-1-piperazinyl, 2-Oxo-1-pyrrolidinyl oder Halogen,
(ii) $R_{12}$-O-C(O)-, worin $R_{12}$ für Wasserstoff oder Alkyl steht,
(iii) $R_{13}$NH-, worin $R_{13}$ für Wasserstoff oder einen Rest $R_{14}$-Alkyl-Z- steht, worin Z für CO, SO oder $SO_2$ steht und $R_{14}$ für Wasserstoff, Trifluormethyl oder Alkoxy steht, oder
(iv) $R_{15}$-Alkyl, worin $R_{15}$ für Wasserstoff, Hydroxy, Alkoxy, 1-Pyrrolidinyl, 2-Oxo-1-pyrrolidinyl, Imidazolidin-2,5-dion-1-yl, 5,5-Dialkyloxazolidin-2,4-dion-3-yl oder Alkyl-N(R16)-, worin $R_{16}$ für Wasserstoff oder Alkyl steht, und

$R_2$ steht für

(a) Alkyl, das unsubstituiert oder substituiert ist durch Alkenyl, Indanyl, Cycloalkyl, das unsubstituiert oder mono- oder disubstituiert ist durch Halogen oder Alkyl, Cycloalkenyl, Phenyl, das unsubstituiert oder mono- oder disubstituiert ist durch Halogen, oder Alkyl,
(b) Cycloalkyl, oder
(c) Alkylcarbonyl,

mit der Maßgabe, dass, falls Y für $CH_2$ steht, $R_1$ für 4-Chlorphenyl steht und p für 1 steht, $R_2$ nicht für 1,1-Dimethylethyl, 1-Methylethyl, Cyclopropyl, Cyclohexyl, 2-Methylpropyl oder 2-Ethylpropyl steht,
mit der Maßgabe, dass $R_2$ nicht für 1,1-Dimethylethyl steht, wenn p für 1 steht, Y für $CH_2$ steht und $R_1$ steht für Thienyl, Phenyl, Methoxyphenyl, Propoxyphenyl, 4-Fluorphenyl, 4-Methylphenyl, 4-Ethylphenyl, 4-Butylphenyl, Hydroxymethylphenyl, 4-(5,5-Dimethyloxazolidin-2,4-dion-3-ylmethyl)-phenyl, 4-(Methylsulfonylamino)-phenyl, 4-(n-Butylsulfonylamino)-phenyl, 4-(Ethylsulfonylamino)-phenyl, 4-(n-Propylsulfonylamino)-phenyl, 4-(Isopropylsulfonylamino)-phenyl, 4-Aminophenyl, 4-(Acetylamino)-phenyl, 4-(Butanoylamino)-phenyl oder 4-(Diethylaminomethyl)-phenyl, und
mit der Maßgabe, dass $R_2$ nicht für 1-Methylethyl steht, wenn p für 1 steht, Y für $CH_2$ steht und $R_1$ für Phenyl steht, das unsubstituiert oder substituiert ist durch 4-Acetyl-1-piperazinyl, oder
Y für $-(CH_2)_f-$ steht,
f für 1 oder 2 steht,
p für 1 steht,
$R_1$ steht für

(a) 1,2,3,6-Tetrahydropyrid-1-yl, Alkyl-1,2,3,6-tetrahydropyrid-1-yl, Dialkyl-1,2,3,6-tetrahydropyrid-1-yl, Halogen-1,2,3,6-tetrahydropyrid-1-yl, Phenyl-1,2,3,6-tetrahydropyrid-1-yl, Imidazolyl, Alkylimidazolyl, Dihalogenimidazolyl, Imidazolidin-2,5-dion-1-yl, 5,5-Dialkyloxazolidin-2,4-dion-3-yl, Alkylimidazolidin-2,5-dion-1-yl, Trifluormethyl-3,4-pyrrolin-1-yl, Pyrrolidinyl, Alkyl-1-pyrrolidinyl, Dialkylpyrrolidinyl, Alkoxypyrrolidinyl, Alkyl-2-oxo-1-pyrrolidinyl, Dialkyl-2-oxo-1-pyrrolidinyl, Halogen-1-pyrrolidinyl, Dihalogen-1-pyrrolidinyl, Dihalogen-1-piperidinyl, Triazolyl, Nitrotriazolyl, Phenylimidazolyl, Tetrazolyl, Benzo[b]imidazolyl, (1-(Alkyl-$SO_2$)-4-piperidinyl)-2,3-dihydro-2-oxobenzo[b]-imidazolyl, 3-(Alkylcarbonyl-4-piperidinyl)-2,3-dihydro-2-oxobenzo[b]imidazolyl, Indolyl, Halogen-1-indolyl, 1,3-Dihydro-2-isoindolyl, 2,3-Dihydro-1-indolyl, 2,3-Dihydro-2-oxobenzo[b]thiazolyl, Di-alkoxy-1,2,3,4-tetrahydrochinolin oder Alkoxy-1,2,3,4-tetrahydroisochinolin,
(b) einen Rest der folgenden Substruktur Ic

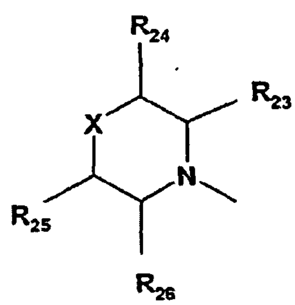
(Ic)

die an das Molekül über das Stickstoffatom gebunden ist, worin
X für -O-, $-(CH_2)_s-CR_{17}R_{18}-$ oder $-NR_{18}$ steht, worin
s für 0, 1 oder 2 steht, $R_{17}$ und $R_{18}$ unabhängig ausgewählt sind aus Wasserstoff, Halogen, Hydroxy, Alkyl, Phenylalkylcarbonyl, Carbamoyl, N-Phenylcarbamoyl, Cyano, Pyridyl, Piperidinyl und Phenyl, das unsubstituiert oder mono- oder disubstituiert ist durch Halogen oder Alkoxy, oder, wenn X für $CR_{17}R_{18}$ steht, dann $R_{17}$ und $R_{18}$ zusammen eine Oxogruppe oder eine Gruppe HO-C(O)-CH= bilden, und $R_{23}$, $R_{24}$, $R_{25}$ und $R_{26}$ unabhängig ausgewählt sind aus Wasserstoff und Alkyl,
(c) einen Rest der folgenden Substruktur Id

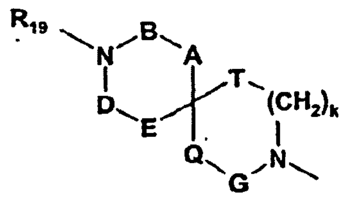

(Id)

die an das Molekül über das Stickstoffatom gebunden ist, worin k für 0, 1 oder 2 steht, A für $CH_2$ oder eine Bindung steht, B für $CH_2$ oder Carbonyl steht, D für $CH_2$ oder Carbonyl steht, E für $CH_2$ oder $NR_{22}$ steht, G für $CH_2$ oder eine Bindung steht, Q für $CH_2$ oder Carbonyl steht, T für $CH_2$ oder $NR_{29}$ steht, $R_{19}$ für Wasserstoff, Alkyl, Phenylalkyl, Alkylcarbonyl oder Alkyl-$SO_2$- steht, $R_{22}$ für Wasserstoff oder Alkyl steht und $R_{29}$ für Phenyl steht,

(d) einen Rest der folgenden Substruktur Ie

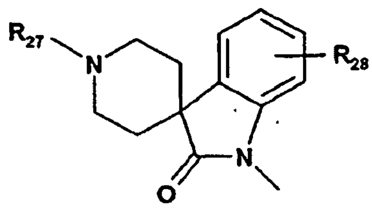

(Ie)

die an das Molekül über das Stickstoffatom gebunden ist, worin

$R_{27}$ für Alkyl oder Alkylcarbonyl steht und $R_{28}$ für Wasserstoff, Alkoxy oder Halogen steht, oder

(e) $NR_{20}R_{21}$, worin $R_{20}$ und $R_{21}$ unabhängig ausgewählt sind aus Wasserstoff, Alkyl, Cycloalkyl, das unsubstituiert oder mono- oder disubstituiert ist durch Hydroxy, und Phenyl, das unsubstituiert oder mono- oder disubstituiert ist durch 1,2,3-Thiadiazolyl, mit der Maßgabe, dass $R_{20}$ und $R_{21}$ nicht gleichzeitig für Wasserstoff stehen können, und

$R_2$ für Alkyl steht, das unsubstituiert oder substituiert ist durch Cycloalkyl, das unsubstituiert oder mono- oder disubstituiert ist durch Halogen, oder für Phenyl steht, das mono- oder disubstituiert ist durch Halogen,
mit der Maßgabe, dass $R_2$ nicht für 1,1-Dimethylethyl steht, wenn

(a) $R_1$ für Benzo[b]imidazol-1-yl, 1-Imidazolyl, 4,5-Dichlor-1-imidazolyl, 2-($C_1$-$C_4$-Alkyl)-1-imidazolyl, Imidazolidin-2,5-dion-1-yl, 5,5-Dimethyloxazolidin-2,4-dion-3-yl, 1H-1,2,3-Triazol-1-yl, 2H-1,2,3-Triazol-2-yl, 3-Nitro-1H-1,2,4-triazol-1-yl, 2H-Tetrazol-2-yl oder 1 H-Tetrazol-1-yl steht, oder wenn $R_1$ für einen Rest der Substruktur Ic steht, $R_{23}$ bis $R_{26}$ für Wasserstoff stehen, X für $NR_{18}$ steht und $R_{18}$ für Wasserstoff, Methyl, Ethyl, Acetyl, 4-Pyridyl, 1-Piperidinyl, Phenyl, Methoxyphenyl, Ethoxyphenyl, Fluorphenyl oder Chlorphenyl steht,
(b) $R_1$ für einen Rest der Substruktur Ic steht, $R_{23}$ bis $R_{26}$ für Wasserstoff stehen, X für $-(CH_2)_s-CR_{17}R_{18}-$ steht, s für 0 steht und $R_{17}$ und $R_{18}$ ausgewählt sind aus Hydroxyl und Phenyl, das monosubstituiert ist durch Chlor, oder $R_{17}$ und $R_{18}$ ausgewählt sind aus Wasserstoff, Methoxyphenyl und N-Phenylcarbamoyl, oder
(c) $R_1$ für einen Rest der Substruktur Id steht, k für 1 steht, A für eine Bindung steht, E für $NR_{22}$ steht, $R_{22}$ für Wasserstoff steht, G, Q und T für $CH_2$ stehen, B und D für Carbonyl stehen und $R_{19}$ für Methyl, n-Propyl oder Isobutyl steht,

mit der Maßgabe, dass $R_2$ nicht für 2-Methylpropyl steht, wenn $R_1$ für einen Rest der Substruktur Id steht, k für 1 steht, A für eine Bindung steht, E für $NR_{22}$ steht, $R_{22}$ für Wasserstoff steht, G, Q und T für $CH_2$ stehen, B und D für Carbonyl stehen und $R_{19}$ für Methyl steht, oder, wenn $R_1$ für einen Rest der Substruktur Ic steht, $R_{23}$ bis $R_{26}$ für Wasserstoff stehen, X für $-(CH_2)_s-CR_{17}R_{18}-$ steht, s für 0 steht, und $R_{17}$ und $R_{18}$ ausgewählt sind aus Wasserstoff und Phenyl, das monosubstituiert ist durch Methoxy, und
mit der Maßgabe, dass $R_2$ nicht für 1-Methylethyl steht, wenn $R_1$ für einen Rest der Substruktur Ic steht, $R_{23}$ bis $R_{26}$ für Wasserstoff stehen, X für $NR_{18}$ steht und $R_{18}$ für Methoxyphenyl oder Ethoxyphenyl steht, oder X für $CR_{17}R_{18}$ steht, und $R_{17}$ und $R_{18}$ ausgewählt sind aus Wasserstoff und Methoxyphenyl,
oder ein N-Oxid oder ein Tautomer hiervon,
oder ein Salz eines solchen Pyrrolopyrimidins, eines N-Oxids oder eines Tautomers hiervon.

**2.** Pyrrolopyrimidin der Formel I nach Anspruch 1, worin Y für $-CH_2$-O- oder $-CH_2$-S- steht, p für 1 steht, $R_1$ steht für

(o) Phenyl, das unsubstituiert oder mono- oder disubstituiert ist durch

(α) Halogen, Carboxy, $C_1$-$C_4$-Alkoxy, Nitro, $C_1$-$C_4$-Alkyl-C(O)-NH-, $C_3$-$C_4$-Cycloalkyl-C(O)-NH-, $C_1$-$C_4$-Alkyl-C(O)-N($C_1$-$C_4$-alkyl)-, Formyl, $C_1$-$C_4$-Alkyl-C(O)-, $C_1$-$C_4$-Alkyl-$S(O)_2$-NH-, $CF_3$-$C_1$-$C_3$-Alkyl-$S(O)_2$-NH-, 1-Pyrrolidinylcarbonyl, 1-Piperidinylcarbonyl, 4-Morpholinylcarbonyl, 4-($C_1$-$C_4$-Alkyl)-1-piperazinylcarbonyl, 4-Piperidinyl, 1-Piperidinyl, 1-($C_1$-$C_4$-Alkylcarbonyl)-4-piperidinyl, 1,2,3,6-Tetrahydro-4-pyridyl, 1-($C_1$-$C_4$-Alkylcarbonyl)-1,2,3,6-tetrahydro-4-pyridyl, 1-Piperazinyl, 4-($C_1$-$C_4$-Alkyl)-1-piperazinyl, 4-($C_1$-$C_4$-Alkylcarbonyl)-1-piperazinyl, 4-($C_3$-$C_5$-Cycloalkylcarbonyl)-1-piperazinyl, 4-($C_1$-$C_4$-Alkoxycarbonyl)-1-piperazinyl, 4-($C_1$-$C_4$-Alkyl-$SO_2$)-1-piperazinyl, 1,4-Diazacyclohept-1-yl, 4-($C_1$-$C_4$-Alkylcarbonyl)-1,4-diazacyclohept-1-yl, 2-Oxo-1-pyrrolidinyl oder 3,3-Di-($C_1$-$C_4$-alkyl)-2-oxo-1-pyrrolidinyl,
(β) $R_3$-$C_1$-$C_4$-Alkyl, worin $R_3$ für Wasserstoff, Hydroxyl, Carboxy, $C_1$-$C_4$-Alkyl-N($C_1$-$C_4$-alkyl)-, $C_1$-$C_4$-Alkyl-NH-,1-Pyrrolidinyl,1-Piperidyl,4-($C_1$-$C_4$-Alkyl)-1-piperazinylcarbonyl,2,4-Dioxa-5,5-(di-$C_1$-$C_4$-alkyl)-oxazolidin-3-yl oder $R_4R_5$N-C(O)- steht, worin $R_4$ und $R_5$ jeweils unabhängig voneinander für Wasserstoff oder $C_1$-$C_4$-Alkyl stehen, oder
(γ) $R_6R_7$N-C(O)-, worin $R_6$ und $R_7$ jeweils unabhängig voneinander für Wasserstoff, $C_1$-$C_4$-Alkyl, $C_5$-$C_7$-Cycloalkyl-$C_1$-$C_4$-alkyl, $CF_3$-$C_1$-$C_3$-Alkyl oder Pyridyl-$C_1$-$C_4$-alkyl stehen,

(p) Pyridyl, das unsubstituiert oder mono- oder disubstituiert ist durch Halogen oder $C_1$-$C_4$-Alkyl, das di- oder trisubstituiert ist durch Halogen,
(q) Pyrimidyl,
(r) Indolyl, das monosubstituiert ist durch $C_1$-$C_4$-Alkyl-C(O)-NH-$C_1$-$C_4$-alkyl,
(s) 2-($C_1$-$C_4$-Alkyl)-benzothiazolyl,
(t) einen Rest der Subformel Ia
worin $R_8$ für Wasserstoff steht, $R_9$ für Wasserstoff steht und m für 2 oder 3 steht, oder
(u) einen Rest der Subformel Ib
worin $R_{10}$ für Wasserstoff steht, $R_{11}$ für Wasserstoff steht, und n für 2 oder 3 steht,

$R_2$ für $C_1$-$C_5$-Alkyl steht, das unsubstituiert oder substituiert ist durch $C_5$-$C_7$-Cycloalkyl, das unsubstituiert oder disubstituiert ist durch Halogen, oder Phenyl, das mono- oder disubstituiert ist durch Halogen,
mit der Maßgabe, dass $R_2$ nicht für 1,1-Dimethylethyl steht, wenn Y für O steht und $R_1$ ausgewählt ist aus 3-Pyridyl, 4-Pyridyl, 5-Chlor-3-pyridyl, 6-Chlor-3-pyridyl, 2-Chlor-4-pyridyl, 2-Trifluormethyl-4-pyridyl, 2-Difluormethyl-4-pyridyl, 4-Acetyl-1-piperazinylphenyl oder 4-Methyl-1-piperazinylmethylphenyl, und
mit der Maßgabe, dass $R_2$ nicht für 1,1-Dimethylethyl steht, wenn Y für S steht und $R_1$ für 4-Pyridyl steht, oder
Y für $-CH_2$- oder -CH=CH- steht,
p für 1 oder 2 steht,
$R_1$ steht für

(e) Thienyl, Thiazolyl oder 1-Piperidinylcarbonyl, oder
(f) Phenyl, das unsubstituiert oder mono- oder disubstituiert ist durch

(i) $C_1$-$C_4$-Alkoxy, $H_2$N-C(O)-, 4-($C_1$-$C_4$-Alkylcarbonyl)-1-piperazinyl, 2-Oxo-1-pyrrolidinyl oder Halogen,
(ii) $R_{12}$-O-C(O)-, worin $R_{12}$ für Wasserstoff oder $C_1$-$C_4$-Alkyl steht,
(iii) $R_{13}$NH-, worin $R_{13}$ für Wasserstoff oder einen Rest $R_{14}$-$C_1$-$C_4$-Alkyl-Z- steht, worin Z für CO oder $SO_2$ steht und $R_{14}$ für Wasserstoff, Trifluormethyl oder $C_1$-$C_4$-Alkoxy steht, und
(iv) $R_{15}$-$C_1$-$C_4$-Alkyl, worin $R_{15}$ für Wasserstoff, Hydroxy, Niederalkoxy, 1-Pyrrolidinyl, 2-Oxo-1-pyrrolidinyl, Imidazolidin-2,5-dion-1-yl, 5,5-Dialkyloxazolidin-2,4-dion-3-yl oder $C_1$-$C_4$-Alkyl-N($R_{16}$)- steht, worin $R_{16}$ für Wasserstoff oder $C_1$-$C_4$-Alkyl steht, und

$R_2$ steht für

(a) $C_1$-$C_7$-Alkyl, das unsubstituiert oder substituiert ist durch $C_2$-$C_3$-Alkenyl, Indanyl, $C_3$-$C_7$-Cycloalkyl, das unsubstituiert oder disubstituiert ist durch Halogen oder $C_1$-$C_4$-Alkyl, $C_3$-$C_7$-Cycloalkenyl, Phenyl, das unsub-

stituiert oder mono- oder disubstituiert ist durch Halogen, oder $C_1$-$C_4$-Alkyl,
(b) $C_3$-$C_7$-Cycloalkyl, oder
(c) $C_1$-$C_4$-Alkylcarbonyl,

mit der Maßgabe, dass, falls Y für $CH_2$ steht, $R_1$ für 4-Chlorphenyl steht und p für 1 steht, $R_2$ nicht für 1,1-Dimethylethyl, 1-Methylethyl, Cyclopropyl, Cyclohexyl, 2-Methylpropyl oder 2-Ethylpropyl steht,
mit der Maßgabe, dass $R_2$ nicht für 1,1-Dimethylethyl steht, wenn p für 1 steht, Y für $CH_2$ steht und $R_1$ steht für Thienyl, Phenyl, Methoxyphenyl, Propoxyphenyl, 4-Fluorphenyl, 4-Methylphenyl, 4-Ethylphenyl, 4-Butylphenyl, Hydroxymethylphenyl, 4-(5,5-Dimethyloxazolidin-2,4-dion-3-ylmethyl)-phenyl, 4-(Methylsulfonylamino)-phenyl, 4-(n-Butylsulfonylamino)-phenyl, 4-(Ethylsulfonylamino)-phenyl, 4-(n-Propylsulfonylamino)-phenyl, 4-(Isopropylsulfonylamino)-phenyl, 4-Aminophenyl, 4-(Acetylamino)-phenyl, 4-(Butanoylamino)-phenyl oder 4-(Diethylaminomethyl)-phenyl, und
mit der Maßgabe, dass $R_2$ nicht für 1-Methylethyl steht, wenn p für 1 steht, Y für $CH_2$ steht und $R_1$ für Phenyl steht, das unsubstituiert oder substituiert ist durch 4-Acetyl-1-piperazinyl, oder
Y für -$CH_2$- steht,
p für 1 steht,
$R_1$ steht für

(a) 1,2,3,6-Tetrahydropyrid-1-yl, 4-($C_1$-$C_4$-Alkyl)-1,2,3,6-tetrahydropyrid-1-yl, 4,5-Di($C_1$-$C_4$-alkyl)-1,2,3,6-tetrahydropyrid-1-yl, 5-Chlor-1,2,3,6-tetrahydropyrid-1-yl, 4-Phenyl-1,2,3,6-tetrahydropyrid-1-yl, 1-Imidazolyl, 2-($C_1$-$C_4$-Alkyl)-1-imidazolyl, 4,5-Dihalogen-1-imidazolyl, Imidazolidin-2,5-dion-1-yl, 5,5-Dimethyloxazolidin-2,4-dion-3-yl, 3-($C_1$-$C_4$-Alkyl)-imidazolidin-2,5-dion-1-yl, 3-Trifluormethyl-3,4-pyrrolin-1-yl, 1-Pyrrolidinyl, 3-$C_1$-$C_4$-Alkyl-1-pyrrolidinyl, 3,3-Di($C_1$-$C_4$-alkyl)-1-pyrrolidinyl, 3-$C_1$-$C_4$-Alkoxy-1-pyrrolidinyl, 3-$C_1$-$C_4$-Alkyl-2-oxo-1-pyrrolidinyl, 3,3-Di($C_1$-$C_4$-alkyl)-2-oxo-1-pyrrolidinyl, 3-Halogen-1-pyrrolidinyl, 3,3-Dihalogen-1-pyrrolidinyl, 3,3-Dihalogen-1-piperidinyl, 1 H-1,2,3-Triazol-1-yl, 2H-1,2,3-Triazol-2-yl, 1H-1,2,4-Triazol-1-yl, 3-Nitro-1H-1,2,4-triazol-1-yl, 2-Phenyl-1-imidazolyl, 2H-Tetrazol-2-yl, 1 H-Tetrazol-1-yl, Benzo[b]imidazol-1-yl, 3-(1-($C_1$-$C_4$-Alkyl-$SO_2$)-4-piperidinyl)-2,3-dihydro-2-oxobenzo[b]imidazol-1-yl, 3-(1-$C_1$-$C_4$-Alkylcarbonyl-4-piperidinyl)-2,3-dihydro-2-oxobenzo[b]imidazol-1-yl, 1-Indolyl, 6-Halogen-1-indolyl, 1,3-Dihydro-2-isoindolyl, 2,3-Dihydro-1-indolyl, 2,3-Dihydro-2-oxobenzo[b]thiazol-3-yl, 6,7-Di($C_1$-$C_4$-alkoxy)-1,2,3,4-tetrahydrochinolin, 6-$C_1$-$C_4$-Alkoxy-1,2,3,4-tetrahydroisochinolin oder 7-$C_1$-$C_4$-Alkoxy-1,2,3,4-tetrahydroisochinolin,
(b) einen Rest der Substruktur Ic
die an das Molekül über das Stickstoffatom gebunden ist, worin
X für -O-, -$(CH_2)_s$-$CR_{17}R_{18}$- oder -$NR_{18}$ steht, worin
s für 0 oder 1 steht, $R_{17}$ und $R_{18}$ unabhängig ausgewählt sind aus Wasserstoff, Halogen, Hydroxy, $C_1$-$C_4$-Alkyl, Phenyl-$C_1$-$C_4$-alkylcarbonyl, Carbamoyl, N-Phenylcarbamoyl, Cyano, 4-Pyridyl, 1-Piperidinyl und Phenyl, das unsubstituiert oder monosubstituiert ist durch Halogen oder $C_1$-$C_4$-Alkoxy, oder, wenn X für $CR_{17}R_{18}$ steht, dann $R_{17}$ und $R_{18}$ zusammen eine Oxogruppe oder eine Gruppe HO-C(O)-CH= bilden, und $R_{23}$, $R_{24}$, $R_{25}$ und $R_{26}$ unabhängig ausgewählt sind aus Wasserstoff und $C_1$-$C_4$-Alkyl,
(c) einen Rest der Substruktur Id
die an das Molekül über das Stickstoffatom gebunden ist, worin
k für 0 oder 1 steht, A für $CH_2$ oder eine Bindung steht, B für $CH_2$ oder Carbonyl steht, D für $CH_2$ oder Carbonyl steht, E für $CH_2$ oder $NR_{22}$ steht, G für $CH_2$ oder eine Bindung steht, Q für $CH_2$ oder Carbonyl steht, T für $CH_2$ oder $NR_{29}$ steht, $R_{19}$ für Wasserstoff, $C_1$-$C_4$-Alkyl, Phenyl-$C_1$-$C_4$-alkyl, $C_1$-$C_4$-Alkylcarbonyl oder $C_1$-$C_4$-Alkyl-$SO_2$- steht, $R_{22}$ für Wasserstoff steht und $R_{29}$ für Phenyl steht,
(d) einen Rest der Substruktur Ie
die an das Molekül über das Stickstoffatom gebunden ist, worin
$R_{27}$ für $C_1$-$C_4$-Alkyl oder $C_1$-$C_4$-Alkylcarbonyl steht und $R_{28}$ für Wasserstoff, $C_1$-$C_4$-Alkoxy oder Halogen steht, oder
(e) $NR_{20}R_{21}$, worin $R_{20}$ und $R_{21}$ unabhängig ausgewählt sind aus Wasserstoff, $C_1$-$C_4$-Alkyl, $C_3$-$C_7$-Cycloalkyl, das unsubstituiert oder monosubstituiert ist durch Hydroxy, und Phenyl, das unsubstituiert oder monosubstituiert ist durch 1,2,3-Thiadiazol-4-yl, mit der Maßgabe, dass $R_{20}$ und $R_{21}$ nicht gleichzeitig für Wasserstoff stehen können, und

$R_2$ für $C_1$-$C_8$-Alkyl steht, das unsubstituiert oder substituiert ist durch $C_3$-$C_7$-Cycloalkyl, das unsubstituiert oder disubstituiert ist durch Halogen, oder für Phenyl steht, das mono- oder disubstituiert ist durch Halogen,
mit der Maßgabe, dass $R_2$ nicht für 1,1-Dimethylethyl steht, wenn

(a) $R_1$ für Benzo[b]imidazol-1-yl, 1-Imidazolyl, 4,5-Dichlor-1-imidazolyl, 2-($C_1$-$C_4$-Alkyl)-1-imidazolyl, Imidazo-

lidin-2,5-dion-1-yl, 5,5-Dimethyloxazolidin-2,4-dion-3-yl, 1H-1,2,3-Triazol-1-yl, 2H-1,2,3-Triazol-2-yl, 3-Nitro-1H-1,2,4-triazol-1-yl, 2H-Tetrazol-2-yl oder 1 H-Tetrazol-1-yl steht, oder wenn $R_1$ für einen Rest der Substruktur Ic steht, $R_{23}$ bis $R_{26}$ für Wasserstoff stehen, X für $NR_{18}$ steht und $R_{18}$ für Wasserstoff, Methyl, Ethyl, Acetyl, 4-Pyridyl, 1-Piperidinyl, Phenyl, Methoxyphenyl, Ethoxyphenyl, Fluorphenyl oder Chlorphenyl steht,
(b) $R_1$ für einen Rest der Substruktur Ic steht, $R_{23}$ bis $R_{26}$ für Wasserstoff stehen, X für $-(CH_2)_s-CR_{17}R_{18}-$ steht, s für 0 steht und $R_{17}$ und $R_{18}$ ausgewählt sind aus Hydroxyl und Phenyl, das monosubstituiert ist durch Chlor, oder $R_{17}$ und $R_{18}$ ausgewählt sind aus Wasserstoff, Methoxyphenyl und N-Phenylcarbamoyl, oder
(c) $R_1$ für einen Rest der Substruktur Id steht, k für 1 steht, A für eine Bindung steht, E für $NR_{22}$ steht, $R_{22}$ für Wasserstoff steht, G, Q und T für $CH_2$ stehen, B und D für Carbonyl stehen und $R_{19}$ für Methyl, n-Propyl oder Isobutyl steht,

mit der Maßgabe, dass $R_2$ nicht für 2-Methylpropyl steht, wenn $R_1$ für einen Rest der Substruktur Id steht, k für 1 steht, A für eine Bindung steht, E für $NR_{22}$ steht, $R_{22}$ für Wasserstoff steht, G, Q und T für $CH_2$ stehen, B und D für Carbonyl stehen und $R_{19}$ für Methyl steht, oder, wenn $R_1$ für einen Rest der Substruktur Ic steht, $R_{23}$ bis $R_{26}$ für Wasserstoff stehen, X für $-(CH_2)_s-CR_{17}R_{18}-$ steht, s für 0 steht, und $R_{17}$ und $R_{18}$ ausgewählt sind aus Wasserstoff und Phenyl, das monosubstituiert ist durch Methoxy, und
mit der Maßgabe, dass $R_2$ nicht für 1-Methylethyl steht, wenn $R_1$ für einen Rest der Substruktur Ic steht, $R_{23}$ bis $R_{26}$ für Wasserstoff stehen, X für $NR_{18}$ steht und $R_{18}$ für Methoxyphenyl oder Ethoxyphenyl steht, oder X für $CR_{17}R_{18}$ steht, und $R_{17}$ und $R_{18}$ ausgewählt sind aus Wasserstoff und Methoxyphenyl, oder ein Tautomer hiervon, oder ein Salz eines solchen Pyrrolopyrimidins oder eines Tautomers hiervon.

3. Pyrrolopyrimidin der Formel I nach Anspruch 1 oder 2 oder ein N-Oxid oder ein Tautomer hiervon oder ein pharmazeutisch akzeptables Salz einer solchen Verbindung zur Verwendung bei einem Verfahren für die Behandlung des menschlichen oder tierischen Körpers.

4. Verwendung eines Pyrrolopyrimidins der Formel 1 nach Anspruch 1 oder 2 oder eines N-Oxids oder eines Tautomers hiervon oder eines pharmazeutisch akzeptablen Salzes einer solchen Verbindung zur Herstellung eines Arzneimittels für die Behandlung von neuropathischem Schmerz.

5. Pharmazeutisches Präparat, umfassend ein Pyrrolopyrimidin der Formel I nach Anspruch 1 oder 2, oder ein N-Oxid oder ein Tautomer hiervon oder ein pharmazeutisch akzeptables Salz einer solchen Verbindung oder ein Hydrat oder ein Solvat hiervon, und wenigstens einen pharmazeutisch akzeptablen Träger.

6. Verfahren zur Herstellung eines Pyrrolopyrimidins der Formel I

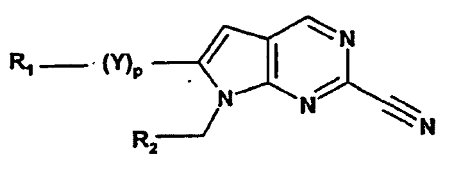

(I)

worin
Y für $-(CH_2)_t-O-$ oder $-(CH_2)_r-S-$ steht,
p für 1 oder 2 steht,
r für 1, 2 oder 3 steht,
t für 1, 2 oder 3 steht,
$R_1$ steht für

(v) Phenyl, das unsubstituiert oder mono-, di- oder trisubstituiert ist durch

(a) Halogen, Carboxy, Alkoxy, Nitro, Alkyl-C(O)-NH-, Cycloalkyl-C(O)-NH-, Alkyl-C(O)-N(alkyl)-, Formyl, Alkyl-C(O)-, Alkyl-$S(O)_2$-NH-, $CF_3$-Alkyl-$S(O)_2$-NH-, Pyrrolidinylcarbonyl, Piperidinylcarbonyl, Morpholinylcarbonyl, N-Alkylpiperazinylcarbonyl, Piperidinyl, 1-(Alkylcarbonyl)-piperidinyl, 1,2,3,6-Tetrahydropyridyl, Alkylcarbonyl-1,2,3,6-tetrahydropyridyl, Piperazinyl, Alkylpiperazinyl, Alkylcarbonylpiperazinyl, Cycloalkylcarbonylpiperazinyl, Alkoxycarbonylpiperazinyl, Alkyl-$SO_2$-piperazinyl, Diazacycloheptyl, Alkylcarbonyldiazacycloheptyl, 2-Oxo-1-pyrrolidinyl oder 3,3-Dialkyl-2-oxo-1-pyrrolidinyl,
(β) $R_3$-Alkyl, worin $R_3$ für Wasserstoff, Hydroxy, Carboxy, Alkyl-N(alkyl)-, Alkyl-NH-, 1-Pyrrolidinyl, 1-Piperidyl, 4-Alkyl-1-piperazinylcarbonyl, 2,4-Dioxa-5,5-(dialkyl)-oxazolidin-3-yl oder $R_4R_5N$-C(O)- steht, worin $R_4$ und $R_5$ jeweils unabhängig voneinander für Wasserstoff oder Alkyl stehen, oder
(γ) $R_6R_7N$-C(O)-, worin $R_6$ und $R_7$ jeweils unabhängig voneinander für Wasserstoff, Alkyl, Cycloalkylalkyl, $CF_3$-Alkyl oder Pyridylalkyl stehen,

(w) Pyridyl, das unsubstituiert oder mono-, di- oder trisubstituiert ist durch Halogen oder Alkyl, das mono-, di- oder trisubstituiert ist durch Halogen,
(x) Pyrimidyl,
(y) Indolyl, das mono- oder disubstituiert ist durch Alkyl-C(O)-NH-alkyl,
(z) 2-(Alkyl)-benzothiazolyl,

(aa) einen Rest der folgenden Subformel Ia

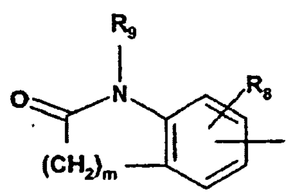

(Ia)

worin $R_8$ für Wasserstoff, Halogen oder Alkyl steht, $R_9$ für Wasserstoff oder Alkyl steht und m für 1, 2, 3 oder 4 steht, oder
(bb) einen Rest der folgenden Subformel Ib

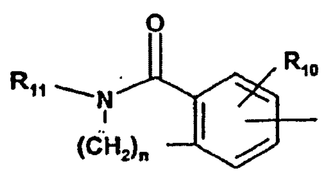

(Ib)

worin $R_{10}$ für Wasserstoff, Halogen oder Alkyl steht, $R_{11}$ für Wasserstoff oder Alkyl steht, und n für 1, 2, 3 oder 4 steht,

$R_2$ für Alkyl, das unsubstituiert oder substituiert ist durch Cycloalkyl, das unsubstituiert oder mono- oder disubstituiert ist durch Halogen, oder für Phenyl steht, das mono- oder disubstituiert ist durch Halogen,
mit der Maßgabe, dass $R_2$ nicht für 1,1-Dimethylethyl steht, wenn Y für O steht und $R_1$ ausgewählt ist aus 3-Pyridyl, 4-Pyridyl, 5-Chlor-3-pyridyl, 6-Chlor-3-pyridyl, 2-Chlor-4-pyridyl, 2-Trifluormethyl-4-pyridyl, 2-Difluormethyl-4-pyridyl, 4-Acetyl-1-piperazinylphenyl, 4-Methyl-1-piperazinylmethylphenyl, und
mit der Maßgabe, dass $R_2$ nicht für 1,1-Dimethylethyl steht, wenn Y für S steht und $R_1$ für 4-Pyridyl steht, durch Alkylierung eines Alkohols oder eines Thiols der Formel II

**$R_1$-$(Y)_p$-H (II)**

worin Y für $-(CH_2)_t$-O- oder $(CH_2)_r$-S- steht und t, r und $R_1$ die oben für eine Verbindung der Formel angegebenen Bedeutungen haben, mit einem Pyrrolopyrimidin der Formel III

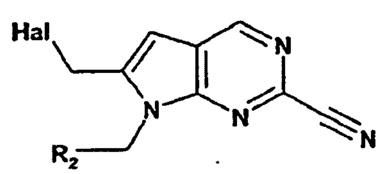

(III)

worin $R_2$ die oben für eine Verbindung der Formel I angegebenen Bedeutungen hat und Hal für Halogen, vorzugsweise für Brom, steht,
worin die Ausgangsverbindungen der Formeln II und III erforderlichenfalls auch mit funktionalen Gruppen in geschützter Form vorliegen können, und/oder in Form von Salzen, sofern eine ein Salz bildende Gruppe vorhanden ist und die Umsetzung in Form eines Salzes möglich ist,
wobei eventuelle Schutzgruppen in einem geschützten Derivat einer Verbindung der Formel I entfernt werden, und gewünschtenfalls eine erhaltene Verbindung der Formel I in eine andere Verbindung der Formel I oder ein N-Oxid hiervon umgewandelt wird, eine freie Verbindung der Formel I in ein Salz umgewandelt wird, ein erhaltenes Salz einer Verbindung der Formel I in die freie Verbindung oder ein anderes Salz umgewandelt wird, und/oder ein Gemisch von isomeren Verbindungen der Formel I in die einzelnen Isomeren aufgetrennt wird.

## Revendications

**1.** Pyrrolopyrimidine de formule 1

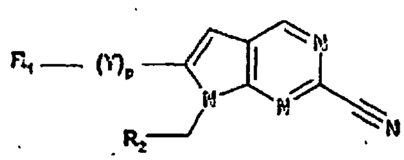

(I)

dans laquelle
Y représente $-(CH_2)_t-O-$ ou $-(CH_2)_r-S-$,
p est égal à 1 ou 2,
r est égal à 1, 2 ou 3,
t est égal à 1, 2 ou 3,
$R_1$ représente

(h) un phényle qui est non substitué ou mono-, di- ou trisubstitué par

(α) un halogène, un groupe carboxy, alcoxy, nitro, alkyl-C(O)-NH-, cycloalkyl-C(O)-NH-, alkyl-C(O)-N(alkyl)-, formyle, alkyl-C(O)-, alkyl-$S(O)_2$-NH-, $CF_3$-alkyl-$S(O)_2$-NH-, pyrrolidinylcarbonyle, pipéridinylcarbonyle, morpholinylcarbonyle, N-alkyl-pipérazinylcarbonyle, pipéridinyle, 1-(alkylcarbonyl)pipéridinyle, 1,2,3,6-tétrahydropyridyle, alkylcarbonyl-1,2,3,6-tétrahydropyridyle, pipérazinyle, alkylpipérazinyle, alkylcarbonylpipérazinyle, cycloalkylcarbonylpipérazinyle, alcoxycarbonylpipérazinyle, alkyl-$SO_2$-pipérazinyle, diazacycloheptyle, alkylcarbonyldiazacycloheptyle, 2-oxo-1-pyrrolidinyle, 3,3-di-alkyl-2-oxo-1-pyrrolidinyle;
(β) un groupe $R_3$-alkyle, dans lequel $R_3$ représente un atome d'hydrogène, un groupe hydroxy, carboxy, alkyl-N(alkyl)-, alkyl-NH-, 1-pyrrolidinyle, 1-pipéridyle, 4-alkyl-1-pipérazinylcarbonyle, 2,4-dioxa-5,5-(di-alkyl)-oxazolidin-3-yle, $R_4R_5N$-C(O)-, où $R_4$ et $R_5$ représentent chacun, indépendamment l'un de l'autre, un atome d'hydrogène ou un groupe alkyle; ou
(γ) $R_6R_7N$-C(O)-, où $R_6$ et $R_7$ représentent chacun, indépendamment l'un de l'autre, un atome d'hydrogène, un groupe alkyle, cycloalkyle, $CF_3$-alkyle ou pyridylalkyle;

(i) un pyridyle qui est non substitué ou mono-, di- ou trisubstitué par un halogène ou un groupe alkyle qui est mono-, di- ou trisubstitué par un halogène;
(j) un pyrimidyle;
(k) un indolyle, qui est mono- ou disubstitué par un groupe alkyl-C(O)-NH-alkyle;
(1) un 2-(alkyl)-benzothiazolyle;
(m) un radical de sous-formule Ia

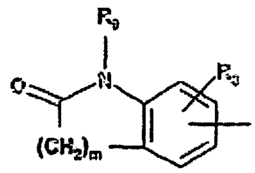

(Ia)

dans laquelle $R_8$ est un atome d'hydrogène, d'halogène ou un groupe alkyle, $R_9$ est un atome d'hydrogène ou un groupe alkyle, et m est égal à 1, 2, 3 ou 4; ou

(n) un radical de sous-formule Ib

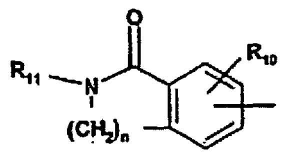

(Ib)

dans laquelle $R_{10}$ est un atome d'hydrogène, d'halogène ou un groupe alkyle, $R_{11}$ est un atome d'hydrogène ou un groupe alkyle, et n est égal à 1, 2, 3 ou 4;

$R_2$ représente un groupe alkyle, qui est non substitué ou substitué par un cycloalkyle, qui est non substitué ou mono- ou disubstitué par un halogène, ou un phényle, qui est mono- ou disubstitué par un halogène;

à condition que $R_2$ ne représente pas un groupe 1,1-diméthyléthyle si Y représente O et $R_1$ est choisi parmi les groupes 3-pyridyle, 4-pyridyle, 5-chloro-3-pyridyle, 6-chloro-3-pyridyle, 2-chloro-4-pyridyle, 2-trifluorométhyl-4-pyridyle, 2-difluorométhyl-4-pyridyle, 4-acétyl-1-pipérazinyl-phényle, 4-méthyl-1-pipérazinyl-méthyl-phényle, et

à condition que $R_2$ ne représente pas un groupe 1,1-diméthyléthyle, si Y représente S et $R_1$ représente un groupe 4-pyridyle;

ou

Y représente $-(CH_2)_j-$ ou -CH=CH-,

j est égal à 1 ou 2;

p est égal à 1 ou 2;

$R_1$ représente

(c) un groupe thiényle, thiazolyle, 1-pipéridinyl-carbonyle, ou

(d) un phényle qui est non substitué ou mono-, di- ou trisubstitué par

(i) un groupe alcoxy, $H_2N$-C(O)-, 4-(alkylcarbonyl)-1-pipérazinyle, 2-oxo-1-pyrrolidinyle, ou un halogène;

(ii) $R_{12}$-O-C(O)-, où $R_{12}$ représente un atome d'hydrogène ou un groupe alkyle, ou

(iii) $R_{13}$NH-, où $R_{13}$ représente un atome d'hydrogène ou un radical $R_{14}$-alkyl-Z-, dans lequel Z représente CO, SO ou $SO_2$ et $R_{14}$ désigne un atome d'hydrogène, un groupe trifluorométhyle ou alcoxy,

(iv) un groupe $R_{15}$-alkyle, où $R_{15}$ désigne un atome d'hydrogène, un groupe hydroxy, alcoxy, 1-pyrrolidinyle, 2-oxo-1-pyrrolidinyle, imidazolidin-2,5-dione-1-yle, 5,5-di-alkyl-oxazolidin-2,4-dione-3-yle ou alkyl-N($R_{16}$)-, où $R_{16}$ représente un atome d'hydrogène ou un groupe alkyle; et

$R_2$ représente

(a) un groupe alkyle, qui est non substitué ou substitué par un groupe alcényle, indanyle, cycloalkyle qui est non substitué ou mono- ou disubstitué par un halogène ou un groupe alkyle, cycloalcényle, phényle, qui est non substitué ou mono- ou disubstitué par un halogène ou par un groupe alkyle;

(b) un groupe cycloalkyle; ou

(c) un groupe alkylcarbonyle;

à condition que, si Y représente $CH_2$, $R_1$ représente un 4-chlorophényle et p est égal à 1, $R_2$ ne désigne pas un groupe 1,1-diméthyléthyle, 1-méthyléthyle, cyclopropyle, cyclohexyle, 2-méthylpropyle ou 2-éthyl-propyle;

à condition que $R_2$ ne représente pas un groupe 1,1-diméthyléthyle, si p est égal à 1, Y représente $CH_2$ et $R_1$

représente un groupe thiényle, phényle, méthoxyphényle, propoxyphényle, 4-fluorophényle, 4-méthylphényle, 4-éthylphényle, 4-butylphényle, hydroxyméthylphényle, 4-(5,5-diméthyloxazolidin-2,4-dione-3-yl-méthyl)-phényle, 4-(méthylsulfonylamino)-phényle, 4-(n-butylsulfonylamino)-phényle, 4-(éthylsulfonylamino)-phényle, 4-(n-propylsulfonylamino)-phényle, 4-(iso-propylsulfonylamino)-phényle, 4-aminophényle, 4-(acétylamino)-phényle, 4-(butanoylamino)-phényle ou 4-(diéthylaminométhyl)-phényle;
et à condition que $R_2$ ne représente pas un groupe 1-méthyléthyle, si p est égal à 1, Y représente $CH_2$ et $R_1$ représente un groupe phényle qui est non substitué ou substitué par un groupe 4-acétyl-1-pipérazinyle; ou
Y représente $-(CH_2)_f-$
f est égal à 1 ou 2;
p est égal 1,
$R_1$ représente

(a) un groupe 1,2,3,6-tétrahydropyrid-1-yle, alkyl-1,2,3,6-tétrahydropyrid-1-yle, di-alkyl-1,2,3,6-tétrahydropyrid-1-yle, halogéno-1,2,3,6-tétrahydropyrid-1-yle, phényl-1,2,3,6-tétrahydropyrid-1-yle, imidazolyle, alkylimidazolyle, di-halogénoimidazolyle, imidazolidin-2,5-dione-1-yle, 5,5-dialkyloxazolidin-2,4-dione-3-yle, alkylimidazolidin-2,5-dione-1-yle, trifluorométhyl-3,4-pyrrolin-1-yle, pyrrolidinyle, alkyl-1-pyrrolidinyle, di-(alkyl)pyrrolidinyle, alcoxypyrrolidinyle, alkyl-2-oxo-1-pyrrolidinyle, dialkyl-2-oxo-1-pyrrolidinyle, halogéno-1-pyrrolidinyle, di-halogéno-1-pyrrolidinyle, di-halogéno-1-pipéridinyle, triazolyle, nitrotriazolyle, phénylimidazolyle, tétrazolyle, benzo[b]imidazolyle, (1-(alkyl-$SO_2$)-4-pipéridinyl)-2,3-dihydro-2-oxo-benzo[b]imidazolyle, 3-(alkylcarbonyl-4-pipéridinyl)-2,3-dihydro-2-oxo-benzo[b]imidazolyle, indolyle, halogéno-1-indolyle, 1,3-dihydro-2-isoindolyle, 2,3-dihydro-1-indolyle, 2,3-dihydro-2-oxo-benzo[b]thiazolyle, di-alcoxy-1,2,3,4-tétrahydroquinoléine, alcoxy-1,2,3,4-tétrahydroisoquinoléine;
(b) un radical de sous-structure Ic

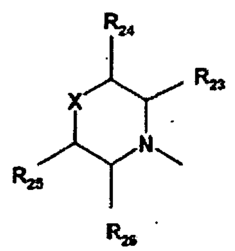

(Ic)

qui est lié à la molécule par l'intermédiaire de l'atome d'azote, dans laquelle X représente -O-, $-(CH_2)_sCR_{17}R_{18}-$ ou $-NR_{18}$, où s est égal à 0, 1 ou 2, $R_{17}$ et $R_{18}$ sont indépendamment choisis parmi un atome d'hydrogène, un atome d'halogène, un groupe hydroxy, alkyle, phénylalkylcarbonyle, carbamoyle, N-phénylcarbamoyle, cyano, pyridyle, pipéridinyle et phényle qui est non substitué ou mono- ou disubstitué par un halogène ou un groupe alcoxy, ou, si X représente $CR_{17}R_{18}$, $R_{17}$ et $R_{18}$ forment ensemble un groupe oxo ou un groupe HO-C(O)-CH=, et $R_{23}$, $R_{24}$, $R_{25}$ et $R_{26}$ sont indépendamment choisis parmi un atome d'hydrogène et un groupe alkyle;
(c) un radical de sous-structure Id

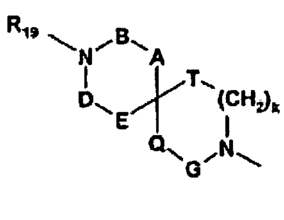

(Id)

qui est lié à la molécule par l'intermédiaire de l'atome d'azote, où k est égal à 0, 1 ou 2, A représente $CH_2$ ou une liaison, B représente $CH_2$ ou un carbonyle, D représente $CH_2$ ou un carbonyle, E représente $CH_2$ ou $NR_{22}$, G représente $CH_2$ ou une liaison, Q représente $CH_2$ ou un carbonyle, T représente $CH_2$ ou $NR_{29}$, $R_{19}$ représente un atome d'hydrogène, un groupe alkyle, phénylalkyle, alkylcarbonyle ou alkyl-$SO_2$-, $R_{22}$ représente un atome d'hydrogène ou un groupe alkyle et $R_{29}$ est un phényle;
(d) un radical de sous-structure Ie

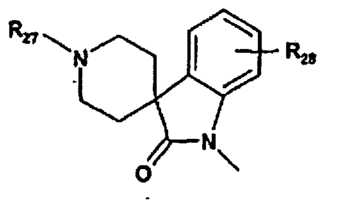

(Ie)

qui est lié à la molécule par l'intermédiaire de l'atome d'azote, où $R_{27}$ est un groupe alkyle ou alkylcarbonyle et $R_{28}$ est un atome d'hydrogène, un groupe alcoxy ou un atome d'halogène; ou

(e) $NR_{20}R_{21}$; où $R_{20}$ et $R_{21}$ sont indépendamment choisis parmi un atome d'hydrogène, un groupe alkyle, cycloalkyle qui est non substitué ou mono- ou disubstitué par un groupe hydroxy; et phényle qui est non substitué ou mono- ou disubstitué par un groupe 1,2,3-thiadiazolyle, à condition que $R_{20}$ et $R_{21}$ ne puissent pas tous deux représenter en même temps un atome d'hydrogène; et

$R_2$ désigne un groupe alkyle, qui est non substitué ou substitué par un groupe cycloalkyle qui est non substitué ou mono- ou disubstitué par un halogène; ou un phényle qui est mono- ou disubstitué par un halogène;

à condition que $R_2$ ne représente pas un groupe 1,1-diméthyléthyle, si

(a) $R_1$ est un groupe benzo[b]imidazol-1-yle, 1-imidazolyle, 4,5-dichloro-1-imidazolyle, 2-(alkyl en $C_1$-$C_4$)-1-imidazolyle, imidazolidin-2,5-dione-1-yle, 5,5-diméthyl-oxazolidin-2,4-dione-3-yle, 1H-1,2,3-triazol-1-yle, 2H-1,2,3-triazol-2-yle, 3-nitro-1H-1,2,4-triazol-1-yle, 2H-tétrazol-2-yle ou 1H-tétrazol-1-yle, ou bien si $R_1$ est un radical de sous-structure Ic, $R_{23}$ à $R_{26}$ représentent un atome d'hydrogène, X représente $NR_{18}$ et $R_{18}$ est un atome d'hydrogène, un groupe méthyle, éthyle, acétyle, 4-pyridyle, 1-pipéridinyle, phényle, méthoxyphényle, éthoxyphényle, fluorophényle ou chlorophényle;

(b) $R_1$ est un radical de sous-structure Ic, $R_{23}$ à $R_{26}$ représentent un atome d'hydrogène, X représente $-(CH_2)_s-CR_{17}R_{18}-$, s est égal à 0, et $R_{17}$ et $R_{18}$ sont choisis parmi un groupe hydroxyle et un groupe phényle qui est monosubstitué par un chloro ou $R_{17}$ et $R_{18}$ sont choisis parmi un atome d'hydrogène, un groupe méthoxyphényle et N-phényl-carbamoyle; ou

(c) $R_1$ est un radical de sous-structure Id, k est égal à 1, A est une liaison, E représente $NR_{22}$, $R_{22}$ est un atome d'hydrogène, G, Q et T représentent $CH_2$, B et D représentent un groupe carbonyle et $R_{19}$ est un groupe méthyle, n-propyle ou isobutyle;

à condition que $R_2$ ne représente pas un groupe 2-méthylpropyle, si $R_1$ est un radical de sous-structure Id, k est égal à 1, A est une liaison, E représente $NR_{22}$, $R_{22}$ est un atome d'hydrogène, G, Q et T représentent $CH_2$, B et D représentent un groupe carbonyle et $R_{19}$ est un groupe méthyle, ou bien si $R_1$ est un radical de sous-structure Ic, $R_{23}$ à $R_{26}$ représentent un atome d'hydrogène, X représente $-(CH_2)_s-CR_{17}R_{18}-$, s est égal à 0, et $R_{17}$ et $R_{18}$ sont choisis parmi un atome d'hydrogène et un groupe phényle qui est monosubstitué par un groupe méthoxy;
et à condition que $R_2$ ne représente pas un groupe 1-méthyléthyle, si $R_1$ est un radical de sous-structure Ic, $R_{23}$ à $R_{26}$ représentent un atome d'hydrogène, X représente $NR_{18}$ et $R_{18}$ est un groupe méthoxyphényle ou éthoxyphényle, ou X représente $CR_{17}R_{18}$ et $R_{17}$ et $R_{18}$ sont choisis parmi un atome d'hydrogène et un groupe méthoxyphényle;
ou un N-oxyde ou un tautomère de celle-ci,
ou un sel d'une telle pyrrolopyrimidine, son N-oxyde ou son tautomère.

**2.** Pyrrolopyrimidine de formule I selon la revendication 1, dans laquelle
Y représente $-CH_2-O-$ ou $-CH_2-S$,
p est égal à 1,
$R_1$ représente

(o) un phényle qui est non substitué ou mono- ou disubstitué par

(α) un halogène, un groupe carboxy, alcoxy en $C_1$-$C_4$, nitro, alkyl(en $C_1$-$C_4$)-C(O)-NH-, cycloalkyl(en $C_3$-$C_4$)-C(O)-NH-, alkyl(en $C_1$-$C_4$)-C(O)-N-alkyl(en $C_1$-$C_4$)-, formyle, alkyl(en $C_1$-$C_4$)-C(O)-, alkyl(en $C_1$-$C_4$)-$S(O)_2$-NH-, $CF_3$-alkyl(en $C_1$-$C_3$)-$S(O)_2$-NH-, 1-pyrrolidinyl-carbonyle, 1-pipéridinyl-carbonyle, 4-morpholinyl-carbonyle, 4-alkyl(en $C_1$-$C_4$)-1-pipérazinyl-carbonyle, 4-pipéridinyle, 1-pipéridinyle, 1-(alkyl(en $C_1$-$C_4$)-carbonyl)-4-pipéridinyle, 1,2,3,6-tétrahydro-4-pyridyle, 1-(alkyl(en $C_1$-$C_4$)-carbonyl)-1,2,3,6-tétrahydro-4-pyridyle, 1-pipérazinyle, 4-alkyl(en $C_1$-$C_4$)-1-pipérazinyle, 4-(alkyl(en $C_1$-$C_4$)-carbonyl)-1-pipéra-

zinyle, 4-(cycloalkyl(en $C_3$-$C_5$)-carbonyl)-1-pipérazinyle, 4-(alcoxy(en $C_1$-$C_4$)-carbonyl)-1-pipérazinyle, 4-(alkyl(en $C_1$-$C_4$)-$SO_2$)-1-pipérazinyle, 1,4-diazacyclohept-1-yle, 4-(alkyl(en $C_1$-$C_4$)-carbonyl)-1,4-diazacyclohept-1-yle, 2-oxo-1-pyrrolidinyle, 3,3-di-alkyl(en $C_1$-$C_4$)-2-oxo-1-pyrrolidinyle;

(β) un groupe $R_3$-alkyle (en $C_1$-$C_4$), où $R_3$ représente un atome d'hydrogène, un groupe hydroxyle, carboxy, alkyl(en $C_1$-$C_4$)-N(alkyl en $C_1$-$C_4$)-, alkyl(en $C_1$-$C_4$)-NH-, 1-pyrrolidinyle, 1-pipéridyle, 4-alkyl(en $C_1$-$C_4$)-1-pipérazinylcarbonyle, 2,4-dioxa-5,5-(di-alkyl(en $C_1$-$C_4$))-oxazolidin-3-yle, $R_4R_5N$-C(O)-, où $R_4$ et $R_5$ représentent chacun, indépendamment l'un de l'autre, un atome d'hydrogène ou un groupe alkyle en $C_1$-$C_4$; ou

(γ) $R_6R_7N$-C(O)-, où $R_6$ et $R_7$ représentent chacun, indépendamment l'un de l'autre, un atome d'hydrogène, un groupe alkyle en $C_1$-$C_4$, cycloalkyl(en $C_5$-$C_7$)-alkyle en $C_1$-$C_4$, $CF_3$-alkyle en $C_1$-$C_3$ ou pyridyl-alkyle en $C_1$-$C_4$;

(p) un pyridyle qui est non substitué ou mono- ou disubstitué par un halogène ou un groupe alkyle en $C_1$-$C_4$ qui est di- ou trisubstitué par un halogène;
(q) un pyrimidyle;
(r) un indolyle qui est monosubstitué par un groupe alkyl(en $C_1$-$C_4$)-C(O)-NH-alkyle en $C_1$-$C_4$;
(s) un groupe 2-alkyl(en $C_1$-$C_4$)-benzothiazolyle;
(t) un radical de sous-formule Ia
où $R_8$ est un atome d'hydrogène, $R_9$ est un atome d'hydrogène, et m est égal à 2 ou 3; ou
(u) un radical de sous-formule Ib
où $R_{10}$ est un atome d'hydrogène, $R_{11}$ est un atome d'hydrogène, et n est égal à 2 ou 3;

$R_2$ représente un groupe alkyle en $C_1$-$C_5$ qui est non substitué ou substitué par un groupe cycloalkyle en $C_5$-$C_7$ qui est non substitué ou disubstitué par un halogène, ou un phényle qui est mono ou disubstitué par un halogène;
à condition que $R_2$ ne représente pas un groupe 1,1-diméthyléthyle si Y représente O et $R_1$ est choisi parmi les groupes 3-pyridyle, 4-pyridyle, 5-chloro-3-pyridyle, 6-chloro-3-pyridyle, 2-chloro-4-pyridyle, 2-trifluorométhyl-4-pyridyle, 2-difluorométhyl-4-pyridyle, 4-acétyl-1-pipérazinyl-phényle, 4-méthyl-1-pipérazinyl-méthyl-phényle, et
à condition que $R_2$ ne représente pas un groupe 1,1-diméthyléthyle, si Y représente S et $R_1$ représente un groupe 4-pyridyle;
ou
Y représente $CH_2$ ou -CH=CH-,
p est égal à 1 ou 2,
$R_1$ représente

(e) un groupe thiényle, thiazolyle, 1-pipéridinyl-carbonyle, ou
(f) un phényle qui est non substitué ou mono- ou disubstitué par

(i) un groupe alcoxy en $C_1$-$C_4$, $H_2N$-C(O)-, 4-(alkyl(en $C_1$-$C_4$)-carbonyle)-1-pipérazinyle, 2-oxo-1-pyrrolidinyle, ou un halogène;
(ii) $R_{12}$-O-C(O)-, où $R_{12}$ est un atome d'hydrogène ou un groupe alkyle en $C_1$-$C_4$, ou
(iii) $R_{13}$NH-, où $R_{13}$ représente un atome d'hydrogène ou un radical $R_{14}$-alkyl(en $C_1$-$C_4$)-Z-, où Z représente CO ou $SO_2$ et $R_{14}$ désigne un atome d'hydrogène, un groupe trifluorométhyle ou alcoxy en $C_1$-$C_4$,
(iv) $R_{15}$-alkyle en $C_1$-$C_4$, où $R_{15}$ désigne un atome d'hydrogène, un groupe hydroxy, alcoxy inférieur, 1-pyrrolidinyle, 2-oxo-1-pyrrolidinyle, imidazolidin-2,5-dione-1-yle, 5,5-diméthyloxazolidin-2,4-dione-3-yle ou alkyl(en $C_1$-$C_4$)-N($R_{16}$)-, où $R_{16}$ représente un atome d'hydrogène ou un groupe alkyle en $C_1$-$C_4$; et

$R_2$ représente

(a) un groupe alkyle en $C_1$-$C_7$, qui est non substitué ou substitué par un groupe alcényle en $C_2$-$C_3$, indanyle, cycloalkyle en $C_3$-$C_7$ qui est non substitué ou disubstitué par un halogène ou un groupe alkyle en $C_1$-$C_4$, cycloalcényle en $C_3$-$C_7$, phényle, qui est non substitué ou mono- ou disubstitué par un halogène ou par un groupe alkyle en $C_1$-$C_4$;
(b) un groupe cycloalkyle en $C_3$-$C_7$; ou
(c) un groupe alkyl(en $C_1$-$C_4$)carbonyle;

à condition que, si Y représente $CH_2$, $R_1$ représente un groupe 4-chlorophényle et p est égal à 1, $R_2$ ne désigne pas un groupe 1,1-diméthyléthyle, 1-méthyléthyle, cyclopropyle, cyclohexyle, 2-méthylpropyle ou 2-éthyl-propyle;
à condition que $R_2$ ne représente pas un groupe 1,1-diméthyléthyle, si p est égal à 1, Y représente $CH_2$ et $R_1$ représente un groupe thiényle, phényle, méthoxyphényle, propoxyphényle, 4-fluorophényle, 4-méthylphényle,

4-éthylphényle, 4-butylphényle, hydroxyméthylphényle, 4-(5,5-diméthyl-oxazolidin-2,4-dione-3-yl-méthyl)phényle, 4-(méthylsulfonylamino)phényle, 4-(n-butylsulfonylamino)phényle, 4-(éthylsulfonylamino)phényle, 4-(n-propylsulfonylamino)phényle, 4-(isopropylsulfonylamino)phényle, 4-aminophényle, 4-(acétylamino)phényle, 4-(butanoylamino)phényle ou 4-(diéthylaminométhyl)phényle;
et à condition que $R_2$ ne représente pas un groupe 1-méthyléthyle, si p est égal à 1, Y représente $CH_2$ et $R_1$ représente un phényle qui est non substitué ou substitué par un groupe 4-acétyl-1-pipérazinyle; ou
Y représente $CH_2$,
p est égal à 1,
$R_1$ représente

(a) un groupe 1,2,3,6-tétrahydropyrid-1-yle, 4-alkyl(en $C_1$-$C_4$)-1,2,3,6-tétrahydropyrid-1-yle, 4,5-di(alkyl en $C_1$-$C_4$)-1,2,3,6-tétrahydropyrid-1-yle, 5-chloro-1,2,3,6-tétrahydropyrid-1-yle, 4-phényl-1,2,3,6-tétrahydropyrid-1-yle, 1-imidazolyle, 2-alkyl(en $C_1$-$C_4$)-1-imidazolyle, 4,5-dihalogéno-1-imidazolyle, imidazolidin-2,5-dione-1-yle, 5,5-diméthyl-oxazolidin-2,4-dione-3-yle, 3-alkyl(en $C_1$-$C_4$)-imidazolidin-2,5-dione-1-yle, 3-trifluorométhyl-3,4-pyrrolin-1-yle, 1-pyrrolidinyle, 3-alkyl(en $C_1$-$C_4$)-1-pyrrolidinyle, 3,3-di(alkyl en $C_1$-$C_4$)-1-pyrrolidinyle, 3-alcoxy(en $C_1$-$C_4$)-1-pyrrolidinyle, 3-alkyl(en $C_1$-$C_4$)-2-oxo-1-pyrrolidinyle, 3,3-di(alkyl en $C_1$-$C_4$)-2-oxo-1-pyrrolidinyle, 3-halogéno-1-pyrrolidinyle, 3,3-dihalogéno-1-pyrrolidinyle, 3,3-di-halogéno-1-pipéridinyle, 1H-1,2,3-triazol-1-yle, 2H-1,2,3-triazol-2-yle, 1H-1,2,4-triazol-1-yle, 3-nitro-1H-1,2,4-triazol-1-yle, 2-phényl-1-imidazolyle, 2H-tétrazol-2-yle, 1H-tétrazol-1-yle, benzo[b]imidazol-1-yle, 3-(1-(alkyl(en $C_1$-$C_4$)-$SO_2$)-4-pipéridinyl)-2,3-dihydro-2-oxo-benzo[b]imidazol-1-yle, 3-1-(alkyl(en $C_1$-$C_4$)-carbonyl-4-pipéridinyl)-2,3-dihydro-2-oxo-benzo[b]imidazol-1-yle, 1-indolyle, 6-halogéno-1-indolyle, 1,3-dihydro-2-isoindolyle, 2,3-dihydro-1-indolyle, 2,3-dihydro-2-oxo-benzo[b]thiazol-3-yle, 6,7-di-(alcoxy en $C_1$-$C_4$)-1,2,3,4-tétrahydroquinoléine, 6-(alcoxy en $C_1$-$C_4$)-1,2,3,4-tétrahydroisoquinoléine, 7-(alcoxy en $C_1$-$C_4$)-1,2,3,4-tétrahydroisoquinoléine;
(b) un radical de sous-structure Ic
qui est lié à la molécule par l'intermédiaire de l'atome d'azote, où X représente -O-, $-(CH_2)_s-CR_{17}R_{18}-$ ou $-NR_{18}$, où s est égal à 0 ou 1, $R_{17}$ et $R_{18}$ sont indépendamment choisis parmi un atome d'hydrogène, un atome d'halogène, un groupe hydroxy, alkyle en $C_1$-$C_4$, phényl-alkyl(en $C_1$-$C_4$)carbonyle, carbamoyle, N-phénylcarbamoyle, cyano, 4-pyridyle, 1-pipéridinyle et phényle qui est non substitué ou monosubstitué par un halogène ou un groupe alcoxy en $C_1$-$C_4$, ou bien, si X représente $CR_{17}R_{18}$, $R_{17}$ et $R_{18}$ forment ensemble un groupe oxo ou un groupe HO-C(O)-CH=, et
$R_{23}$, $R_{24}$, $R_{25}$ et $R_{26}$ sont indépendamment choisis parmi un atome d'hydrogène et un groupe alkyle en $C_1$-$C_4$;
(c) un radical de sous-structure Id
qui est lié à la molécule par l'intermédiaire de l'atome d'azote, où k est égal à 0 ou 1, A représente $CH_2$ ou une liaison, B représente $CH_2$ ou un groupe carbonyle, D représente $CH_2$ ou un groupe carbonyle, E représente $CH_2$ ou $NR_{22}$, G représente $CH_2$ ou une liaison, Q représente $CH_2$ ou un groupe carbonyle, T représente $CH_2$ ou $NR_{29}$, $R_{19}$ représente un atome d'hydrogène, un groupe alkyle en $C_1$-$C_4$, phényl(alkyle en $C_1$-$C_4$), alkyl(en $C_1$-$C_4$)carbonyle ou alkyl(en $C_1$-$C_4$)-$SO_2$-, $R_{22}$ représente un atome d'hydrogène et $R_{29}$ représente un groupe phényle,
(d) un radical de sous-structure Ie
qui est lié à la molécule par l'intermédiaire de l'atome d'azote, où $R_{27}$ est un groupe alkyle en $C_1$-$C_4$ ou alkyl (en $C_1$-$C_4$)carbonyle et $R_{28}$ est un atome d'hydrogène, un groupe alcoxy en $C_1$-$C_4$ ou un atome d'halogène; ou
(e) $NR_{20}R_{21}$, où $R_{20}$ et $R_{21}$ sont indépendamment choisis parmi un atome d'hydrogène, un groupe alkyle en $C_1$-$C_4$, cycloalkyle en $C_3$-$C_7$ qui est non substitué ou monosubstitué par un groupe hydroxy; et phényle qui est non substitué ou monosubstitué par un groupe 1,2,3-thiadiazol-4-yle, à condition que $R_{20}$ et $R_{21}$ ne puissent pas tous deux représenter en même temps un atome d'hydrogène; et
$R_2$ désigne un groupe alkyle en $C_1$-$C_8$, qui est non substitué ou substitué par un groupe cycloalkyle en $C_3$-$C_7$ qui est non substitué ou disubstitué par un halogène; un groupe phényle qui est mono- ou disubstitué par un halogène;

à condition que $R_2$ ne représente pas un groupe 1,1-diméthyléthyle, si

(a) $R_1$ est un groupe benzo[b]imidazol-1-yle, 1-imidazolyle, 4,5-dichloro-1-imidazolyle, 2-alkyl(en $C_1$-$C_4$)-1-imidazolyle, imidazolidin-2,5-dione-1-yle, 5,5-diméthyl-oxazolidin-2,4-dione-3-yle, 1H-1,2,3-triazol-1-yle, 2H-1,2,3-triazol-2-yle, 3-nitro-1H-1,2,4-triazol-1-yle, 2H-tétrazol-2-yle ou 1H-tétrazol-1-yle, ou si $R_1$ est un radical de sous-structure Ic, $R_{23}$ à $R_{26}$ représentent un atome d'hydrogène, X représente $NR_{18}$ et $R_{18}$ est un atome d'hydrogène, un groupe méthyle, éthyle, acétyle, 4-pyridyle, 1-pipéridinyle, phényle, méthoxyphényle, éthoxyphényle, fluorophényle ou chlorophényle;
(b) $R_1$ est un radical de sous-structure Ic, $R_{23}$ à $R_{26}$ représentent un atome d'hydrogène, X représente

$-(CH_2)_s-CR_{17}R_{18}-$, s est égal à 0 et $R_{17}$ et $R_{18}$ sont choisis parmi un groupe hydroxyle et phényle qui est monosubstitué par un chloro ou $R_{17}$ et $R_{18}$ sont choisis parmi un atome d'hydrogène, un groupe méthoxyphényle et N-phénylcarbamoyle; ou

(c) $R_1$ est un radical de sous-structure Id, k est égal à 1, A est une liaison, E représente $NR_{22}$, $R_{22}$ est un atome d'hydrogène, G, Q et T représentent $CH_2$, B et D représentent un groupe carbonyle et $R_{19}$ est un groupe méthyle, n-propyle ou isobutyle;

à condition que $R_2$ ne représente pas un groupe 2-méthylpropyle, si $R_1$ est un radical de sous-structure Id, k est égal à 1, A est une liaison, E représente $NR_{22}$, $R_{22}$ est un atome d'hydrogène, G, Q et T représentent $CH_2$, B et D représentent un groupe carbonyle et $R_{19}$ est un groupe méthyle, ou si $R_1$ est un radical de sous-structure Ic, $R_{23}$ à $R_{26}$ représentent un atome d'hydrogène, X représente $-(CH_2)_s-CR_{17}R_{18}-$, s est égal à 0 et $R_{17}$ et $R_{18}$ sont choisis parmi un atome d'hydrogène et un phényle qui est monosubstitué par un groupe méthoxy;
et à condition que $R_2$ ne représente pas un groupe 1-méthyléthyle, si $R_1$ est un radical de sous-structure Ic, $R_{23}$ à $R_{26}$ représentent un atome d'hydrogène, X représente $N-R_{18}$ et $R_{18}$ est un groupe méthoxyphényle ou éthoxyphényle, ou X représente $CR_{17}R_{18}$ et $R_{17}$ et $R_{18}$ sont choisis parmi un atome d'hydrogène et un groupe méthoxyphényle;
ou un tautomère de celle-ci;
ou un sel d'une telle pyrrolopyrimidine ou son tautomère.

**3.** Pyrrolopyrimidine de formule I selon la revendication 1 ou 2, ou un N-oxyde ou un tautomère de celle-ci, ou un sel pharmaceutiquement acceptable d'un tel composé, à utiliser dans un procédé pour le traitement du corps humain ou animal.

**4.** Utilisation d'une pyrrolopyrimidine de formule I selon la revendication 1 ou 2, ou d'un N-oxyde ou d'un tautomère de celle-ci, ou d'un sel pharmaceutiquement acceptable d'un tel composé, pour la préparation d'un médicament destiné au traitement d'une douleur neuropathique.

**5.** Préparation pharmaceutique comprenant une pyrrolopyrimidine de formule I selon la revendication 1 ou 2, ou un N-oxyde ou un tautomère de celle-ci, ou un sel pharmaceutiquement acceptable d'un tel composé, ou un hydrate ou solvate de celle-ci, et au moins un véhicule pharmaceutiquement acceptable.

**6.** Procédé pour la préparation d'une pyrrolopyrimidine de formule I

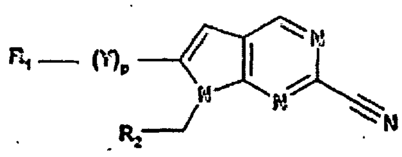

(I)

dans laquelle Y représente $-(CH_2)_tO-$ ou $(CH_2)_r-S-$
p est égal à 1 ou 2,
r est égal à 1, 2 ou 3,
t est égal à 1, 2 ou 3,
$R_1$ représente

(v) un phényle qui est non substitué ou mono-, di- ou trisubstitué par

(α) un halogène, un groupe carboxy, alcoxy, nitro, alkyl-C(O)-NH, cycloalkyl-C(O)-NH-, alkyl-C(O)-N(alkyl)-, formyle, alkyl(C(O)-, alkyl-$S(O)_2$-NH-, $CF_3$-alkyl-$S(O)_2$-NH-, pyrrolidinylcarbonyle, pipéridinylcarbonyle, morpholinylcarbonyle, N-alkyl-pipérazinylcarbonyle, pipéridinyle, 1-(alkylcarbonyl)pipéridinyle, 1,2,3,6-tétrahydropyridyle, alkylcarbonyle-1,2,3,6-tétrahydropyridyle, pipérazinyle, alkylpipérazinyle, alkylcarbonylpipérazinyle, cycloalkylcarbonylpipérazinyle, alcoxycarbonylpipérazinyle, alkyl-$SO_2$-pipérazinyle, diazacycloheptyle, alkylcarbonyldiazacycloheptyle, 2-oxo-1-pyrrolidinyle, 3,3-di-alkyl-2-oxo-1-pyrrolidinyle;
(β) un groupe $R_3$-alkyle, dans lequel $R_3$ représente un atome d'hydrogène, un groupe hydroxy, carboxy, alkyl-N(alkyl)-, alkyl-NH-, 1-pyrrolidinyle, 1-pipéridyle, 4-alkyl-1-pipérazinylcarbonyle, 2,4-dioxa-5,5-(di-alkyl)oxazolidin-3-yle, $R_4R_5N$-C(O)-, où $R_4$ et $R_5$ représentent chacun indépendamment l'un de l'autre un atome d'hydrogène ou un groupe alkyle; ou
(γ) $R_6R_7N$-C(O)-, où $R_6$ et $R_7$ représentent chacun indépendamment l'un de l'autre un atome d'hydrogène

ou un groupe alkyle, cycloalkylalkyle, $CF_3$-alkyle ou pyridylalkyle;

(w) un pyridyle qui est non substitué ou mono-, di- ou trisubstitué par un halogène ou un groupe alkyle qui est mono-, di- ou trisubstitué par un halogène;
(x) un pyrimidyle;
(y) un indolyle, qui est mono- ou disubstitué par un groupe alkyl-C(O)-NH-alkyle;
(z) un 2-(alkyl)benzothiazolyle;

(aa) un radical de sous-formule Ia

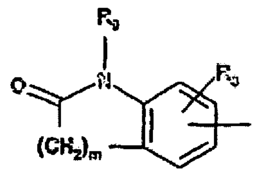
(Ia)

dans laquelle $R_8$ est un atome d'hydrogène, d'halogène ou un groupe alkyle, $R_9$ est un atome d'hydrogène ou un groupe alkyle, et m est égal à 1, 2, 3 ou 4; ou
(bb) un radical de sous-formule Ib

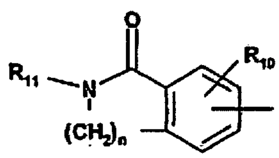
(Ib)

dans laquelle $R_{10}$ est un atome d'hydrogène, d'halogène ou un groupe alkyle, $R_{11}$ est un atome d'hydrogène ou un groupe alkyle, et n est égal à 1, 2, 3 ou 4;

$R_2$ représente un groupe alkyle, qui est non substitué ou substitué par un cycloalkyle, qui est non substitué ou mono- ou disubstitué par un halogène, ou un groupe phényle, qui est mono ou disubstitué par un halogène;

à condition que $R_2$ ne représente pas un groupe 1,1-diméthyléthyle si Y représente O et $R_1$ est choisi parmi un groupe 3-pyridyle, 4-pyridyle, 5-chloro-3-pyridyle, 6-chloro-3-pyridyle, 2-chloro-4-pyridyle, 2-trifluorométhyl-4-pyridyle, 2-difluorométhyl-4-pyridyle, 4-acétyl-1-pipérazinyl-phényle, 4-méthyl-1-pipérazinyl-méthyl-phényle, et
à condition que $R_2$ ne représente pas un groupe 1, 1-diméthyléthyle si Y représente S et $R_1$ est un groupe 4-pyridyle;
où un alcool ou un thiol de formule II

$R_1$-$(Y)_p$-H (II)

dans laquelle Y représente -$(CH_2)_t$-O- ou $(CH_2)_r$-S- et t, r et $R_1$ ont les significations données ci-dessus pour un composé de formule I, est alkylé avec une pyrrolopyrimidine de formule III

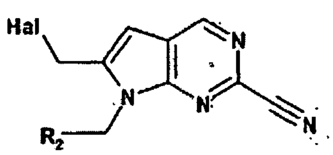
(III)

dans laquelle $R_2$ a la signification donnée ci-dessus pour un composé de formule I et Hal désigne un halogéno, de préférence bromo,

où les composés de départ de formules II et III peuvent être aussi présents avec des groupes fonctionnels sous forme protégée, si nécessaire, et/ou sous forme de sels, pourvu qu'un groupe formant un sel soit présent et que la réaction soit possible sous forme de sel;
où les groupes protecteurs éventuels dans un dérivé protégé d'un composé de formule I sont éliminés;
et si on le souhaite, un composé de formule I susceptible d'être obtenu est converti en un autre composé de formule I ou en un N-oxyde de celui-ci, un composé libre de formule I est converti en un sel, un sel susceptible d'être obtenu d'un composé de formule I est converti en composé libre ou en un autre sel, et/ou mélange de composés isomères de formule I est séparé pour donner les isomères individuels.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03020721 A
- WO 03020287 A


### Non-patent literature cited in the description


- **STEIN, C. et al.** *Pharmacol. Biochem. Behav.,* 1988, vol. 31, 445-451
- **SELTZER et al.** *Pain,* 1990, vol. 43, 205-218
- **BENNETT, G.J. ; XIE, Y.K.** *Pain,* 1988, vol. 33, 87-107
- **KIM, S.O. ; CHUNG, J.M.** *Pain,* 1992, vol. 50, 355-363
- **COLOMBO et al.** *Arth. Rheum.,* 1993, vol. 26, 875-886
- **O'BYRNE et al.** *Inflamm Res,* 1995, vol. 44, S117-S118
- **J. F. W. MCOMIE.** Protective Groups in Organic Chemistry. Plenum Press, 1973
- **T. W. GREENE.** Protective Groups in Organic Synthesis. Wiley, 1981
- The Peptides. Academic Press, 1981, vol. 3
- Methoden der organischen Chemie. Georg Thieme Verlag, 1974, vol. 15/l
- **H.-D. JAKUBKE ; H. JESCHEIT.** Aminosäuren, Peptide, Proteine. Verlag Chemie, 1982
- **JOCHEN LEHMANN.** Chemie der Kohlenhydrate: Monosaccharide und Derivate. Georg Thieme Verlag, 1974